# EUROPÄISCHE PATENTSCHRIFT

(11) **EP 3 728 235 B1**
(45) Veröffentlichungstag und Bekanntmachung des Hinweises auf die Patenterteilung: **09.02.2022**
(21) Anmeldenummer: 18816094.9
(22) Anmeldetag: 17.12.2018
(51) Int. Cl.: C07D 403/12, A01N 43/54, C07D 401/12, C07D 405/12, C07D 239/54, C07D 413/12, A01P 13/00, A01P 21/00

(54) **SUBSTITUIERTE THIOPHENYLURACILE SOWIE DEREN SALZE UND IHRE VERWENDUNG ALS HERBIZIDE WIRKSTOFFE**
SUBSTITUTED THIOPHENYLURACILS , THEIR SALTS AND USE OF SAID COMPOUNDS AS HERBICIDAL AGENTS
THIOPHÉNYL-URACILES SUBSTITUÉS AINSI QUE LEURS SELS ET LEUR UTILISATION EN TANT QU'AGENTS HERBICIDES

(30) Priorität: 19.12.2017 EP 17208493
(43) Veröffentlichungstag der Anmeldung: 28.10.2020
(73) Patentinhaber: Syngenta Crop Protection AG, 4058 Basel (CH)
(72) Erfinder: HEINEMANN, Ines, 65719 Hofheim (DE); FRACKENPOHL, Jens, 60322 Frankfurt (DE); WILLMS, Lothar, 56204 Hillscheid (DE); BEFFA, Roland, 65835 Liederbach (DE); DIETRICH, Hansjörg, 65835 Liederbach am Taunus (DE); GATZWEILER, Elmar, 61231 Bad Nauheim (DE); MACHETTIRA, Anu Bheemaiah, 60326 Frankfurt am Main (DE); ROSINGER, Christopher Hugh, 65719 Hofheim (DE); LÜMMEN, Peter, 65510 Idstein (DE); ASMUS, Elisabeth, 63768 Hösbach (DE)
(74) Vertreter: SYNGENTA IP
(86) Internationale Anmeldenummer: PCT/EP2018/085262
(87) Internationale Veröffentlichungsnummer: WO 2019/121543

(56) Entgegenhaltungen:
- WO-A1-2013/154396
- US-A- 5 084 084

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft diese Erfindung substituierte Thiophenyluracile sowie deren Salze, Verfahren zu ihrer Herstellung und ihre Verwendung als Herbizide, inbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Es ist aus verschiedenen Schriften bekannt, daß bestimmte substituierte N-verknüpfte Aryluracile als herbizide Wirkstoffe verwendet werden können (vgl. EP408382, EP473551, EP648749, US4943309, US5084084, US5127935, WO91/00278, WO95/29168, WO95/30661, WO96/35679, WO97/01541, WO98/25909, WO1801/39597). Viele bekannte Aryluracile weisen jedoch eine Reihe von Wirkungslücken, insbesondere gegenüber monokotylen Unkräutern auf. Eine Reihe von herbiziden Wirkstoffkombinationen auf Basis von N-verknüpften Aryluracilen sind ebenfalls bekannt geworden (vgl. DE4437197, EP714602, WO96/07323, WO96/08151, JP11189506). Die Eigenschaften dieser Wirkstoffkombinationen waren jedoch auch nicht in allen Belangen zufriedenstellend.

Es ist weiterhin bekannt, daß bestimmte N-Aryluracile mit gegebenenfalls weiter substituierten Milchsäuregruppen auch als herbizide Wirkstoffe eingesetzt werden können (vgl. JP1800/302764, JP1801/172265, US6403534, EP408382). Es ist darüberhinaus bekannt, daß N-Aryluracile mit gegebenenfalls weiter substituierten Thiomilchsäuregruppen ebenfalls herbizide Wirkungen zeigen (vgl. WO2010/038953, KR2011110420).

Substituierte Thiophenyluracile mit gegebenenfalls weiter substituierten Thioessigsäureestern sind dagegen im Wesentlichen noch nicht beschrieben. Überraschenderweise wurde nun gefunden, dass bestimmte substituierte Thiophenyluracile oder deren Salze als Herbizide gut geeignet sind und besonders vorteilhaft als Wirkstoffe zur Bekämpfung von monokotylen und dikotylen Unkräutern in Nutzpflanzenkulturen eingesetzt werden können.

Ein Gegenstand der vorliegenden Erfindung sind damit substituierte Thiophenyluracile der allgemeinen Formel (I) oder deren Salze 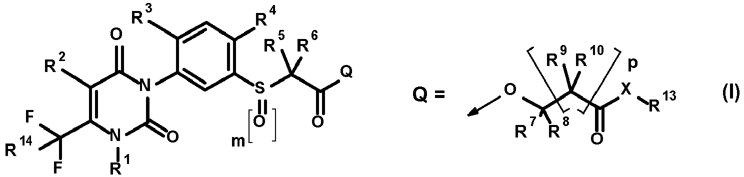 worin
- R¹: für (C₁-C₈)-Alkyl, Amino, Bis-[(C₁-C₈)-alkyl]amino steht,
- R²: für Wasserstoff, (C₁-C₈)-Alkyl steht,
- R³: für Wasserstoff, Halogen, (C₁-C₈)-Alkoxy steht,
- R⁴: für Halogen, Cyano, NO₂, C(O)NH₂, C(S)NH₂, (C₁-C₈)-Haloalkyl, (C₂-C₈)-Alkinyl steht,
- R⁵ und R⁶: unabhängig voneinander für Wasserstoff, Halogen, (C₁-C₈)-Alkyl, (C₃-C₈)-Cycloalkyl, (C₃-C₈)-Cycloalkyl-(C₁-C₈)-alkyl, (C₂-C₈)-Alkenyl, (C₂-C₈)-Alkinyl, (C₁-C₁₀)-Haloalkyl, (C₂-C₈)-Haloalkenyl, (C₂-C₈)-Haloalkinyl, (C₃-C₁₀)-Halocycloalkyl, (C₄-C₁₀)-Cycloalkenyl, (C₄-C₁₀)-Halocycloalkenyl, (C₁-C₈)-Alkoxy, (C₁-C₈)-Alkoxy-(C₁-C₈)-alkyl, (C₁-C₈)-Alkoxy-(C₁-C₈)-haloalkyl, (C₁-C₈)-Haloalkoxy-(C₁-C₈)-haloalkyl, (C₁-C₈)-Haloalkoxy-(C₁-C₈)-alkyl, Aryl, Aryl-(C₁-C₈)-alkyl, Heteroaryl, Heteroaryl-(C₁-C₈)-alkyl, (C₄-C₁₀)-Cycloalkenyl-(C₁-C₈)-alkyl, Heterocyclyl, Heterocyclyl-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkylcarbonyl-(C₁-C₈)-alkyl, C(O)OR¹⁷, C(O)NR¹⁵R¹⁶, C(O)R¹⁷ , R¹⁷O(O)C-(C₁-C₈)-alkyl, R¹⁵R¹⁶N(O)C-(C₁-C₈)-alkyl, R¹⁵R¹⁶N-(C₁-C₈)-alkyl stehen, oder
- R⁵ und R⁶: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁵ und R⁶: mit dem Kohlenstoffatom, an das sie gebunden sind, eine gegebenenfalls weiter durch R¹⁹ und R²⁰ substituierte Doppelbindung, gemäß nachfolgender Formel (I'), bilden 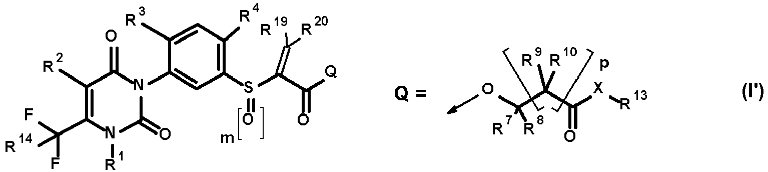
- m: für 0, 1, 2 steht,
- p: für 0, 1, 2, 3, 4, 5 steht,
- X: für O (Sauerstoff) oder die Gruppierungen N-R¹¹ oder N-O-R¹² steht und wobei R¹¹ und R¹² in den Gruppierung N-R¹¹ und N-O-R¹² unabhängig voneinander die Bedeutungen gemäß der nachfolgenden Definitionen haben,
- R⁷, R⁸, R⁹ und R¹⁰: unabhängig voneinander für Wasserstoff, Fluor, (C₁-C₈)-Alkyl, (C₃-C₈)-Cycloalkyl, (C₃-C₈)-Cycloalkyl-(C₁-C₈)-alkyl, (C₂-C₈)-Alkenyl, (C₂-C₈)-Alkinyl, (C₁-C₁₀)-Haloalkyl, (C₂-C₈)-Haloalkenyl, (C₂-C₈)-Haloalkinyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-haloalkyl, (C₁-C₈)-Haloalkoxy-(C₁-C₈)-haloalkyl, Aryl, Aryl-(C₁-C₈)-alkyl, Heteroaryl, Heteroaryl-(C₁-C₈)-alkyl, (C₄-C₁₀)-Cycloalkenyl-(C₁-C₈)-alkyl, Heterocyclyl, Heterocyclyl-(C₁-C₈)-alkyl, R¹⁷O-(C₁-C₈)-alkyl, R¹⁸S-(C₁-C₈)-alkyl, (C₁-C₈)-Alkylcarbonyl-(C₁-C₈)-alkyl, C(O)OR¹⁷, C(O)NR¹⁵R¹⁶, C(O)R¹⁷, R¹⁷O(O)C-(C₁-C₈)-alkyl, R¹⁵R¹⁶N(O)C-(C₁-C₈)-alkyl, R¹⁵R¹⁶N-(C₁-C₈)-alkyl stehen, oder
- R⁵ und R⁷: mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 8-gliedrigen monocyclischen oder bicyclischen Ring bilden, oder
- R⁷ und R⁸: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹⁰: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹⁰: mit dem Kohlenstoffatom, an das sie gebunden sind, eine Exomethylengruppe bilden, oder
- R⁷ und R⁹: mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹¹: für Wasserstoff, (C₁-C₈)-Alkyl, (C₃-C₈)-Cycloalkyl, Cyano-(C₁-C₈)-alkyl, (C₃-C₈)-Cycloalkyl-(C₁-C₈)-alkyl, (C₁-C₈)-Alkylsulfonyl, Arylsulfonyl, Heteroarylsulfonyl, (C₃-C₈)-Cycloalkylsulfonyl, Heterocyclylsulfonyl, Aryl-(C₁-C₈)-alkylsulfonyl, (C₁-C₈)-Alkylcarbonyl, Arylcarbonyl, Heteroarylcarbonyl, (C₃-C₈)-Cycloalkylcarbonyl, Heterocyclylcarbonyl, (C₁-C₈)-Alkoxycarbonyl, (C₁-C₈)-Alkoxy, (C₂-C₈)-Alkenyloxy, Aryl-(C₁-C₈)-alkoxycarbonyl, (C₁-C₈)-Haloalkylcarbonyl, (C₂-C₈)-Alkenyl, (C₂-C₈)-Alkinyl, (C₁-C₈)-Haloalkyl, Halo-(C₂-C₈)-alkinyl, Halo-(C₂-C₈)-alkenyl, (C₁-C₈)-Alkoxy-(C₁-C₈)-alkyl, Amino, (C₁-C₈)-Alkylamino, Bis[(C₁-C₈)-alkyl]amino, (C₁-C₈)-Alkoxy-(C₁-C₈)-alkoxy-(C₁-C₈)-alkyl, Heteroaryl-(C₁-C₈)-alkylsulfonyl, Heterocyclyl-(C₁-C₈)-alkylsulfonyl, (C₂-C₈)-Alkenyloxycarbonyl, (C₂-C₈)-Alkinyloxycarbonyl, (C₁-C₈)-Alkylaminocarbonyl, (C₃-C₈)-Cycloalkylaminocarbonyl, Bis-[(C₁-C₈)-alkyl]aminocarbonyl steht,
- R¹²: für Wasserstoff, (C₁-C₈)-Alkyl, (C₃-C₈)-Cycloalkyl-(C₁-C₈)-alkyl, (C₂-C₈)-Alkenyl, (C₂-C₈)-Alkinyl, (C₁-C₈)-Alkoxy-(C₁-C₈)-alkyl, Aryl, Aryl-(C₁-C₈)-alkyl, R¹⁷O(O)C-(C₁-C₈)-alkyl, R¹⁵R¹⁶N(O)C-(C₁-C₈)-alkyl stehen,
- R¹³: für Wasserstoff, (C₁-C₈)-Alkyl, (C₁-C₈)-Haloalkyl, (C₃-C₈)-Cycloalkyl, (C₃-C₈)-Cycloalkyl-(C₁-C₈)-alkyl, (C₂-C₈)-Alkenyl, (C₂-C₈)-Alkinyl, (C₁-C₈)-Alkoxy-(C₁-C₈)-alkyl, (C₁-C₈)-Haloalkoxy-(C₁-C₈)-alkyl, Aryl, Aryl-(C₁-C₈)-alkyl, Heteroaryl, Heteroaryl-(C₁-C₈)-alkyl, Heterocyclyl, Heterocyclyl-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkylcarbonyl-(C₁-C₈)-alkyl, R¹⁷O(O)C-(C₁-C₈)-alkyl, R¹⁵R¹⁶N(O)C-(C₁-C₈)-alkyl, R¹⁵R¹⁶N-(C₁-C₈)-alkyl stehen, oder
- R⁷ und R¹³: mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹³: mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R¹¹ und R¹³: in dem Fall, daß X für N-R¹¹ steht, mit dem Stickstoffatom, an das sie gebunden sind, einen vollständig gesättigten oder gegebenenfalls durch Heteroatome unterbrochenen und gegebenenfalls weiter substituierten 4 bis 10-gliedrigen monocyclischen oder bicyclischen Ring bilden, oder
- R¹² und R¹³: in dem Fall, daß X für N-O-R¹² steht, mit dem Stickstoffatom, an das sie gebunden sind, einen vollständig gesättigten und gegebenenfalls weiter substituierten 4 bis 10-gliedrigen monocyclischen oder bicyclischen Ring bilden,
- R¹⁴: für Wasserstoff, Fluor, Chlor, Brom, (C₁-C₅)-Haloalkyl, (C₁-C₈)-Alkoxy steht,
- R¹⁵ und R¹⁶: gleich oder verschieden sind und unabhängig voneinander für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₂-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, COR¹⁷, SO₂R¹⁸, (C₁-C₈)-Alkyl-HNO₂S-, (C₃-C₈)-Cycloalkyl-HNO₂S-, Heterocyclyl, (C₁-C₈)-Alkoxycarbonyl-(C₁-C₈)-alkyl, (C₁-C₈)-Alkoxycarbonyl, Aryl-(C₁-C₈)-Alkoxycarbonyl-(C₁-C₈)-alkyl, Aryl-(C₁-C₈)-Alkoxycarbonyl, Heteroaryl-(C₁-C₈)-Alkoxycarbonyl, (C₂-C₈)-Alkenyloxycarbonyl, (C₂-C₈)-Alkinyloxycarbonyl, Heterocyclyl-(C₁-C₈)-alkyl stehen,
- R¹⁷: für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₂-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, (C₁-C₈)-Alkoxycarbonyl-(C₁-C₈)-alkyl, (C₂-C₈)-Alkenyloxycarbonyl-(C₁-C₈)-alkyl, Aryl-(C₁-C₈)-Alkoxycarbonyl-(C₁-C₈)-alkyl, Hydroxycarbonyl-(C₁-C₈)-alkyl, Heterocyclyl, Heterocyclyl-(C₁-C₈)-alkyl steht,
- R¹⁸: für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₂-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₁₀)-Cycloalkenyl-(C₁-C₈)-alkyl, NR¹⁵R¹⁶ steht
und
- R¹⁹ und R²⁰: unabhängig voneinander für Wasserstoff, Halogen, (C₁-C₈)-Alkyl, (C₃-C₈)-Cycloalkyl, (C₂-C₈)-Alkenyl, (C₂-C₈)-Alkinyl, (C₁-C₁₀)-Haloalkyl, Aryl stehen, oder
- R¹⁹ und R²⁰: mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden.

Die Verbindungen der allgemeinen Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetall-salze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Im Folgenden werden die erfindungsgemäß verwendeten Verbindungen der Formel (I) und ihre Salze "Verbindungen der allgemeinen Formel (I)" bezeichnet.

Bevorzugter Erfindungsgegenstand sind Verbindungen der allgemeinen Formel (I), worin
- R¹: für (C₁-C₇)-Alkyl, Amino, Bis-[(C₁-C₇)-alkyl]amino steht,
- R²: für Wasserstoff, (C₁-C₇)-Alkyl steht,
- R³: für Wasserstoff, Halogen, (C₁-C₇)-Alkoxy steht,
- R⁴: für Halogen, Cyano, NO₂, C(O)NH₂, C(S)NH₂, (C₁-C₇)-Haloalkyl, (C₂-C₇)-Alkinyl steht,
- R⁵ und R⁶: unabhängig voneinander für Wasserstoff, Halogen, (C₁-C₇)-Alkyl, (C₃-C₇)-Cycloalkyl, (C₃-C₇)-Cycloalkyl-(C₁-C₇)-alkyl, (C₂-C₇)-Alkenyl, (C₂-C₇)-Alkinyl, (C₁-C₁₀)-Haloalkyl, (C₂-C₇)-Haloalkenyl, (C₂-C₇)-Haloalkinyl, (C₃-C₁₀)-Halocycloalkyl, (C₄-C₁₀)-Cycloalkenyl, (C₄-C₁₀)-Halocycloalkenyl, (C₁-C₇)-Alkoxy, (C₁-C₇)-Alkoxy-(C₁-C₇)-alkyl, (C₁-C₇)-Alkoxy-(C₁-C₇)-haloalkyl, (C₁-C₇)-Haloalkoxy-(C₁-C₇)-haloalkyl, (C₁-C₇)-Haloalkoxy-(C₁-C₇)-alkyl, Aryl, Aryl-(C₁-C₇)-alkyl, Heteroaryl, Heteroaryl-(C₁-C₇)-alkyl, (C₄-C₁₀)-Cycloalkenyl-(C₁-C₇)-alkyl, Heterocyclyl, Heterocyclyl-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkylcarbonyl-(C₁-C₇)-alkyl, C(O)OR¹⁷, C(O)NR¹⁵R¹⁶, C(O)R¹⁷, R¹⁷O(O)C-(C₁-C₇)-alkyl, R¹⁵R¹⁶N(O)C-(C₁-C₇)-alkyl, R¹⁵R¹⁶N-(C₁-C₇)-alkyl stehen, oder
- R⁵ und R⁶: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁵ und R⁶: mit dem Kohlenstoffatom, an das sie gebunden sind, eine gegebenenfalls weiter durch R¹⁹ und R²⁰ substituierte Doppelbindung, gemäß nachfolgender Formel (I'), bilden 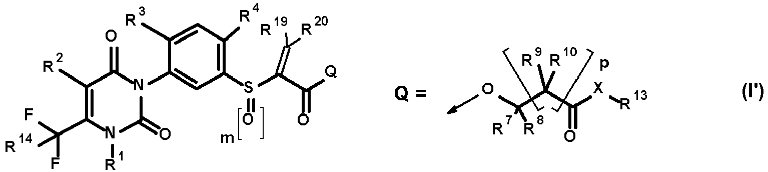
- m: für 0, 1, 2 steht,
- p: für 0, 1, 2, 3 steht,
- X: für O (Sauerstoff), N (Stickstoff) oder die Gruppierungen N-R¹¹ oder N-O-R¹² steht und wobei R¹¹ und R¹² in den Gruppierung N-R¹¹ und N-O-R¹² unabhängig voneinander die Bedeutungen gemäß der nachfolgenden Definitionen haben,
- R⁷, R⁸, R⁹ und R¹⁰: unabhängig voneinander für Wasserstoff, Fluor, (C₁-C₇)-Alkyl, (C₃-C₇)-Cycloalkyl, (C₃-C₇)-Cycloalkyl-(C₁-C₇)-alkyl, (C₂-C₇)-Alkenyl, (C₂-C₇)-Alkinyl, (C₁-C₁₀)-Haloalkyl, (C₂-C₇)-Haloalkenyl, (C₂-C₇)-Haloalkinyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-haloalkyl, (C₁-C₇)-Haloalkoxy-(C₁-C₇)-haloalkyl, Aryl, Aryl-(C₁-C₇)-alkyl, Heteroaryl, Heteroaryl-(C₁-C₇)-alkyl, (C₄-C₁₀)-Cycloalkenyl-(C₁-C₇)-alkyl, Heterocyclyl, Heterocyclyl-(C₁-C₇)-alkyl, R¹⁷O-(C₁-C₇)-alkyl, R¹⁸S-(C₁-C₇)-alkyl, (C₁-C₇)-Alkylcarbonyl-(C₁-C₇)-alkyl, C(O)OR¹⁷, C(O)NR¹⁵R¹⁶, C(O)R¹⁷, R¹⁷O(O)C-(C₁-C₇)-alkyl, R¹⁵R¹⁶N(O)C-(C₁-C₇)-alkyl, R¹⁵R¹⁶N-(C₁-C₇)-alkyl stehen, oder
- R⁵ und R⁷: mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 8-gliedrigen monocyclischen oder bicyclischen Ring bilden, oder
- R⁷ und R⁸: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹⁰: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹⁰: mit dem Kohlenstoffatom, an das sie gebunden sind, eine Exomethylengruppe bilden oder
- R⁷ und R⁹: mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹¹: für Wasserstoff, (C₁-C₇)-Alkyl, (C₃-C₇)-Cycloalkyl, Cyano-(C₁-C₇)-alkyl, (C₃-C₇)-Cycloalkyl-(C₁-C₇)-alkyl, (C₁-C₇)-Alkylsulfonyl, Arylsulfonyl, Heteroarylsulfonyl, (C₃-C₇)-Cycloalkylsulfonyl, Heterocyclylsulfonyl, Aryl-(C₁-C₇)-alkylsulfonyl, (C₁-C₇)-Alkylcarbonyl, Arylcarbonyl, Heteroarylcarbonyl, (C₃-C₇)-Cycloalkylcarbonyl, Heterocyclylcarbonyl, (C₁-C₇)-Alkoxycarbonyl, (C₁-C₇)-Alkoxy, (C₂-C₇)-Alkenyloxy, Aryl-(C₁-C₇)-alkoxycarbonyl, (C₁-C₇)-Haloalkylcarbonyl, (C₂-C₇)-Alkenyl, (C₂-C₇)-Alkinyl, (C₁-C₇)-Haloaₗkyl, Halo-(C₂-C₇)-alkinyl, Halo-(C₂-C₇)-alkenyl, (C₁-C₇)-Alkoxy-(C₁-C₇)-alkyl, Amino, (C₁-C₇)-Alkylamino, Bis[(C₁-C₇)-alkyl]amino, (C₁-C₇)-Alkoxy-(C₁-C₇)-alkoxy-(C₁-C₇)-alkyl, Heteroaryl-(C₁-C₇)-alkylsulfonyl, Heterocyclyl-(C₁-C₇)-alkylsulfonyl, (C₂-C₇)-Alkenyloxycarbonyl, (C₂-C₇)-Alkinyloxycarbonyl, (C₁-C₇)-Alkylaminocarbonyl, (C₃-C₇)-Cycloalkylaminocarbonyl, Bis-[(C₁-C₇)-alkyl]aminocarbonyl steht,
- R¹²: für Wasserstoff, (C₁-C₇)-Alkyl, (C₃-C₇)-Cycloalkyl-(C₁-C₇)-alkyl, (C₂-C₇)-Alkenyl, (C₂-C₇)-Alkinyl, (C₁-C₇)-Alkoxy-(C₁-C₇)-alkyl, Aryl, Aryl-(C₁-C₇)-alkyl, R¹⁷O(O)C-(C₁-C₇)-alkyl, R¹⁵R¹⁶N(O)C-(C₁-C₇)-alkyl stehen,
- R¹³: für Wasserstoff, (C₁-C₇)-Alkyl, (C₁-C₇)-Haloalkyl, (C₃-C₇)-Cycloalkyl, (C₃-C₇)-Cycloalkyl-(C₁-C₇)-alkyl, (C₂-C₇)-Alkenyl, (C₂-C₇)-Alkinyl, (C₁-C₇)-Alkoxy-(C₁-C₇)-alkyl, (C₁-C₇)-Haloalkoxy-(C₁-C₇)-alkyl, Aryl, Aryl-(C₁-C₇)-alkyl, Heteroaryl, Heteroaryl-(C₁-C₇)-alkyl, Heterocyclyl, Heterocyclyl-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkylcarbonyl-(C₁-C₇)-alkyl, R¹⁷O(O)C-(C₁-C₇)-alkyl, R¹⁵R¹⁶N(O)C-(C₁-C₇)-alkyl, R¹⁵R¹⁶N-(C₁-C₇)-alkyl stehen, oder
- R⁷ und R¹³: mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹³: mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R¹¹ und R¹³: in dem Fall, daß X für N-R¹¹ steht, mit dem Stickstoffatom, an das sie gebunden sind, einen vollständig gesättigten oder gegebenenfalls durch Heteroatome unterbrochenen und gegebenenfalls weiter substituierten 4 bis 10-gliedrigen monocyclischen oder bicyclischen Ring bilden, oder
- R¹² und R¹³: in dem Fall, daß X für N-O-R¹² steht, mit dem Stickstoffatom, an das sie gebunden sind, einen vollständig gesättigten und gegebenenfalls weiter substituierten 4 bis 10-gliedrigen monocyclischen oder bicyclischen Ring bilden,
- R¹⁴: für Wasserstoff, Fluor, Chlor, Brom, (C₁-C₅)-Haloalkyl, (C₁-C₇)-Alkoxy steht,
- R¹⁵ und R¹⁶: gleich oder verschieden sind und unabhängig voneinander für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₂-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Haloalkoxy-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, COR¹⁷, SO₂R¹⁸, (C₁-C₇)-Alkyl-HNO₂S-, (C₃-C₇)-Cycloalkyl-HNO₂S-, Heterocyclyl, (C₁-C₇)-Alkoxycarbonyl-(C₁-C₇)-alkyl, (C₁-C₇)-Alkoxycarbonyl, Aryl-(C₁-C₇)-Alkoxycarbonyl-(C₁-C₇)-alkyl, Aryl-(C₁-C₇)-Alkoxycarbonyl, Heteroaryl-(C₁-C₇)-Alkoxycarbonyl, (C₂-C₇)-Alkenyloxycarbonyl, (C₂-C₇)-Alkinyloxycarbonyl, Heterocyclyl-(C₁-C₇)-alkyl stehen,
- R¹⁷: für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₂-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, (C₁-C₇)-Alkoxycarbonyl-(C₁-C₇)-alkyl, (C₂-C₇)-Alkenyloxycarbonyl-(C₁-C₇)-alkyl, Aryl-(C₁-C₇)-Alkoxycarbonyl-(C₁-C₇)-alkyl, Hydroxycarbonyl-(C₁-C₇)-alkyl, Heterocyclyl, Heterocyclyl-(C₁-C₇)-alkyl steht
und
- R¹⁸: für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₂-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, Heterocyclyl-(C₁-C₇)-alkyl, (C₃-C₇)-Cycloalkyl-(C₁-C₇)-alkyl, (C₄-C₁₀)-Cycloalkenyl-(C₁-C₇)-alkyl, NR¹⁵R¹⁶ steht
und
- R¹⁹ und R²⁰: unabhängig voneinander für Wasserstoff, Halogen, (C₁-C₇)-Alkyl, (C₃-C₇)-Cycloalkyl, (C₂-C₇)-Alkenyl, (C₂-C₇)-Alkinyl, (C₁-C₇)-Haloalkyl, Aryl stehen, oder
- R¹⁹ und R²⁰: mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden. Besonders bevorzugter Erfindungsgegenstand sind Verbindungen der allgemeinen Formel (I), worin
- R¹: für (C₁-C₆)-Alkyl, Amino, Bis-[(C₁-C₆)-alkyl]amino steht,
- R²: für Wasserstoff, (C₁-C₆)-Alkyl steht,
- R³: für Wasserstoff, Halogen, (C₁-C₆)-Alkoxy steht,
- R⁴: für Halogen, Cyano, NO₂, C(O)NH₂, C(S)NH₂, (C₁-C₆)-Haloalkyl, (C₂-C₆)-Alkinyl steht,
- R⁵ und R⁶: unabhängig voneinander für Wasserstoff, Halogen, (C₁-C₆)-Alkyl, (C₃-C₆)-Cycloalkyl, (C₃-C₆)-Cycloalkyl-(C₁-C₆)-alkyl, (C₂-C₆)-Alkenyl, (C₂-C₆)-Alkinyl, (C₁-C₁₀)-Haloalkyl, (C₂-C₆)-Haloalkenyl, (C₂-C₆)-Haloalkinyl, (C₃-C₁₀)-Halocycloalkyl, (C₄-C₁₀)-Cycloalkenyl, (C₄-C₁₀)-Halocycloalkenyl, (C₁-C₆)-Alkoxy, (C₁-C₆)-Alkoxy-(C₁-C₆)-alkyl, (C₁-C₆)-Alkoxy-(C₁-C₆)-haloalkyl, (C₁-C₆)-Haloalkoxy-(C₁-C₆)-haloalkyl, (C₁-C₆)-Haloalkoxy-(C₁-C₆)-alkyl, Aryl, Aryl-(C₁-C₆)-alkyl, Heteroaryl, Heteroaryl-(C₁-C₆)-alkyl, (C₄-C₁₀)-Cycloalkenyl-(C₁-C₆)-alkyl, Heterocyclyl, Heterocyclyl-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkylcarbonyl-(C₁-C₆)-alkyl, C(O)OR¹⁷, C(O)NR¹⁵R¹⁶, C(O)R¹⁷, R¹⁷O(O)C-(C₁-C₆)-alkyl, R¹⁵R¹⁶N(O)C-(C₁-C₆)-alkyl, R¹⁵R¹⁶N-(C₁-C₆)-alkyl stehen, oder
- R⁵ und R⁶: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁵ und R⁶: mit dem Kohlenstoffatom, an das sie gebunden sind, eine gegebenenfalls weiter durch R¹⁹ und R²⁰ substituierte Doppelbindung, gemäß nachfolgender Formel (I'), bilden 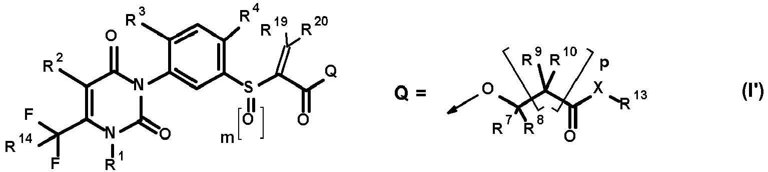
- m: für 0, 1, 2 steht,
- p: für 0, 1, 2, 3 steht,
- X: für O (Sauerstoff), N (Stickstoff) oder die Gruppierungen N-R¹¹ oder N-O-R¹² steht und wobei R¹¹ und R¹² in den Gruppierung N-R¹¹ und N-O-R¹² unabhängig voneinander die Bedeutungen gemäß der nachfolgenden Definitionen haben,
- R⁷, R⁸, R⁹ und R¹⁰: unabhängig voneinander für Wasserstoff, Fluor, (C₁-C₆)-Alkyl, (C₃-C₆)-Cycloalkyl, (C₃-C₆)-Cycloalkyl-(C₁-C₆)-alkyl, (C₂-C₆)-Alkenyl, (C₂-C₆)-Alkinyl, (C₁-C₁₀)-Haloalkyl, (C₂-C₆)-Haloalkenyl, (C₂-C₆)-Haloalkinyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-haloalkyl, (C₁-C₆)-Haloalkoxy-(C₁-C₆)-haloalkyl, Aryl, Aryl-(C₁-C₆)-alkyl, Heteroaryl, Heteroaryl-(C₁-C₆)-alkyl, (C₄-C₁₀)-Cycloalkenyl-(C₁-C₆)-alkyl, Heterocyclyl, Heterocyclyl-(C₁-C₆)-alkyl, R¹⁷O-(C₁-C₆)-alkyl, R¹⁸S-(C₁-C₆)-alkyl, (C₁-C₆)-Alkylcarbonyl-(C₁-C₆)-alkyl, C(O)OR¹⁷, C(O)NR¹⁵R¹⁶, C(O)R¹⁷, R¹⁷O(O)C-(C₁-C₆)-alkyl, R¹⁵R¹⁶N(O)C-(C₁-C₆)-alkyl, R¹⁵R¹⁶N-(C₁-C₆)-alkyl stehen, oder
- R⁵ und R⁷: mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 8-gliedrigen monocyclischen oder bicyclischen Ring bilden, oder
- R⁷ und R⁸: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹⁰: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹⁰: mit dem Kohlenstoffatom, an das sie gebunden sind, eine Exomethylengruppe bilden oder
- R⁷ und R⁹: mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹¹: für Wasserstoff, (C₁-C₆)-Alkyl, (C₃-C₆)-Cycloalkyl, Cyano-(C₁-C₆)-alkyl, (C₃-C₆)-Cycloalkyl-(C₁-C₆)-alkyl, (C₁-C₆)-Alkylsulfonyl, Arylsulfonyl, Heteroarylsulfonyl, (C₃-C₆)-Cycloalkylsulfonyl, Heterocyclylsulfonyl, Aryl-(C₁-C₆)-alkylsulfonyl, (C₁-C₆)-Alkylcarbonyl, Arylcarbonyl, Heteroarylcarbonyl, (C₃-C₆)-Cycloalkylcarbonyl, Heterocyclylcarbonyl, (C₁-C₆)-Alkoxycarbonyl, (C₁-C₆)-Alkoxy, (C₂-C₆)-Alkenyloxy, Aryl-(C₁-C₆)-alkoxycarbonyl, (C₁-C₆)-Haloalkylcarbonyl, (C₂-C₆)-Alkenyl, (C₂-C₆)-Alkinyl, (C₁-C₆)-Haloalkyl, Halo-(C₂-C₆)-alkinyl, Halo-(C₂-C₆)-alkenyl, (C₁-C₆)-Alkoxy-(C₁-C₆)-alkyl, Amino, (C₁-C₆)-Alkylamino, Bis[(C₁-C₆)-alkyl]amino, (C₁-C₆)-Alkoxy-(C₁-C₆)-alkoxy-(C₁-C₆)-alkyl, Heteroaryl-(C₁-C₆)-alkylsulfonyl, Heterocyclyl-(C₁-C₆)-alkylsulfonyl, (C₂-C₆)-Alkenyloxycarbonyl, (C₂-C₆)-Alkinyloxycarbonyl, (C₁-C₆)-Alkylaminocarbonyl, (C₃-C₆)-Cycloalkylaminocarbonyl, Bis-[(C₁-C₆)-alkyl]aminocarbonyl steht,
- R¹²: für Wasserstoff, (C₁-C₆)-Alkyl, (C₃-C₆)-Cycloalkyl-(C₁-C₆)-alkyl, (C₂-C₆)-Alkenyl, (C₂-C₆)-Alkinyl, (C₁-C₆)-Alkoxy-(C₁-C₆)-alkyl, Aryl, Aryl-(C₁-C₆)-alkyl, R¹⁷O(O)C-(C₁-C₆)-alkyl, R¹⁵R¹⁶N(O)C-(C₁-C₆)-alkyl stehen,
- R¹³: für Wasserstoff, (C₁-C₆)-Alkyl, (C₁-C₆)-Haloalkyl, (C₃-C₆)-Cycloalkyl, (C₃-C₆)-Cycloalkyl-(C₁-C₆)-alkyl, (C₂-C₆)-Alkenyl, (C₂-C₆)-Alkinyl, (C₁-C₆)-Alkoxy-(C₁-C₆)-alkyl, (C₁-C₆)-Haloalkoxy-(C₁-C₆)-alkyl, Aryl, Aryl-(C₁-C₆)-alkyl, Heteroaryl, Heteroaryl-(C₁-C₆)-alkyl, Heterocyclyl, Heterocyclyl-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkylcarbonyl-(C₁-C₆)-alkyl, R¹⁷O(O)C-(C₁-C₆)-alkyl, R¹⁵R¹⁶N(O)C-(C₁-C₆)-alkyl, R¹⁵R¹⁶N-(C₁-C₆)-alkyl stehen, oder
- R⁷ und R¹³: mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁹ und R¹³: mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R¹¹ und R¹³: in dem Fall, daß X für N-R¹¹ steht, mit dem Stickstoffatom, an das sie gebunden sind, einen vollständig gesättigten oder gegebenenfalls durch Heteroatome unterbrochenen und gegebenenfalls weiter substituierten 4 bis 10-gliedrigen monocyclischen oder bicyclischen Ring bilden, oder
- R¹² und R¹³: in dem Fall, daß X für N-O-R¹² steht, mit dem Stickstoffatom, an das sie gebunden sind, einen vollständig gesättigten und gegebenenfalls weiter substituierten 4 bis 10-gliedrigen monocyclischen oder bicyclischen Ring bilden,
- R¹⁴: für Wasserstoff, Fluor, Chlor, Brom, (C₁-C₅)-Haloalkyl, (C₁-C₆)-Alkoxy steht,
- R¹⁵ und R¹⁶: gleich oder verschieden sind und unabhängig voneinander für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₂-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, COR¹⁷, SO₂R¹⁸, (C₁-C₆)-Alkyl-HNO₂S-, (C₃-C₆)-Cycloalkyl-HNO₂S-, Heterocyclyl, (C₁-C₆)-Alkoxycarbonyl-(C₁-C₆)-alkyl, (C₁-C₆)-Alkoxycarbonyl, Aryl-(C₁-C₆)-Alkoxycarbonyl-(C₁-C₆)-alkyl, Aryl-(C₁-C₆)-Alkoxycarbonyl, Heteroaryl-(C₁-C₆)-Alkoxycarbonyl, (C₂-C₆)-Alkenyloxycarbonyl, (C₂-C₆)-Alkinyloxycarbonyl, Heterocyclyl-(C₁-C₆)-alkyl stehen,
- R¹⁷: für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₂-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, (C₁-C₆)-Alkoxycarbonyl-(C₁-C₆)-alkyl, (C₂-C₆)-Alkenyloxycarbonyl-(C₁-C₆)-alkyl, Aryl-(C₁-C₆)-Alkoxycarbonyl-(C₁-C₆)-alkyl, Hydroxycarbonyl-(C₁-C₆)-alkyl, Heterocyclyl, Heterocyclyl-(C₁-C₆)-alkyl steht
und
- R¹⁸: für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₂-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₁₀)-Cycloalkenyl-(C₁-C₆)-alkyl, NR¹⁵R¹⁶ steht
und
- R¹⁹ und R²⁰: unabhängig voneinander für Wasserstoff, Fluor, Chlor, Brom, (C₁-C₆)-Alkyl, (C₃-C₆)-Cycloalkyl, (C₂-C₆)-Alkenyl, (C₂-C₆)-Alkinyl, (C₁-C₆)-Haloalkyl, Aryl stehen, oder
- R¹⁹ und R²⁰: mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden.

Ganz besonders bevorzugter Erfindungsgegenstand sind Verbindungen der allgemeinen Formel (I), worin
- R¹: für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Amino, Dimethylamino, Diethylamino, Methyl(ethyl)amino, Methyl(n-propyl)amino steht,
- R²: für Wasserstoff, Methyl, Ethyl, n-Propyl, iso-Propyl steht,
- R³: für Wasserstoff, Fluor, Chlor, Brom, Methoxy, Ethoxy steht,
- R⁴: für Halogen, Cyano, NO₂, C(O)NH₂, C(S)NH₂, Difluormethyl, Trifluormethyl, Ethinyl, Propin-1-yl, 1-Butin-1-yl, Pentin-1-yl, Hexin-1-yl steht,
- R⁵ und R⁶: unabhängig voneinander für Wasserstoff, Fluor, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl,Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanocyclopropyl, 2-Cyanocyclopropyl, 1-Methylcyclobutyl, 2-Methylcyclobutyl, 3-Methylcyclobutyl, 3,3-Dimethylcyclobut-1-yl, 1-Cyanocyclobutyl, 2-Cyanocyclobutyl, 3-Cyanocyclobutyl, 3,3-Difluorcyclobut-1-yl, 3-Fluorcyclobut-1-yl, 2,2-Difluorcycloprop-1-yl, 1-Fluorcycloprop-1-yl, 2-Fluorcycloprop-1-yl, 1-Allylcyclopropyl, 1-Vinylcyclobutyl, 1-Vinylcyclopropyl, 1-Ethylcyclopropyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, 2-Fluorcycloprop-1-yl, 4-Fluorcyclohexyl, 4,4-Difluorcyclohexyl, Cyclopropylmethyl, Cyclobutylmethyl, Cyclopentylmethyl, Cyclohexylmethyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Ioddifluormethyl, Bromfluormethyl, 1-Fluorethyl, 2-Fluorethyl, Fluormethyl, Difluormethyl, 2,2-Difluorethyl, 2,2,2-Trifluorethyl, Difluor-tert.-butyl, Chlormethyl, Brommethyl, Methoxy, Ethoxy, n-Propyloxy, iso-Propyloxy, n-Butyloxy, tert-Butyloxy, Methoxymethyl, Ethoxymethyl, n-Propyloxymethyl, iso-Propyloxymethyl, Methoxyethyl, Ethoxyethyl, n-Propyloxyethyl, iso-Propyloxyethyl, Methoxy-n-propyl, Methoxydifluormethyl, Ethoxydifluormethyl, n-propyloxydifluormethyl, n-Butyloxydifluormethyl, Trifluormethoxymethyl, Trifluormethoxyethyl, Trifluormethoxy-n-propyl, Phenyl, 2-Fluor-Phenyl, 3-Fluor-Phenyl, 4-Fluor-Phenyl, 2,4-Difluor-Phenyl, 2,5-Difluor-Phenyl, 2,6-Difluor-Phenyl, 2,3-Difluor-Phenyl, 3,4-Difluor-Phenyl, 3,5-Difluor-Phenyl, 2,4,5-Trifluor-Phenyl, 3,4,5-Trifluor-Phenyl, 2-Chlor-Phenyl, 3-Chlor-Phenyl, 4-Chlor-Phenyl, 2,4-Dichlor-Phenyl, 2,5-Dichlor-Phenyl, 2,6-Dichlor-Phenyl, 2,3-Dichlor-Phenyl, 3,4-Dichlor-Phenyl, 3,5-Dichlor-Phenyl, 2,4,5-Trichlor-Phenyl, 3,4,5-Trichlor-Phenyl, 2,4,6-Trichlor-Phenyl, 2-Brom-Phenyl, 3-Brom-Phenyl, 4-Brom-Phenyl, 2-Iod-Phenyl, 3-Iod-Phenyl, 4-Iod-Phenyl, 2-Brom-4-Fluor-Phenyl, 2-Brom-4-Chlor-Phenyl, 3-Brom-4-Fluor-Phenyl, 3-Brom-4-Chlor-Phenyl, 3-Brom-5-Fluor-Phenyl, 3-Brom-5-Chlor-Phenyl, 2-Fluor-4-Brom-Phenyl, 2-Chlor-4-Brom-Phenyl, 3-Fluor-4-Brom-Phenyl, 3-Chlor-4-Brom-Phenyl, 2-Chlor-4-Fluor-Phenyl, 3-Chlor-4-Fluor-Phenyl, 2-Fluor-3-Chlor-Phenyl, 2-Fluor-4-Chlor-Phenyl, 2-Fluor-5-Chlor-Phenyl, 3-Fluor-4-Chlor-Phenyl, 3-Fluor-5-Chlor-Phenyl, 2-Fluor-6-Chlor-Phenyl, 2-Methyl-Phenyl, 3-Methyl-Phenyl, 4-Methyl-Phenyl, 2,4-Dimethyl-Phenyl, 2,5-Dimethyl-Phenyl, 2,6-Dimethyl-Phenyl, 2,3-Dimethyl-Phenyl, 3,4-Dimethyl-Phenyl, 3,5-Dimethyl-Phenyl, 2,4,5-Trimethyl-Phenyl, 3,4,5-Trimethyl-Phenyl, 2,4,6-Trimethyl-Phenyl, 2-Methoxy-Phenyl, 3-Methoxy-Phenyl, 4-Methoxy-Phenyl, 2,4-Dimethoxy-Phenyl, 2,5-Dimethoxy-Phenyl, 2,6-Dimethoxy-Phenyl, 2,3-Dimethoxy-Phenyl, 3,4-Dimethoxy-Phenyl, 3,5-Dimethoxy-Phenyl, 2,4,5-Trimethoxy-Phenyl, 3,4,5-Trimethoxy-Phenyl, 2,4,6-Trimethoxy-Phenyl, 2-Trifluormethoxy-Phenyl, 3-Trifluormethoxy-Phenyl, 4-Trifluormethoxy-Phenyl, 2-Difluormethoxy-Phenyl, 3-Difluormethoxy-Phenyl, 4-Difluormethoxy-Phenyl, 2-Trifluormethyl-Phenyl, 3-Trifluormethyl-Phenyl, 4-Trifluormethyl-Phenyl, 2-Difluormethyl-Phenyl, 3-Difluormethyl-Phenyl, 4-Difluormethyl-Phenyl, 3,5-Bis(Trifluormethyl)-Phenyl, 3-Trifluormethyl-5-Fluor-Phenyl, 3-Trifluormethyl-5-Chlor-Phenyl, 3-Methyl-5-Fluor-Phenyl, 3-Methyl-5-Chlor-Phenyl, 3-Methoxy-5-Fluor-Phenyl, 3-Methoxy-5-Chlor-Phenyl, 3-Trifluormethoxy-5-Chlor-Phenyl, 2-Ethoxy-Phenyl, 3-Ethoxy-Phenyl, 4-Ethoxy-Phenyl, 2-Methylthio-Phenyl, 3-Methylthio-Phenyl, 4-Methylthio-Phenyl, 2-Trifluormethylthio-Phenyl, 3-Trifluormethylthio-Phenyl, 4-Trifluormethylthio-Phenyl, 2-Ethyl-Phenyl, 3-Ethyl-Phenyl, 4-Ethyl-Phenyl, 2-Methoxycarbonyl-Phenyl, 3-Methoxycarbonyl-Phenyl, 4-Methoxycarbonyl-Phenyl, 2-Ethoxycarbonyl-Phenyl, 3-Ethoxycarbonyl-Phenyl, 4-Ethoxycarbonyl-Phenyl, Pyridin-2-yl, Pyridin-3-yl, Pyridin-4-yl, Pyrazin-2-yl, Pyridazin-3-yl, Pyridazin-4-yl, Pyrimidin-2-yl, Pyrimidin-5-yl, Pyrimidin-4-yl, Pyridazin-3-ylmethyl, Pyridazin-4-ylmethyl, Pyrimidin-2-ylmethyl, Pyrimidin-5-ylmethyl, Pyrimidin-4-ylmethyl, Pyrazin-2-ylmethyl, 3-Chlor-Pyrazin-2-yl, 3-Brom-Pyrazin-2-yl, 3-Methoxy-Pyrazin-2-yl, 3-Ethoxy-Pyrazin-2-yl, 3-Trifluormethylpyrazin-2-yl, 3-Cyanopyrazin-2-yl, Naphth-2-yl, Naphth-1-yl, Chinolin-4-yl, Chinolin-6-yl, Chinolin-8-yl, Chinolin-2-yl, Chinoxalin-2-yl, 2-Naphthylmethyl, 1-Naphthylmethyl, Chinolin-4-ylmethyl, Chinolin-6-ylmethyl, Chinolin-8-ylmethyl, Chinolin-2-ylmethyl, Chinoxalin-2-ylmethyl, Pyrazin-2-ylmethyl, 4-Chloropyridin-2-yl, 3-Chloropyridin-4-yl, 2-Chloropyridin-3-yl, 2-Chloropyridin-4-yl, 2-Chlorpyridin-5-yl, 2,6-Dichlorpyridin-4-yl, 3-Chlorpyridin-5-yl, 3,5-Dichlorpyridin-2-yl, 3-Chlor-5-Trifluormethylpyridin-2-yl, (4-Chloropyridin-2-yl)methyl, (3-Chloropyridin-4-yl)methyl, (2-Chloropyridin-3-yl)methyl, (2-Chloropyridin-4-yl)methyl, (2-Chlorpyridin-5-yl)methyl, (2,6-Dichlorpyridin-4-yl)methyl, (3-Chlorpyridin-5-yl)methyl, (3,5-Dichlorpyridin-2-yl)methyl, Thiophen-2-yl, Thiophen-3-yl, 5-Methylthiophen-2-yl, 5-Ethylthiophen-2-yl, 5-Chlorthiophen-2-yl, 5-Bromthiophen-2-yl, 4-Methylthiophen-2-yl, 3-Methylthiophen-2-yl, 5-Fluorthiophen-3-yl, 3,5-Dimethylthiophen-2-yl, 3-Ethylthiophen-2-yl, 4,5-Dimethylthiophen-2-yl, 3,4-Dimethylthiophen-2-yl, 4-Chlorthiophen-2-yl, Furan-2-yl, 5-Methylfuran-2-yl, 5-Ethylfuran-2-yl, 5-Methoxycarbonylfuran-2-yl, 5-Chlorfuran-2-yl, 5-Bromfuran-2-yl, Thiophan-2-yl, Thiophan-3-yl, Sulfolan-2-yl, Sulfolan-3-yl, Tetrahydrothiopyran-4-yl, Tetrahydropyran-4-yl, Tetrahydrofuran-2-yl, Tetrahydrofuran-3-yl, 1-(4-Methylphenyl)ethyl, 1-(3-Methylphenyl)ethyl, 1-(2-Methylphenyl)ethyl, 1-(4-Chlorphenyl)ethyl, 1-(3-Chlorphenyl)ethyl, 1-(2-Chlorphenyl)ethyl, Benzyl, (4-Fluorphenyl)methyl, (3-Fluorphenyl)methyl, (2-Fluorphenyl)methyl, (2,4-Difluorphenyl)methyl, (3,5-Difluorphenyl)methyl, (2,5-Difluorphenyl)methyl, (2,6-Difluorphenyl)methyl, (2,4,5-Trifluorphenyl)methyl, (2,4,6-Trifluorphenyl)methyl, (4-Chlorphenyl)methyl, (3-Chlorphenyl)methyl, (2-Chlorphenyl)methyl, (2,4-Dichlorphenyl)methyl, (3,5-Dichlorphenyl)methyl, (2,5-Dichlorphenyl)methyl, (2,6-Dichlorphenyl)methyl, (2,4,5-Trichlorphenyl)methyl, (2,4,6-Trichlorphenyl)methyl, (4-Bromphenyl)methyl, (3-Bromphenyl)methyl, (2-Bromphenyl)methyl, (4-Iodphenyl)methyl, (3-Iodphenyl)methyl, (2-Iodphenyl)methyl, (3-Chlor-5-Trifluormethyl-pyridin-2-yl)methyl, (2-Brom-4-Fluorphenl)methyl, (2-Brom-4-Chlorphenyl)methyl, (3-Brom-4-Fluorphenyl)methyl, (3-Brom-4-Chlorphenyl)methyl, (3-Brom-5-Fluorphenyl)methyl, (3-Brom-5-Chlorphenyl)methyl, (2-Fluor-4-Bromphenyl)methyl, (2-Chlor-4-Bromphenyl)methyl, (3-Fluor-4-Bromphenyl)methyl, (3-Chlor-4-Bromphenyl)methyl, (2-Chlor-4-Fluorphenyl)methyl, (3-Chlor-4-Fluorphenyl)methyl, (2-Fluor-3-Chlorphenyl)methyl, (2-Fluor-4-Chlorphenyl)methyl, (2-Fluor-5-Chlorphenyl)methyl, (3-Fluor-4-Chlorphenyl)methyl, (3-Fluor-5-Chlorphenyl)methyl, (2-Fluor-6-Chlorphenyl)methyl, 2-Phenyleth-1-yl, 3-Trifluormethyl-4-Chlorphenyl, 3-Chlor-4-Trifluormethylphenyl, 2-Chlor-4-Trifluormethylphenyl, 3,5-Dfluorpyridin-2-yl, (3,6-Dichlor-pyridin-2-yl)methyl, (4-Trifluormethylphenyl)methyl, (3-Trifluormethylphenyl)methyl, (2-Trifluormethylphenyl)methyl, (4-Trifluormethoxyphenyl)methyl, (3-Trifluormethoxyphenyl)methyl, (2-Trifluormethoxyphenyl)methyl, (4-Methoxyphenyl)methyl, (3-Methoxyphenyl)methyl, (2-Methoxyphenyl)methyl, (4-Methylphenyl)methyl, (3-Methylphenyl)methyl, (2-Methylphenyl)methyl, (4-Cyanophenyl)methyl, (3-Cyanophenyl)methyl, (2-Cyanophenyl)methyl, (2,4-Diethylphenyl)methyl, (3,5-Diethylphenyl)methyl, (3,4-Dimethylphenyl)methyl, (3,5-Dimethoxyphenyl)methyl, 1-Phenyleth-1-yl, 1-(o-Chlorphenyl)eth-1-yl, 1,3-Thiazol-2-yl, 4-Methyl-1,3-thiazol-2-yl, 1,3-Thiazol-2-yl, Methylthiomethyl, Ethylthiomethyl, Ethylthioethyl, Methylthioethyl, n-Propylthiomethyl, iso-Propylthiomethyl, Trifluormethylthiomethyl, trifluormethylthioethyl, Methylcarbonylmethyl, Ethylcarbonylmethyl, n-Propylcarbonylmethyl, iso-Propylcarbonylmethyl, Methylcarbonylethyl, Hydroxycarbonyl, Methoxycarbonyl, Ethoxycarbonyl, n-Propyloxycarbonyl, iso-Propyloxycarbonyl, n-Butyloxycarbonyl, tert-Butyloxycarbonyl, Allyloxycarbonyl, Benzyloxycarbonyl, Aminocarbonyl, Methylaminocarbonyl, Ethylaminocarbonyl, n-Propylaminocarbonyl, iso-Propylaminocarbonyl, Dimethylaminocarbonyl, Diethylaminocarbonyl, Methyl(ethyl)aminocarbonyl, Cyclopropylaminocarbonyl, Cyclobutylaminocarbonyl, Cyclopentylaminocarbonyl, Cyclohexylaminocarbonyl, Allylaminocarbonyl, benzylaminocarbonyl, tert-Butyloxycarbonylaminocarbonyl, Hydroxycarbonylmethyl, Methoxycarbonylmethyl, Ethoxycarbonylmethyl, n-Propyloxycarbonylmethyl, iso-Propyloxycarbonylmethyl, n-Butyloxycarbonylmethyl, tert-Butyloxycarbonylmethyl, Allyloxycarbonylmethyl, Benzyloxycarbonylmethyl, Aminocarbonylmethyl, Methylaminocarbonylmethyl, Ethylaminocarbonylmethyl, n-Propylaminocarbonylmethyl, iso-Propylaminocarbonylmethyl, Dimethylaminocarbonylmethyl, Diethylaminocarbonylmethyl, Methyl(ethyl)aminocarbonylmethyl, Cyclopropylaminocarbonylmethyl, Cyclobutylaminocarbonylmethyl, Cyclopentylaminocarbonylmethyl, Cyclohexylaminocarbonylmethyl, Allylaminocarbonylmethyl, Benzylaminocarbonylmethyl, 1-(Hydroxycarbonyl)eth-1-yl, 1-(Methoxycarbonyl)eth-1-yl, 1-(Ethoxycarbonyl)eth-1-yl, 1-(tert-Butyloxycarbonyl)eth-1-yl, 1-(Benzyloxycarbonyl)eth-1-yl, 2-(Hydroxycarbonyl)eth-1-yl, 2-(Methoxycarbonyl)eth-1-yl, 2-(Ethoxycarbonyl)eth-1-yl, 2-(tert-Butyloxycarbonyl)eth-1-yl, 2-(Benzyloxycarbonyl)eth-1-yl, 1-(Hydroxycarbonyl)prop-1-yl, 1-(Methoxycarbonyl)prop-1-yl, 1-(Ethoxycarbonyl)prop-1-yl, 1-(tert-Butyloxycarbonyl)prop-1-yl, 1-(Benzyloxycarbonyl)prop-1-yl, 2-(Hydroxycarbonyl)prop-1-yl, 2-(Methoxycarbonyl)prop-1-yl, 2-(Ethoxycarbonyl)prop-1-yl, 2-(tert-Butyloxycarbonyl)prop-1-yl, 2-(Benzyloxycarbonyl)prop-1-yl, 1-(Hydroxycarbonyl)prop-2-yl, 1-(Methoxycarbonyl)prop-2-yl, 1-(Ethoxycarbonyl)prop-2-yl, 1-(tert-Butyloxycarbonyl)prop-2-yl, 1-(Benzyloxycarbonyl)prop-2-yl, 3-(Hydroxycarbonyl)prop-1-yl, 3-(Methoxycarbonyl)prop-1-yl, 3-(Ethoxycarbonyl)prop-1-yl, 3-(tert-Butyloxycarbonyl)prop-1-yl, 3-(Benzyloxycarbonyl)prop-1-yl, Aminomethyl, 2-Aminoeth-1-yl, 1-Aminoethl-yl, 1-Amino-prop-1-yl, 3-Amino-prop-1-yl, Methylaminomethyl, Dimethylaminomethyl, Diethylaminomethyl, Ethylaminomethyl, iso-Propylaminomethyl, Cyclopropylaminomethyl, Cyclobutylaminomethyl, Cyclopentylaminomethyl, Cyclohexylaminomethyl, Methoxycarbonylaminomethyl, Ethoxycarbonylaminomethyl tert-Butyloxycarbonylaminomethyl, Methylcarbonylaminomethyl, Ethylcarbonylaminomethyl, n-propylcarbonylaminomethyl, iso-Propylcarbonylaminomethyl, 2-(Methylamino)eth-1-yl, 2-(Dieth-1-ylamino)eth-1-yl, 2-(Diethylamino)eth-1-yl, 2-(Ethylamino)eth-1-yl, 2-(iso-Propylamino)eth-1-yl, 2-(Cyclopropylamino)eth-1-yl, 2-(Cyclobutylamino)eth-1-yl, 2-(Cyclopentylamino)eth-1-yl, 2-(Cyclohexylamino)eth-1-yl, 2-(Methoxycarbonylamino)eth-1-yl, 2-(Ethoxycarbonylamino)eth-1-yl, 2-(tert-Butyloxycarbonylamino)eth-1-yl, 2-(Methylcarbonylamino)eth-1-yl, 2-(Ethylcarbonylamino)eth-1-yl, 2-(n-Propylcarbonylamino)eth-1-yl, 2-(iso-Propylcarbonylamino)eth-1-yl, 1-(Methylamino)eth-1-yl, 1-(Dieth-1-ylamino)eth-1-yl, 1-(Diethylamino)eth-1-yl, 1-(Ethylamino)eth-1-yl, 1-(iso-Propylamino)eth-1-yl, 1-(Cyclopropylamino)eth-1-yl, 1-(Cyclobutylamino)eth-1-yl, 1-(Cyclopentylamino)eth-1-yl, 1-(Cyclohexylamino)eth-1-yl, 1-(Methoxycarbonylamino)eth-1-yl, 1-(Ethoxycarbonylamino)eth-1-yl, 1-(tert-Butyloxycarbonylamino)eth-1-yl, 1-(Methylcarbonylamino)eth-1-yl, 1-(Ethylcarbonylamino)eth-1-yl, 1-(n-Propylcarbonylamino)eth-1-yl, 1-(iso-Propylcarbonylamino)eth-1-yl stehen, oder
- R⁵ und R⁶: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁵ und R⁶: mit dem Kohlenstoffatom, an das sie gebunden sind, eine gegebenenfalls weiter durch R¹⁹ und R²⁰ substituierte Doppelbindung, gemäß nachfolgender Formel (I'), bilden 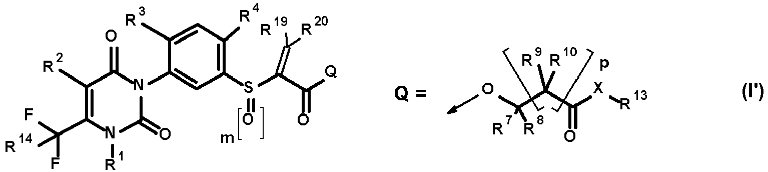
- R¹⁴: für Wasserstoff, Fluor, Chlor, Brom, Trifluormethyl, Methoxy, Ethoxy, n-Propyloxy, n-Butyloxy steht,
- m: für 0, 1, 2 steht,
- R¹⁹ und R²⁰: unabhängig voneinander für Wasserstoff, Fluor, Chlor, Brom, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, Cyclopropyl, Cyclobutyl, Cyclopentyl, Cyclohexyl, Trifluormethyl, Difluormethyl, Pentafluorethyl, Ethenyl, 1-Propenyl, 1-Methyl-ethenyl, 1-Butenyl, Phenyl stehen, oder
- R¹⁹ und R²⁰: mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden
und Q für eine der nachfolgend spezifisch genannten Gruppierungen Q-1 bis Q-300 steht:

| | | | | |
|---|---|---|---|---|
| 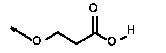 | 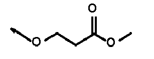 | 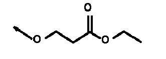 | 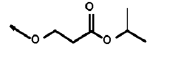 | 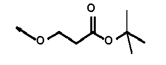 |
| Q-1 | Q-2 | Q-3 | Q-4 | Q-5 |
| | | | | |
| 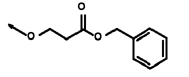 | 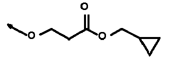 | 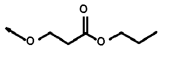 | 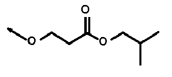 | 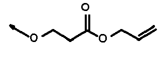 |
| Q-6 | Q-7 | Q-8 | Q-9 | Q-10 |
| 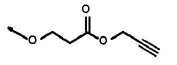 | 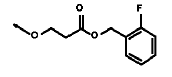 | 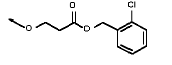 | 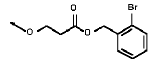 | 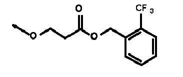 |
| Q-11 | Q-12 | Q-13 | Q-14 | Q-15 |
| | | | | |
| 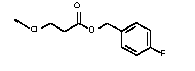 | 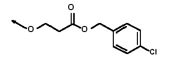 | 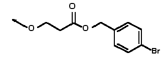 | 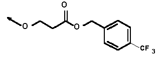 | 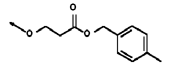 |
| Q-16 | Q-17 | Q-18 | Q-19 | Q-20 |
| | | | | |
| 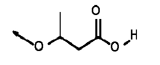 | 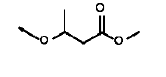 | 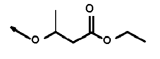 | 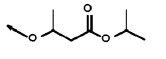 | 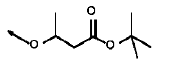 |
| Q-21 | Q-22 | Q-23 | Q-24 | Q-25 |
| | | | | |
| 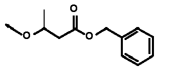 | 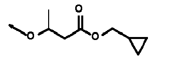 | 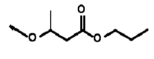 | 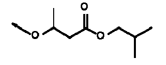 | 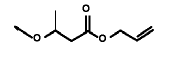 |
| Q-26 | Q-27 | Q-28 | Q-29 | Q-30 |
| | | | | |
| 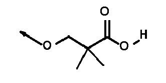 | 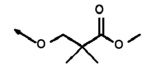 | 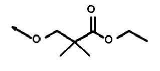 | 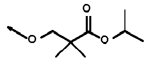 | 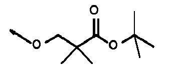 |
| Q-31 | Q-32 | Q-33 | Q-34 | Q-35 |
| | | | | |
| 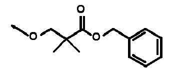 | 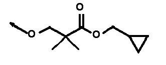 | 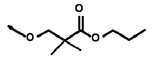 | 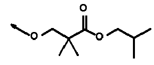 | 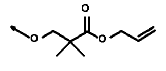 |
| Q-36 | Q-37 | Q-38 | Q-39 | Q-40 |
| | | | | |
| 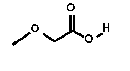 | 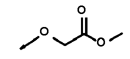 | 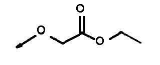 | 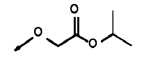 | 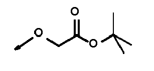 |
| Q-41 | Q-42 | Q-43 | Q-44 | Q-45 |
| | | | | |
| 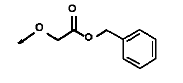 | 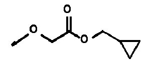 | 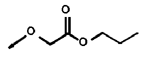 | 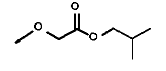 | 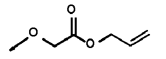 |
| Q-46 | Q-47 | Q-48 | Q-49 | Q-50 |
| | | | | |
| 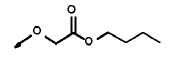 | 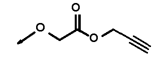 | 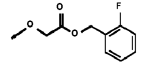 | 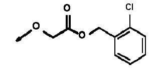 | 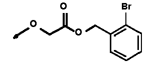 |
| Q-51 | Q-52 | Q-53 | Q-54 | Q-55 |
| 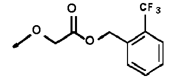 | 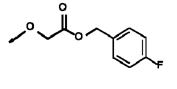 | 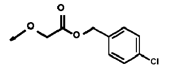 | 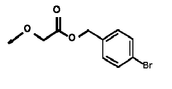 | 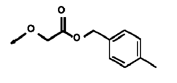 |
| Q-56 | Q-57 | Q-58 | Q-59 | Q-50 |
| | | | | |
| 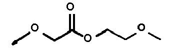 | 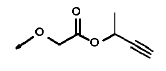 | 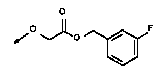 | 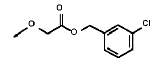 | 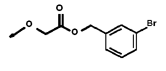 |
| Q-61 | Q-62 | Q-63 | Q-64 | Q-65 |
| | | | | |
| 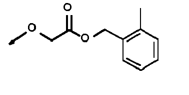 | 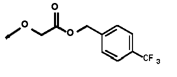 | 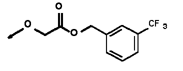 | 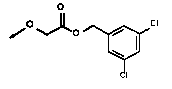 | 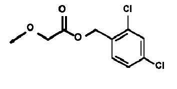 |
| Q-66 | Q-67 | Q-68 | Q-69 | Q-70 |
| | | | | |
| 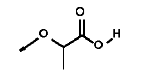 | 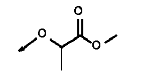 | 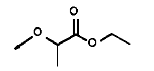 | 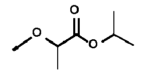 | 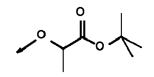 |
| Q-71 | Q-72 | Q-73 | Q-74 | Q-75 |
| | | | | |
| 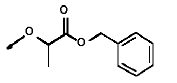 | 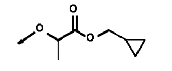 | 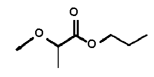 | 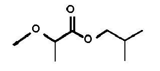 | 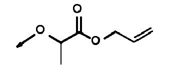 |
| Q-76 | Q-77 | Q-78 | Q-79 | Q-80 |
| | | | | |
| 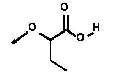 | 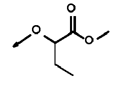 | 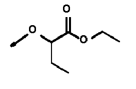 | 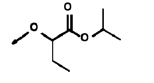 | 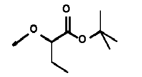 |
| Q-81 | Q-82 | Q-83 | Q-84 | Q-85 |
| | | | | |
| 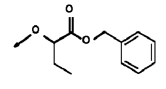 | 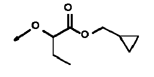 | 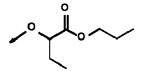 | 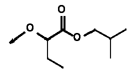 | 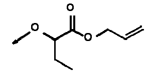 |
| Q-86 | Q-87 | Q-88 | Q-89 | Q-90 |
| | | | | |
| 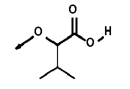 | 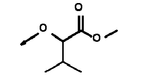 | 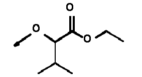 | 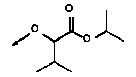 | 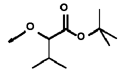 |
| Q-91 | Q-92 | Q-93 | Q-94 | Q-95 |
| 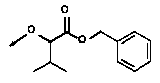 | 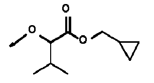 | 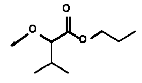 | 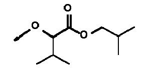 | 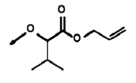 |
| Q-96 | Q-97 | Q-98 | Q-99 | Q-100 |
| | | | | |
| 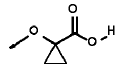 | 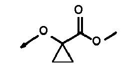 | 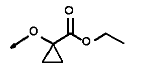 | 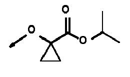 | 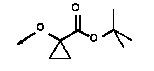 |
| Q-101 | Q-102 | Q-103 | Q-104 | Q-105 |
| | | | | |
| 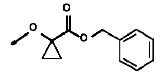 | 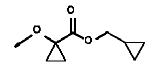 | 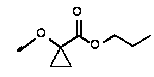 | 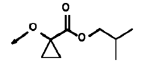 | 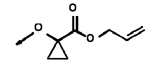 |
| Q-106 | Q-107 | Q-108 | Q-109 | Q-110 |
| | | | | |
| 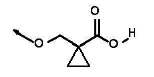 | 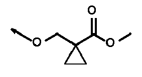 | 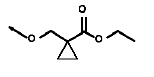 | 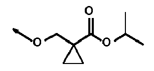 | 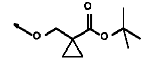 |
| Q-111 | Q-112 | Q-113 | Q-114 | Q-115 |
| | | | | |
| 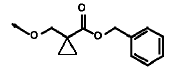 | 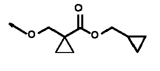 | 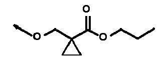 | 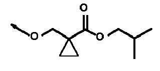 | 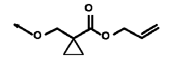 |
| Q-116 | Q-117 | Q-118 | Q-119 | Q-120 |
| | | | | |
| 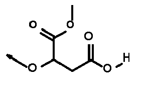 | 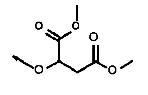 | 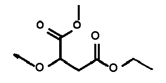 | 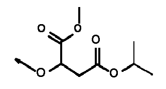 | 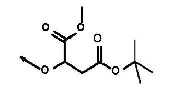 |
| Q-121 | Q-122 | Q-123 | Q-124 | Q-125 |
| | | | | |
| 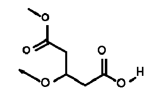 | 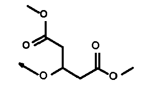 | 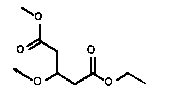 | 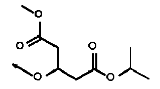 | 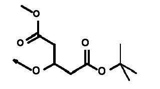 |
| Q-126 | Q-127 | Q-128 | Q-129 | Q-130 |
| | | | | |
| 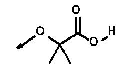 | 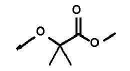 | 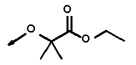 | 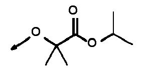 | 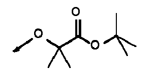 |
| Q-131 | Q-132 | Q-133 | Q-134 | Q-135 |
| 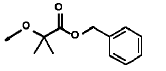 | 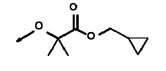 | 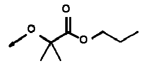 | 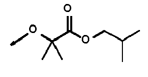 | 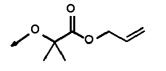 |
| Q-136 | Q-137 | Q-138 | Q-139 | Q-140 |
| | | | | |
| 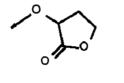 | 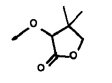 | 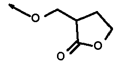 | 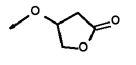 | 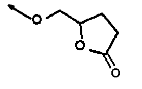 |
| Q-141 | Q-142 | Q-143 | Q-144 | Q-145 |
| | | | | |
| 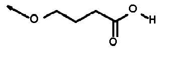 | 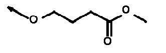 | 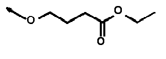 | 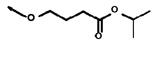 | 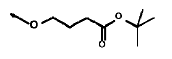 |
| Q-146 | Q-147 | Q-148 | Q-149 | Q-150 |
| | | | | |
| 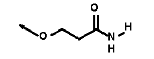 | 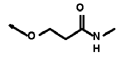 | 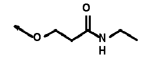 | 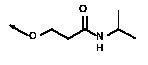 | 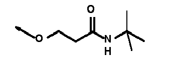 |
| Q-151 | Q-152 | Q-153 | Q-154 | Q-155 |
| | | | | |
| 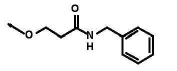 | 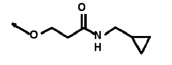 | 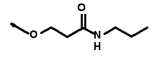 | 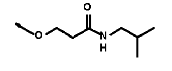 | 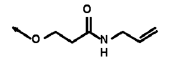 |
| Q-156 | Q-157 | Q-158 | Q-159 | Q-160 |
| | | | | |
| 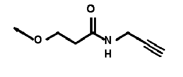 | 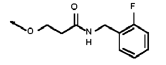 | 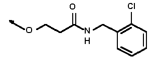 | 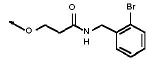 | 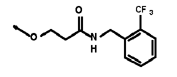 |
| Q-161 | Q-162 | Q-163 | Q-164 | Q-165 |
| | | | | |
| 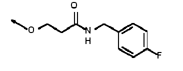 | 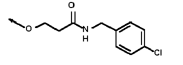 | 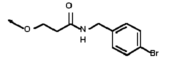 | 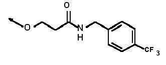 | 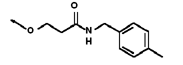 |
| Q-166 | Q-167 | Q-168 | Q-169 | Q-170 |
| | | | | |
| 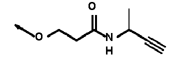 | 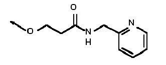 | 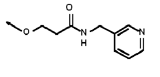 | 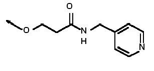 | 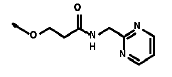 |
| Q-171 | Q-172 | Q-173 | Q-174 | Q-175 |
| | | | | |
| 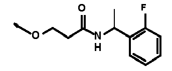 | 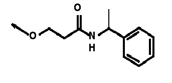 | 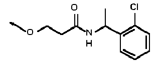 | 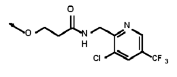 | 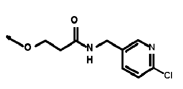 |
| Q-176 | Q-177 | Q-178 | Q-179 | Q-180 |
| 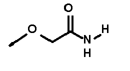 | 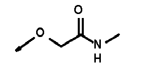 | 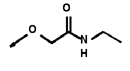 | 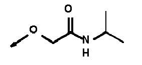 | 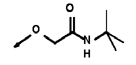 |
| Q-181 | Q-182 | Q-183 | Q-184 | Q-185 |
| | | | | |
| 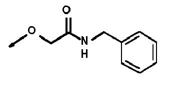 | 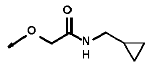 | 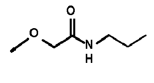 | 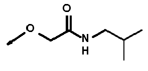 | 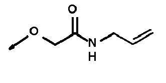 |
| Q-186 | Q-187 | Q-188 | Q-189 | Q-190 |
| | | | | |
| 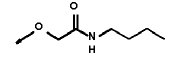 | 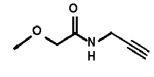 | 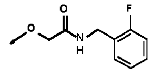 | 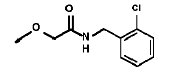 | 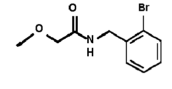 |
| Q-191 | Q-192 | Q-193 | Q-194 | Q-195 |
| | | | | |
| 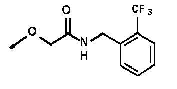 | 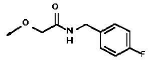 | 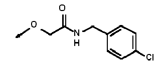 | 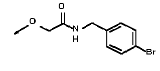 | 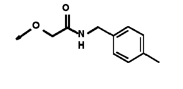 |
| Q-196 | Q-197 | Q-198 | Q-199 | Q-200 |
| | | | | |
| 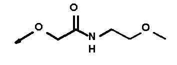 | 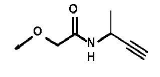 | 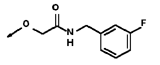 | 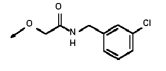 | 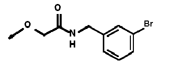 |
| Q-201 | Q-202 | Q-203 | Q-204 | Q-205 |
| | | | | |
| 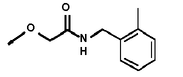 | 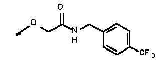 | 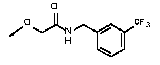 | 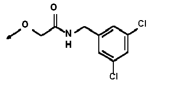 | 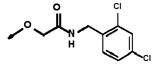 |
| Q-206 | Q-207 | Q-208 | Q-209 | Q-210 |
| | | | | |
| 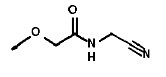 | 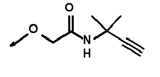 | 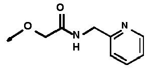 | 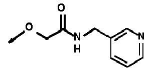 | 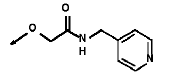 |
| Q-211 | Q-212 | Q-213 | Q-214 | Q-215 |
| | | | | |
| 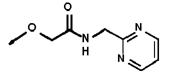 | 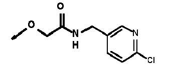 | 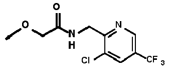 | 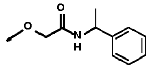 | 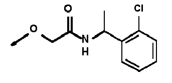 |
| Q-216 | Q-217 | Q-218 | Q-219 | Q-220 |
| 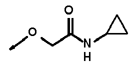 | 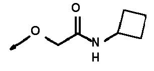 | 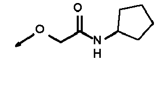 | 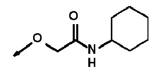 | 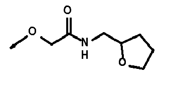 |
| Q-221 | Q-222 | Q-223 | Q-224 | Q-225 |
| | | | | |
| 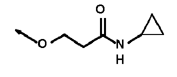 | 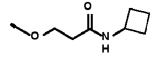 | 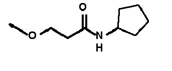 | 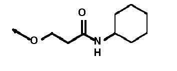 | 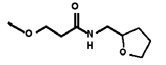 |
| Q-226 | Q-227 | Q-228 | Q-229 | Q-230 |
| | | | | |
| 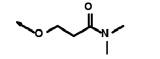 | 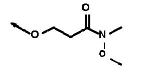 | 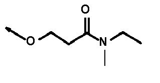 | 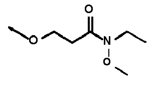 | 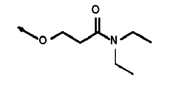 |
| Q-231 | Q-232 | Q-233 | Q-234 | Q-235 |
| | | | | |
| 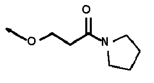 | 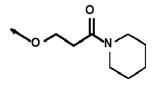 | 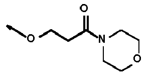 | 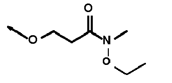 | 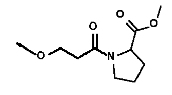 |
| Q-236 | Q-237 | Q-238 | Q-239 | Q-240 |
| | | | | |
| 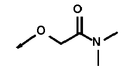 | 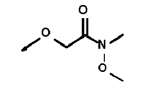 | 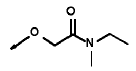 | 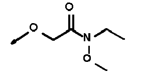 | 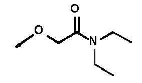 |
| Q-241 | Q-242 | Q-243 | Q-244 | Q-245 |
| | | | | |
| 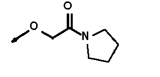 | 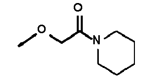 | 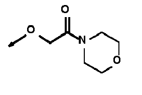 | 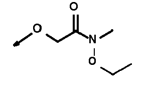 | 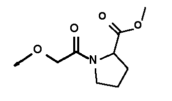 |
| Q-246 | Q-247 | Q-248 | Q-249 | Q-250 |
| | | | | |
| 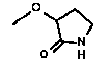 | 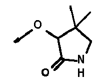 | 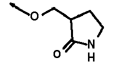 | 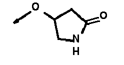 | 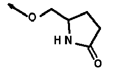 |
| Q-251 | Q-252 | Q-253 | Q-254 | Q-255 |
| | | | | |
| 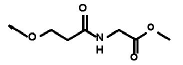 | 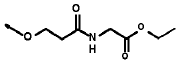 | 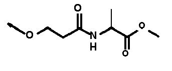 | 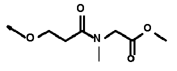 | 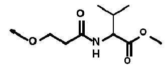 |
| Q-256 | Q-257 | Q-258 | Q-259 | Q-260 |
| 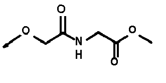 | 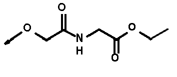 | 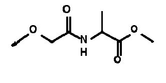 | 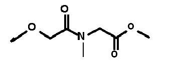 | 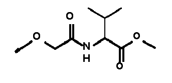 |
| Q-261 | Q-262 | Q-263 | Q-264 | Q-265 |
| | | | | |
| 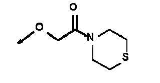 | 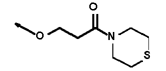 | 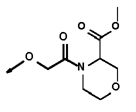 | 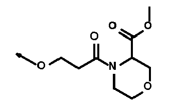 | 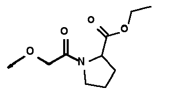 |
| Q-266 | Q-267 | Q-268 | Q-269 | Q-270 |
| | | | | |
| 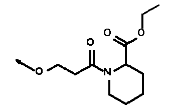 | 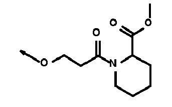 | 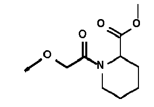 | 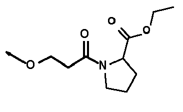 | 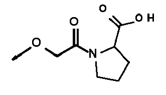 |
| Q-271 | Q-272 | Q-273 | Q-274 | Q-275 |
| | | | | |
| 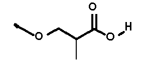 | 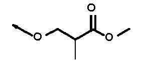 | 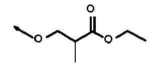 | 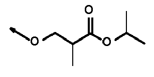 | 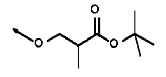 |
| Q-276 | Q-277 | Q-278 | Q-279 | Q-280 |
| | | | | |
| 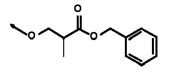 | 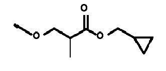 | 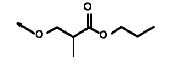 | 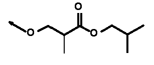 | 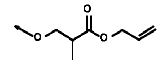 |
| Q-281 | Q-282 | Q-283 | Q-284 | Q-285 |
| | | | | |
| 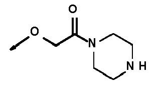 | 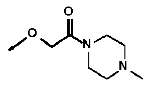 | 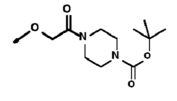 | 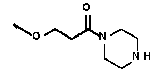 | 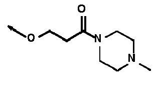 |
| Q-286 | Q-287 | Q-288 | Q-289 | Q-290 |
| | | | | |
| 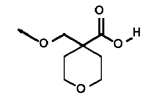 | 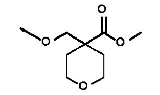 | 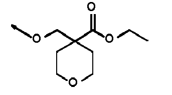 | 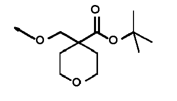 | 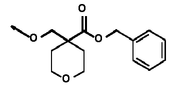 |
| Q-291 | Q-292 | Q-293 | Q-294 | Q-295 |
| 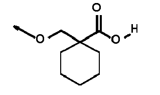 | 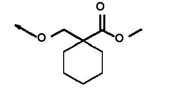 | 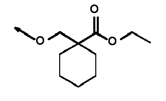 | 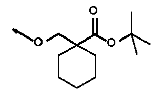 | 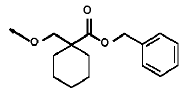 |
| Q-296 | Q-297 | Q-298 | Q-299 | Q-300 |

Im Speziellen bevorzugter Erfindungsgegenstand sind Verbindungen der allgemeinen Formel (I), worin
- R¹: für Methyl, Ethyl, n-Propyl, 1-Methylethyl, n-Butyl, 1-Methylpropyl, 2-Methylpropyl, 1,1-Dimethylethyl, n-Pentyl, Amino, Dimethylamino, Diethylamino steht,
- R²: für Wasserstoff, Methyl, Ethyl, n-Propyl, iso-Propyl steht,
- R³: für Wasserstoff, Fluor, Chlor, Brom, Methoxy, Ethoxy steht,
- R⁴: für Halogen, Cyano, C(O)NH₂, C(S)NH₂, Difluormethyl, Trifluormethyl, Ethinyl, Propin-1-yl steht,
- R⁵ und R⁶: unabhängig voneinander für Wasserstoff, Fluor, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclobutylmethyl, Cyclopentylmethyl, Cyclohexylmethyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Ioddifluormethyl, Bromfluormethyl, 1-Fluorethyl, 2-Fluorethyl, Fluormethyl, Difluormethyl, 2,2-Difluorethyl, 2,2,2-Trifluorethyl, Difluor-tert.-butyl, Methoxy, Ethoxy, n-Propyloxy, iso-Propyloxy, n-Butyloxy, tert-Butyloxy, Methoxymethyl, Ethoxymethyl, n-Propyloxymethyl, iso-Propyloxymethyl, Methoxyethyl, Ethoxyethyl, n-Propyloxyethyl, iso-Propyloxyethyl, Methoxy-n-propyl, Methoxydifluormethyl, Ethoxydifluormethyl, n-propyloxydifluormethyl, n-Butyloxydifluormethyl, Trifluormethoxymethyl, Trifluormethoxyethyl, Trifluormethoxy-n-propyl, Phenyl, 2-Fluor-Phenyl, 3-Fluor-Phenyl, 4-Fluor-Phenyl, 2,4-Difluor-Phenyl, 2,5-Difluor-Phenyl, 2,6-Difluor-Phenyl, 2,3-Difluor-Phenyl, 3,4-Difluor-Phenyl, 3,5-Difluor-Phenyl, 2,4,5-Trifluor-Phenyl, 3,4,5-Trifluor-Phenyl, 2-Chlor-Phenyl, 3-Chlor-Phenyl, 4-Chlor-Phenyl, 2,4-Dichlor-Phenyl, 2,5-Dichlor-Phenyl, 2,6-Dichlor-Phenyl, 2,3-Dichlor-Phenyl, 3,4-Dichlor-Phenyl, 3,5-Dichlor-Phenyl, 2,4,5-Trichlor-Phenyl, 3,4,5-Trichlor-Phenyl, 2,4,6-Trichlor-Phenyl, Pyridin-2-yl, Pyridin-3-yl, Pyridin-4-yl, Thiophen-2-yl, Thiophen-3-yl, Furan-2-yl, Tetrahydrofuran-2-yl, Tetrahydrofuran-3-yl, 1-(4-Methylphenyl)ethyl, 1-(3-Methylphenyl)ethyl, 1-(2-Methylphenyl)ethyl, 1-(4-Chlorphenyl)ethyl, 1-(3-Chlorphenyl)ethyl, 1-(2-Chlorphenyl)ethyl, Benzyl, (4-Fluorphenyl)methyl, (3-Fluorphenyl)methyl, (2-Fluorphenyl)methyl, (2,4-Difluorphenyl)methyl, (3,5-Difluorphenyl)methyl, (2,5-Difluorphenyl)methyl, (2,6-Difluorphenyl)methyl, (2,4,5-Trifluorphenyl)methyl, (2,4,6-Trifluorphenyl)methyl, (4-Chlorphenyl)methyl, (3-Chlorphenyl)methyl, (2-Chlorphenyl)methyl, (2,4-Dichlorphenyl)methyl, (3,5-Dichlorphenyl)methyl, (2,5-Dichlorphenyl)methyl, (2,6-Dichlorphenyl)methyl, (2,4,5-Trichlorphenyl)methyl, (2,4,6-Trichlorphenyl)methyl, Methylthiomethyl, Ethylthiomethyl, Ethylthioethyl, Methylthioethyl, n-Propylthiomethyl, iso-Propylthiomethyl, Trifluormethylthiomethyl, trifluormethylthioethyl, Methylcarbonylmethyl, Ethylcarbonylmethyl, n-Propylcarbonylmethyl, iso-Propylcarbonylmethyl, Methylcarbonylethyl, Hydroxycarbonyl, Methoxycarbonyl, Ethoxycarbonyl, n-Propyloxycarbonyl, iso-Propyloxycarbonyl, n-Butyloxycarbonyl, tert-Butyloxycarbonyl, Allyloxycarbonyl, Benzyloxycarbonyl, Aminocarbonyl, Methylaminocarbonyl, Ethylaminocarbonyl, n-Propylaminocarbonyl, iso-Propylaminocarbonyl, Dimethylaminocarbonyl, Diethylaminocarbonyl, Methyl(ethyl)aminocarbonyl, Cyclopropylaminocarbonyl, Cyclobutylaminocarbonyl, Cyclopentylaminocarbonyl, Cyclohexylaminocarbonyl, Allylaminocarbonyl, benzylaminocarbonyl, tert-Butyloxycarbonylaminocarbonyl, Hydroxycarbonylmethyl, Methoxycarbonylmethyl, Ethoxycarbonylmethyl, n-Propyloxycarbonylmethyl, iso-Propyloxycarbonylmethyl, n-Butyloxycarbonylmethyl, tert-Butyloxycarbonylmethyl, Allyloxycarbonylmethyl, Benzyloxycarbonylmethyl, Aminocarbonylmethyl, Methylaminocarbonylmethyl, Ethylaminocarbonylmethyl, n-Propylaminocarbonylmethyl, iso-Propylaminocarbonylmethyl, Dimethylaminocarbonylmethyl, Diethylaminocarbonylmethyl, Methyl(ethyl)aminocarbonylmethyl, Cyclopropylaminocarbonylmethyl, Cyclobutylaminocarbonylmethyl, Cyclopentylaminocarbonylmethyl, Cyclohexylaminocarbonylmethyl, Allylaminocarbonylmethyl, Benzylaminocarbonylmethyl, 1-(Hydroxycarbonyl)eth-1-yl, 1-(Methoxycarbonyl)eth-1-yl, 1-(Ethoxycarbonyl)eth-1-yl, 1-(tert-Butyloxycarbonyl)eth-1-yl, 1-(Benzyloxycarbonyl)eth-1-yl, 2-(Hydroxycarbonyl)eth-1-yl, 2-(Methoxycarbonyl)eth-1-yl, 2-(Ethoxycarbonyl)eth-1-yl, 2-(tert-Butyloxycarbonyl)eth-1-yl, 2-(Benzyloxycarbonyl)eth-1-yl, 1-(Hydroxycarbonyl)prop-1-yl, 1-(Methoxycarbonyl)prop-1-yl, 1-(Ethoxycarbonyl)prop-1-yl, 1-(tert-Butyloxycarbonyl)prop-1-yl, 1-(Benzyloxycarbonyl)prop-1-yl, 2-(Hydroxycarbonyl)prop-1-yl, 2-(Methoxycarbonyl)prop-1-yl, 2-(Ethoxycarbonyl)prop-1-yl, 2-(tert-Butyloxycarbonyl)prop-1-yl, 2-(Benzyloxycarbonyl)prop-1-yl, 1-(Hydroxycarbonyl)prop-2-yl, 1-(Methoxycarbonyl)prop-2-yl, 1-(Ethoxycarbonyl)prop-2-yl, 1-(tert-Butyloxycarbonyl)prop-2-yl, 1-(Benzyloxycarbonyl)prop-2-yl, 3-(Hydroxycarbonyl)prop-1-yl, 3-(Methoxycarbonyl)prop-1-yl, 3-(Ethoxycarbonyl)prop-1-yl, 3-(tert-Butyloxycarbonyl)prop-1-yl, 3-(Benzyloxycarbonyl)prop-1-yl, Aminomethyl, 2-Aminoeth-1-yl, 1-Aminoethl-yl, 1-Amino-prop-1-yl, 3-Amino-prop-1-yl, Methylaminomethyl, Dimethylaminomethyl, Diethylaminomethyl, Ethylaminomethyl, iso-Propylaminomethyl, Cyclopropylaminomethyl, Cyclobutylaminomethyl, Cyclopentylaminomethyl, Cyclohexylaminomethyl, Methoxycarbonylaminomethyl, Ethoxycarbonylaminomethyl tert-Butyloxycarbonylaminomethyl, Methylcarbonylaminomethyl, Ethylcarbonylaminomethyl, n-propylcarbonylaminomethyl, iso-Propylcarbonylaminomethyl, 2-(Methylamino)eth-1-yl, 2-(Dieth-1-ylamino)eth-1-yl, 2-(Diethylamino)eth-1-yl, 2-(Ethylamino)eth-1-yl, 2-(iso-Propylamino)eth-1-yl, 2-(Cyclopropylamino)eth-1-yl, 2-(Cyclobutylamino)eth-1-yl, 2-(Cyclopentylamino)eth-1-yl, 2-(Cyclohexylamino)eth-1-yl, 2-(Methoxycarbonylamino)eth-1-yl, 2-(Ethoxycarbonylamino)eth-1-yl, 2-(tert-Butyloxycarbonylamino)eth-1-yl, 2-(Methylcarbonylamino)eth-1-yl, 2-(Ethylcarbonylamino)eth-1-yl, 2-(n-Propylcarbonylamino)eth-1-yl, 2-(iso-Propylcarbonylamino)eth-1-yl, 1-(Methylamino)eth-1-yl, 1-(Dieth-1-ylamino)eth-1-yl, 1-(Diethylamino)eth-1-yl, 1-(Ethylamino)eth-1-yl, 1-(iso-Propylamino)eth-1-yl, 1-(Cyclopropylamino)eth-1-yl, 1-(Cyclobutylamino)eth-1-yl, 1-(Cyclopentylamino)eth-1 -yl, 1-(Cyclohexylamino)eth-1-yl, 1-(Methoxycarbonylamino)eth-1-yl, 1-(Ethoxycarbonylamino)eth-1-yl, 1-(tert-Butyloxycarbonylamino)eth-1-yl, 1-(Methylcarbonylamino)eth-1-yl, 1-(Ethylcarbonylamino)eth-1-yl, 1-(n-Propylcarbonylamino)eth-1-yl, 1-(iso-Propylcarbonylamino)eth-1-yl stehen, oder
- R⁵ und R⁶: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁵ und R⁶: mit dem Kohlenstoffatom, an das sie gebunden sind, eine gegebenenfalls durch R¹⁹ und R²⁰ substituierte Doppelbindung, gemäß nachfolgender Formel (I'), bilden 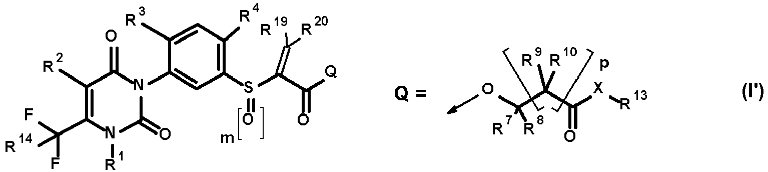
- R¹⁴: für Wasserstoff, Fluor, Chlor, Brom, Trifluormethyl, Methoxy, Ethoxy, n-Propyloxy, n-Butyloxy steht,
- m: für 0, 1, 2 steht,
- R¹⁹ und: R²⁰ unabhängig voneinander für Wasserstoff, Fluor, Chlor, Brom, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, Cyclopropyl, Cyclobutyl, Cyclopentyl, Cyclohexyl, Trifluormethyl, Difluormethyl, Pentafluorethyl, Ethenyl, 1-Propenyl, 1-Methyl-ethenyl, 1-Butenyl, Phenyl stehen, oder
- R¹⁹ und: R²⁰ mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden
und
- Q: für eine der in der vorstehend spezifisch genannten Gruppierungen Q-1 bis Q-300 steht.

Im ganz Speziellen bevorzugter Erfindungsgegenstand sind Verbindungen der allgemeinen Formel (I), worin
- R¹: für Methyl, Ethyl, n-Propyl, 1-Methylethyl, n-Butyl, 1-Methylpropyl, 2-Methylpropyl, 1,1-Dimethylethyl, Amino, Dimethylamino steht,
- R²: für Wasserstoff, Methyl, Ethyl, n-Propyl, iso-Propyl steht,
- R³: für Wasserstoff, Fluor, Chlor, Brom, Methoxy, Ethoxy steht,
- R⁴: für Fluor, Chlor, Brom, Cyano, C(O)NH₂, C(S)NH₂, Difluormethyl, Trifluormethyl, Ethinyl, Propin-1-yl steht,
- R⁵ und R⁶: unabhängig voneinander für Wasserstoff, Fluor, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclobutylmethyl, Cyclopentylmethyl, Cyclohexylmethyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, Trifluormethyl, Pentafluorethyl, 1,1,2,2-Tetrafluorethyl, Heptafluorpropyl, Difluormethyl, 2,2-Difluorethyl, 2,2,2-Trifluorethyl, Methoxy, Ethoxy, n-Propyloxy, iso-Propyloxy, n-Butyloxy, Methoxymethyl, Ethoxymethyl, n-Propyloxymethyl, iso-Propyloxymethyl, Methoxyethyl, Ethoxyethyl, n-Propyloxyethyl, iso-Propyloxyethyl, Methoxy-n-propyl, Trifluormethoxymethyl, Trifluormethoxyethyl, Trifluormethoxy-n-propyl, Phenyl, Pyridin-2-yl, Pyridin-3-yl, Pyridin-4-yl, Thiophen-2-yl, Thiophen-3-yl, Furan-2-yl, Tetrahydrofuran-2-yl, Tetrahydrofuran-3-yl, Benzyl, Methylthiomethyl, Ethylthiomethyl, Ethylthioethyl, Methylthioethyl, n-Propylthiomethyl, iso-Propylthiomethyl, Trifluormethylthiomethyl, Trifluormethylthioethyl stehen, oder
- R⁵ und R⁶: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
- R⁵ und R⁶: mit dem Kohlenstoffatom, an das sie gebunden sind, eine gegebenenfalls durch R¹⁹ und R²⁰ substituierte Doppelbindung, gemäß nachfolgender Formel (I'), bilden 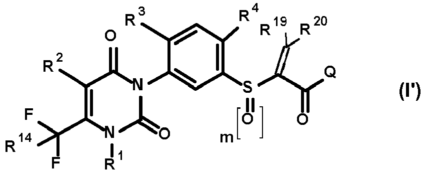
- R¹⁴: für Wasserstoff, Fluor, Chlor, Brom, Trifluormethyl, Methoxy, Ethoxy steht,
- m: für 0, 1, 2 steht,
- R¹⁹ und R²⁰: unabhängig voneinander für Wasserstoff, Fluor, Chlor, Brom, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, Cyclopropyl, Cyclobutyl, Cyclopentyl, Cyclohexyl, Trifluormethyl, Difluormethyl, Pentafluorethyl, Ethenyl, 1-Propenyl, 1-Methyl-ethenyl, 1-Butenyl, Phenyl stehen, oder
- R¹⁹ und R²⁰: mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden
und
Q für eine der in der vorstehend spezifisch genannten Gruppierungen Q-1, Q-2, Q-3, Q-4, Q-5, Q-6, Q-7, Q-8, Q-10, Q-11, Q-12, Q-16, Q-17, Q-18, Q-19, Q-20, Q-21, Q-22, Q-23, Q-31, Q-32, Q-33, Q-41, Q-42, Q-43, Q-44, Q-45, Q-46, Q-47, Q-48, Q-49, Q-50, Q-51, Q-52, Q-53, Q-57, Q-61, Q-71, Q-72, Q-73, Q-74, Q-75, Q-76, Q-102, Q-103, Q-127, Q-132, Q-133, Q-141, Q-142, Q-144, Q-145, Q-146, Q-147, Q-148, Q-151, Q-152, Q-153, Q-154, Q-155, Q-156, Q-160, Q-161, Q-166, Q-167, Q-181, Q-182, Q-183, Q-184, Q-185, Q-186, Q-187, Q-188, Q-189, Q-190, Q-191, Q-192, Q-201, Q-211, Q-231, Q-232, Q-233, Q-241, Q-242, Q-251, Q-254, Q-255, Q-292, Q-293 steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Vor allem aus den Gründen der höheren herbiziden Wirkung, besseren Selektivität und/oder besseren Herstellbarkeit sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Wenn die Verbindungen durch Wasserstoffverschiebung Tautomere bilden können, welche strukturell formal nicht durch die Formel (I) erfasst würden, so sind diese Tautomere gleichwohl von der Definition der erfindungsgemäßen Verbindungen der Formel (I) umfasst, sofern nicht ein bestimmtes Tautomer Gegenstand der Betrachtung ist. So können beispielsweise viele Carbonylverbindungen sowohl in der Ketoform wie auch in der Enolform vorliegen, wobei beide Formen durch die Definition der Verbindung der Formel (I) umfasst werden.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Sofern die Verbindungen als Feststoffe erhalten werden, kann die Reinigung auch durch Umkristallisieren oder Digerieren erfolgen. Sofern einzelne Verbindungen (I) nicht auf den nachstehend beschriebenen Wegen zufriedenstellend zugänglich sind, können sie durch Derivatisierung anderer Verbindungen (I) hergestellt werden.

Als Isolierungs-, Reinigungs- und Stereoisomerenauftrennungsverfahren von Verbindungen der Formel (I) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
Sofern nicht anders definiert, gilt generell für die Bezeichnung von chemischen Gruppen, dass die Anbindung an das Gerüst bzw. den Rest des Moleküls über das zuletzt genannte Strukturelement der betreffenden chemischen Gruppe erfolgt, d.h. beispielsweise im Falle von (C₂-C₈)-Alkenyloxy über das Sauerstoffatom, und im Falle von Heterocyclyl-(C₁-C₈)-alkyl oder R¹⁷O(O)C-(C₁-C₈)-Alkyl jeweils über das C-Atom der Alkylgruppe.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, oder mit 1 bis 6 Kohlenstoffatomen, z.B. (aber nicht beschränkt auf) (C₁-C₆)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl.

Erfindungsgemäß steht "Heteroarylsulfonyl" für gegebenenfalls substituiertes Pyridylsulfonyl, Pyrimidinylsulfonyl, Pyrazinylsulfonyl oder gegebenenfalls substituiertes polycyclisches Heteroarylsulfonyl, hier insbesondere gegebenenfalls substituiertes Chinolinylsulfonyl, beispielsweise substituiert durch Fluor, Chlor, Brom, Iod, Cyano, Nitro, Alkyl-, Haloalkyl-, Haloalkoxy-, Amino-, Alkylamino-, Alkylcarbonylamino-, Dialkylamino- oder Alkoxygruppen.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie (C₁-C₁₀)-, (C₁-C₆)- oder (C₁-C₄)-Alkylthio, z.B. (aber nicht beschränkt auf) (C₁-C₆)-Alkylthio wie Methylthio, Ethylthio, Propylthio, 1 -Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio.

"Alkenylthio" bedeutet erfindungsgemäßt ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

"Alkylsulfinyl (Alkyl-S(=O)-)", soweit nicht an anderer Stelle anders definiert steht erfindungsgemäß für Alkylreste, die über -S(=O)- an das Gerüst gebunden sind, wie (C₁-C₁₀)-, (C₁-C₆)- oder (C₁-C₄)-Alkylsulfinyl, z. B. (aber nicht beschränkt auf) (C₁-C₆)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl.

Analog sind "Alkenylsulfinyl" und "Alkinylsulfinyl", erfindungsgemäß definiert als Alkenyl- bzw. Alkinylreste, die über -S(=O)- an das Gerüst gebunden sind, wie (C₂-C₁₀)-, (C₂-C₆)- oder (C₂-C₄)-Alkenylsulfinyl bzw. (C₃-C₁₀)-, (C₃-C₆)- oder (C₃-C₄)-Alkinylsulfinyl.

Analog sind "Alkenylsulfonyl" und "Alkinylsulfonyl" erfindungsgemäß definiert als Alkenyl- bzw. Alkinylreste, die über -S(=O)₂- an das Gerüst gebunden sind, wie (C₂-C₁₀)-, (C₂-C₆)- oder (C₂-C₄)-Alkenylsulfonyl bzw. (C₃-C₁₀)-, (C₃-C₆)- oder (C₃-C₄)-Alkinylsulfonyl.

"Alkoxy" bedeutet ein über ein Sauerstoffatom gebundenen Alkylrest, z. B. (aber nicht beschränkt auf) (C₁-C₆)-Alkoxy wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Alkenyloxy bedeutet ein über ein Sauerstoffatom gebundenen Alkenylrest, Alkinyloxy bedeutet ein über ein Sauerstoffatom gebundenen Alkinylrest wie (C₂-C₁₀)-, (C₂-C₆)- oder (C₂-C₄)-Alkenoxy bzw. (C₃-C₁₀)-, (C₃-C₆)- oder (C₃-C₄)-Alkinoxy.

"Cycloalkyloxy" bedeutet ein über ein Sauerstoffatom gebundenen Cycloalkylrest und Cycloalkenyloxy bedeutet ein über ein Sauerstoffatom gebundenen Cycloalkenylrest.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₁₀)-, (C₁-C₆)- oder (C₁-C₄)-Alkylcarbonyl. Die Anzahl der C-Atome bezieht sich dabei auf den Alkylrest in der Alkylcarbonylgruppe.

Analog stehen "Alkenylcarbonyl" und "Alkinylcarbonyl", soweit nicht an anderer Stelle anders definiert, erfindungsgemäß für Alkenyl- bzw. Alkinylreste, die über -C(=O)- an das Gerüst gebunden sind, wie (C₂-C₁₀)-, (C₂-C₆)- oder (C₂-C₄)-Alkenylcarbonyl bzw. (C₂-C₁₀)-, (C₂-C₆)- oder (C₂-C₄)-Alkinylcarbonyl. Die Anzahl der C-Atome bezieht sich dabei auf den Alkenyl- bzw. Alkinylrest in der Alkenyl- bzw. Alkinylcarbonylgruppe.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₁₀)-, (C₁-C₆)- oder (C₁-C₄)-Alkoxycarbonyl. Die Anzahl der C-Atome bezieht sich dabei auf den Alkylrest in der Alkoxycarbonylgruppe. Analog stehen "Alkenyloxycarbonyl" und "Alkinyloxycarbonyl", soweit nicht an anderer Stelle anders definiert, erfindungsgemäß für Alkenyl- bzw. Alkinylreste, die über -O-C(=O)- an das Gerüst gebunden sind, wie (C₂-C₁₀)-, (C₂-C₆)- oder (C₂-C₄)-Alkenyloxycarbonyl bzw. (C₃-C₁₀)-, (C₃-C₆)- oder (C₃-C₄)-Alkinyloxycarbonyl. Die Anzahl der C-Atome bezieht sich dabei auf den Alkenyl- bzw. Alkinylrest in der Alken- bzw. Alkinyloxycarbonylgruppe.

Der Begriff "Alkylcarbonyloxy" (Alkyl-C(=O)-O-) steht erfindungsgemäß, soweit nicht an anderer Stelle anders definiert, für Alkylreste, die über eine Carbonyloxygruppe (-C(=O)-O-) mit dem Sauerstoff an das Gerüst gebunden sind, wie (C₁-C₁₀)-, (C₁-C₆)- oder (C₁-C₄)-Alkylcarbonyloxy. Die Anzahl der C-Atome bezieht sich dabei auf den Alkylrest in der Alkylcarbonyloxygruppe.

Analog sind "Alkenylcarbonyloxy" und "Alkinylcarbonyloxy" erfindungsgemäß definiert als Alkenyl- bzw. Alkinylreste, die über (-C(=O)-O-) mit dem Sauerstoff an das Gerüst gebunden sind, wie (C₂-C₁₀)-, (C₂-C₆)- oder (C₂-C₄)-Alkenylcarbonyloxy bzw. (C₂-C₁₀)-, (C₂-C₆)- oder (C₂-C₄)-Alkinylcarbonyloxy. Die Anzahl der C-Atome bezieht sich dabei auf den Alkenyl- bzw. Alkinylrest in der Alkenyl- bzw. Alkinylcarbonyloxygruppe.

In Kurzformen wie z.B. C(O)R¹⁷, C(O)OR¹⁷, OC(O)NR¹⁵R¹⁶, oder C(O)NR¹⁵R¹⁶ steht die in Klammern aufgeführte Kurzform O für ein über eine Doppelbindung an das benachbarte Kohlenstoffatom gebundenes Sauerstoffatom.

In Kurzformen wie z.B. OC(S)OR¹⁷, OC(S)SR¹⁸, OC(S)NR¹⁵R¹⁶, steht die in Klammern aufgeführte Kurzform S für ein über eine Doppelbindung an das benachbarte Kohlenstoffatom gebundenes Schwefelatom.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst. Bevorzugte Aryl-Substituenten sind hier zum Beispiel Wasserstoff, Halogen, Alkyl, Cycloalkyl, Cycloalkylalkyl, Cycloalkenyl, Halocycloalkyl, Alkenyl, Alkinyl, Aryl, Arylalkyl, Arylalkenyl, Heteroaryl, Heteroarylalkyl, Heterocyclyl, Heterocyclylalkyl, Alkoxyalkyl, Alkylthio, Haloalkylthio, Haloalkyl, Alkoxy, Haloalkoxy, Cycloalkoxy, Cycloalkylalkoxy, Aryloxy, Heteroraryloxy, Alkoxyalkoxy, Alkinylalkoxy, Alkenyloxy, Bis-alkylaminoalkoxy, Tris-[alkyl]silyl, Bis-[alkyl]arylsilyl, Bis-[alkyl]alkylsilyl, Tris-[alkyl]silylalkinyl, Arylalkinyl, Heteroarylalkinyl, Alkylalkinyl, Cycloalkylalkinyl, Haloalkylalkinyl, Heterocyclyl-N-alkoxy, Nitro, Cyano, Amino, Alkylamino, Bis-alkylamino, Alkylcarbonylamino, Cycloalkylcarbonylamino, Arylcarbonylamino, Alkoxycarbonylamino, Alkoxycarbonylalkylamino, Arylalkoxycarbonylalkylamino, Hydroxycarbonyl, Alkoxycarbonyl, Aminocarbonyl, Alkylaminocarbonyl, Cycloalkylaminocarbonyl, Bis-Alkylaminocarbonyl, Heteroarylalkoxy, Arylalkoxy

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfasst, wie beispielsweise 8-Aza-bicyclo[3.2.1]octanyl, 8-Aza-bicyclo[2.2.2]octanyl oder 1-Aza-bicyclo[2.2.1]heptyl. Im Falle von gegebenenfalls substituiertem Heterocyclyl werden auch spirocyclische Systeme umfasst, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2- oder 3- oder 4- oder 5- oder 6- oder 7-yl; 2,5-Dihydro-1,2-oxazepin-2- oder 3- oder 4- oder 5- oder 6- oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5- oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5- oder 6- oder 7-yl; 1,3-Oxazepin-2- oder 4- oder 5- oder 6- oder 7-yl; 1,4-Oxazepan-2- oder 3- oder 5- oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-l,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2- oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5- oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5- oder 6- oder 7-yl; 5H-1,4,2-Dioxazepin-3- oder 5- oder 6- oder 7-yl; 7H-1,4,2-Dioxazepin-3- oder 5- oder 6- oder 7-yl. Strukturbeispiele für gegebenenfalls weiter substituierte Heterocyclen sind auch im Folgenden aufgeführt:

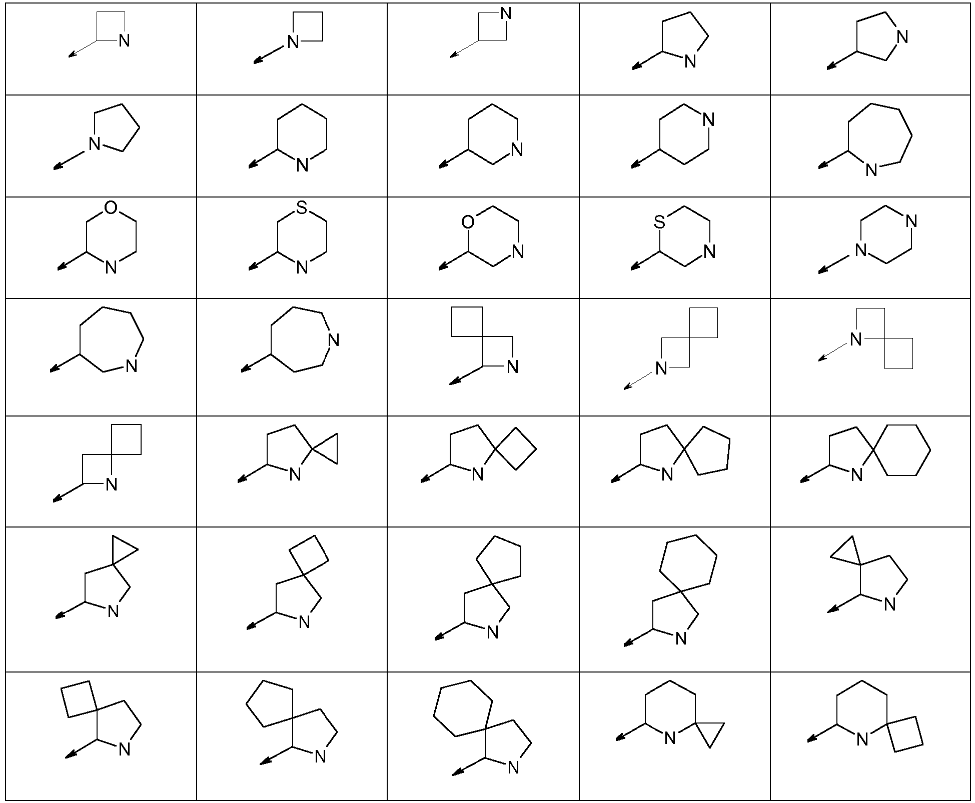

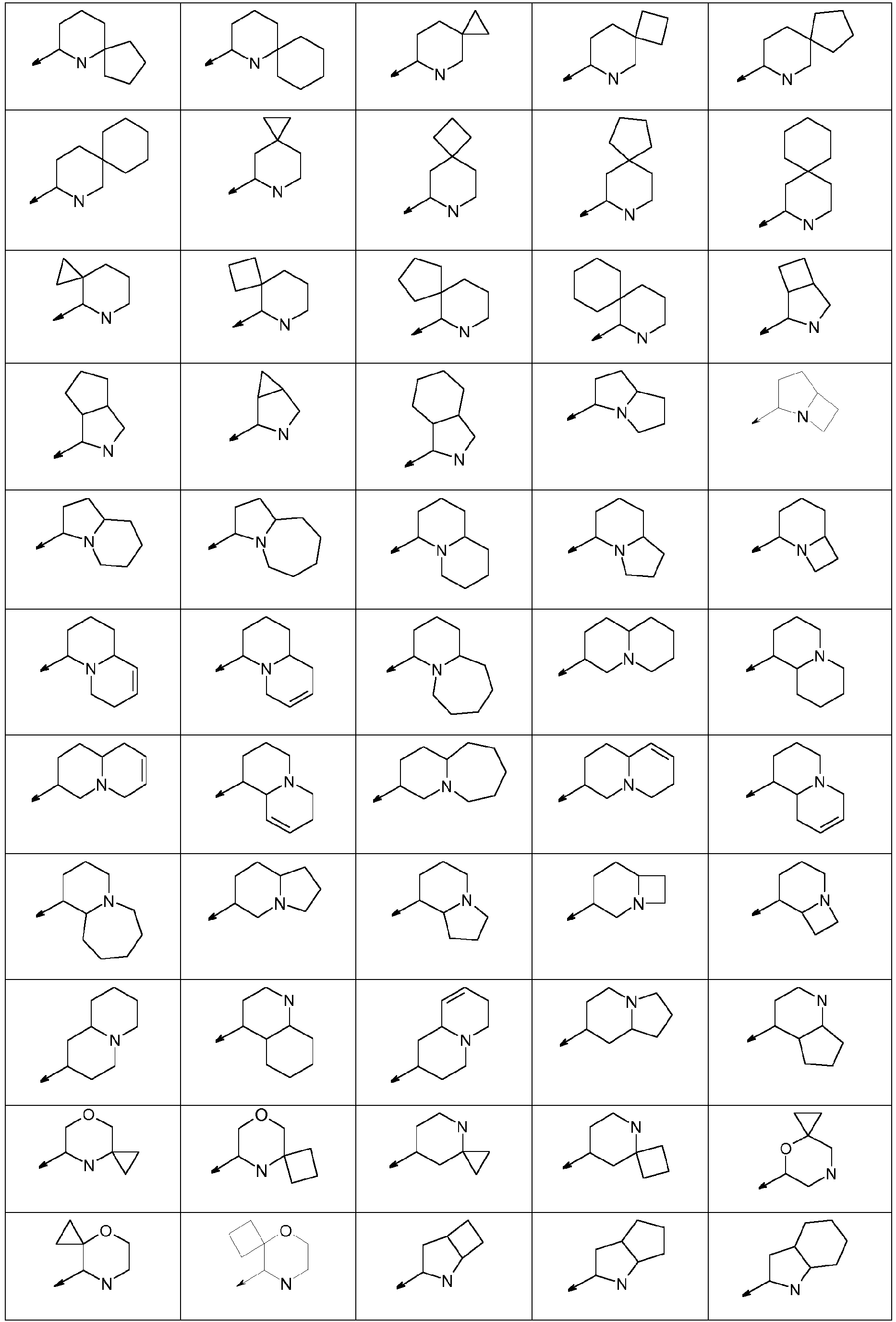

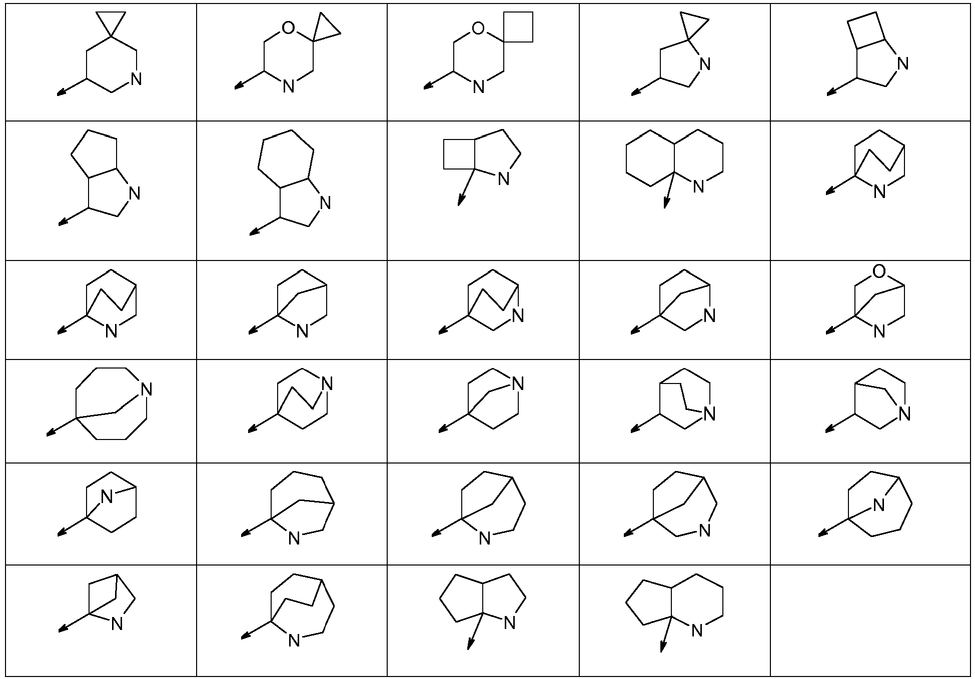

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O) , S(O) (auch kurz SO) und S(O)₂ (auch kurz SO₂) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CC1₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Teilfluoriertes Alkyl" bedeutet einen geradkettigen oder verzweigten, gesättigten Kohlenwasserstoff, der einfach oder mehrfach durch Fluor substituiert ist, wobei sich die entsprechenden Fluoratome als Substituenten an einem oder mehreren verschiedenen Kohlenstoffatomen der geradkettigen oder verzweigten Kohlenwasserstoffkette befinden können, wie z. B. CHFCH₃, CH₂CH₂F, CH₂CH₂CF₃, CHF₂, CH₂F, CHFCF₂CF₃

"Teilfluoriertes Haloalkyl" bedeutet einen geradkettigen oder verzweigten, gesättigten Kohlenwasserstoff, der durch verschiedenene Halogenatomen mit mindestens einem Fluoratom substituiert ist, wobei alle anderen gegebenenfalls vorhandenen Halogenatome ausgewählt sind aus der Gruppe Fluor, Chlor oder Brom, Iod. Die entsprechenden Halogenatome können sich dabei als Substituenten an einem oder mehreren verschiedenen Kohlenstoffatomen der geradkettigen oder verzweigten Kohlenwasserstoffkette befinden. Teilfluoriertes Haloalkyl schließt auch die vollständige Substitution der geradkettigen oder verzweigten Kette durch Halogen unter Beteiligung von mindestens einem Fluoratom ein.

"Haloalkoxy" ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z B. (aber nicht beschränkt auf) (C₂-C₆)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Di-methyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₇)-Cycloalkyl" bedeutet eine Kurzschreibweise für Cycloalkyl mit drei bis 7 Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Cycloalkyliden bedeutet ein carbocyclischer Rest, der über eine Zweifachbindung gebunden ist.

"Cycloalkylalkyloxy" bedeutet ein über ein Sauerstoffatom gebundenen Cycloalkylalkylrest und "Arylalkyloxy" bedeutet ein über ein Sauerstoffatom gebundenen Arylalkylrest.

"Alkoxyalkyl" steht für einen über eine Alkylgruppe gebundenen Alkoxyrest und "Alkoxyalkoxy" bedeutet einen über ein Sauerstoffatom gebundenen Alkoxyalkylrest, z.B. (aber nicht beschränkt auf) Methoxymethoxy, Methoxyethoxy, Ethoxyethoxy, Methoxy-n-propyloxy.

"Alkylthioalkyl" steht für einen über eine Alkylgruppe gebundenen Alkylthiorest und "Alkylthioalkylthio" bedeutet einen über ein Sauerstoffatom gebundenen Alkylthioalkylrest.

"Arylalkoxyalkyl" steht für einen über eine Alkylgruppe gebundenen Aryloxyrest und "Heteroaryloxyalkyl" bedeutet einen über eine Alkylgruppe gebundenen Heteroaryloxyrest.

"Haloalkoxyalkyl" steht für einen gebundenen Haloalkoxyrest und "Haloalkylthioalkyl" bedeutet einen über eine Alkylgruppe gebundenen Haloalkylthiorest.

"Arylalkyl" steht für einen über eine Alkylgruppe gebundenen Arylrest, "Heteroarylalkyl" bedeutet einen über eine Alkylgruppe gebundenen Heteroarylrest, und "Heterocyclylalkyl" bedeutet einen über eine Alkylgruppe gebundenen Heterocyclylrest.

"Cycloalkylalkyl" steht für einen über eine Alkylgruppe gebundenen Cycloalkylrest, z. B. (aber nicht beschränkt auf) Cyclopropylmethyl, Cyclobutylmethyl, Cyclopentylmethyl, Cyclohexylmethyl, 1-Cyclopropyleth-1-yl, 2-Cyclopropyleth-1-yl, 1-Cyclopropylprop-1-yl, 3-Cyclopropylprop-1-yl.

"Arylalkenyl" steht für einen über eine Alkenylgruppe gebundenen Arylrest, "Heteroarylalkenyl" bedeutet einen über eine Alkenylgruppe gebundenen Heteroarylrest, und "Heterocyclylalkenyl" bedeutet einen über eine Alkenylgruppe gebundenen Heterocyclylrest.

"Arylalkinyl" steht für einen über eine Alkinylgruppe gebundenen Arylrest, "Heteroarylalkinyl" bedeutet einen über eine Alkinylgruppe gebundenen Heteroarylrest, und "Heterocyclylalkinyl" bedeutet einen über eine Alkinylgruppe gebundenen Heterocyclylrest.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 8, oder mit 1 bis 6 Kohlenstoffatomen, wie (C₁-C₈)-, (C₁-C₆)- oder (C₁-C₄)-Haloalkylthio, z.B. (aber nicht beschränkt auf) Trifluormethylthio, Pentafluorethylthio, Difluormethyl, 2,2-Difluoreth-1-ylthio, 2,2,2-Difluoreth-1-ylthio, 3,3,3-prop-1-ylthio.

"Halocycloalkyl" und "Halocycloalkenyl" bedeuten durch gleiche oder verschiedene Halogenatome, wie z. B. F, Cl und Br, oder durch Haloalkyl, wie z. B. Trifluormethyl oder Difluormethyl teilweise oder vollständig substituiertes Cycloalkyl oder Cycloalkenyl, z.B. 1-Fluorcycloprop-1-yl, 2-Fluorcycloprop-1-yl, 2,2-Difluorcycloprop-1-yl, 1-Fluorcyclobut-1-yl, 1-Trifluormethylcycloprop-1-yl, 2-Trifluormethylcycloprop-1-yl, 1-Chlor-cycloprop-1-yl, 2-Chlorcycloprop-1-yl, 2,2-Dichlorcycloprop-1-yl, 3,3-Difluorcyclobutyl,

Erfindungsgemäß steht "Trialkylsilyl" - in Alleinstellung oder als Bestandteil einer chemischen Gruppe - für geradkettiges oder verzweigtes Si-Alkyl, vorzugsweise mit 1 bis 8, oder mit 1 bis 6 Kohlenstoffatomen, wie Tri-[(C₁-C₈)-, (C₁-C₆)- oder (C₁-C₄)-alkyl]silyl, z.B. (aber nicht beschränkt auf) Trimethylsilyl, Triethylsilyl, Tri-(n-propyl)silyl, Tri-(iso-propyl)silyl, Tri-(n-butyl)silyl, Tri-(1-methylprop-1-yl)silyl, Tri-(2-methylprop-1-yl)silyl, Tri(1,1-Dimethyleth-1-yl)silyl, Tri(2,2-Dimethyleth-1-yl)silyl.

"Trialkylsilylalkinyl" steht für einen über eine Alkinylgruppe gebundenen Trialkylsilylrest.

Synthese von substituierten Thiophenyluracilen der allgemeinen Formel (I).

Die erfindungsgemäßen substituierten Thiophenyluracile der allgemeinen Formel (I) können ausgehend von bekannten Verfahren hergestellt werden. Die eingesetzten und untersuchten Syntheserouten gehen dabei von kommerziell erhältlichen oder leicht herstellbaren heteroaromatischen Aminen und von entsprechend substituierten Hydroxyestern aus. Die Gruppierungen Q, W, R¹, R², R³, R⁴, R⁵, R⁶ und R¹⁴ der allgemeinen Formel (I) haben in den nachfolgenden Schemata die zuvor definierten Bedeutungen, sofern nicht beispielhafte, aber nicht einschränkende, Definitionen erfolgen. Als erstes Schlüsselintermediat für die Synthese der erfindungsgemäßen Verbindungen der allgemeinen Formel (I) wird ein gegebenenfalls weiter substituiertes Mercaptophenyl-1H-pyrimidin-2,4-dion hergestellt. Beispielhaft, aber nicht einschränkend, wird dies an der Synthese von 3-(4-Chlor-2-fluor-5-mercaptophenyl)-1-methyl-6-trifluormethyl-1H-pyrimidin-2,4-dion (IIa) gezeigt (Schema 1). Dazu wird ein geeignetes substituiertes Anilin, beispielhaft, aber nicht einschränkend, 2-Fluor-4-Chloranilin, mit einem geeigneten Reagenz (z. B. Triphosgen) in einem geeigneten polar-aprotischen Lösemittel (z. B. Dichlormethan) in das entsprechende Isocyanat überführt, das im nächsten Schritt durch Umsetzung mit einem geeigneten Aminoacrylsäureester unter Verwendung einer geeigneten Base (z. B. Natriumhydrid oder Kalium-tert-butylat) in einem geeigneten polar-aprotischen Lösemittel (z. B. N,N-Dimethylformamid) in das entsprechende gegebenenfalls weiter substituierte Pyrimidin-2,4-dion, beispielhaft, aber nicht einschränkend, 3-(4-Chlor-2-fluorphenyl)-1-methyl-6-trifluormethyl-1H-pyrimidin-2,4-dion, überführt (Schema 1). Durch nachfolgende Sulfochlorierung mit einem geeigneten Reagenz (z. B. Chlorsulfonsäure) und anschließende Reduktion mit einem geeigneten Reduktionsmittel (z. B. Zn in EtOH und HCl, Zinn(II)chlorid-Hydrat oder Triphenylphosphin) kann das gewünschte weiter substituierte Mercaptophenyl-1H-pyrimidin-2,4-dion, beispielhaft, aber nicht einschränkend, 3-(4-Chlor-2-fluor-5-mercaptophenyl)-1-methyl-6-trifluormethyl-1H-pyrimidin-2,4-dion (IIa), hergestellt werden (vgl. KR1345394; EP1122244; EP408382; WO 2003/029226; WO2010/038953; US2011/0224083; KR2011/110420). Im nachfolgenden Schema 1 stehen W beispielhaft, aber nicht einschränkend für O, R¹ beispielhaft, aber nicht einschränkend für CH₃, R² beispielhaft, aber nicht einschränkend für Wasserstoff, R³ beispielhaft, aber nicht einschränkend für Fluor, R⁴ beispielhaft, aber nicht einschränkend für Chlor und R¹⁴ beispielhaft, aber nicht einschränkend für Fluor.

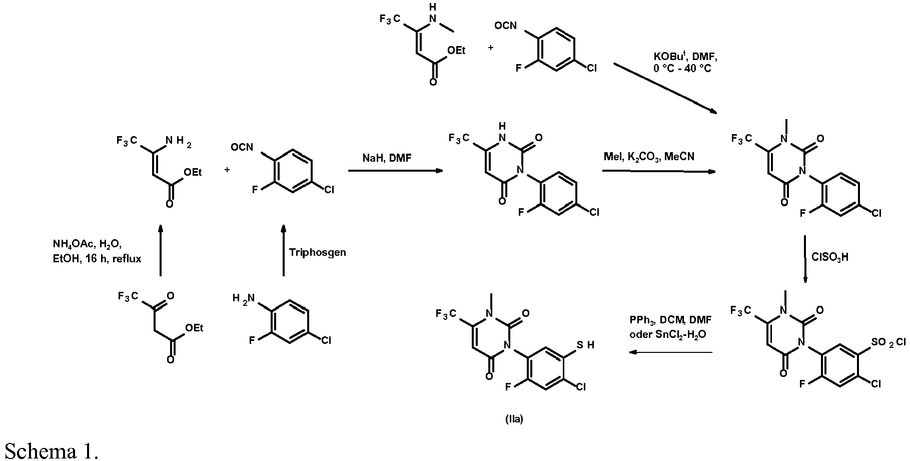

Die in Schema 1 beschriebene Synthese des Schlüsselintermediats (IIa) kann auch auf die Herstellung ähnlicher Intermediate angewendet werden, z. B. 3-(4-Chlor-2-fluor-5-mercaptophenyl)-1,5-dimethyl - 6-trifluormethyl-1H-pyrimidin-2,4-dion (IIb). Im nachfolgenden Schema 2 stehen R¹ beispielhaft, aber nicht einschränkend für CH₃, R² beispielhaft, aber nicht einschränkend für Methyl, R³ beispielhaft, aber nicht einschränkend für Fluor, R⁴ beispielhaft, aber nicht einschränkend für Chlor und R¹⁴ beispielhaft, aber nicht einschränkend für Fluor.

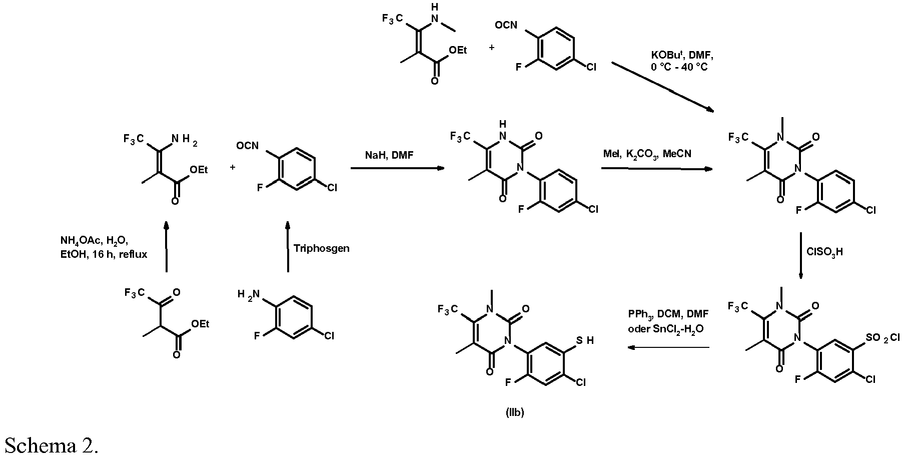

Die in Schema 1 beschriebene Synthese des Schlüsselintermediats (IIa) kann weiterhin auf die Herstellung von Intermediaten angewendet werden, bei denen die Gruppe R¹ für eine Aminogruppe steht, z. B. 3-(4-Chlor-2-fluor-5-mercaptophenyl)-1,5-dimethyl -6-trifluormethyl-1H-pyrimidin-2,4-dion (IIc). Hierbei wird ein geeignetes Phthalimid als Schutzgruppe für die Aminogruppe verwendet. Im nachfolgenden Schema 3 stehen R¹ beispielhaft, aber nicht einschränkend für NH₂, R² beispielhaft, aber nicht einschränkend für Wasserstoff, R³ beispielhaft, aber nicht einschränkend für Fluor, R⁴ beispielhaft, aber nicht einschränkend für Chlor und R¹⁴ beispielhaft, aber nicht einschränkend für Fluor.

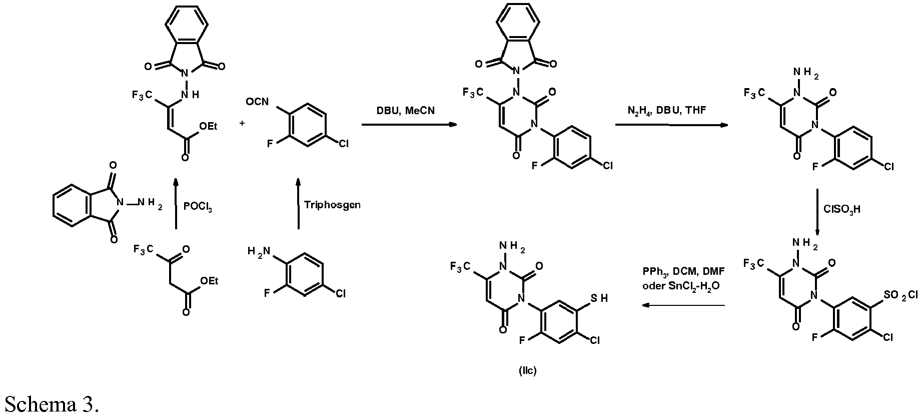

Die betreffenden intermediären weiter substituierten 5-Mercaptophenyl-1H-pyrimidin-2,4-dione (II) können daraufhin auf zwei verschiedenen Wegen in die gewünschten erfindungsmeäßen Verbindungen der allgemeinen Formel (I) überführt werden (Schema 4), (i) durch Umsetzung mit einem geeigneten gegebenenfalls weiter substituierten 2-Halogenacetester (III) unter Verwendung einer geeigneten Base (z. B. Kaliumcarbonat, Caesiumcarbonat oder Natriumcarbonat) in einem geeigneten polar-aprotischen Lösemittel oder (ii) durch Umsetzung der Intermediate (II) mit einer geeigneten gegebenenfalls weiter substituierten 2-Halogenessigsäure in ein entsprechendes Thioessigsäure-intermediat (IV) unter Verwendung einer geeigneten Base (z. B. Kaliumcarbonat, Caesiumcarbonat) und nachfolgende Reaktion des entsprechenden Intermediates (IV) mit einem geeigneten gegebenenfalls weiter substituierten Hydroxyester unter Verwendung von geeigneten Kupplungsreagenzien (z. B. HOBt = 1-Hydroxybenzotriazol, EDC = 1-Ethyl-3-(3-dimethylaminopropyl)carbodiimid, HATU = O-(7-Azabenzotriazol-1-yl)-N,N,N',N'-tetramethyluronium-hexafluorphosphat, T3P = 2,4,6-Tripropyl-1,3,5,2,4,6-trioxatriphosphorinane-2,4,6-trioxid) und geeigneten Basen (z. B. Diisopropylethylamin, Triethylamin) in einem geeigneten polar-aprotischen Lösemittel (z. B. Dichlormethan, Chloroform). Im nachfolgenden Schema 4 stehen R² und R⁶ beispielhaft, aber nicht einschränkend für Wasserstoff, und Q beispielhaft, aber nicht einschränkend für die Gruppierungen Q-2 und Q-42.

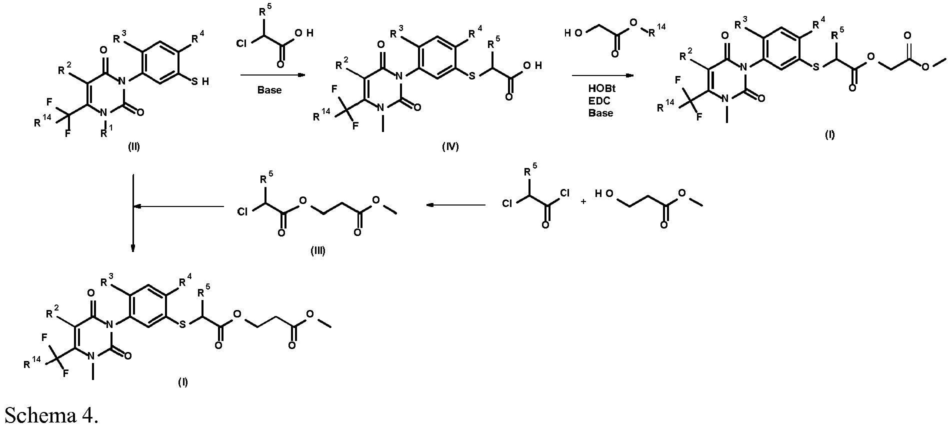

Ausgewählte detaillierte Synthesebeispiele für die erfindungsgemäßen Verbindungen der allgemeinen Formeln (I) sind im Folgenden aufgeführt. Die angegebenen Beispielnummern entsprechen den in den nachstehenden Tabellen I.1 bis 1.50 genannten Numerierungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, i-Pr = verzweigtes Propyl, c-Pr = Cyclopropyl, c-Hex = Cyclohexyl.

### Synthesebeispiele:

No. I.2-3: 3-Ethoxy-3-oxopropyl-2-({2-chlor-4-fluor-5-[3-methyl-2,6-dioxo-4-(trifluormethyl)-3,6-dihydropyrimidin-1(2H)-yl]phenyl} sulfanyl)butanoat 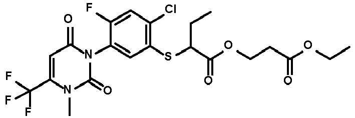 2-Fluor-4-Chloranilin (145 g, 996 mmol) und Triethylamin (202 g, 2000 mmol) wurden nacheinander vorsichtig zu einer Lösung von Triphosgen (119 g, 401 mmol) in abs . Dichlormethan (1000 mL) gegeben, sodaß die Temperatur des resultierenden Reaktionsgemisches unter 20 °C blieb. Das Reaktionsgemisch wurde nach dem Ende der Zugabe über Nacht bei Raumtemperatur gerührt und danach mit Wasser (3 mal 500 mL) und IN Salzsäure (500 mL) gewaschen, über Natriumsulfat getrocknet, abfiltriert und bei vermindertem Druck eingeengt. Das so erhaltene 2-Fluor-4-Chlorphenylisocyanat wurde ohne weitere Reinigung im nächsten Schritt eingesetzt. Natriumhydrid (5.60 g, 140 mmol, 60%ige Dispersion in Mineralöl) wurde in abs. N,N-Dimethylformamid suspendiert und mit Ethyl-(2E)-3-amino-4,4,4-trifluorbut-2-enoat (14.2 g, 77.5 mmol) versetzt. Das Reaktionsgemisch wurde 1 h lang bei Raumtemperatur gerührt, danach auf eine Temperatur von -30 °C eingekühlt und mit 2-Fluor-4-Chlorphenylisocyanat (12.0 g, 70.0 mmol) versetzt. Das resultierende Reaktionsgemisch wurde nach vollständiger Zugabe 4 h lang bei Raumtemperatur nachgerührt und anschließend auf Eiswasser gegeben. Nach der Zugabe von Essigester und Ansäuern mit IN Salzsäure wurde die wäßrige Phase gründlich mit Essigester extrahiert. Die vereinigten organischen Phasen wurden mit Wasser gewaschen, über Natriumsulfat getrocknet, ab filtriert und unter vermindertem Druck eingeengt. Auf diese Weise wurde 3-(4-Chlor-2-fluorphenyl)-6-(trifluormethyl)pyrimidin-2,4(1H,3H)-dion (15.2 g, 50.2 mmol, 65%) erhalten, das ohne weitere Reinigung im nächsten Schritt eingesetzt wurde. Dieser Reaktionsschritt konnte auch im größeren Maßstab erfolgreich wiederholt werden. 3-(4-Chlor-2-fluorphenyl)-6-(trifluormethyl)pyrimidin-2,4(1H,3H)-dion (238 g, 770 mmol) wurde in abs. N,N-Dimethylformamid (800 mL) gelöst und mit Kaliumcarbonat (117 g, 850 mmol) versetzt. Danach wurde eine Lösung von Methyliodid (120 g, 850 mmol) in abs. N,N-Dimethylformamid (100 mL) zugegeben und das resultierende Reaktionsgemisch wurde 1 h lang bei Raumtemperatur nachgerührt. Nach vollständigem Umsatz wurde das Reaktionsgemsich auf eine Temperatur von 0°C eingekühlt, vorsichtig mit Wasser (2000 mL) versetzt und anschließend gründlich mit Dichlormethan extrahiert. Die vereinigten organischen Phasen wurden über Natriumsulfat getrocknet, abflitriert und unter vermindertem Druck eingeengt. Auf diese Weise wurde 3-(4-Chlor-2-fluorphenyl)-1-methyl-6-(trifluormethyl)pyrimidin-2,4(1H,3H)-dion (241 g, 747 mmol, 97% der Theorie) erhalten, das im nächsten Schritt ohne weitere Reinigung umgesetzt wurde. 3-(4-Chlor-2-fluorphenyl)-1-methyl-6-(trifluormethyl)pyrimidin-2,4(1H,3H)-dion (100 g, 310 mmol) wurde daraufhin schrittweise zu Chlorsulfonsäure in einem ausgeheizten Rundkolben gegeben. Das resultierende Reaktionsgemisch wurde danach 20 h lang bei einer Temperatur von 110 °C gerührt und nach dem Abkühlen auf Raumtemperatur auf Eiswasser gegeben und mehrfach mit Essigester extrahiert (3 mal 300 mL). Die vereinigten organischen Phasen wurden über Natriumsulfat getrocknet, abfiltriert und unter vermindertem Druck eingeengt. Dadurch wurde 2-Chlor-4-fluor-5-[3-methyl-2,6-dioxo-4-(trifluormethyl)-3,6-dihydropyrimidin-l(2H)-yl]benzensulfonylchlorid (75.0 g, 178 mmol, 57% der Theorie) erhalten, das ohne weitere Reinigung im nächsten Schritt eingesetzt wurde. 2-Chlor-4-fluor-5-[3-methyl-2,6-dioxo-4-(trifluormethyl)-3,6-dihydropyrimidin-1(2H)-yl]sulfonylchlorid (100.0 g, 237 mmol) wurde in einem Rundkolben vorgelegt und nacheinander mit Salzsäure (500 mL), Essigsäure (500 mL) und Zinndichlorid-Dihydrat (270 g, 1197 mmol) versetzt. Das resultierende reaktionsgemisch wurde 10 h lang bei einer Temperatur von 100 °C gerührt, nach dem Abkühlen auf Raumtemperatur auf Eiswasser gegeben und gründlich mit Dichlormethan (3 mal 400 mL) extrahiert. Die vereinigten organischen Phasen wurden über Natriumsulfat getrocknet, abfiltriert und unter vermindertem Druck eingeengt. Durch abschließende säulenchromatographische Reinigung wurde 3-(4-Chlor-2-fluor-5-sulfanylphenyl)-1-methyl-6-(trifluormethyl)pyrimidin-2,4(1H,3H)-dion (73.0 g, 206 mmol, 83% der Theorie) in Form eines farblosen Feststoffs erhalten. 2-Chlorbutancarbonsäure (691 mg, 5.64 mmol) wurde unter Argon in einem ausgeheizten Rundkolben in abs. Acetonitril gelöst und danach mit Caesiumcarbonat (3.67 g, 11.28 mmol) sowie 3-(4-Chlor-2-fluor-5-sulfanylphenyl)-1-methyl-6-(trifluormethyl)pyrimidin-2,4(1H,3H)-dion (2.0 g, 5.64 mmol) versetzt. Das resultierende Reaktionsgemisch wurde 1 h lang bei einer Temperatur von 50 °C gerührt und nach dem Abkühlen auf Raumtemperatur mit Wasser und Dichlormethan versetzt und gründlich extrahiert. Die wäßrige Phase wurde daraufhin mit 10%iger Salzsäure sauer gestellt und erneut mehrfach mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 2-({2-Chlor-4-fluor-5-[3-methyl-2,6-dioxo-4-(trifluormethyl)-3,6-dihydropyrimidin-1(2H)-yl]phenyl}sulfanyl)butansäure (2.0 g, 80 % der Theorie) wurde in Form eines farblosen Feststoffs erhalten. ¹H-NMR (CDCl₃ δ, ppm) 7.53 (d, 1H), 7.38 (d, 1H), 6.36 (d, 1H), 3.68 (m, 1H), 3.55 (s, 3H), 2.05-1.95 (m, 1H), 1.93-1.82 (m, 1H), 1.09 (t, 3H). 2-({2-Chlor-4-fluor-5-[3-methyl-2,6-dioxo-4-(trifluormethyl)-3,6-dihydropyrimidin-1(2H)-yl]phenyl}sulfanyl)butansäure (100 mg, 0.23 mmol), 1-Hydroxy-1H-benzotriazol (40 mg, 0.29 mmol) und 1-(3-Dimethylaminopropyl)-3-ethylcarbodiimidhydrochlorid (57 mg, 0.29 mmol) wurden in einem ausgeheizten Rundkolben unter Argon in abs. Dichlormethan gelöst und nach 5 Minuten Rühren bei Raumtemperatur mit Ethyl-3-hydroxypropanoat (35 mg, 0.29 mmol) sowie Triethylamin (0.08 ml, 0.57 mmol) versetzt. Das resultierende Reaktionsgemisch wurde 6 h lang bei Raumtemperatur gerührt, danach mit Wasser und Dichlormethan versetzt und gründlich mit Dichlormethan extrahiert.. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 3-Ethoxy-3-oxopropyl-2-({2-chlor-4-fluor-5-[3-methyl-2,6-dioxo-4-(trifluormethyl)-3,6-dihydropyrimidin-1(2H)-yl]phenyl}sulfanyl)butanoat (44 mg g, 36 % der Theorie) wurde in Form eines hochviskosen farblosen Öls erhalten. ¹H-NMR (CDCl₃ δ, ppm) 7.53 (m, 1H), 7.36 (m, 1H), 6.35 (d, 1H), 4.36-4.27 (m, 2H), 4.17-4.10 (m, 2H), 3.71-3.64 (m, 1H), 3.55 (s, 3H),2.59-2.52 (m, 2H), 2.02-1.91 (m, 1H), 1.89-1.80 (m, 1H), 1.24 (t, 3H), 1.05 (t, 3H).

No. I.2-43: 2-Ethoxy-2-oxoethyl-2-({2-chlor-4-fluor-5-[3-methyl-2,6-dioxo-4-(trifluormethyl)-3,6-dihydropyrimidin-1(2H)-yl]phenyl} sulfanyl)butanoat 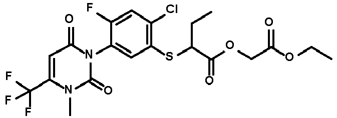 2-({2-Chlor-4-fluor-5-[3-methyl-2,6-dioxo-4-(trifluormethyl)-3,6-dihydropyrimidin-1(2H)-yl]phenyl}sulfanyl)butansäure (100 mg, 0.23 mmol), 1-Hydroxy-1H-benzotriazol (40 mg, 0.29 mmol) und 1-(3-Dimethylaminopropyl)-3-ethylcarbodiimidhydrochlorid (57 mg, 0.29 mmol) wurden in einem ausgeheizten Rundkolben unter Argon in abs. Dichlormethan gelöst und nach 5 Minuten Rühren bei Raumtemperatur mit Ethylhydroxyacetat (31 mg, 0.29 mmol) sowie Triethylamin (0.08 ml, 0.57 mmol) versetzt. Das resultierende Reaktionsgemisch wurde 6 h lang bei Raumtemperatur gerührt, danach mit Wasser und Dichlormethan versetzt und gründlich mit Dichlormethan extrahiert.. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 2-Ethoxy-2-oxoethyl-2-({2-chlor-4-fluor-5-[3-methyl-2,6-dioxo-4-(trifluormethyl)-3,6-dihydropyrimidin-1(2H)-yl]phenyl}sulfanyl)butanoat (60 mg" 50 % der Theorie) wurde in Form eines hochviskosen farblosen Öls erhalten. ¹H-NMR (CDCl₃ δ, ppm) 7.58 (m, 1H), 7.36 (m, 1H), 6.34 (m, 1H), 4.63-4.53 (m, 2H), 4.19-4.12 (m, 2H), 3.77-3.72 (m, 1H), 3.55 (s, 3H),2.09-1.99 (m, 1H), 1.93-1.84 (m, 1H), 1.25 (t, 3H), 1.12 (t, 3H).

No. 1.2-145: (5-Oxotetrahydrofuran-2-yl)methyl-2-({2-chlor-4-fluor-5-[3-methyl-2,6-dioxo-4-(trifluormethyl)-3,6-dihydropyrimidin-1(2H)-yl]phenyl} sulfanyl)butanoat

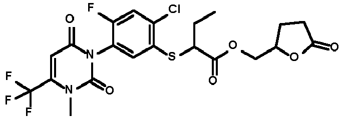

2-({2-Chlor-4-fluor-5-[3-methyl-2,6-dioxo-4-(trifluormethyl)-3,6-dihydropyrimidin-1(2H)-yl]phenyl}sulfanyl)butansäure (90 mg, 0.20 mmol), 1-Hydroxy-1H-benzotriazol (36 mg, 0.27 mmol) und 1-(3-Dimethylaminopropyl)-3-ethylcarbodiimidhydrochlorid (51 mg, 0.27 mmol) wurden in einem ausgeheizten Rundkolben unter Argon in abs. Dichlormethan gelöst und nach 5 Minuten Rühren bei Raumtemperatur mit 5-(Hydroxymethyl)dihydrofuran-2(3H)-on (31 mg, 0.27 mmol) sowie Triethylamin (0.08 ml, 0.57 mmol) versetzt. Das resultierende Reaktionsgemisch wurde 2 h lang bei Raumtemperatur gerührt, danach mit Wasser und Dichlormethan versetzt und gründlich mit Dichlormethan extrahiert.. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und (5-Oxotetrahydrofuran-2-yl)methyl-2-({2-chlor-4-fluor-5-[3-methyl-2,6-dioxo-4-(trifluormethyl)-3,6-dihydropyrimidin-1(2H)-yl]phenyl}sulfanyl)butanoat (73 mg" 66 % der Theorie) wurde in Form eines hochviskosen farblosen Öls erhalten. ¹H-NMR (CDCl₃ δ, ppm) 7.54 (m, 1H), 7.38 (m, 1H), 6.33 (m, 1H), 4.64-4.57 (m, 1H), 4.29-4.21 (m, 1H), 4.10-4.04 (m, 1H), 3.82-3.76 (m, 1H), 3.56 (s, 3H), 2.58-2.48 (m, 2H), 2.33-2.20 (m, 1H), 2.06-1.97 (m, 1H), 1.93-1.78 (m, 2H), 1.11-1.06 (m, 3H).

No. 1.4-3: 3-Ethoxy-3-oxopropyl-2-({2-chlor-4-fluor-5-[3-methyl-2,6-dioxo-4-(trifluormethyl)-3,6-dihydropyrimidin-1(2H)-yl]phenyl} sulfanyl)-4-methylpentanoat

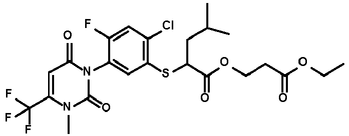

2-Chlor-4-methylpentancarbonsäure (849 mg, 5.64 mmol) wurde unter Argon in einem ausgeheizten Rundkolben in abs. Acetonitril gelöst und danach mit Caesiumcarbonat (3.67 g, 11.28 mmol) sowie 3-(4-Chlor-2-fluor-5-sulfanylphenyl)-1-methyl-6-(trifluormethyl)pyrimidin-2,4(1H,3H)-dion (2.0 g, 5.64 mmol) versetzt. Das resultierende Reaktionsgemisch wurde 1 h lang bei einer Temperatur von 50 °C gerührt und nach dem Abkühlen auf Raumtemperatur mit Wasser und Dichlormethan versetzt und gründlich extrahiert. Die wäßrige Phase wurde daraufhin mit 10%iger Salzsäure sauer gestellt und erneut mehrfach mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 2-({2-Chlor-4-fluor-5-[3-methyl-2,6-dioxo-4-(trifluormethyl)-3,6-dihydropyrimidin-1(2H)-yl]phenyl}sulfanyl)-4-methylpentansäure (1.62 g, 61 % der Theorie) wurde in Form eines farblosen Feststoffs erhalten. ¹H-NMR (CDCl₃ δ, ppm) 7.53 (d, 1H), 7.37 (d, 1H), 6.37 (m, 1H), 3.82-3.78 (m, 1H), 3.55 (s, 3H), 1.90-1.78 (m, 2H), 1.73-1.65 (m, 1H), 0.99-0.92 (m, 6H). 2-({2-Chlor-4-fluor-5-[3-methyl-2,6-dioxo-4-(trifluormethyl)-3,6-dihydropyrimidin-1(2H)-yl]phenyl}sulfanyl)-4-methylpentansäure (100 mg, 0.21 mmol), 1-Hydroxy-1H-benzotriazol (38 mg, 0.28 mmol) und 1-(3-Dimethylaminopropyl)-3-ethylcarbodiimidhydrochlorid (53 mg, 0.28 mmol) wurden in einem ausgeheizten Rundkolben unter Argon in abs. Dichlormethan gelöst und nach 5 Minuten Rühren bei Raumtemperatur mit Ethyl-3-hydroxypropanoat (33 mg, 0.28 mmol) sowie Triethylamin (0.07 ml, 0.51 mmol) versetzt. Das resultierende Reaktionsgemisch wurde 6 h lang bei Raumtemperatur gerührt, danach mit Wasser und Dichlormethan versetzt und gründlich mit Dichlormethan extrahiert.. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 3-Ethoxy-3-oxopropyl-2-({2-chlor-4-fluor-5-[3-methyl-2,6-dioxo-4-(trifluormethyl)-3,6-dihydropyrimidin-l(2H)-yl]phenyl}sulfanyl)-4-methylpentanoat (43 mg, 35 % der Theorie) wurde in Form eines hochviskosen farblosen Öls erhalten. ¹H-NMR (CDCl₃ δ, ppm) 7.47 (m, 1H), 7.35 (m, 1H), 6.35 (d, 1H), 4.37-4.30 (m, 1H), 4.28-4.22 (m, 1H), 4.14-4.09 (q, 2H), 3.84-3.80 (m, 1H), 3.55 (s, 3H), 2.58-2.51 (m, 2H), 1.89-1.83 (m, 1H), 1.80-1.72 (m, 1H), 1.69-1.62 (m, 1H), 1.25 (t, 3H), 0.94 (d, 3H), 0.92 (d, 3H).

No. 1.6-3: 3-Ethoxy-3-oxopropyl-2-({2-chlor-4-fluor-5-[3-methyl-2,6-dioxo-4-(trifluormethyl)-3,6-dihydropyrimidin-1(2H)-yl]phenyl} sulfanyl)-3-methylbutanoat

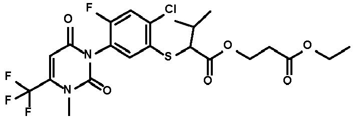

2-({2-Chlor-4-fluor-5-[3-methyl-2,6-dioxo-4-(trifluormethyl)-3,6-dihydropyrimidin-1(2H)-yl]phenyl}sulfanyl)-3-methylbutansäure (100 mg, 0.22 mmol), 1-Hydroxy-1H-benzotriazol (39 mg, 0.29 mmol) und 1-(3-Dimethylaminopropyl)-3-ethylcarbodiimidhydrochlorid (55 mg, 0.29 mmol) wurden in einem ausgeheizten Rundkolben unter Argon in abs. Dichlormethan gelöst und nach 5 Minuten Rühren bei Raumtemperatur mit Ethyl-3-hydroxypropanoat (34 mg, 0.29 mmol) sowie Triethylamin (0.07 ml, 0.53 mmol) versetzt. Das resultierende Reaktionsgemisch wurde 6 h lang bei Raumtemperatur gerührt, danach mit Wasser und Dichlormethan versetzt und gründlich mit Dichlormethan extrahiert.. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 3-Ethoxy-3-oxopropyl-2-({2-chlor-4-fluor-5-[3-methyl-2,6-dioxo-4-(trifluormethyl)-3,6-dihydropyrimidin-l(2H)-yl]phenyl}sulfanyl)-3-methylbutanoat (45 mg, 37 % der Theorie) wurde in Form eines hochviskosen farblosen Öls erhalten. ¹H-NMR (CDCl₃ δ, ppm) 7.45 (m, 1H), 7.34 (m, 1H), 6.35 (m, 1H), 4.37-4.23 (m, 2H), 4.15-4.09 (m, 2H), 3.55 (s, 3H), 3.52 (m, 1H), 2.58-2.54 (m, 2H), 2.24-2.16 (m, 1H), 1.19 (t, 3H), 1.07 (t, 3H).

No. 1.6-43: 2-Ethoxy-2-oxoethyl-2-({2-chlor-4-fluor-5-[3-methyl-2,6-dioxo-4-(trifluormethyl)-3,6-dihydropyrimidin-1(2H)-yl]phenyl} sulfanyl)-3-methylbutanoat

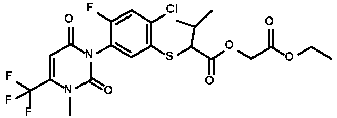

2-Chlor-3-methylbutancarbonsäure (770 mg, 5.64 mmol) wurde unter Argon in einem ausgeheizten Rundkolben in abs. Acetonitril gelöst und danach mit Caesiumcarbonat (3.67 g, 11.28 mmol) sowie 3-(4-Chlor-2-fluor-5-sulfanylphenyl)-1-methyl-6-(trifluormethyl)pyrimidin-2,4(1H,3H)-dion (2.0 g, 5.64 mmol) versetzt. Das resultierende Reaktionsgemisch wurde 1 h lang bei einer Temperatur von 50 °C gerührt und nach dem Abkühlen auf Raumtemperatur mit Wasser und Dichlormethan versetzt und gründlich extrahiert. Die wäßrige Phase wurde daraufhin mit 10%iger Salzsäure sauer gestellt und erneut mehrfach mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 2-({2-Chlor-4-fluor-5-[3-methyl-2,6-dioxo-4-(trifluormethyl)-3,6-dihydropyrimidin-1(2H)-yl]phenyl}sulfanyl)-3-methylbutansäure (0.47 g, 21 % der Theorie) wurde in Form eines farblosen Feststoffs erhalten. ¹H-NMR (CDCl₃ δ, ppm) 7.49 (d, 1H), 7.37 (d, 1H), 6.33 (m, 1H), 3.58-3.48 (m, 1H), 3.53 (s, 3H), 2.25-2.17 (m, 1H), 1.18 (d, 3H), 1.11 (d, 3H). 2-({2-Chlor-4-fluor-5-[3-methyl-2,6-dioxo-4-(trifluormethyl)-3,6-dihydropyrimidin-l(2H)-yl]phenyl}sulfanyl)-3-methylbutansäure (100 mg, 0.22 mmol), 1-Hydroxy-1H-benzotriazol (39 mg, 0.29 mmol) und 1-(3-Dimethylaminopropyl)-3-ethylcarbodiimidhydrochlorid (55 mg, 0.29 mmol) wurden in einem ausgeheizten Rundkolben unter Argon in abs. Dichlormethan gelöst und nach 5 Minuten Rühren bei Raumtemperatur mit Ethyl-3-hydroxyacetat (30 mg, 0.29 mmol) sowie Triethylamin (0.07 ml, 0.53 mmol) versetzt. Das resultierende Reaktionsgemisch wurde 6 h lang bei Raumtemperatur gerührt, danach mit Wasser und Dichlormethan versetzt und gründlich mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 2-Ethoxy-2-oxoethyl-2-({2-chlor-4-fluor-5-[3-methyl-2,6-dioxo-4-(trifluormethyl)-3,6-dihydropyrimidin-1(2H)-yl]phenyl}sulfanyl)-3-methylbutanoat (54 mg, 45 % der Theorie) wurde in Form eines hochviskosen farblosen Öls erhalten. ¹H-NMR (CDCl₃ δ, ppm) 7.56 (m, 1H), 7.36 (m, 1H), 6.34 (m, 1H), 4.60-4.50 (m, 2H), 4.17-4.12 (m, 1H), 3.64-3.58 (m, 1H), 3.54 (s, 3H), 2.32-2.24 (m, 1H), 1.26 (t, 3H), 1.20 (d, 3H), 1.16 (d, 3H).

No. 1.44-3: Ethyl-3-[2-({2-chlor-4-fluor-5-[3-methyl-2,6-dioxo-4-(trifluormethyl)-3,6-dihydropyrimidin-1(2H)-yl]phenyl} sulfanyl)(cyclopropyl)acetoxy]propanoat

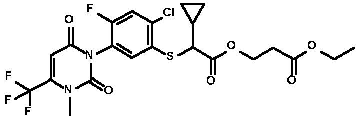

Chlor(cyclopropyl)essigsäureethylester (409 mg, 1.97 mmol) wurde unter Argon in einem ausgeheizten Rundkolben in abs. Acetonitril gelöst und danach mit Caesiumcarbonat (273 mg, 1.97 mmol) sowie 3-(4-Chlor-2-fluor-5-sulfanylphenyl)-1-methyl-6-(trifluormethyl)pyrimidin-2,4(1H,3H)-dion (700 mg, 1.97 mmol) versetzt. Das resultierende Reaktionsgemisch wurde 2 h lang bei einer Temperatur von 50 °C gerührt und nach dem Abkühlen auf Raumtemperatur mit Wasser und Dichlormethan versetzt und gründlich extrahiert. Die wäßrige Phase wurde daraufhin mit 10%iger Salzsäure sauer gestellt und erneut mehrfach mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und Ethyl-({2-chlor-4-fluor-5-[3-methyl-2,6-dioxo-4-(trifluormethyl)-3,6-dihydropyrimidin-1(2H)-yl]phenyl}sulfanyl)(cyclopropyl)acetat (940 mg, 99 % der Theorie) wurde in Form eines farblosen Feststoffs erhalten. ¹H-NMR (CDCl₃ δ, ppm) 7.51 (d, 1H), 7.35 (d, 1H), 6.34 (s, 1H), 4.18-4.05 (m, 2H), 3.53 (s, 3H), 3.13-3.10 (m, 1H), 1.33-1.26 (m, 1H), 1.15 (t, 3H), 0.75-0.66 (m, 2H), 0.48-0.44 (m, 1H), 0.42-0.36 (m, 1H). Ethyl-({2-chlor-4-fluor-5-[3-methyl-2,6-dioxo-4-(trifluormethyl)-3,6-dihydropyrimidin-1(2H)-yl]phenyl}sulfanyl)(cyclopropyl)acetat (160 mg, 0.33 mmol) wurde anschließend in Esigsäure gelöst und mit 6N HCl versetzt. Das erhaltene Reaktionsgemisch wurde 3 h lang bei einer Temperatur von 100 °C gerührt, nach dem Abkühlen auf Raumtemperatur mit Wasser versetzt und gründlich mit abs. Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und ({2-Chlor-4-fluor-5-[3-methyl-2,6-dioxo-4-(trifluormethyl)-3,6-dihydropyrimidin-1(2H)-yl]phenyl}sulfanyl)(cyclopropyl)essigsäure (120 mg, 79 % der Theorie) wurde in Form eines farblosen Feststoffs erhalten. ¹H-NMR (CDCl₃ δ, ppm) 7.54 (dd, 1H), 7.36 (d, 1H), 6.34 (m, 1H), 3.55 (s, 3H), 3.11-3.08 (m, 1H), 1.32-1.24 (m, 1H), 0.77-0.69 (m, 2H), 0.52-0.38 (m, 2H). ({2-Chlor-4-fluor-5-[3-methyl-2,6-dioxo-4-(trifluormethyl)-3,6-dihydropyrimidin-1(2H)-yl]phenyl}sulfanyl)(cyclopropyl)-essigsäure (120 mg, 0.27 mmol), 1-Hydroxy-1H-benzotriazol (47 mg, 0.35 mmol) und 1-(3-Dimethylaminopropyl)-3-ethylcarbodiimidhydrochlorid (66 mg, 0.35 mmol) wurden in einem ausgeheizten Rundkolben unter Argon in abs. Dichlormethan gelöst und nach 5 Minuten Rühren bei Raumtemperatur mit Ethyl-3-hydroxypropionat (41 mg, 0.35 mmol) sowie Triethylamin (0.09 ml, 0.64 mmol) versetzt. Das resultierende Reaktionsgemisch wurde 2 h lang bei Raumtemperatur gerührt, danach mit Wasser und Dichlormethan versetzt und gründlich mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und Ethyl-3-[2-({2-chlor-4-fluor-5-[3-methyl-2,6-dioxo-4-(trifluormethyl)-3,6-dihydropyrimidin-l(2H)-yl]phenyl}sulfanyl)(cyclopropyl)acetoxy]-propanoat (81 mg, 54 % der Theorie) wurde in Form eines hochviskosen farblosen Öls erhalten. ¹H-NMR (d₆-DMSO δ, ppm) 7.83 (d, 1H), 7.68 (m, 1H), 6.62 (m, 1H), 4.27-4.18 (m, 1H), 4.17-4.10 (m, 1H), 4.07-3.98 (m, 2H), 3.52-3.48 (m, 1H), 3.41 (s, 3H), 2.71-2.58 (m, 2H) 1.23-1.11 (m, 4H), 0.72-0.56 (m, 2H), 0.49-0.34 (m, 2H).

No. 1.44-6: Benzyl-3-[2-({2-chlor-4-fluor-5-[3-methyl-2,6-dioxo-4-(trifluormethyl)-3,6-dihydropyrimidin-1(2H)-yl]phenyl} sulfanyl)(cyclopropyl)acetoxy]propanoat

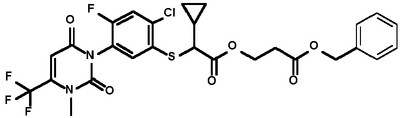

({2-Chlor-4-fluor-5-[3-methyl-2,6-dioxo-4-(trifluormethyl)-3,6-dihydropyrimidin-1(2H)-yl]phenyl}sulfanyl)(cyclopropyl)-essigsäure (120 mg, 0.27 mmol), 1-Hydroxy-1H-benzotriazol (47 mg, 0.35 mmol) und 1-(3-Dimethylaminopropyl)-3-ethylcarbodiimidhydrochlorid (66 mg, 0.35 mmol) wurden in einem ausgeheizten Rundkolben unter Argon in abs. Dichlormethan gelöst und nach 5 Minuten Rühren bei Raumtemperatur mit Benzyl-3-hydroxypropionat (62 mg, 0.35 mmol) sowie Triethylamin (0.09 ml, 0.64 mmol) versetzt. Das resultierende Reaktionsgemisch wurde 2 h lang bei Raumtemperatur gerührt, danach mit Wasser und Dichlormethan versetzt und gründlich mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und Benzyl-3-[2-({2-chlor-4-fluor-5-[3-methyl-2,6-dioxo-4-(trifluormethyl)-3,6-dihydropyrimidin-1(2H)-yl]phenyl}sulfanyl)(cyclopropyl)acetoxy]propanoat (76 mg, 45 % der Theorie) wurde in Form eines hochviskosen farblosen Öls erhalten. ¹H-NMR (d₆-DMSO δ, ppm) 7.83 (d, 1H), 7.67 (m, 1H), 6.60 (m, 1H), 5.09 (s, 2H), 4.30-4.23 (m, 1H), 4.21-4.15 (m, 1H), 3.41 (s, 3H), 3.39-3.31 (m, 1H), 2.72-2.57 (m, 2H), 1.18-1.09 (m, 1H), 0.68-0.53 (m, 2H), 0.40-0.34 (m, 2H).

No. 1.48-2: 3-Methoxy-3-oxopropyl-2-({2-chlor-5-[5-ethyl-3-methyl-2,6-dioxo-4-(trifluormethyl)-3,6-dihydropyrimidin-1(2H)-yl]-4-fluorphenyl}sulfanyl)propanoat

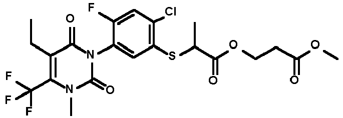

Eine Lösung von n-Butyllithium in Hexan wurde analog zur Synthese von Beispiel No. I.12-72 zu einer auf-10°C eingekühlten Lösung von Diisopropylamin in abs. Tetrahydrofuran gegeben. Das resultierende Reaktionsgemisch wurde 40 Minuten lang bei einer Temperatur von -10 °C gerührt und danach weiter auf -78 °C eingekühlt. Anschließend erfolgte die vorsichtige Zugabe von Ethylbutanoat. Die Reaktionsmischung wurde 1 h lang bei -78 °C gerührt, mit einer geeigneten Menge 2,2,2-Trifluoroethyl trifluoroacetat versetzt und abschließend über Nacht bei Raumtemperatur gerührt. Nach vollständiger Umsetzung wurde mit verd. Salzsäure (1M) angesäuert und mehrfach gründlich mit Essigester extrahiert. Die vereinigten organischen Phasen wurden über Natriumsulfat getrocknet, abfiltriert und unter vermindertem Druck eingeengt, und das auf diese Weise erhaltene Ethyl 4,4,4-trifluoro-2-ethyl-3-oxobutanoat (67% der Theorie) wurde ohne weitere Reinigung in einem Rundkolben in Toluol gelöst und mit Ammoniumacetat und Essigsäure versetzt. Das resultierende Reaktionsgemisch wurde unter Verwendung eines Wasserabscheiders mehrere Stunden unter Rückflußbedingungen gerührt bis kein Wasser mehr abgeschieden wurde. Das Reaktionsgemisch wurde nach dem Abkühlen auf Raumtemperatur unter vermindertem Druck eingeengt und der Rückstand danach mit Essigester und Wasser aufgenommen. Die Wasserphase wurde daraufhin gründlich mit Essigester extrahiert und die vereinigten organischen Phasen wurden über Natriumsulfat getrocknet, abfiltriert und unter vermindertem Druck eingeengt. Durch destillative Auftrennung des erhaltenen Rückstandes wurde Ethyl-(2Z)-3-amino-4,4,4-trifluor-2-ethylbut-2-enoat (58% der Theorie) erhalten. 2-Fluor-4-Chloranilin und Triethylamin wurden nacheinander vorsichtig zu einer Lösung von Triphosgen in abs. Dichlormethan gegeben, sodaß die Temperatur des resultierenden Reaktionsgemisches unter 20 °C blieb. Das Reaktionsgemisch wurde nach dem Ende der Zugabe über Nacht bei Raumtemperatur gerührt und danach mit Wasser und IN Salzsäure gewaschen, über Natriumsulfat getrocknet, abfiltriert und bei vermindertem Druck eingeengt. Das so erhaltene 2-Fluor-4-Chlorphenylisocyanat wurde ohne weitere Reinigung im nächsten Schritt eingesetzt. Natriumhydrid (60%ige Dispersion in Mineralöl) wurde in abs. N,N-Dimethylformamid suspendiert und mit Ethyl-(2Z)-3-amino-4,4,4-trifluor-2-ethylbut-2-enoat versetzt. Das Reaktionsgemisch wurde 1 h lang bei Raumtemperatur gerührt, danach auf eine Temperatur von -30 °C eingekühlt und mit 2-Fluor-4-Chlorphenylisocyanat versetzt. Das resultierende Reaktionsgemisch wurde nach vollständiger Zugabe 4 h lang bei Raumtemperatur nachgerührt und anschließend auf Eiswasser gegeben. Nach der Zugabe von Essigester und Ansäuern mit IN Salzsäure wurde die wäßrige Phase gründlich mit Essigester extrahiert. Die vereinigten organischen Phasen wurden mit Wasser gewaschen, über Natriumsulfat getrocknet, abfiltriert und unter vermindertem Druck eingeengt. Auf diese Weise wurde 3-(4-Chlor-2-fluorphenyl)-5-ethyl-6-(trifluormethyl)pyrimidin-2,4(1H,3H)-dion (66%) erhalten, das ohne weitere Reinigung im nächsten Schritt eingesetzt wurde. 3-(4-Chlor-2-fluorphenyl)-5-methyl-6-(trifluormethyl)pyrimidin-2,4(1H,3H)-dion (1 equiv.) wurde in abs. N,N-Dimethylformamid gelöst und mit Kaliumcarbonat (1.2 equiv.) versetzt. Danach wurde eine Lösung von Methyliodid (1.2 equiv.) in abs. N,N-Dimethylformamid zugegeben und das resultierende Reaktionsgemisch wurde 1 h lang bei Raumtemperatur nachgerührt. Nach vollständigem Umsatz wurde das Reaktionsgemsich auf eine Temperatur von 0°C eingekühlt, vorsichtig mit Wasser versetzt und anschließend gründlich mit Dichlormethan extrahiert. Die vereinigten organischen Phasen wurden über Natriumsulfat getrocknet, abflitriert und unter vermindertem Druck eingeengt. Auf diese Weise wurde 3-(4-Chlor-2-fluorphenyl)-5-ethyl-1-methyl-6-(trifluormethyl)pyrimidin-2,4(1H,3H)-dion (97% der Theorie) erhalten, das im nächsten Schritt ohne weitere Reinigung umgesetzt wurde. 3-(4-Chlor-2-fluorphenyl)-5-ethyl-1-methyl-6-(trifluormethyl)pyrimidin-2,4(1H,3H)-dion wurde daraufhin schrittweise zu Chlorsulfonsäure in einem ausgeheizten Rundkolben gegeben. Das resultierende Reaktionsgemisch wurde danach 20 h lang bei einer Temperatur von 110 °C gerührt und nach dem Abkühlen auf Raumtemperatur auf Eiswasser gegeben und mehrfach mit Essigester extrahiert. Die vereinigten organischen Phasen wurden über Natriumsulfat getrocknet, abfiltriert und unter vermindertem Druck eingeengt. Dadurch wurde 2-Chlor-4-fluor-5-[5-ethyl-3-methyl-2,6-dioxo-4-(trifluormethyl)-3,6-dihydropyrimidin-1(2H)-yl]benzensulfonylchlorid (54% der Theorie) erhalten, das ohne weitere Reinigung im nächsten Schritt eingesetzt wurde. 2-Chlor-4-fluor-5-[5-ethyl-3-methyl-2,6-dioxo-4-(trifluormethyl)-3,6-dihydropyrimidin-1(2H)-yl]sulfonylchlorid (1 equiv.) wurde in einem Rundkolben vorgelegt und nacheinander mit Salzsäure (2mL/mmol), Essigsäure (2.5 mL/mmol) und Zinndichlorid-Dihydrat (3 equiv.) versetzt. Das resultierende Reaktionsgemisch wurde 10 h lang bei einer Temperatur von 100 °C gerührt, nach dem Abkühlen auf Raumtemperatur auf Eiswasser gegeben und gründlich mit Dichlormethan extrahiert. Die vereinigten organischen Phasen wurden über Natriumsulfat getrocknet, abfiltriert und unter vermindertem Druck eingeengt. Durch abschließende säulenchromatographische Reinigung wurde 3-(4-Chlor-2-fluor-5-sulfanylphenyl)-5-ethyl-1-methyl-6-(trifluormethyl)pyrimidin-2,4(1H,3H)-dion (84% der Theorie) in Form eines farblosen Feststoffs erhalten. 2-Chlorpropancarbonsäure (567 mg, 5.23 mmol) wurde unter Argon in einem ausgeheizten Rundkolben in abs. Acetonitril gelöst und danach mit Caesiumcarbonat (3.41g, 10.45 mmol) sowie 3-(4-Chlor-2-fluor-5-sulfanylphenyl)-5-ethyl-1-methyl-6-(trifluormethyl)pyrimidin-2,4(1H,3H)-dion (2.0 g, 5.23 mmol) versetzt. Das resultierende Reaktionsgemisch wurde 1 h lang bei einer Temperatur von 50 °C gerührt und nach dem Abkühlen auf Raumtemperatur mit Wasser und Dichlormethan versetzt und gründlich extrahiert. Die wäßrige Phase wurde daraufhin mit 10%iger Salzsäure sauer gestellt und erneut mehrfach mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 2-({2-Chlor-5-[5-ethyl-3-methyl-2,6-dioxo-4-(trifluormethyl)-3,6-dihydropyrimidin-1(2H)-yl]-4-fluorphenyl}sulfanyl)propansäure (1.30 g, 54 % der Theorie) wurde in Form eines farblosen Feststoffs erhalten. ¹H-NMR (CDCl₃ δ, ppm) 7.54 (d, 1H), 7.39 (d, 1H), 3.90-3.83 (m, 1H), 3.54 (s, 3H), 2.72-2.69 (m, 2H), 1.55 (d, 3H), 1.13 (t, 3H). 2-({2-Chlor-5-[5-ethyl-3-methyl-2,6-dioxo-4-(trifluormethyl)-3,6-dihydropyrimidin-1(2H)-yl]-4-fluorphenyl}sulfanyl)propansäure (110 mg, 0.24 mmol), 1-Hydroxy-1H-benzotriazol (42 mg, 0.31 mmol) und 1-(3-Dimethylaminopropyl)-3-ethylcarbodiimidhydrochlorid (60 mg, 0.31 mmol) wurden in einem ausgeheizten Rundkolben unter Argon in abs. Dichlormethan gelöst und nach 5 Minuten Rühren bei Raumtemperatur mit Methyl-3-hydroxypropanoat (33 mg, 0.31 mmol) sowie Triethylamin (0.08 ml, 0.61 mmol) versetzt. Das resultierende Reaktionsgemisch wurde 6 h lang bei Raumtemperatur gerührt, danach mit Wasser und Dichlormethan versetzt und gründlich mit Dichlormethan extrahiert. Die vereinigten organischen Phasen wurden über Natriumsulfat getrocknet, gefiltert und unter vermindertem Druck eingeengt. Das Rohprodukt wurde anschließend säulenchromatographisch gereinigt (Gradient Essigsäureethylester/Heptan), und 3-Methoxy-3-oxopropyl-2-({2-chlor-5-[5-ethyl-3-methyl-2,6-dioxo-4-(trifluormethyl)-3,6-dihydropyrimidin-1(2H)-yl]-4-fluorphenyl}sulfanyl)propanoat (61 mg, 45 % der Theorie) wurde in Form eines hochviskosen farblosen Öls erhalten. ¹H-NMR (CDCl₃ δ, ppm) 7.49 (d, 1H), 7.37 (d, 1H), 4.37-4.25 (m, 2H), 3.92-3.87 (m, 1H), 3.67 (s, 3H), 3.54 (s, 3H), 2.74-2.68 (m, 2H), 2.60-2.53 (m, 2H), 1.51 (t, 3H), 1.12 (t, 3H).

In Analogie zu den oben angeführten und an entsprechender Stelle rezitierten Herstellungsbeispielen und unter Berücksichtigung der allgemeinen Angaben zur Herstellung von substituierten Thiophenyluracilen erhält man die nachfolgend genannten Verbindungen. Wenn in Tabelle 1 ein Strukturelement durch eine Strukurformel definiert ist, welches eine gestrichelte Linie enthält, so bedeutet diese gestrichelte Linie, dass an dieser Position die betreffende Gruppe mit dem Rest des Moleküls verbunden ist. Wenn in Tabelle 1 ein Strukturelement durch eine Strukturformel definiert ist, welches einen Pfeil enthält, so steht der Pfeil für eine Bindung der jeweiligen Gruppe Q zur Carbonylgruppe in der allgemeinen Formel (I).

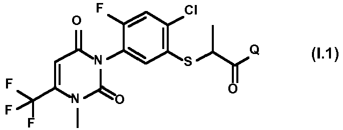

Tabelle I.1: Bevorzugte Verbindungen der Formel (I.1) sind die Verbindungen I.1-1 bis I.1-300, worin Q die in der jeweiligen Zeile angegebenen Bedeutungen der Tabelle 1 hat. Die Verbindungen I.1-1 bis I.1-300 der Tabelle I.1 sind somit durch die Bedeutung der jeweiligen Einträge No. 1 bis 300 für Q der Tabelle 1 definiert.

**Tabelle 1:**

| No. | Q |
|---|---|
| 1 | Q-1 |
| 2 | Q-2 |
| 3 | Q-3 |
| 4 | Q-4 |
| 5 | Q-5 |
| 6 | Q-6 |
| 7 | Q-7 |
| 8 | Q-8 |
| 9 | Q-9 |
| 10 | Q-10 |
| 11 | Q-11 |
| 12 | Q-12 |
| 13 | Q-13 |
| 14 | Q-14 |
| 15 | Q-15 |
| 16 | Q-16 |
| 17 | Q-17 |
| 18 | Q-18 |
| 19 | Q-19 |
| 20 | Q-20 |
| 21 | Q-21 |
| 22 | Q-22 |
| 23 | Q-23 |
| 24 | Q-24 |
| 25 | Q-25 |
| 26 | Q-26 |
| 27 | Q-27 |
| 28 | Q-28 |
| 29 | Q-29 |
| 30 | Q-30 |
| 31 | Q-31 |
| 32 | Q-32 |
| 33 | Q-33 |
| 34 | Q-34 |
| 35 | Q-35 |
| 36 | Q-36 |
| 37 | Q-37 |
| 38 | Q-38 |
| 39 | Q-39 |
| 40 | Q-40 |
| 41 | Q-41 |
| 42 | Q-42 |
| 43 | Q-43 |
| 44 | Q-44 |
| 45 | Q-45 |
| 46 | Q-46 |
| 47 | Q-47 |
| 48 | Q-48 |
| 49 | Q-49 |
| 50 | Q-50 |
| 51 | Q-51 |
| 52 | Q-52 |
| 53 | Q-53 |
| 54 | Q-54 |
| 55 | Q-55 |
| 56 | Q-56 |
| 57 | Q-57 |
| 58 | Q-58 |
| 59 | Q-59 |
| 60 | Q-60 |
| 61 | Q-61 |
| 62 | Q-62 |
| 63 | Q-63 |
| 64 | Q-64 |
| 65 | Q-65 |
| 66 | Q-66 |
| 67 | Q-67 |
| 68 | Q-68 |
| 69 | Q-69 |
| 70 | Q-70 |
| 71 | Q-71 |
| 72 | Q-72 |
| 73 | Q-73 |
| 74 | Q-74 |
| 75 | Q-75 |
| 76 | Q-76 |
| 77 | Q-77 |
| 78 | Q-78 |
| 79 | Q-79 |
| 80 | Q-80 |
| 81 | Q-81 |
| 82 | Q-82 |
| 83 | Q-83 |
| 84 | Q-84 |
| 85 | Q-85 |
| 86 | Q-86 |
| 87 | Q-87 |
| 88 | Q-88 |
| 89 | Q-89 |
| 90 | Q-90 |
| 91 | Q-91 |
| 92 | Q-92 |
| 93 | Q-93 |
| 94 | Q-94 |
| 95 | Q-95 |
| 96 | Q-96 |
| 97 | Q-97 |
| 98 | Q-98 |
| 99 | Q-99 |
| 100 | Q-100 |
| 101 | Q-101 |
| 102 | Q-102 |
| 103 | Q-103 |
| 104 | Q-104 |
| 105 | Q-105 |
| 106 | Q-106 |
| 107 | Q-107 |
| 108 | Q-108 |
| 109 | Q-109 |
| 110 | Q-110 |
| 111 | Q-111 |
| 112 | Q-112 |
| 113 | Q-113 |
| 114 | Q-114 |
| 115 | Q-115 |
| 116 | Q-116 |
| 117 | Q-117 |
| 118 | Q-118 |
| 119 | Q-119 |
| 120 | Q-120 |
| 121 | Q-121 |
| 122 | Q-122 |
| 123 | Q-123 |
| 124 | Q-124 |
| 125 | Q-125 |
| 126 | Q-126 |
| 127 | Q-127 |
| 128 | Q-128 |
| 129 | Q-129 |
| 130 | Q-130 |
| 131 | Q-131 |
| 132 | Q-132 |
| 133 | Q-133 |
| 134 | Q-134 |
| 135 | Q-135 |
| 136 | Q-136 |
| 137 | Q-137 |
| 138 | Q-138 |
| 139 | Q-139 |
| 140 | Q-140 |
| 141 | Q-141 |
| 142 | Q-142 |
| 143 | Q-143 |
| 144 | Q-144 |
| 145 | Q-145 |
| 146 | Q-146 |
| 147 | Q-147 |
| 148 | Q-148 |
| 149 | Q-149 |
| 150 | Q-150 |
| 151 | Q-151 |
| 152 | Q-152 |
| 153 | Q-153 |
| 154 | Q-154 |
| 155 | Q-155 |
| 156 | Q-156 |
| 157 | Q-157 |
| 158 | Q-158 |
| 159 | Q-159 |
| 160 | Q-160 |
| 161 | Q-161 |
| 162 | Q-162 |
| 163 | Q-163 |
| 164 | Q-164 |
| 165 | Q-165 |
| 166 | Q-166 |
| 167 | Q-167 |
| 168 | Q-168 |
| 169 | Q-169 |
| 170 | Q-170 |
| 171 | Q-171 |
| 172 | Q-172 |
| 173 | Q-173 |
| 174 | Q-174 |
| 175 | Q-175 |
| 176 | Q-176 |
| 177 | Q-177 |
| 178 | Q-178 |
| 179 | Q-179 |
| 180 | Q-180 |
| 181 | Q-181 |
| 182 | Q-182 |
| 183 | Q-183 |
| 184 | Q-184 |
| 185 | Q-185 |
| 186 | Q-186 |
| 187 | Q-187 |
| 188 | Q-188 |
| 189 | Q-189 |
| 190 | Q-190 |
| 191 | Q-191 |
| 192 | Q-192 |
| 193 | Q-193 |
| 194 | Q-194 |
| 195 | Q-195 |
| 196 | Q-196 |
| 197 | Q-197 |
| 198 | Q-198 |
| 199 | Q-199 |
| 200 | Q-200 |
| 201 | Q-201 |
| 202 | Q-202 |
| 203 | Q-203 |
| 204 | Q-204 |
| 205 | Q-205 |
| 206 | Q-206 |
| 207 | Q-207 |
| 208 | Q-208 |
| 209 | Q-209 |
| 210 | Q-210 |
| 211 | Q-211 |
| 212 | Q-212 |
| 213 | Q-213 |
| 214 | Q-214 |
| 215 | Q-215 |
| 216 | Q-216 |
| 217 | Q-217 |
| 218 | Q-218 |
| 219 | Q-219 |
| 220 | Q-220 |
| 221 | Q-221 |
| 222 | Q-222 |
| 223 | Q-223 |
| 224 | Q-224 |
| 225 | Q-225 |
| 226 | Q-226 |
| 227 | Q-227 |
| 228 | Q-228 |
| 229 | Q-229 |
| 230 | Q-230 |
| 231 | Q-231 |
| 232 | Q-232 |
| 233 | Q-233 |
| 234 | Q-234 |
| 235 | Q-235 |
| 236 | Q-236 |
| 237 | Q-237 |
| 238 | Q-238 |
| 239 | Q-239 |
| 240 | Q-240 |
| 241 | Q-241 |
| 242 | Q-242 |
| 243 | Q-243 |
| 244 | Q-244 |
| 245 | Q-245 |
| 246 | Q-246 |
| 247 | Q-247 |
| 248 | Q-248 |
| 249 | Q-249 |
| 250 | Q-250 |
| 251 | Q-251 |
| 252 | Q-252 |
| 253 | Q-253 |
| 254 | Q-254 |
| 255 | Q-255 |
| 256 | Q-256 |
| 257 | Q-257 |
| 258 | Q-258 |
| 259 | Q-259 |
| 260 | Q-260 |
| 261 | Q-261 |
| 262 | Q-262 |
| 263 | Q-263 |
| 264 | Q-264 |
| 265 | Q-265 |
| 266 | Q-266 |
| 267 | Q-267 |
| 268 | Q-268 |
| 269 | Q-269 |
| 270 | Q-270 |
| 271 | Q-271 |
| 272 | Q-272 |
| 273 | Q-273 |
| 274 | Q-274 |
| 275 | Q-275 |
| 276 | Q-276 |
| 277 | Q-277 |
| 278 | Q-278 |
| 279 | Q-279 |
| 280 | Q-280 |
| 281 | Q-281 |
| 282 | Q-282 |
| 283 | Q-283 |
| 284 | Q-284 |
| 285 | Q-285 |
| 286 | Q-286 |
| 287 | Q-287 |
| 288 | Q-288 |
| 289 | Q-289 |
| 290 | Q-290 |
| 291 | Q-291 |
| 292 | Q-292 |
| 293 | Q-293 |
| 294 | Q-294 |
| 295 | Q-295 |
| 296 | Q-296 |
| 297 | Q-297 |
| 298 | Q-298 |
| 299 | Q-299 |
| 300 | Q-300 |

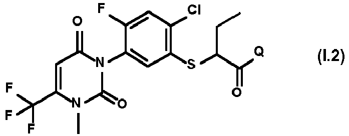

Tabelle I.2: Bevorzugte Verbindungen der Formel (1.2) sind die Verbindungen 1.2-1 bis I.2-300, worin Q die in der jeweiligen Zeile angegebenen Bedeutungen der Tabelle 1 hat. Die Verbindungen I.2-1 bis I.2-300 der Tabelle I.2 sind somit durch die Bedeutung der jeweiligen Einträge No. 1 bis 300 für Q der Tabelle 1 definiert.

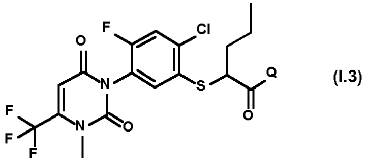

Tabelle I.3: Bevorzugte Verbindungen der Formel (I.3) sind die Verbindungen I.3-1 bis I.3-300, worin Q die in der jeweiligen Zeile angegebenen Bedeutungen der Tabelle 1 hat. Die Verbindungen I.3-1 bis I.3-300 der Tabelle I.3 sind somit durch die Bedeutung der jeweiligen Einträge No. 1 bis 300 für Q der Tabelle 1 definiert.

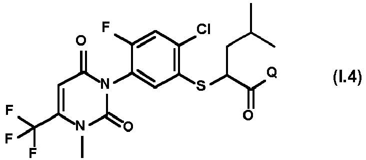

Tabelle I.4: Bevorzugte Verbindungen der Formel (I.4) sind die Verbindungen I.4-1 bis I.4-300, worin Q die in der jeweiligen Zeile angegebenen Bedeutungen der Tabelle 1 hat. Die Verbindungen I.4-1 bis I.4-300 der Tabelle I.4 sind somit durch die Bedeutung der jeweiligen Einträge No. 1 bis 300 für Q der Tabelle 1 definiert.

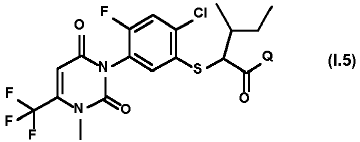

Tabelle I.5: Bevorzugte Verbindungen der Formel (I.5) sind die Verbindungen I.5-1 bis I.5-300, worin Q die in der jeweiligen Zeile angegebenen Bedeutungen der Tabelle 1 hat. Die Verbindungen I.5-1 bis I.5-300 der Tabelle I.5 sind somit durch die Bedeutung der jeweiligen Einträge No. 1 bis 300 für Q der Tabelle 1 definiert.

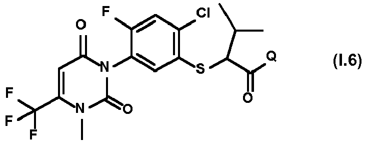

Tabelle I.6: Bevorzugte Verbindungen der Formel (I.6) sind die Verbindungen I.6-1 bis I.6-300, worin Q die in der jeweiligen Zeile angegebenen Bedeutungen der Tabelle 1 hat. Die Verbindungen I.6-1 bis I.6-300 der Tabelle I.6 sind somit durch die Bedeutung der jeweiligen Einträge No. 1 bis 300 für Q der Tabelle 1 definiert.

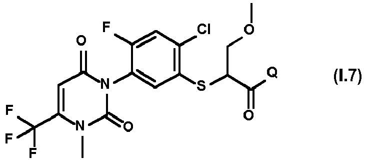

Tabelle I.7: Bevorzugte Verbindungen der Formel (I.7) sind die Verbindungen I.7-1 bis I.7-300, worin Q die in der jeweiligen Zeile angegebenen Bedeutungen der Tabelle 1 hat. Die Verbindungen I.7-1 bis I.7-300 der Tabelle I.7 sind somit durch die Bedeutung der jeweiligen Einträge No. 1 bis 300 für Q der Tabelle 1 definiert.

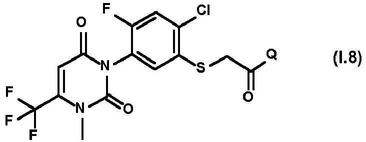

Tabelle I.8: Bevorzugte Verbindungen der Formel (I.8) sind die Verbindungen I.8-1 bis I.8-300, worin Q die in der jeweiligen Zeile angegebenen Bedeutungen der Tabelle 1 hat. Die Verbindungen I.8-1 bis I.8-300 der Tabelle I.8 sind somit durch die Bedeutung der jeweiligen Einträge No. 1 bis 300 für Q der Tabelle 1 definiert.

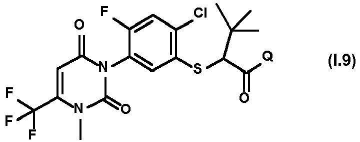

Tabelle I.9: Bevorzugte Verbindungen der Formel (I.9) sind die Verbindungen I.9-1 bis I.9-300, worin Q die in der jeweiligen Zeile angegebenen Bedeutungen der Tabelle 1 hat. Die Verbindungen I.9-1 bis I.9-300 der Tabelle I.9 sind somit durch die Bedeutung der jeweiligen Einträge No. 1 bis 300 für Q der Tabelle 1 definiert.

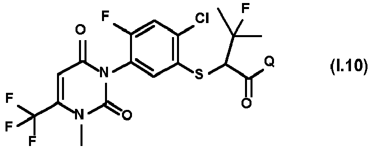

Tabelle I.10: Bevorzugte Verbindungen der Formel (I.10) sind die Verbindungen I.10-1 bis I.10-300, worin Q die in der jeweiligen Zeile angegebenen Bedeutungen der Tabelle 1 hat. Die Verbindungen I.10-1 bis I.10-300 der Tabelle I.10 sind somit durch die Bedeutung der jeweiligen Einträge No. 1 bis 300 für Q der Tabelle 1 definiert.

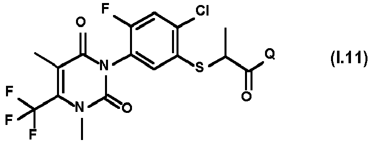

Tabelle I.11: Bevorzugte Verbindungen der Formel (I.11) sind die Verbindungen I.11-1 bis I.11-300, worin Q die in der jeweiligen Zeile angegebenen Bedeutungen der Tabelle 1 hat. Die Verbindungen I.11-1 bis I.11-300 der Tabelle I.11 sind somit durch die Bedeutung der jeweiligen Einträge No. 1 bis 300 für Q der Tabelle 1 definiert.

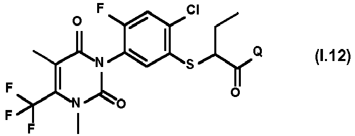

Tabelle I.12: Bevorzugte Verbindungen der Formel (I.12) sind die Verbindungen I.12-1 bis I.12-300, worin Q die in der jeweiligen Zeile angegebenen Bedeutungen der Tabelle 1 hat. Die Verbindungen I.12-1 bis I.12-300 der Tabelle I.12 sind somit durch die Bedeutung der jeweiligen Einträge No. 1 bis 300 für Q der Tabelle 1 definiert.

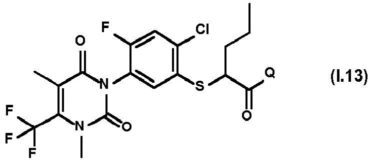

Tabelle I.13: Bevorzugte Verbindungen der Formel (I.13) sind die Verbindungen I.13-1 bis I.13-300, worin Q die in der jeweiligen Zeile angegebenen Bedeutungen der Tabelle 1 hat. Die Verbindungen I.13-1 bis I.13-300 der Tabelle I.13 sind somit durch die Bedeutung der jeweiligen Einträge No. 1 bis 300 für Q der Tabelle 1 definiert.

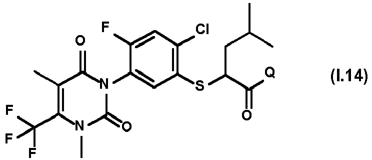

Tabelle I.14: Bevorzugte Verbindungen der Formel (I.14) sind die Verbindungen I.14-1 bis I.14-300, worin Q die in der jeweiligen Zeile angegebenen Bedeutungen der Tabelle 1 hat. Die Verbindungen I.14-1 bis I.14-300 der Tabelle I.14 sind somit durch die Bedeutung der jeweiligen Einträge No. 1 bis 300 für Q der Tabelle 1 definiert.

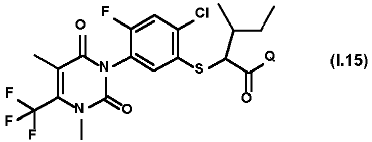

Tabelle I.15: Bevorzugte Verbindungen der Formel (I.15) sind die Verbindungen I.15-1 bis I.15-300, worin Q die in der jeweiligen Zeile angegebenen Bedeutungen der Tabelle 1 hat. Die Verbindungen I.15-1 bis I.15-300 der Tabelle I.15 sind somit durch die Bedeutung der jeweiligen Einträge No. 1 bis 300 für Q der Tabelle 1 definiert.

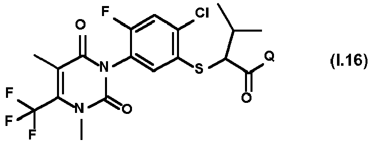

Tabelle I.16: Bevorzugte Verbindungen der Formel (I.16) sind die Verbindungen I.16-1 bis I.16-300, worin Q die in der jeweiligen Zeile angegebenen Bedeutungen der Tabelle 1 hat. Die Verbindungen I.16-1 bis I.16-300 der Tabelle I.16 sind somit durch die Bedeutung der jeweiligen Einträge No. 1 bis 300 für Q der Tabelle 1 definiert.

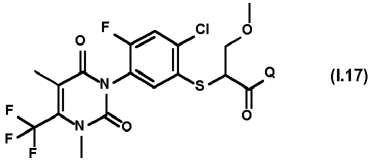

Tabelle I.17: Bevorzugte Verbindungen der Formel (I.17) sind die Verbindungen I.17-1 bis I.17-300, worin Q die in der jeweiligen Zeile angegebenen Bedeutungen der Tabelle 1 hat. Die Verbindungen I.17-1 bis I.17-300 der Tabelle I.17 sind somit durch die Bedeutung der jeweiligen Einträge No. 1 bis 300 für Q der Tabelle 1 definiert.

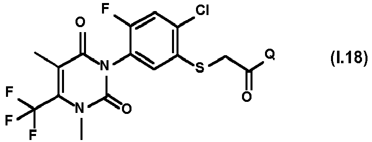

Tabelle I.18: Bevorzugte Verbindungen der Formel (I.18) sind die Verbindungen I.18-1 bis I.18-300, worin Q die in der jeweiligen Zeile angegebenen Bedeutungen der Tabelle 1 hat. Die Verbindungen I.18-1 bis I.18-300 der Tabelle I.18 sind somit durch die Bedeutung der jeweiligen Einträge No. 1 bis 300 für Q der Tabelle 1 definiert.

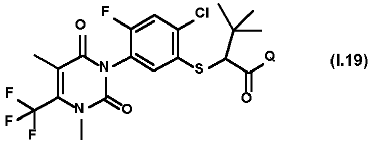

Tabelle I.19: Bevorzugte Verbindungen der Formel (I.19) sind die Verbindungen I.19-1 bis I.19-300, worin Q die in der jeweiligen Zeile angegebenen Bedeutungen der Tabelle 1 hat. Die Verbindungen I.19-1 bis I.19-300 der Tabelle I.19 sind somit durch die Bedeutung der jeweiligen Einträge No. 1 bis 300 für Q der Tabelle 1 definiert.

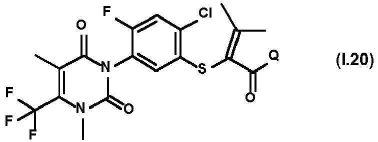

Tabelle I.20: Bevorzugte Verbindungen der Formel (I.20) sind die Verbindungen I.20-1 bis I.20-300, worin Q die in der jeweiligen Zeile angegebenen Bedeutungen der Tabelle 1 hat. Die Verbindungen I.20-1 bis I.20-300 der Tabelle I.20 sind somit durch die Bedeutung der jeweiligen Einträge No. 1 bis 300 für Q der Tabelle 1 definiert.

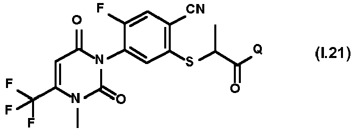

Tabelle I.21: Bevorzugte Verbindungen der Formel (I.21) sind die Verbindungen I.21-1 bis I.21-300, worin Q die in der jeweiligen Zeile angegebenen Bedeutungen der Tabelle 1 hat. Die Verbindungen I.21-1 bis I.21-300 der Tabelle I.21 sind somit durch die Bedeutung der jeweiligen Einträge No. 1 bis 300 für Q der Tabelle 1 definiert.

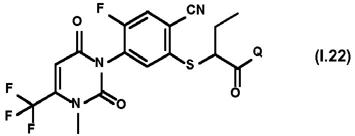

Tabelle I.22: Bevorzugte Verbindungen der Formel (I.22) sind die Verbindungen I.22-1 bis I.22-300, worin Q die in der jeweiligen Zeile angegebenen Bedeutungen der Tabelle 1 hat. Die Verbindungen I.22-1 bis I.22-300 der Tabelle I.22 sind somit durch die Bedeutung der jeweiligen Einträge No. 1 bis 300 für Q der Tabelle 1 definiert.

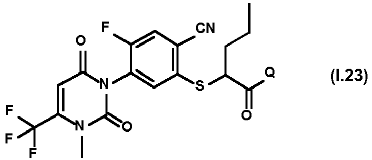

Tabelle I.23: Bevorzugte Verbindungen der Formel (1.23) sind die Verbindungen |.23-1 bis I.23-300, worin Q die in der jeweiligen Zeile angegebenen Bedeutungen der Tabelle 1 hat. Die Verbindungen I.23-1 bis I.23-300 der Tabelle I.23 sind somit durch die Bedeutung der jeweiligen Einträge No. 1 bis 300 für Q der Tabelle 1 definiert.

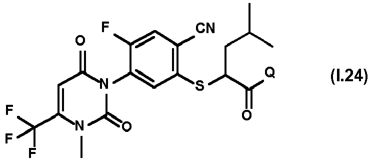

Tabelle I.24: Bevorzugte Verbindungen der Formel (I.24) sind die Verbindungen I.24-1 bis I.24-300, worin Q die in der jeweiligen Zeile angegebenen Bedeutungen der Tabelle 1 hat. Die Verbindungen I.24-1 bis I.24-300 der Tabelle I.24 sind somit durch die Bedeutung der jeweiligen Einträge No. 1 bis 300 für Q der Tabelle 1 definiert.

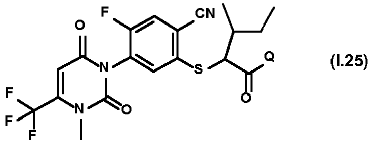

Tabelle I.25: Bevorzugte Verbindungen der Formel (I.25) sind die Verbindungen I.25-1 bis I.25-300, worin Q die in der jeweiligen Zeile angegebenen Bedeutungen der Tabelle 1 hat. Die Verbindungen I.25-1 bis I.25-300 der Tabelle I.25 sind somit durch die Bedeutung der jeweiligen Einträge No. 1 bis 300 für Q der Tabelle 1 definiert.

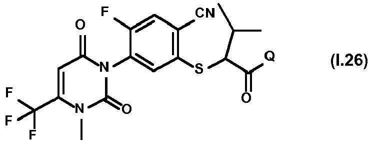

Tabelle I.26: Bevorzugte Verbindungen der Formel (I.26) sind die Verbindungen I.26-1 bis I.26-300, worin Q die in der jeweiligen Zeile angegebenen Bedeutungen der Tabelle 1 hat. Die Verbindungen I.26-1 bis I.26-300 der Tabelle I.26 sind somit durch die Bedeutung der jeweiligen Einträge No. 1 bis 300 für Q der Tabelle 1 definiert.

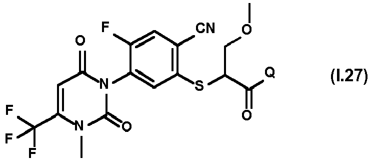

Tabelle I.27: Bevorzugte Verbindungen der Formel (I.27) sind die Verbindungen I.27-1 bis I.27-300, worin Q die in der jeweiligen Zeile angegebenen Bedeutungen der Tabelle 1 hat. Die Verbindungen I.27-1 bis I.27-300 der Tabelle I.27 sind somit durch die Bedeutung der jeweiligen Einträge No. 1 bis 300 für Q der Tabelle 1 definiert.

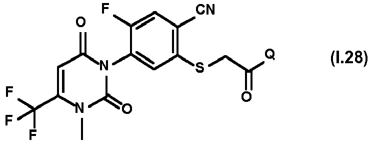

Tabelle I.28: Bevorzugte Verbindungen der Formel (I.28) sind die Verbindungen I.28-1 bis I.28-300, worin Q die in der jeweiligen Zeile angegebenen Bedeutungen der Tabelle 1 hat. Die Verbindungen I.28-1 bis I.28-300 der Tabelle I.28 sind somit durch die Bedeutung der jeweiligen Einträge No. 1 bis 300 für Q der Tabelle 1 definiert.

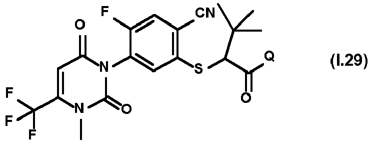

Tabelle I.29: Bevorzugte Verbindungen der Formel (I.29) sind die Verbindungen I.29-1 bis I.29-300, worin Q die in der jeweiligen Zeile angegebenen Bedeutungen der Tabelle 1 hat. Die Verbindungen I.29-1 bis I.29-300 der Tabelle I.29 sind somit durch die Bedeutung der jeweiligen Einträge No. 1 bis 300 für Q der Tabelle 1 definiert.

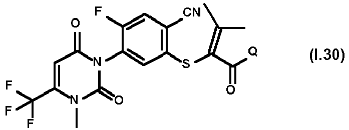

Tabelle I.30: Bevorzugte Verbindungen der Formel (I.30) sind die Verbindungen I.30-1 bis I.30-300, worin Q die in der jeweiligen Zeile angegebenen Bedeutungen der Tabelle 1 hat. Die Verbindungen I.30-1 bis I.30-300 der Tabelle I.30 sind somit durch die Bedeutung der jeweiligen Einträge No. 1 bis 300 für Q der Tabelle 1 definiert.

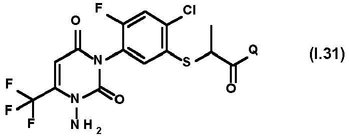

Tabelle I.31: Bevorzugte Verbindungen der Formel (I.31) sind die Verbindungen I.31-1 bis I.31-300, worin Q die in der jeweiligen Zeile angegebenen Bedeutungen der Tabelle 1 hat. Die Verbindungen I.31-1 bis I.31-300 der Tabelle I.31 sind somit durch die Bedeutung der jeweiligen Einträge No. 1 bis 300 für Q der Tabelle 1 definiert.

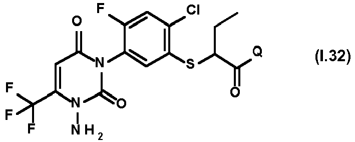

Tabelle I.32: Bevorzugte Verbindungen der Formel (I.32) sind die Verbindungen I.32-1 bis I.32-300, worin Q die in der jeweiligen Zeile angegebenen Bedeutungen der Tabelle 1 hat. Die Verbindungen I.32-1 bis I.32-300 der Tabelle I.32 sind somit durch die Bedeutung der jeweiligen Einträge No. 1 bis 300 für Q der Tabelle 1 definiert.

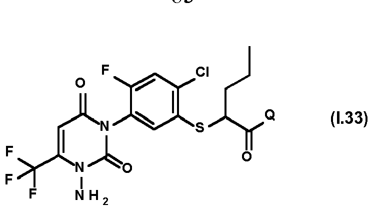

Tabelle I.33: Bevorzugte Verbindungen der Formel (I.33) sind die Verbindungen I.33-1 bis I.33-300, worin Q die in der jeweiligen Zeile angegebenen Bedeutungen der Tabelle 1 hat. Die Verbindungen I.33-1 bis I.33-300 der Tabelle I.33 sind somit durch die Bedeutung der jeweiligen Einträge No. 1 bis 300 für Q der Tabelle 1 definiert.

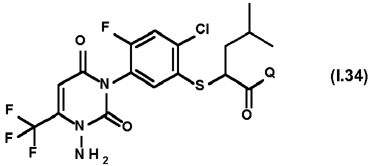

Tabelle I.34: Bevorzugte Verbindungen der Formel (I.34) sind die Verbindungen I.34-1 bis I.34-300, worin Q die in der jeweiligen Zeile angegebenen Bedeutungen der Tabelle 1 hat. Die Verbindungen I.34-1 bis I.34-300 der Tabelle I.34 sind somit durch die Bedeutung der jeweiligen Einträge No. 1 bis 300 für Q der Tabelle 1 definiert.

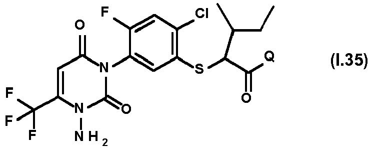

Tabelle I.35: Bevorzugte Verbindungen der Formel (I.35) sind die Verbindungen I.35-1 bis I.35-300, worin Q die in der jeweiligen Zeile angegebenen Bedeutungen der Tabelle 1 hat. Die Verbindungen I.35-1 bis I.35-300 der Tabelle I.35 sind somit durch die Bedeutung der jeweiligen Einträge No. 1 bis 300 für Q der Tabelle 1 definiert.

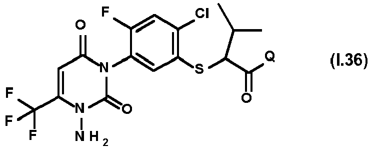

Tabelle I.36: Bevorzugte Verbindungen der Formel (I.36) sind die Verbindungen I.36-1 bis I.36-300, worin Q die in der jeweiligen Zeile angegebenen Bedeutungen der Tabelle 1 hat. Die Verbindungen I.36-1 bis I.36-300 der Tabelle I.36 sind somit durch die Bedeutung der jeweiligen Einträge No. 1 bis 300 für Q der Tabelle 1 definiert.

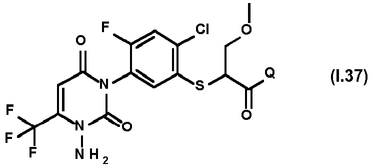

Tabelle I.37: Bevorzugte Verbindungen der Formel (I.37) sind die Verbindungen I.37-1 bis I.37-300, worin Q die in der jeweiligen Zeile angegebenen Bedeutungen der Tabelle 1 hat. Die Verbindungen I.37-1 bis I.37-300 der Tabelle I.37 sind somit durch die Bedeutung der jeweiligen Einträge No. 1 bis 300 für Q der Tabelle 1 definiert.

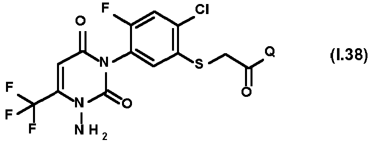

Tabelle I.38: Bevorzugte Verbindungen der Formel (I.38) sind die Verbindungen I.38-1 bis I.38-300, worin Q die in der jeweiligen Zeile angegebenen Bedeutungen der Tabelle 1 hat. Die Verbindungen I.38-1 bis I.38-300 der Tabelle I.38 sind somit durch die Bedeutung der jeweiligen Einträge No. 1 bis 300 für Q der Tabelle 1 definiert.

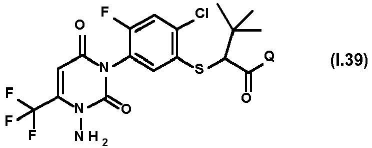

Tabelle I.39: Bevorzugte Verbindungen der Formel (I.39) sind die Verbindungen I.39-1 bis I.39-300, worin Q die in der jeweiligen Zeile angegebenen Bedeutungen der Tabelle 1 hat. Die Verbindungen I.39-1 bis I.39-300 der Tabelle I.39 sind somit durch die Bedeutung der jeweiligen Einträge No. 1 bis 300 für Q der Tabelle 1 definiert.

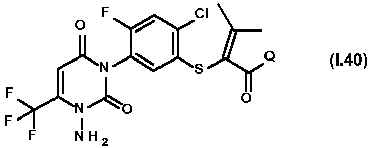

Tabelle I.40: Bevorzugte Verbindungen der Formel (I.40) sind die Verbindungen I.40-1 bis I.40-300, worin Q die in der jeweiligen Zeile angegebenen Bedeutungen der Tabelle 1 hat. Die Verbindungen I.40-1 bis I.40-300 der Tabelle I.40 sind somit durch die Bedeutung der jeweiligen Einträge No. 1 bis 300 für Q der Tabelle 1 definiert.

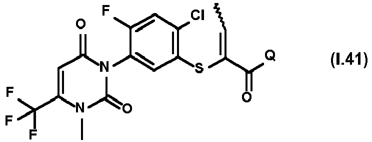

Tabelle I.41: Bevorzugte Verbindungen der Formel (I.41) sind die Verbindungen I.41-1 bis I.41-300, worin Q die in der jeweiligen Zeile angegebenen Bedeutungen der Tabelle 1 hat. Die Verbindungen I.41-1 bis I.41-300 der Tabelle I.41 sind somit durch die Bedeutung der jeweiligen Einträge No. 1 bis 300 für Q der Tabelle 1 definiert.

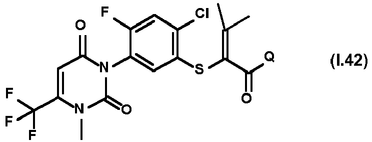

Tabelle I.42: Bevorzugte Verbindungen der Formel (I.42) sind die Verbindungen I.42-1 bis I.42-300, worin Q die in der jeweiligen Zeile angegebenen Bedeutungen der Tabelle 1 hat. Die Verbindungen I.42-1 bis I.42-300 der Tabelle I.42 sind somit durch die Bedeutung der jeweiligen Einträge No. 1 bis 300 für Q der Tabelle 1 definiert.

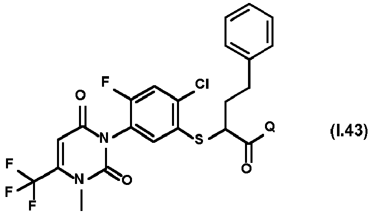

Tabelle I.43: Bevorzugte Verbindungen der Formel (I.43) sind die Verbindungen I.43-1 bis I.43-300, worin Q die in der jeweiligen Zeile angegebenen Bedeutungen der Tabelle 1 hat. Die Verbindungen I.43-1 bis I.43-300 der Tabelle I.43 sind somit durch die Bedeutung der jeweiligen Einträge No. 1 bis 300 für Q der Tabelle 1 definiert.

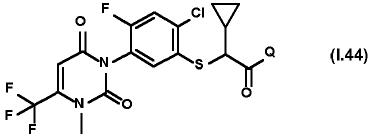

Tabelle I.44: Bevorzugte Verbindungen der Formel (I.44) sind die Verbindungen I.44-1 bis I.44-300, worin Q die in der jeweiligen Zeile angegebenen Bedeutungen der Tabelle 1 hat. Die Verbindungen I.44-1 bis I.44-300 der Tabelle I.44 sind somit durch die Bedeutung der jeweiligen Einträge No. 1 bis 300 für Q der Tabelle 1 definiert.

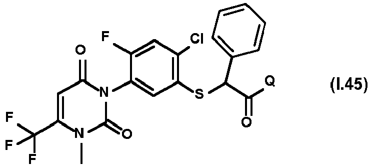

Tabelle I.45: Bevorzugte Verbindungen der Formel (I.45) sind die Verbindungen I.45-1 bis I.45-300, worin Q die in der jeweiligen Zeile angegebenen Bedeutungen der Tabelle 1 hat. Die Verbindungen I.45-1 bis I.45-300 der Tabelle I.45 sind somit durch die Bedeutung der jeweiligen Einträge No. 1 bis 300 für Q der Tabelle 1 definiert.

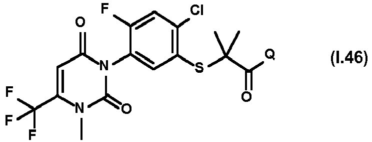

Tabelle I.46: Bevorzugte Verbindungen der Formel (I.46) sind die Verbindungen I.46-1 bis I.46-300, worin Q die in der jeweiligen Zeile angegebenen Bedeutungen der Tabelle 1 hat. Die Verbindungen I.46-1 bis I.46-300 der Tabelle I.46 sind somit durch die Bedeutung der jeweiligen Einträge No. 1 bis 300 für Q der Tabelle 1 definiert.

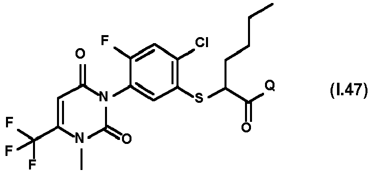

Tabelle I.47: Bevorzugte Verbindungen der Formel (I.47) sind die Verbindungen I.47-1 bis I.47-300, worin Q die in der jeweiligen Zeile angegebenen Bedeutungen der Tabelle 1 hat. Die Verbindungen I.47-1 bis I.47-300 der Tabelle I.47 sind somit durch die Bedeutung der jeweiligen Einträge No. 1 bis 300 für Q der Tabelle 1 definiert.

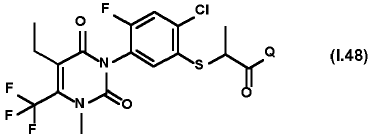

Tabelle I.48: Bevorzugte Verbindungen der Formel (I.48) sind die Verbindungen I.48-1 bis I.48-300, worin Q die in der jeweiligen Zeile angegebenen Bedeutungen der Tabelle 1 hat. Die Verbindungen I.48-1 bis I.48-300 der Tabelle I.48 sind somit durch die Bedeutung der jeweiligen Einträge No. 1 bis 300 für Q der Tabelle 1 definiert.

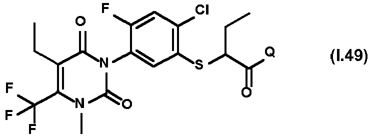

Tabelle I.49: Bevorzugte Verbindungen der Formel (I.49) sind die Verbindungen I.49-1 bis I.49-300, worin Q die in der jeweiligen Zeile angegebenen Bedeutungen der Tabelle 1 hat. Die Verbindungen I.49-1 bis I.49-300 der Tabelle I.49 sind somit durch die Bedeutung der jeweiligen Einträge No. 1 bis 300 für Q der Tabelle 1 definiert.

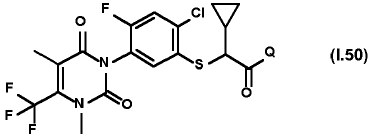

Tabelle I.50: Bevorzugte Verbindungen der Formel (I.50) sind die Verbindungen I.50-1 bis I.50-300, worin Q die in der jeweiligen Zeile angegebenen Bedeutungen der Tabelle 1 hat. Die Verbindungen I.50-1 bis I.50-300 der Tabelle I.50 sind somit durch die Bedeutung der jeweiligen Einträge No. 1 bis 300 für Q der Tabelle 1 definiert.

Spektroskopische Daten ausgewählter Tabellenbeispiele:
a) klassische NMR-Interpretation:
   Beispiel Nr. I.1-43:
      ¹H-NMR (CDCl₃ δ, ppm) 7.61 (m, 1H), 7.37 (d, 1H), 6.34 (s, 1H), 4.63-4.53 (m, 2H), 4.20-4.13 (m, 2H), 3.98-3.91 (m, 1H), 3.55 (s, 3H), 1.57 (d, 3H), 1.25 (t, 3H).
   Beispiel Nr. I.1-46:
      ¹H-NMR (CDCl₃ δ, ppm) 7.59 (m, 1H), 7.39-7.27 (m, 6H), 6.32 (m, 1H), 5.18-5.11 (m, 2H), 4.70-4.59 (m, 2H), 3.98-3.91 (m, 1H), 3.53 (m, 3H), 1.55 (m, 3H).
   Beispiel Nr. I.1-51:
      ¹H-NMR (CDCl₃ δ, ppm) 7.63 (m, 1H), 7.37 (d, 1H), 6.34 (m, 1H), 4.63-4.53 (m, 2H), 4.13-4.09 (m, 2H), 3.96-3.93 (m, 1H), 3.55 (s, 3H), 1.62-1.55 (m, 5H), 1.39-1.33 (m, 2H), 0.92 (t, 3H).
   Beispiel Nr. I.1-233:
      ¹H-NMR (CDCl₃ δ, ppm) 7.52 (m, 1H), 7.37 (d, 1H), 6.34 (s, 1H), 4.43-4.32 (m, 2H), 3.91-3.8 (m, 1H), 3.55 (s, 3H), 3.43-3.37 (m, 1H), 3.32-3.27 (m, 1H), 2.92/2.88 (s, 3H), 2.57-2.51 (m, 2H), 1.52 (d, 3H), 1.15 / 1.07 (t, 3H).
   Beispiel Nr. I.2-6:
      ¹H-NMR (CDCl₃ δ, ppm) 7.48 (m, 1H), 7.37-7.31 (m, 6H), 6.33 (m, 1H), 5.11 (s, 2H), 4.39-4.26 (m, 2H), 3.66 (m, 1H), 3.52 (m, 3H), 2.62-2.56 (m, 2H), 1.97-1.89 (m, 1H), 1.86-1.75 (m, 1H), 1.04 (t, 3H).
   Beispiel Nr. I.8-54:
      ¹H-NMR (CDCl₃ δ, ppm) 7.53 (d, 1H), 7.35 (d, 1H), 6.34 (s, 1H), 5.03-4.97 (sept, 1H), 4.59 (s, 2H), 3.74 (s, 2H), 3.55 (s, 3H), 1.24 (d, 6H).
   Beispiel Nr. I.8-46:
      ¹H-NMR (CDCl₃ δ, ppm) 7.51 (m, 1H), 7.39-7.28 (m, 6H), 6.32 (s, 1H), 5.14 (s, 2H), 4.69 (s, 2H), 3.98-3.74 (s, 2H), 3.53 (s, 3H).
   Beispiel Nr. I.8-51:
      ¹H-NMR (CDCl₃ δ, ppm) 7.52 (d, 1H), 7.35 (d, 1H), 6.34 (s, 1H), 4.63 (s, 2H), 4.10 (t, 2H), 3.74 (s, 2H), 3.55 (s, 3H), 1.63-1.56 (m, 5H), 1.42-1.32 (m, 2H), 0.93 (t, 3H).
   Beispiel Nr. I.12-3:
      ¹H-NMR (CDCl₃ δ, ppm) 7.47 (m, 1H), 7.35 (m, 1H), 4.39-4.24 (m, 2H), 4.17-4.09 (m, 2H), 3.70-3.67 (m, 1H), 3.54 (s, 3H), 2.62-2.55 (m, 2H), 2.27-2.22 (m, 3H), 1.99-1.91 (m, 1H), 1.89-1.81 (m, 1H), 1.25 (t, 3H), 1.06 (t, 3H).
   Beispiel Nr. I.14-3:
      ¹H-NMR (CDCl₃ δ, ppm) 7.47 (m, 1H), 7.36 (m, 1H), 4.37-4.29 (m, 1H), 4.28-4.20 (m, 1H), 4.13-4.10 (q, 2H), 3.84-3.80 (m, 1H), 3.54 (s, 3H), 2.58-2.52 (m, 2H), 2.24-2.20 (m, 3H), 1.89-1.82 (m, 1H), 1.78-1.73 (m, 1H), 1.69-1.60 (m, 2H), 1.31-1.22 (m, 4H), 0.96-0.92 (m, 6H).
   Beispiel Nr. I.47-3:
      ¹H-NMR (d₆-DMSO δ, ppm) 7.85 (m, 1H), 7.70 (m, 1H), 6.60 (m, 1H), 4.27-4.21 (m, 1H), 4.20-4.14 (m, 1H), 4.06 (q, 2H), 3.92-3.87 (m, 1H), 3.47 (s, 3H), 2.59-2.55 (m, 2H), 1.87-1.78 (m, 1H), 1.76-1.68 (m, 1H), 1.42-1.24 (m, 4H), 1.17 (t, 3H), 0.85 (t, 3H).
   Beispiel Nr. I.47-6:
      ¹H-NMR (CDCl₃ δ, ppm) 7.56 (m, 1H), 7.36-7.32 (m, 6H), 6.33 (m, 1H), 5.11 (s, 2H), 4.39-4.25 (m, 2H), 3.72 (m, 1H), 3.52 (m, 3H), 2.62-2.58 (m, 2H), 1.93-1.88 (m, 1H), 1.81-1.73 (m, 1H), 1.45-1.33 (m, 4H), 0.91 (t, 3H).

NMR-Peak-Listenverfahren - Die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form: δ₁ (Intensität₁⁾; δ₂ (lntensität₂);........; δᵢ (lntensitätᵢ⁾;......; δₙ (Intensitätₙ). Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen. Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen. Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation. Weitere Details zu ¹H-NMR-Peaklistenkönnen der Research Disclosure Database Number 564025 entnommen werden.
Beispiel Nr. I.1-1:
   ¹H-NMR (400 MHz, CDCl₃ δ, ppm) 7.5183 (3.8); 7.4518 (5.2); 7.4451 (5.5); 7.4331 (5.2); 7.4266 (5.5); 7.3877 (5.3); 7.3802 (5.6); 7.3647 (5.4); 7.3574 (5.8); 7.3089 (3.1); 7.2966 (1.7); 7.2594 (648.9); 7.2081 (1.9); 6.9953 (3.5); 6.4114 (10.7); 5.2986 (13.6); 4.4561 (1.0); 4.4428 (1.1); 4.4391 (1.3); 4.4283 (2.6); 4.4157 (2.3); 4.3991 (2.7); 4.3848 (2.0); 4.3717 (1.5); 4.3320 (1.5); 4.3188 (2.4); 4.3120 (1.7); 4.3038 (2.4); 4.2998 (2.2); 4.2958 (1.8); 4.2902 (1.9); 4.2840 (2.6); 4.2715 (1.5); 4.2561 (1.0); 3.9480 (1.3); 3.9424 (1.4); 3.9298 (4.3); 3.9245 (4.4); 3.9117 (4.2); 3.9064 (4.3); 3.8938 (1.4); 3.8883 (1.3); 3.5810 (13.9); 3.5780 (15.6); 3.5735 (16.0); 3.5705 (14.1); 2.5391 (5.6); 2.5241 (9.8); 2.5174 (3.5); 2.5104 (4.4); 2.0054 (1.3); 1.5698 (18.9); 1.5676 (18.0); 1.5516 (18.5); 1.5495 (17.3); 0.1462 (1.0); 0.0494 (1.1); 0.0079 (8.6); -0.0002 (238.4); -0.0084 (6.9); -0.0515 (0.7); -0.1495 (0.9)
Beispiel Nr. 1.1-2:
   ¹H-NMR (400 MHz, CDCl₃ δ, ppm) 7.4907 (0.6); 7.4847 (0.7); 7.4719 (0.7); 7.4659 (0.7); 7.3814 (0.7); 7.3777 (0.7); 7.3587 (0.7); 7.3549 (0.7); 7.2595 (75.2); 6.3512 (1.2); 6.3475 (1.2); 4.2934 (0.7); 3.8837 (1.0); 3.8658 (1.0); 3.6668 (6.2); 3.5515 (3.0); 3.5489 (3.0); 2.5821 (0.6); 2.5665 (1.0); 2.5548 (0.7); 2.5517 (0.7); 1.5327 (16.0); 1.5232 (3.7); 1.5053 (3.6); 1.3686 (0.7); 0.0078 (0.9); -0.0002 (26.8); - 0.0085 (0.8)
Beispiel Nr. I.1-3:
   ¹H-NMR (400 MHz, CDCl₃ δ, ppm) 7.5184 (2.3); 7.4974 (3.4); 7.4908 (3.6); 7.4786 (3.5); 7.4720 (3.5); 7.3800 (3.7); 7.3760 (3.7); 7.3572 (3.8); 7.3532 (3.7); 7.2914 (1.0); 7.2841 (0.8); 7.2595 (404.0); 6.9955 (2.3); 6.3497 (5.6); 6.3444 (5.7); 4.3686 (0.5); 4.3513 (0.7); 4.3409 (1.5); 4.3340 (1.5); 4.3262 (1.6); 4.3237 (1.9); 4.3190 (1.7); 4.3162 (2.0); 4.3130 (1.7); 4.3092 (2.6); 4.3012 (1.8); 4.2976 (2.5); 4.2944 (2.7); 4.2819 (2.0); 4.2783 (1.6); 4.2665 (1.0); 4.2542 (0.5); 4.1591 (0.7); 4.1509 (2.2); 4.1487 (2.3); 4.1412 (0.8); 4.1331 (6.4); 4.1308 (6.6); 4.1152 (6.5); 4.1129 (6.6); 4.0974 (2.2); 4.0951 (2.3); 3.8988 (1.3); 3.8809 (4.4); 3.8630 (4.4); 3.8452 (1.3); 3.5504 (15.3); 3.5475 (15.8); 2.6517 (0.5); 2.5959 (0.7); 2.5805 (0.6); 2.5704 (3.0); 2.5552 (5.2); 2.5447 (2.1); 2.5415 (3.8); 2.5393 (4.7); 2.5272 (1.7); 2.5239 (1.6); 2.5005 (0.5); 2.0331 (1.0); 2.0277 (1.2); 1.5691 (1.8); 1.5531 (1.8); 1.5223 (20.8); 1.5044 (20.3); 1.2744 (0.8); 1.2652 (7.8); 1.2634 (8.0); 1.2565 (1.8); 1.2473 (15.5); 1.2455 (16.0); 1.2388 (1.2); 1.2295 (7.5); 1.2277 (7.6); 0.3308 (0.7); 0.2375 (0.5); 0.1574 (0.7); 0.1459 (0.6); 0.0079 (5.2); -0.0002 (177.4); -0.0085 (5.4); -0.1495 (0.7)
Beispiel Nr. I.1-5:
   ¹H-NMR (400 MHz, CDCl₃ δ, ppm) 7.5186 (1.7); 7.5125 (3.7); 7.5068 (3.8); 7.4938 (3.7); 7.4880 (3.8); 7.3787 (3.8); 7.3750 (4.0); 7.3559 (3.8); 7.3522 (4.0); 7.2861 (0.6); 7.2597 (283.3); 6.9957 (1.6); 6.3505 (5.9); 6.3439 (6.1); 5.2986 (3.6); 4.3112 (0.7); 4.3083 (0.6); 4.3026 (1.5); 4.2984 (1.8); 4.2932 (0.7); 4.2877 (1.9); 4.2839 (3.7); 4.2807 (2.4); 4.2779 (1.8); 4.2689 (3.3); 4.2624 (2.8); 4.2562 (0.8); 4.2527 (1.7); 4.2466 (1.5); 4.2409 (0.8); 4.2348 (0.8); 3.8956 (0.8); 3.8918 (0.9); 3.8776 (3.0); 3.8739 (3.2); 3.8597 (3.1); 3.8560 (3.2); 3.8419 (0.9); 3.8382 (0.9); 3.5503 (15.5); 3.5477 (16.0); 2.5151 (0.8); 2.4994 (0.5); 2.4896 (2.6); 2.4741 (5.8); 2.4616 (2.7); 2.4587 (4.9); 2.4443 (1.7); 2.4400 (1.6); 1.6097 (7.4); 1.5210 (20.5); 1.5031 (20.2); 1.4472 (1.0); 1.4308 (92.0); 1.4293 (95.4); 0.0080 (3.2); -0.0002 (102.5); - 0.0085 (2.9)
Beispiel Nr. I.1-6:
   ¹H-NMR (400 MHz, CDCl₃ δ, ppm) 7.5183 (9.6); 7.4723 (6.0); 7.4603 (6.4); 7.4536 (6.0); 7.4416 (6.4); 7.3726 (3.6); 7.3599 (3.3); 7.3550 (7.3); 7.3498 (9.4); 7.3418 (23.8); 7.3352 (29.0); 7.3271 (10.7); 7.3217 (10.2); 7.3178 (6.1); 7.2979 (2.2); 7.2931 (2.2); 7.2906 (2.5); 7.2867 (2.9); 7.2843 (2.9); 7.2820 (3.1); 7.2772 (4.8); 7.2740 (6.0); 7.2732 (6.2); 7.2724 (6.7); 7.2700 (9.6); 7.2684 (11.8); 7.2668 (15.6); 7.2594 (1724.6); 7.2378 (1.0); 7.2271 (1.1); 7.2088 (1.9); 6.9954 (9.8); 6.3351 (9.3); 6.3182 (9.6); 5.2985 (9.0); 5.1439 (1.0); 5.1092 (16.0); 5.0770 (0.9); 4.3853 (1.1); 4.3707 (1.2); 4.3675 (1.4); 4.3578 (3.2); 4.3529 (1.6); 4.3483 (2.4); 4.3431 (3.5); 4.3399 (3.0); 4.3334 (2.7); 4.3304 (3.2); 4.3251 (2.6); 4.3212 (3.2); 4.3155 (2.7); 4.3051 (6.0); 4.2895 (3.3); 4.2773 (2.4); 4.2611 (1.3); 3.8686 (1.4); 3.8506 (5.2); 3.8354 (5.6); 3.8328 (5.6); 3.8175 (5.6); 3.7997 (1.4); 3.5307 (13.7); 3.5276 (14.2); 3.5048 (13.0); 3.5018 (13.5); 2.6667 (0.9); 2.6514 (1.5); 2.6252 (4.9); 2.6096 (9.3); 2.5942 (8.4); 2.5798 (2.6); 2.5755 (2.4); 2.5526 (1.0); 2.0049 (0.9); 1.5343 (143.3); 1.4845 (21.0); 1.4823 (21.6); 1.4666 (20.2); 1.4644 (21.0); 1.2844 (0.8); 1.2562 (1.5); 0.1459 (2.8); 0.1131 (1.2); 0.0334 (1.0); 0.0080 (27.7); 0.0064 (10.6); 0.0055 (11.6); 0.0047 (13.5); -0.0002 (851.4); -0.0059 (10.4); -0.0067 (9.1); -0.0085 (24.9); -0.0115 (2.7); -0.0163 (1.1); -0.0219 (0.9); -0.0507 (0.8); -0.1496 (2.5)
Beispiel Nr. I.1-8:
   ¹H-NMR (400 MHz, CDCl₃ δ, ppm) 7.4969 (1.0); 7.4900 (1.0); 7.4782 (1.0); 7.4713 (1.0); 7.3794 (1.0); 7.3754 (1.0); 7.3566 (1.0); 7.3527 (1.0); 7.2689 (0.6); 7.2681 (0.7); 7.2673 (0.8); 7.2665 (0.9); 7.2657 (1.1); 7.2649 (1.4); 7.2600 (86.0); 7.2535 (0.9); 7.2527 (0.8); 7.2519 (0.6); 6.3488 (1.6); 6.3429 (1.4); 4.3383 (0.5); 4.3274 (0.5); 4.3231 (0.5); 4.3126 (0.7); 4.2976 (0.6); 4.2934 (0.6); 4.2815 (0.5); 4.0445 (1.1); 4.0420 (1.1); 4.0276 (2.1); 4.0252 (2.2); 4.0107 (1.1); 4.0084 (1.1); 3.8806 (0.9); 3.8789 (0.9); 3.8628 (0.9); 3.8609 (0.9); 3.5496 (4.3); 3.5465 (4.3); 2.5810 (0.7); 2.5657 (1.3); 2.5499 (1.1); 1.6638 (0.8); 1.6452 (1.5); 1.6283 (1.4); 1.6098 (0.8); 1.5759 (16.0); 1.5206 (5.4); 1.5027 (5.4); 0.9465 (2.3); 0.9379 (0.7); 0.9278 (4.6); 0.9094 (2.0); 0.0080 (1.0); -0.0002 (29.7); -0.0085 (0.8)
Beispiel Nr. I.1-22:
   ¹H-NMR (400 MHz, CDCl₃ δ, ppm) 7.5185 (1.1); 7.5088 (1.3); 7.5008 (1.4); 7.4901 (1.3); 7.4821 (1.4); 7.4670 (1.4); 7.4639 (1.3); 7.4486 (1.3); 7.4453 (1.4); 7.3670 (3.9); 7.3442 (3.9); 7.2596 (208.2); 6.9956 (1.1); 6.3503 (5.7); 5.2986 (1.2); 5.2520 (0.7); 5.2411 (0.8); 5.2366 (0.8); 5.2224 (0.9); 5.2065 (0.8); 5.1930 (0.5); 3.8666 (1.0); 3.8595 (1.1); 3.8486 (1.4); 3.8416 (1.2); 3.8304 (1.5); 3.8123 (1.2); 3.6461 (16.0); 3.6371 (15.2); 3.5523 (9.9); 3.5494 (9.4); 2.6192 (1.1); 2.6004 (1.0); 2.5802 (1.9); 2.5614 (1.8); 2.4937 (0.9); 2.4901 (0.9); 2.4800 (0.9); 2.4767 (1.0); 2.4659 (0.8); 2.4574 (0.8); 2.4512 (1.3); 2.4422 (1.0); 2.4182 (0.5); 2.4122 (0.5); 1.7277 (1.0); 1.5155 (9.2); 1.4976 (9.2); 1.2638 (4.1); 1.2611 (4.4); 1.2480 (4.2); 1.2453 (4.3); 1.2018 (8.0); 1.1860 (7.9); 0.0080 (2.1); -0.0002 (73.2); -0.0085 (2.3)
Beispiel Nr. I.1-23:
   ¹H-NMR (400 MHz, CDCl₃ δ, ppm) 7.5184 (3.1); 7.5151 (2.4); 7.5053 (2.4); 7.4965 (2.2); 7.4867 (2.3); 7.4723 (2.2); 7.4696 (2.2); 7.4538 (2.2); 7.3660 (5.9); 7.3433 (5.9); 7.2871 (1.2); 7.2596 (522.0); 7.2267 (1.1); 7.2099 (1.0); 6.9956 (2.9); 6.3471 (8.1); 5.2986 (6.9); 5.2630 (1.0); 5.2486 (1.6); 5.2332 (1.6); 5.2153 (1.2); 5.2003 (0.9); 4.2038 (1.0); 4.1861 (3.1); 4.1682 (3.1); 4.1503 (1.2); 4.1300 (1.4); 4.1242 (2.3); 4.1121 (4.2); 4.1063 (6.7); 4.0942 (4.4); 4.0885 (6.8); 4.0765 (1.5); 4.0707 (2.3); 3.8751 (0.6); 3.8648 (1.7); 3.8577 (1.8); 3.8466 (2.1); 3.8397 (1.9); 3.8272 (3.1); 3.8091 (2.8); 3.7913 (0.8); 3.5514 (16.0); 3.5484 (14.9); 2.9716 (0.9); 2.9634 (0.9); 2.6089 (1.0); 2.6042 (1.0); 2.5897 (1.1); 2.5853 (0.9); 2.5732 (1.7); 2.5697 (1.9); 2.5652 (1.7); 2.5568 (1.5); 2.5506 (1.7); 2.5462 (1.6); 2.4759 (2.1); 2.4646 (1.7); 2.4608 (1.7); 2.4509 (1.3); 2.4429 (2.6); 2.4363 (2.0); 2.4278 (1.3); 2.4212 (2.0); 2.4119 (0.9); 2.4038 (0.9); 2.3973 (1.0); 2.3890 (0.8); 2.0051 (0.6); 1.5347 (122.2); 1.5187 (9.1); 1.5131 (13.4); 1.5008 (9.2); 1.4952 (13.0); 1.2962 (3.6); 1.2783 (7.2); 1.2604 (11.1); 1.2566 (6.1); 1.2443 (11.3); 1.2387 (14.0); 1.2277 (9.9); 1.2256 (11.0); 1.2228 (7.1); 1.2099 (4.8); 1.2078 (5.2); 1.1997 (13.0); 1.1839 (12.6); 0.1459 (0.8); 0.0079 (5.8); -0.0002 (185.2); -0.0085 (5.1); -0.1496 (0.8)
Beispiel Nr. I.1-32:
   ¹H-NMR (400 MHz, CDCl₃ δ, ppm) 7.5184 (0.9); 7.4862 (2.0); 7.4739 (2.2); 7.4677 (2.0); 7.4551 (2.1); 7.3630 (2.5); 7.3400 (2.5); 7.2596 (168.8); 6.9955 (0.9); 6.3526 (3.7); 6.3485 (3.8); 4.1714 (1.6); 4.1624 (1.7); 4.1446 (2.5); 4.1357 (2.6); 4.0597 (2.6); 4.0430 (2.4); 4.0330 (1.6); 4.0163 (1.6); 3.8935 (0.7); 3.8755 (2.1); 3.8580 (2.1); 3.8401 (0.6); 3.6775 (1.6); 3.6454 (14.6); 3.6403 (15.1); 3.5530 (9.5); 2.0126 (0.8); 2.0050 (1.0); 1.5784 (3.1); 1.5245 (11.4); 1.5067 (11.2); 1.2079 (1.5); 1.1642 (12.2); 1.1615 (11.9); 1.1356 (16.0); 0.0079 (2.1); -0.0002 (65.2); -0.0085 (1.9)
Beispiel Nr. I.1-42:
   ¹H-NMR (400 MHz, CDCl₃ δ, ppm) 7.6230 (2.3); 7.6040 (2.4); 7.5965 (2.2); 7.5776 (2.2); 7.5183 (2.5); 7.3813 (2.5); 7.3759 (2.3); 7.3663 (0.6); 7.3585 (2.5); 7.3532 (2.3); 7.3094 (1.0); 7.2920 (0.5); 7.2594 (453.3); 7.2098 (0.6); 6.9954 (2.4); 6.3457 (3.4); 6.3387 (3.8); 4.7127 (0.9); 4.7018 (0.6); 4.6559 (1.0); 4.6504 (1.5); 4.6355 (0.6); 4.6102 (3.4); 4.5959 (4.0); 4.5838 (5.5); 4.5434 (1.2); 3.9686 (1.9); 3.9555 (2.0); 3.9506 (1.9); 3.9375 (1.9); 3.9327 (0.7); 3.9198 (0.6); 3.7402 (1.6); 3.7244 (1.8); 3.7084 (15.2); 3.7040 (16.0); 3.5501 (10.1); 3.5470 (10.8); 3.5295 (0.7); 2.0049 (0.7); 1.5911 (7.4); 1.5852 (8.0); 1.5731 (9.3); 1.5672 (12.1); 1.5606 (8.6); 0.0079 (4.7); -0.0002 (167.9); -0.0085 (5.1); -0.1495 (0.6)
Beispiel Nr. 1-71:
   ¹H-NMR (400 MHz, CDCl₃ δ, ppm) δ= 7.4932 (3.4); 7.4890 (3.3); 7.4743 (3.5); 7.4702 (3.3); 7.3814 (3.8); 7.3784 (3.7); 7.3587 (3.8); 7.3557 (3.6); 7.2603 (78.2); 6.3869 (5.5); 6.3770 (5.2); 5.2985 (14.1); 4.9902 (0.7); 4.9798 (0.8); 4.9724 (2.5); 4.9622 (2.8); 4.9547 (2.6); 4.9445 (2.8); 4.9370 (0.8); 4.9267 (0.8); 4.1333 (0.7); 4.1271 (0.7); 4.1154 (2.1); 4.1093 (2.1); 4.0975 (2.1); 4.0915 (2.1); 4.0797 (0.7); 4.0734 (0.6); 3.5715 (7.7); 3.5687 (7.9); 3.5579 (8.5); 3.5551 (8.8); 1.5699 (10.6); 1.5678 (10.9); 1.5520 (10.6); 1.5500 (10.8); 1.5297 (0.6); 1.5258 (0.5); 1.4225 (16.0); 1.4048 (15.9); 0.0080 (1.0); -0.0002 (30.3); -0.0085 (1.1)
Beispiel Nr. I.1-72:
   ¹H-NMR (400 MHz, CDCl₃ δ, ppm) 7.6037 (1.5); 7.5850 (1.6); 7.5603 (1.9); 7.5572 (2.8); 7.5418 (1.9); 7.5382 (2.7); 7.5183 (2.5); 7.3846 (2.0); 7.3795 (1.9); 7.3611 (3.2); 7.3563 (3.4); 7.3379 (1.7); 7.3334 (1.8); 7.2883 (0.5); 7.2811 (0.6); 7.2803 (0.6); 7.2794 (0.6); 7.2787 (0.7); 7.2779 (0.8); 7.2771 (0.8); 7.2763 (0.8); 7.2755 (0.9); 7.2747 (1.0); 7.2739 (1.1); 7.2731 (1.2); 7.2723 (1.4); 7.2715 (1.5); 7.2707 (1.7); 7.2699 (2.0); 7.2691 (2.2); 7.2683 (2.5); 7.2675 (2.8); 7.2667 (3.2); 7.2659 (4.0); 7.2650 (5.0); 7.2594 (418.8); 7.2530 (3.5); 7.2506 (1.3); 7.2497 (1.0); 7.2489 (0.8); 7.2481 (0.7); 7.2473 (0.7); 7.2465 (0.6); 7.2457 (0.5); 7.2449 (0.6); 6.9954 (2.4); 6.3526 (3.0); 6.3406 (3.2); 6.3369 (2.8); 5.2983 (4.7); 5.0983 (1.5); 5.0808 (2.2); 5.0633 (1.4); 5.0227 (1.4); 5.0144 (1.4); 5.0050 (1.4); 4.9968 (1.5); 4.0133 (1.6); 4.0086 (1.7); 3.9954 (1.7); 3.9908 (1.8); 3.9240 (1.3); 3.9160 (1.3); 3.9059 (1.4); 3.8978 (1.4); 3.7010 (15.3); 3.6982 (16.0); 3.6824 (14.3); 3.6806 (14.0); 3.5592 (4.3); 3.5560 (4.6); 3.5451 (10.1); 1.6000 (11.6); 1.5958 (13.2); 1.5884 (8.5); 1.5776 (6.0); 1.5702 (5.7); 1.5619 (6.3); 1.5577 (6.6); 1.5440 (5.9); 1.5398 (6.2); 1.4603 (5.5); 1.4487 (5.7); 1.4426 (5.6); 1.4310 (5.6); 1.3514 (8.6); 1.3338 (8.7); 0.0079 (5.1); 0.0063 (1.7); 0.0054 (2.0); 0.0046 (2.6); -0.0002 (163.8); -0.0051 (2.6); -0.0060 (2.1); - 0.0069 (2.0); -0.0085 (4.8)
Beispiel Nr. I.1-1 02:
   ¹H-NMR (400 MHz, d₆-DMSO δ, ppm) 7.8549 (1.5); 7.8513 (1.4); 7.8312 (1.5); 7.8274 (1.4); 7.7657 (1.2); 7.7594 (1.3); 7.7466 (1.2); 7.7407 (1.3); 6.6184 (2.6); 6.6151 (2.6); 5.7526 (3.6); 4.1723 (1.0); 4.1610 (1.1); 4.1545 (1.0); 4.1433 (1.0); 3.5974 (16.0); 3.4230 (5.7); 3.3081 (219.0); 2.6738 (1.3); 2.6692 (1.8); 2.6646 (1.3); 2.5226 (9.0); 2.5179 (12.6); 2.5093 (113.1); 2.5047 (230.5); 2.5002 (313.9); 2.4956 (216.7); 2.4911 (97.9); 2.3316 (1.3); 2.3270 (1.9); 2.3224 (1.3); 1.4714 (6.4); 1.4537 (6.3); 1.4125 (0.8); 1.3962 (0.6); 1.3474 (0.6); 1.3294 (0.9); 1.1382 (0.7); 1.1236 (2.0); 1.1084 (1.3); 1.0813 (0.5); 0.0080 (3.6); -0.0002 (112.5); -0.0085 (3.6)
Beispiel Nr. I.1-127:
   ¹H-NMR (400 MHz, CDCl₃ δ, ppm) 7.5184 (1.2); 7.4816 (1.2); 7.4687 (1.2); 7.4630 (1.2); 7.4502 (1.2); 7.3700 (1.2); 7.3650 (1.3); 7.3473 (1.2); 7.3423 (1.2); 7.2595 (203.8); 6.9955 (1.2); 6.3486 (3.6); 5.4824 (0.7); 5.4665 (0.5); 3.8281 (1.3); 3.8106 (1.3); 3.6485 (16.0); 3.6445 (10.0); 3.6424 (9.8); 3.5498 (5.3); 2.6900 (2.0); 2.6862 (1.2); 2.6756 (2.7); 2.6694 (1.2); 2.6590 (2.4); 2.6490 (1.1); 2.6430 (1.8); 2.6334 (1.1); 2.6241 (0.9); 1.5339 (58.3); 1.5154 (5.7); 1.4974 (5.6); 0.0080 (2.4); -0.0002 (77.4); -0.0085 (2.2)
Beispiel Nr. I.1-132:
   ¹H-NMR (400 MHz, CDCl₃ δ, ppm) 7.5307 (1.7); 7.5246 (1.7); 7.5120 (1.8); 7.5059 (1.7); 7.3713 (1.8); 7.3673 (1.8); 7.3486 (1.8); 7.3446 (1.8); 7.2604 (50.2); 6.3441 (5.2); 3.9130 (1.4); 3.9058 (1.4); 3.8950 (1.4); 3.8878 (1.4); 3.6714 (16.0); 3.6698 (15.8); 3.5485 (7.3); 3.5457 (7.6); 2.0048 (3.0); 1.5546 (7.6); 1.5400 (5.4); 1.5360 (5.6); 1.5220 (5.3); 1.5180 (5.4); 1.4765 (9.1); 1.4711 (25.5); 0.0079 (0.6); -0.0002 (21.9); -0.0085 (0.7)
Beispiel Nr. I.1-141:
   ¹H-NMR (400 MHz, CDCl₃ δ, ppm) 7.6862 (3.7); 7.6780 (4.3); 7.6674 (4.0); 7.6592 (8.0); 7.6511 (4.1); 7.6404 (4.0); 7.6324 (4.1); 7.5185 (3.5); 7.3841 (6.6); 7.3802 (7.4); 7.3614 (6.2); 7.3575 (7.6); 7.3178 (0.7); 7.3107 (0.8); 7.3092 (2.1); 7.3020 (0.6); 7.2940 (0.8); 7.2908 (0.7); 7.2892 (0.5); 7.2837 (0.8); 7.2829 (0.7); 7.2820 (0.9); 7.2813 (0.8); 7.2804 (0.9); 7.2797 (1.1); 7.2789 (1.1); 7.2781 (1.1); 7.2773 (1.3); 7.2765 (1.3); 7.2757 (1.5); 7.2749 (1.6); 7.2741 (1.6); 7.2733 (1.6); 7.2725 (1.8); 7.2717 (2.0); 7.2709 (2.0); 7.2701 (2.4); 7.2693 (2.7); 7.2685 (3.1); 7.2677 (3.7); 7.2669 (4.2); 7.2661 (4.9); 7.2653 (6.0); 7.2645 (7.8); 7.2637 (10.4); 7.2596 (611.7); 7.2556 (21.9); 7.2548 (17.5); 7.2540 (14.6); 7.2532 (12.2); 7.2524 (10.4); 7.2516 (8.8); 7.2507 (7.7); 7.2499 (7.2); 7.2491 (6.6); 7.2483 (6.2); 7.2475 (5.7); 7.2467 (5.4); 7.2459 (5.0); 7.2451 (4.6); 7.2443 (4.4); 7.2436 (4.3); 7.2428 (3.9); 7.2419 (3.8); 7.2412 (3.7); 7.2404 (3.5); 7.2396 (3.3); 7.2387 (3.3); 7.2380 (3.2); 7.2372 (3.1); 7.2364 (3.1); 7.2356 (3.0); 7.2340 (3.2); 7.2236 (1.9); 7.2197 (1.8); 7.2149 (1.6); 7.2089 (0.9); 7.2054 (0.9); 7.1974 (0.9); 7.1588 (1.1); 7.1467 (0.6); 6.9956 (3.5); 6.3483 (5.7); 6.3318 (5.9); 6.3215 (7.2); 6.3171 (6.7); 5.4442 (1.8); 5.4366 (1.7); 5.4226 (2.1); 5.4192 (2.2); 5.4148 (2.2); 5.4110 (2.2); 5.3973 (2.0); 5.3892 (1.9); 5.3466 (1.8); 5.3225 (3.9); 5.3005 (4.5); 5.2985 (7.9); 5.2763 (2.2); 4.4636 (0.8); 4.4582 (1.0); 4.4500 (1.0); 4.4407 (2.0); 4.4353 (2.1); 4.4273 (2.5); 4.4221 (2.7); 4.4183 (1.6); 4.4128 (1.3); 4.4062 (3.9); 4.4003 (4.2); 4.3835 (2.1); 4.3776 (2.0); 4.2771 (1.3); 4.2704 (1.3); 4.2615 (2.3); 4.2589 (1.7); 4.2543 (2.6); 4.2519 (1.9); 4.2456 (2.8); 4.2427 (2.1); 4.2375 (3.3); 4.2359 (3.3); 4.2314 (1.9); 4.2289 (2.9); 4.2215 (3.0); 4.2143 (2.0); 4.2111 (1.7); 4.2062 (1.3); 4.1980 (1.4); 4.1949 (1.4); 4.0885 (0.8); 4.0760 (1.1); 4.0707 (3.3); 4.0582 (4.0); 4.0530 (3.6); 4.0404 (4.1); 4.0354 (1.1); 4.0227 (1.1); 3.8910 (0.8); 3.8728 (3.4); 3.8548 (3.7); 3.8381 (3.7); 3.8201 (3.3); 3.8021 (0.9); 3.5512 (10.9); 3.5481 (11.4); 3.5391 (10.4); 3.5358 (13.1); 3.5324 (14.0); 3.5289 (15.1); 3.5247 (12.0); 2.6357 (1.1); 2.6304 (1.1); 2.6199 (1.3); 2.6143 (1.9); 2.6086 (1.3); 2.6038 (1.6); 2.5983 (3.0); 2.5926 (1.8); 2.5880 (1.8); 2.5822 (2.8); 2.5764 (2.4); 2.5660 (2.0); 2.5597 (2.3); 2.5535 (1.1); 2.5490 (1.1); 2.5434 (1.8); 2.5376 (1.2); 2.5271 (1.1); 2.5215 (0.9); 2.3182 (0.6); 2.3052 (0.6); 2.2927 (1.3); 2.2865 (0.7); 2.2800 (1.4); 2.2701 (1.5); 2.2673 (1.0); 2.2609 (1.4); 2.2575 (1.5); 2.2481 (1.3); 2.2447 (0.8); 2.2382 (1.2); 2.2322 (0.8); 2.2256 (1.2); 2.2125 (0.5); 2.1403 (1.4); 2.1333 (1.0); 2.1240 (0.8); 2.1157 (1.9); 2.1082 (0.7); 2.0995 (1.6); 2.0931 (1.6); 2.0834 (1.3); 2.0768 (1.7); 2.0674 (1.7); 2.0610 (1.3); 2.0520 (0.7); 2.0445 (1.4); 2.0365 (0.6); 2.0199 (0.6); 2.0049 (0.9); 1.6350 (4.8); 1.6038 (16.0); 1.6019 (15.7); 1.5857 (15.5); 1.5838 (15.0); 1.5707 (25.7); 1.5529 (24.8); 0.3309 (0.9); 0.2376 (0.8); 0.1579 (1.2); 0.1460 (0.7); 0.1262 (0.6); 0.0494 (1.0); 0.0160 (0.6); 0.0136 (0.7); 0.0128 (0.7); 0.0120 (0.8); 0.0112 (0.9); 0.0104 (1.1); 0.0080 (8.0); 0.0065 (2.2); 0.0057 (2.5); 0.0048 (3.1); 0.0040 (4.2); -0.0002 (278.7); -0.0057 (7.2); -0.0066 (6.3); - 0.0085 (10.3); -0.1497 (0.8)
Beispiel Nr. I.1-142:
   ¹H-NMR (400 MHz, CDCl₃ δ, ppm) 7.7293 (1.5); 7.7157 (1.8); 7.7104 (1.6); 7.6970 (1.7); 7.6551 (1.7); 7.6364 (1.7); 7.5952 (1.7); 7.5768 (1.7); 7.5184 (1.1); 7.3723 (1.6); 7.3620 (3.5); 7.3538 (1.9); 7.3496 (1.7); 7.3394 (3.6); 7.3314 (1.8); 7.2595 (203.2); 6.9955 (1.1); 6.3441 (2.4); 6.3296 (2.8); 6.3216 (4.8); 5.3188 (3.7); 5.3141 (3.8); 5.2962 (3.1); 5.2881 (3.6); 4.0857 (1.2); 4.0761 (1.4); 4.0677 (1.2); 4.0582 (1.4); 4.0321 (1.0); 4.0248 (0.9); 4.0097 (3.1); 4.0014 (3.7); 3.9890 (4.4); 3.9724 (1.4); 3.9661 (1.3); 3.9544 (1.4); 3.9362 (0.7); 3.9177 (1.3); 3.8996 (1.3); 3.5482 (4.2); 3.5452 (4.5); 3.5373 (4.3); 3.5342 (4.8); 3.5300 (4.4); 3.5266 (3.8); 3.5168 (4.1); 3.5138 (4.1); 2.1271 (0.7); 2.0046 (3.7); 1.6429 (5.1); 1.6367 (5.1); 1.6248 (5.4); 1.6186 (5.5); 1.5949 (10.6); 1.5770 (9.4); 1.1561 (8.6); 1.1476 (16.0); 1.1391 (7.4); 1.0342 (11.6); 0.9934 (5.7); 0.9625 (6.7); 0.0079 (2.3); -0.0002 (81.5); -0.0085 (2.7)
Beispiel Nr. I.1-144:
   ¹H-NMR (400 MHz, d₆-DMSO δ, ppm) 7.9102 (3.3); 7.8956 (3.4); 7.8617 (2.7); 7.8542 (3.4); 7.8374 (2.6); 7.8311 (3.1); 7.8257 (2.9); 7.7520 (3.9); 7.7333 (4.2); 7.7205 (2.4); 6.6041 (8.2); 6.5922 (3.8); 6.2417 (1.7); 6.2371 (3.0); 6.2316 (1.9); 6.2227 (2.8); 6.2171 (1.6); 5.7528 (9.5); 5.3130 (3.1); 5.2997 (3.2); 4.9603 (7.1); 4.9550 (11.9); 4.9506 (7.1); 4.4841 (1.9); 4.4715 (2.0); 4.4566 (2.6); 4.4440 (3.1); 4.4263 (1.5); 4.4163 (1.8); 4.4047 (1.2); 4.2561 (1.5); 4.2293 (1.6); 4.2109 (1.3); 4.1083 (2.2); 4.0906 (3.6); 4.0809 (2.0); 4.0731 (2.6); 4.0592 (3.8); 4.0326 (2.8); 3.4257 (20.9); 3.3084 (331.5); 3.0207 (1.8); 3.0033 (2.2); 2.9939 (1.5); 2.9770 (2.5); 2.9569 (2.3); 2.9484 (1.4); 2.9325 (1.4); 2.6691 (5.4); 2.5220 (41.3); 2.5085 (319.6); 2.5043 (620.2); 2.4999 (827.8); 2.4956 (601.1); 2.4914 (298.3); 2.4311 (3.2); 2.3866 (1.2); 2.3267 (5.5); 2.2853 (1.2); 2.2675 (1.1); 2.0711 (1.1); 1.4451 (16.0); 1.4329 (8.0); 1.4274 (15.2); 1.2368 (1.1); 0.0074 (5.2); -0.0002 (123.3); -0.0083 (6.7)
Beispiel Nr. I.1-145:
   ¹H-NMR (400 MHz, CDCl₃ δ, ppm) 7.6028 (3.4); 7.5839 (3.5); 7.5724 (3.3); 7.5581 (3.3); 7.5536 (3.6); 7.5393 (3.2); 7.5211 (3.5); 7.5025 (3.4); 7.4106 (6.4); 7.4029 (3.8); 7.3972 (3.8); 7.3879 (6.7); 7.3801 (3.8); 7.3745 (3.6); 7.2724 (0.8); 7.2610 (127.6); 7.2117 (0.5); 6.9970 (0.7); 6.3471 (4.6); 6.3281 (5.1); 6.3159 (5.1); 6.3001 (5.2); 4.6195 (0.6); 4.6109 (1.1); 4.6018 (1.3); 4.5921 (1.9); 4.5852 (1.7); 4.5742 (2.3); 4.5673 (1.9); 4.5634 (1.4); 4.5561 (2.2); 4.5491 (1.4); 4.5377 (0.9); 4.2747 (1.4); 4.2672 (1.4); 4.2539 (1.6); 4.2469 (1.9); 4.2443 (2.3); 4.2369 (2.4); 4.2308 (3.1); 4.2234 (3.8); 4.2164 (2.3); 4.2080 (2.4); 4.2005 (4.1); 4.1933 (2.3); 4.0909 (2.5); 4.0893 (2.4); 4.0808 (2.4); 4.0759 (2.8); 4.0637 (4.2); 4.0604 (2.4); 4.0506 (1.9); 4.0456 (3.8); 4.0334 (2.9); 4.0155 (1.6); 3.9946 (0.9); 3.9907 (1.0); 3.9856 (0.9); 3.9766 (3.0); 3.9728 (3.2); 3.9677 (2.9); 3.9589 (3.6); 3.9550 (3.4); 3.9499 (3.0); 3.9418 (3.6); 3.9371 (1.2); 3.9321 (1.0); 3.9239 (3.1); 3.9062 (0.9); 3.5502 (7.9); 3.5470 (9.2); 3.5419 (10.3); 3.5355 (16.0); 2.5425 (1.8); 2.5400 (2.0); 2.5373 (2.1); 2.5352 (1.9); 2.5282 (1.8); 2.5253 (2.3); 2.5187 (7.0); 2.5129 (4.5); 2.5085 (2.4); 2.5017 (3.7); 2.4971 (5.2); 2.4873 (4.0); 2.4777 (2.2); 2.4752 (2.3); 2.4650 (2.0); 2.4513 (0.6); 2.4412 (0.5); 2.3289 (0.5); 2.3230 (0.5); 2.3104 (0.9); 2.3046 (0.9); 2.2964 (0.7); 2.2906 (1.3); 2.2843 (0.9); 2.2787 (1.4); 2.2761 (1.4); 2.2731 (1.3); 2.2595 (1.6); 2.2571 (1.7); 2.2434 (1.2); 2.2404 (1.4); 2.2375 (1.1); 2.2344 (1.0); 2.2273 (1.3); 2.2241 (1.1); 2.2210 (1.2); 2.2080 (1.0); 2.2047 (1.1); 2.1857 (0.5); 1.8931 (0.5); 1.8883 (0.5); 1.8752 (0.6); 1.8695 (1.0); 1.8664 (0.8); 1.8595 (0.8); 1.8535 (1.2); 1.8476 (1.2); 1.8421 (1.5); 1.8371 (1.7); 1.8332 (1.6); 1.8293 (1.4); 1.8209 (1.6); 1.8147 (2.0); 1.8093 (1.5); 1.8032 (1.1); 1.7969 (1.5); 1.7910 (1.2); 1.7822 (1.1); 1.7643 (0.7); 1.7582 (0.8); 1.5605 (9.7); 1.5536 (10.2); 1.5445 (14.7); 1.5428 (20.7); 1.5358 (11.0); 1.5267 (12.6); 1.5252 (11.7); 0.0079 (1.5); -0.0002 (51.8); -0.0085 (2.2)
Beispiel Nr. I.1-151:
   ¹H-NMR (400 MHz, d₆-DMSO δ, ppm) 7.8607 (8.7); 7.8370 (9.0); 7.7546 (3.9); 7.7473 (4.3); 7.7354 (4.2); 7.7282 (4.2); 7.3034 (2.4); 6.8591 (2.5); 6.6152 (15.5); 4.2586 (0.9); 4.2419 (1.8); 4.2315 (2.5); 4.2150 (4.1); 4.1987 (4.2); 4.1827 (5.0); 4.1666 (2.6); 4.1556 (2.2); 4.1395 (1.1); 4.0575 (1.3); 4.0407 (5.4); 4.0229 (5.4); 4.0051 (1.4); 3.4363 (17.6); 3.3685 (67.3); 2.6854 (1.5); 2.5388 (5.0); 2.5341 (7.2); 2.5254 (89.1); 2.5208 (186.5); 2.5162 (259.7); 2.5116 (177.7); 2.5071 (79.7); 2.3991 (0.8); 2.3770 (3.0); 2.3696 (3.0); 2.3610 (6.6); 2.3532 (5.8); 2.3448 (3.5); 2.3370 (3.4); 2.0878 (3.1); 1.4301 (16.0); 1.4130 (15.8); 0.0157 (1.0)
Beispiel Nr. I.1-152:
   ¹H-NMR (400 MHz, CDCl₃ δ, ppm) δ= 7.4893 (1.7); 7.4705 (1.7); 7.4609 (1.6); 7.4421 (1.6); 7.3982 (1.7); 7.3909 (1.7); 7.3755 (1.7); 7.3682 (1.7); 7.2598 (84.7); 6.3803 (2.8); 6.3665 (2.9); 4.3498 (0.6); 4.3444 (0.7); 4.3377 (0.8); 4.3287 (1.6); 4.3226 (1.4); 4.3135 (1.4); 4.3081 (1.4); 4.2948 (1.0); 4.2888 (0.8); 4.2744 (0.6); 3.9720 (0.5); 3.9674 (1.3); 3.9543 (1.4); 3.9495 (1.4); 3.9364 (1.3); 3.5750 (6.2); 3.5724 (6.2); 2.6723 (4.9); 2.6651 (5.2); 2.6604 (5.1); 2.6532 (4.6); 2.2788 (0.7); 2.2710 (0.5); 2.2639 (0.9); 2.2566 (1.0); 2.2446 (0.7); 2.2271 (0.7); 2.2224 (0.6); 2.2120 (0.6); 2.2071 (0.8); 2.1883 (0.6); 1.5429 (16.0); 1.5334 (5.1); 1.5256 (5.2); 1.5155 (4.8); 1.5077 (4.9); 0.0079 (2.0); -0.0002 (48.4); - 0.0085 (1.6)
Beispiel Nr. I.1-153:
   ¹H-NMR (400 MHz, CDCl₃ δ, ppm) δ= 7.5194 (0.7); 7.5029 (4.1); 7.4840 (4.2); 7.4740 (4.0); 7.4552 (4.0); 7.3953 (4.3); 7.3871 (4.2); 7.3726 (4.3); 7.3644 (4.2); 7.3112 (0.8); 7.2606 (115.1); 7.2118 (0.8); 6.9965 (0.7); 6.3739 (6.5); 6.3542 (7.0); 6.1415 (0.7); 6.0210 (0.7); 4.3609 (0.5); 4.3541 (0.9); 4.3453 (1.4); 4.3422 (1.8); 4.3387 (0.9); 4.3316 (2.4); 4.3268 (3.6); 4.3179 (4.4); 4.3134 (2.8); 4.3054 (2.1); 4.3030 (2.1); 4.2991 (2.0); 4.2906 (2.0); 4.2864 (2.2); 4.2784 (0.7); 4.2716 (1.8); 4.2635 (0.6); 4.2592 (0.7); 4.2443 (0.6); 3.9695 (0.9); 3.9515 (3.5); 3.9368 (3.5); 3.9336 (3.8); 3.9189 (3.4); 3.9011 (0.9); 3.5717 (16.0); 3.2090 (1.0); 3.2042 (0.6); 3.1908 (3.3); 3.1861 (1.8); 3.1764 (3.7); 3.1725 (4.5); 3.1680 (2.0); 3.1587 (3.3); 3.1542 (2.7); 3.1405 (1.1); 3.1361 (0.6); 2.2940 (1.2); 2.2789 (1.4); 2.2712 (1.2); 2.2638 (0.7); 2.2564 (3.3); 2.2414 (3.5); 2.2334 (1.4); 2.2265 (1.2); 2.2179 (1.6); 2.2152 (1.8); 2.2124 (1.6); 2.1998 (1.5); 2.1962 (2.0); 2.1932 (1.8); 2.1774 (1.9); 2.1591 (0.7); 2.1557 (0.7); 2.1399 (0.5); 1.5580 (12.2); 1.5317 (11.8); 1.5237 (12.8); 1.5138 (11.9); 1.5058 (12.3); 1.0702 (7.7); 1.0668 (7.5); 1.0520 (15.9); 1.0486 (15.3); 1.0338 (7.6); 1.0304 (7.3); 0.0080 (2.0); -0.0002 (67.3); -0.0085 (2.6)
Beispiel Nr. I.1-182:
   ¹H-NMR (400 MHz, CDCl₃ δ, ppm) 7.5411 (5.0); 7.5393 (5.0); 7.5223 (5.1); 7.5205 (5.3); 7.4091 (7.8); 7.3865 (7.8); 7.3100 (1.1); 7.2599 (286.8); 6.9959 (1.6); 6.3716 (6.9); 6.3620 (6.8); 5.9964 (1.0); 4.5648 (1.4); 4.5548 (1.1); 4.5267 (4.7); 4.5171 (5.2); 4.4983 (7.2); 4.4599 (1.6); 3.9302 (1.0); 3.9253 (1.0); 3.9124 (3.5); 3.9075 (3.5); 3.8946 (3.6); 3.8897 (3.5); 3.8769 (1.1); 3.8719 (1.0); 3.5669 (16.0); 2.7704 (10.9); 2.7638 (11.2); 2.7582 (11.4); 2.7516 (10.6); 2.0052 (2.4); 1.6077 (2.6); 1.5674 (19.1); 1.5496 (18.6); 0.1463 (0.6); 0.0499 (0.5); 0.0079 (4.9); -0.0002 (154.1); -0.0085 (5.1); -0.1495 (0.6)
Beispiel Nr. I.1-183:
   ¹H-NMR (400 MHz, CDCl₃ δ, ppm) 7.5291 (4.3); 7.5279 (4.4); 7.5197 (0.9); 7.5105 (4.3); 7.5092 (4.4); 7.4048 (7.0); 7.3822 (7.1); 7.3105 (0.7); 7.2607 (111.8); 7.2269 (0.7); 7.2126 (0.8); 6.9967 (0.6); 6.3651 (6.0); 6.3584 (5.8); 6.0674 (0.9); 4.5655 (1.7); 4.5512 (1.2); 4.5276 (4.6); 4.5136 (5.1); 4.4919 (5.7); 4.4887 (5.2); 4.4544 (1.5); 4.4508 (1.8); 3.9521 (0.9); 3.9495 (0.9); 3.9344 (3.2); 3.9316 (3.2); 3.9166 (3.3); 3.9138 (3.2); 3.8987 (1.0); 3.8961 (1.0); 3.5655 (11.1); 3.5624 (16.0); 3.5591 (11.8); 3.3062 (1.0); 3.2920 (1.3); 3.2879 (2.4); 3.2740 (3.4); 3.2696 (2.6); 3.2604 (2.5); 3.2559 (3.4); 3.2423 (2.4); 3.2379 (1.4); 3.2242 (1.1); 3.2090 (0.5); 2.0049 (1.9); 1.6934 (1.7); 1.5692 (11.6); 1.5662 (11.8); 1.5514 (11.6); 1.5484 (11.7); 1.1152 (7.1); 1.1134 (7.2); 1.0970 (14.4); 1.0952 (14.8); 1.0788 (7.0); 1.0770 (7.1); 0.0080 (1.3); -0.0002 (42.3); -0.0085 (1.4)
Beispiel Nr. I.1-184:
   ¹H-NMR (400 MHz, CDCl₃ δ, ppm) 7.5193 (3.8); 7.5163 (3.4); 7.5007 (3.3); 7.4977 (3.4); 7.4030 (6.1); 7.3804 (6.1); 7.2605 (97.9); 6.9965 (0.5); 6.3586 (7.2); 5.9415 (0.8); 4.5442 (1.7); 4.5297 (1.1); 4.5068 (4.8); 4.4926 (5.4); 4.4757 (5.5); 4.4711 (5.0); 4.4385 (1.2); 4.4336 (1.7); 4.0924 (0.7); 4.0892 (0.9); 4.0760 (1.0); 4.0727 (1.5); 4.0695 (0.8); 4.0594 (0.8); 4.0563 (1.5); 4.0530 (1.0); 4.0398 (0.9); 4.0366 (0.7); 3.9694 (1.3); 3.9516 (4.8); 3.9338 (5.0); 3.9160 (1.3); 3.5597 (13.2); 2.0044 (0.7); 1.5959 (0.7); 1.5680 (9.9); 1.5640 (10.0); 1.5502 (9.7); 1.5462 (9.7); 1.1309 (12.1); 1.1229 (9.3); 1.1131 (16.0); 1.1066 (9.3); 1.0957 (8.6); 0.0080 (1.1); -0.0002 (35.1); -0.0085 (1.0)
Beispiel Nr. I.1-231:
   ¹H-NMR (400 MHz, CDCl₃ δ, ppm) δ= 7.5141 (2.0); 7.5116 (1.9); 7.4954 (2.0); 7.4928 (1.9); 7.3690 (2.7); 7.3463 (2.7); 7.2608 (35.2); 6.3421 (5.3); 4.4087 (0.9); 4.3984 (0.9); 4.3916 (0.5); 4.3812 (1.9); 4.3633 (1.4); 4.3542 (0.6); 4.3461 (1.3); 4.3379 (1.3); 4.3298 (0.7); 4.3214 (0.7); 4.3188 (0.6); 4.3105 (0.6); 3.8958 (0.7); 3.8779 (2.5); 3.8601 (2.6); 3.8422 (0.7); 3.5468 (7.9); 2.9592 (12.4); 2.9563 (12.1); 2.9134 (16.0); 2.5657 (2.1); 2.5491 (4.2); 2.5323 (2.0); 1.5641 (4.9); 1.5242 (5.9); 1.5219 (5.9); 1.5063 (5.9); 1.5040 (5.8); 0.0080 (0.7); -0.0002 (20.1); -0.0085 (0.7)
Beispiel Nr. I.1-241:
   ¹H-NMR (400 MHz, CDCl₃ δ, ppm) 7.6956 (1.7); 7.6767 (1.7); 7.6665 (1.7); 7.6478 (1.6); 7.5187 (0.7); 7.3619 (1.8); 7.3510 (1.8); 7.3391 (1.8); 7.3283 (1.7); 7.2599 (127.1); 6.9958 (0.7); 6.3300 (2.7); 6.3194 (2.8); 4.7569 (0.9); 4.7365 (0.9); 4.7206 (1.6); 4.7002 (1.7); 4.6251 (1.7); 4.6217 (1.8); 4.5889 (1.0); 4.5853 (1.0); 4.0410 (1.5); 4.0242 (1.8); 4.0065 (1.5); 3.5419 (7.8); 3.5390 (8.1); 2.9245 (16.0); 2.9030 (7.7); 2.8991 (7.4); 1.6152 (4.8); 1.6033 (5.2); 1.5972 (5.0); 1.5854 (5.0); 1.5475 (4.8); 1.2553 (0.6); 0.0079 (1.2); -0.0002 (35.0); -0.0085 (1.1)
Beispiel Nr. I.1-242:
   ¹H-NMR (400 MHz, CDCl₃ δ, ppm) 7.7081 (1.6); 7.6892 (1.6); 7.6689 (1.6); 7.6502 (1.6); 7.5188 (0.6); 7.3631 (1.9); 7.3532 (1.7); 7.3402 (1.7); 7.3305 (1.7); 7.2599 (103.8); 6.9959 (0.6); 6.3282 (2.7); 6.3157 (2.6); 4.8718 (0.5); 4.8324 (1.2); 4.8170 (1.4); 4.7909 (1.5); 4.7871 (1.5); 4.7392 (0.6); 4.7346 (0.8); 4.0084 (1.3); 3.9904 (1.4); 3.9877 (1.4); 3.9697 (1.4); 3.7169 (16.0); 3.7149 (14.8); 3.5422 (7.6); 3.5394 (8.1); 3.5110 (0.6); 3.1469 (1.3); 3.1351 (13.9); 3.1168 (1.3); 2.0053 (2.2); 1.6134 (5.0); 1.6027 (5.5); 1.5954 (5.6); 1.5847 (5.6); 1.5771 (1.7); 1.5745 (1.5); 1.5589 (1.1); 1.5565 (1.0); 0.0080 (1.7); -0.0002 (56.2); -0.0085 (1.9)
Beispiel Nr. I.1-251:
   ¹H-NMR (400 MHz, CDCl₃ δ, ppm) 7.5432 (3.2); 7.5322 (3.2); 7.5247 (3.4); 7.5203 (1.2); 7.5137 (6.0); 7.5105 (3.2); 7.4952 (3.0); 7.4918 (2.9); 7.3770 (2.8); 7.3728 (3.0); 7.3615 (3.6); 7.3584 (3.8); 7.3542 (3.2); 7.3501 (3.2); 7.3389 (3.6); 7.3358 (3.7); 7.2727 (0.5); 7.2719 (0.5); 7.2711 (0.6); 7.2703 (0.6); 7.2694 (0.8); 7.2687 (0.9); 7.2679 (1.1); 7.2670 (1.3); 7.2662 (1.7); 7.2654 (2.3); 7.2614 (136.5); 7.2573 (4.1); 7.2565 (3.3); 7.2557 (2.8); 7.2549 (2.4); 7.2541 (2.1); 7.2533 (1.8); 7.2525 (1.5); 7.2517 (1.4); 7.2509 (1.2); 7.2501 (1.1); 7.2493 (1.0); 7.2485 (0.9); 7.2477 (0.9); 7.2469 (0.8); 7.2461 (0.8); 7.2453 (0.7); 7.2445 (0.7); 7.2437 (0.7); 7.2429 (0.7); 7.2421 (0.6); 7.2413 (0.6); 7.2405 (0.6); 7.2397 (0.6); 7.2389 (0.6); 7.2357 (0.5); 7.2167 (0.5); 6.9973 (0.8); 6.3683 (4.9); 6.3574 (5.3); 6.3518 (5.0); 6.3487 (4.8); 5.7628 (0.8); 5.6816 (1.3); 4.4584 (0.9); 4.4501 (1.0); 4.4306 (1.4); 4.4223 (1.5); 4.4001 (0.7); 4.3914 (0.8); 4.3771 (0.9); 4.3726 (1.9); 4.3671 (1.6); 4.3638 (2.0); 4.3575 (0.8); 4.3495 (1.6); 4.3400 (4.0); 4.3294 (4.4); 4.3181 (2.7); 4.3168 (2.8); 4.3138 (1.5); 4.3053 (2.4); 4.3015 (3.3); 4.2884 (3.0); 4.2777 (0.9); 4.2740 (1.1); 4.2605 (1.0); 4.1304 (0.8); 4.1125 (0.8); 3.8846 (0.7); 3.8665 (2.6); 3.8534 (0.8); 3.8485 (3.0); 3.8461 (3.0); 3.8355 (2.6); 3.8327 (2.9); 3.8280 (3.0); 3.8174 (2.6); 3.8147 (2.7); 3.8100 (1.0); 3.7994 (0.8); 3.7968 (0.8); 3.5613 (7.8); 3.5581 (12.0); 3.5549 (16.0); 3.5517 (15.0); 3.5483 (9.2); 3.5468 (8.8); 3.5433 (7.6); 3.2906 (2.5); 3.2760 (3.4); 3.2743 (4.2); 3.2703 (2.8); 3.2677 (1.9); 3.2545 (3.6); 3.2482 (1.2); 3.2287 (1.8); 3.2122 (1.6); 3.1927 (1.2); 3.1861 (0.7); 3.1834 (0.7); 3.1726 (0.8); 3.1699 (0.9); 3.1638 (1.2); 3.1608 (1.0); 3.1548 (0.7); 3.1442 (0.9); 3.1413 (1.0); 3.1346 (0.9); 3.1321 (0.9); 2.6354 (0.9); 2.6259 (1.4); 2.6154 (1.7); 2.6123 (1.7); 2.6029 (1.8); 2.5925 (1.3); 2.5821 (1.0); 2.2307 (0.7); 2.2207 (1.0); 2.2148 (1.0); 2.2109 (0.9); 2.2047 (1.2); 2.2013 (1.2); 2.1973 (1.8); 2.1917 (1.2); 2.1883 (1.4); 2.1819 (1.4); 2.1779 (1.2); 2.1735 (1.4); 2.1686 (1.0); 2.1644 (1.0); 2.1592 (0.8); 2.1547 (0.5); 2.0436 (4.0); 1.9229 (1.0); 1.9104 (0.9); 1.9064 (1.1); 1.9028 (1.1); 1.8899 (1.9); 1.8828 (0.7); 1.8778 (0.8); 1.8698 (1.8); 1.8555 (1.4); 1.8501 (0.9); 1.8364 (1.0); 1.5804 (6.6); 1.5558 (10.0); 1.5517 (14.7); 1.5501 (13.5); 1.5378 (10.4); 1.5349 (12.7); 1.5336 (14.7); 1.5320 (13.6); 1.2764 (1.4); 1.2586 (3.3); 1.2407 (1.3); 0.0079 (1.9); 0.0054 (0.5); 0.0046 (0.7); -0.0002 (70.0); - 0.0052 (1.9); -0.0060 (1.6); -0.0069 (1.5); -0.0085 (2.7); -0.0116 (0.7); -0.0124 (0.7); -0.0140 (0.6); - 0.0148 (0.5)
Beispiel Nr. I.1-254:
   ¹H-NMR (400 MHz, CDCl₃ δ, ppm) 7.5189 (1.0); 7.5114 (2.0); 7.4920 (4.5); 7.4774 (2.7); 7.4726 (3.0); 7.4179 (2.4); 7.4094 (2.8); 7.4047 (2.8); 7.3952 (2.7); 7.3867 (2.8); 7.3820 (2.8); 7.2600 (151.9); 6.9959 (0.9); 6.3656 (5.2); 6.3617 (5.0); 6.3577 (4.3); 6.3449 (4.3); 5.6570 (1.2); 5.6283 (1.3); 5.6085 (1.0); 5.5535 (0.9); 5.2761 (1.7); 5.2615 (3.6); 5.2466 (2.8); 5.2309 (0.7); 4.0852 (0.5); 4.0674 (1.9); 4.0543 (2.1); 4.0498 (2.1); 4.0366 (2.0); 4.0192 (0.6); 3.9183 (0.6); 3.9010 (1.9); 3.8980 (1.8); 3.8830 (1.9); 3.8649 (0.6); 3.6418 (1.0); 3.6368 (1.0); 3.6283 (1.1); 3.6235 (1.0); 3.6126 (1.3); 3.6077 (1.3); 3.5994 (2.4); 3.5861 (1.5); 3.5805 (1.6); 3.5629 (14.5); 3.5540 (16.0); 3.5382 (1.6); 3.2446 (1.2); 3.2316 (1.1); 3.2161 (1.1); 3.2023 (1.0); 3.0583 (1.4); 3.0283 (1.3); 3.0107 (1.4); 2.9816 (1.3); 2.6486 (1.2); 2.6324 (1.3); 2.6183 (1.0); 2.6106 (1.2); 2.6026 (2.4); 2.5941 (1.3); 2.5878 (1.6); 2.5737 (1.3); 2.5662 (1.5); 2.5572 (1.3); 2.5498 (1.3); 2.3091 (1.6); 2.2962 (1.6); 2.2619 (2.4); 2.2515 (2.4); 2.2190 (1.0); 2.2070 (0.9); 2.0045 (1.9); 1.5459 (12.2); 1.5284 (16.1); 1.5147 (10.1); -0.0002 (54.6)
Beispiel Nr. I.1-255:
   ¹H-NMR (400 MHz, CDCl₃ δ, ppm) 7.5387 (3.0); 7.5194 (4.1); 7.5151 (2.9); 7.4963 (2.7); 7.4766 (3.1); 7.4721 (2.8); 7.4579 (3.3); 7.4534 (3.0); 7.4499 (3.8); 7.4256 (6.8); 7.4021 (3.7); 7.2601 (297.0); 7.2104 (1.1); 6.9961 (1.6); 6.3797 (4.2); 6.3614 (8.0); 6.3448 (5.0); 5.8476 (0.9); 5.7945 (1.7); 5.7683 (0.9); 4.1754 (1.3); 4.1720 (1.4); 4.1658 (1.5); 4.1619 (1.6); 4.1475 (1.6); 4.1444 (1.8); 4.1379 (1.7); 4.1343 (1.8); 4.0557 (1.1); 4.0475 (1.1); 4.0268 (2.4); 4.0192 (3.4); 4.0023 (5.7); 3.9981 (2.7); 3.9899 (2.2); 3.9846 (5.7); 3.9806 (2.7); 3.9669 (1.7); 3.9628 (2.4); 3.9451 (0.6); 3.8742 (1.7); 3.8711 (1.4); 3.8585 (2.0); 3.8552 (1.7); 3.8459 (1.2); 3.8427 (1.1); 3.8301 (1.5); 3.8269 (1.4); 3.8040 (1.2); 3.7878 (1.7); 3.7750 (1.8); 3.7597 (3.4); 3.7471 (1.0); 3.7318 (2.2); 3.7146 (1.2); 3.7004 (1.9); 3.6932 (1.8); 3.6858 (1.8); 3.6729 (1.2); 3.5652 (7.4); 3.5622 (8.0); 3.5510 (16.0); 2.3759 (0.6); 2.3505 (1.9); 2.3330 (1.9); 2.3255 (1.6); 2.3082 (2.7); 2.3008 (1.3); 2.2949 (2.1); 2.2911 (2.3); 2.2880 (3.1); 2.2834 (2.9); 2.2767 (1.7); 2.2719 (3.3); 2.2700 (3.1); 2.2662 (3.1); 2.2597 (1.1); 2.2555 (1.8); 2.2498 (1.8); 2.2457 (0.8); 2.2413 (0.8); 2.2323 (1.2); 2.2288 (1.5); 2.2226 (1.1); 2.2146 (1.4); 2.2073 (1.5); 2.1976 (1.2); 2.1946 (1.3); 2.1867 (1.4); 2.1788 (1.6); 2.1749 (1.7); 2.1662 (0.7); 2.1623 (0.9); 2.1561 (1.2); 2.0048 (2.3); 1.8045 (0.9); 1.7920 (1.1); 1.7825 (1.2); 1.7791 (1.3); 1.7705 (1.7); 1.7625 (1.8); 1.7549 (1.5); 1.7500 (1.6); 1.7414 (1.3); 1.7331 (1.7); 1.7247 (1.1); 1.7191 (1.0); 1.7147 (1.0); 1.7011 (1.0); 1.6455 (0.6); 1.5524 (8.4); 1.5434 (11.7); 1.5400 (13.3); 1.5347 (9.3); 1.5257 (11.5); 1.5231 (12.6); 0.0080 (3.3); - 0.0002 (108.7); -0.0085 (3.3)
Beispiel Nr. I.1-293:
   ¹H-NMR (400 MHz, CDCl₃ δ, ppm) 7.4868 (3.5); 7.4794 (3.6); 7.4683 (3.6); 7.4608 (3.6); 7.3655 (4.9); 7.3429 (5.0); 7.2717 (0.5); 7.2709 (0.6); 7.2701 (0.6); 7.2693 (0.7); 7.2685 (0.8); 7.2677 (1.0); 7.2668 (1.1); 7.2660 (1.4); 7.2652 (1.7); 7.2610 (83.8); 6.3517 (7.4); 4.1846 (2.8); 4.1835 (2.9); 4.1789 (2.4); 4.1766 (2.6); 4.1654 (6.9); 4.1586 (10.2); 4.1520 (4.8); 4.1476 (7.3); 4.1409 (7.2); 4.1298 (2.3); 4.1231 (2.4); 4.1148 (4.2); 4.0992 (3.8); 4.0878 (1.9); 4.0722 (2.1); 3.8702 (0.7); 3.8597 (0.8); 3.8522 (2.9); 3.8418 (3.0); 3.8343 (3.0); 3.8239 (3.8); 3.8160 (1.7); 3.8137 (1.7); 3.8055 (2.1); 3.7945 (2.4); 3.7842 (2.5); 3.7745 (1.9); 3.7654 (1.7); 3.7555 (0.9); 3.5597 (9.4); 3.5565 (13.4); 3.5530 (10.0); 3.4871 (1.3); 3.4804 (2.1); 3.4762 (1.1); 3.4737 (1.1); 3.4700 (1.0); 3.4595 (1.8); 3.4572 (1.9); 3.4528 (2.5); 3.4505 (2.7); 3.4464 (1.9); 3.4433 (1.6); 3.4300 (1.4); 3.4233 (1.7); 3.4192 (1.0); 3.4166 (0.8); 3.4130 (0.7); 2.0034 (1.1); 1.9935 (0.8); 1.9785 (2.0); 1.9587 (0.9); 1.9505 (1.3); 1.9448 (1.2); 1.5769 (2.3); 1.5727 (2.3); 1.5679 (2.4); 1.5505 (1.3); 1.5406 (1.5); 1.5308 (2.2); 1.5220 (13.8); 1.5209 (14.0); 1.5041 (14.7); 1.5032 (14.4); 1.4960 (1.8); 1.4922 (1.4); 1.4846 (1.2); 1.4686 (1.2); 1.4574 (0.9); 1.2666 (7.4); 1.2616 (7.5); 1.2488 (15.7); 1.2439 (16.0); 1.2310 (7.1); 1.2260 (7.3); 0.0080 (1.1); 0.0048 (0.5); 0.0040 (0.7); - 0.0002 (32.9); -0.0085 (0.8)
Beispiel Nr. I.2-2:
   ¹H-NMR (400 MHz, CDCl₃ δ, ppm) 7.6080 (0.6); 7.5183 (1.2); 7.4793 (1.8); 7.4715 (1.8); 7.4607 (2.0); 7.4528 (1.9); 7.3708 (2.0); 7.3670 (2.2); 7.3481 (1.9); 7.3444 (2.2); 7.2787 (0.7); 7.2594 (216.1); 7.2456 (0.6); 7.2081 (0.5); 6.9955 (1.2); 6.3517 (3.5); 6.3483 (3.5); 5.2985 (0.8); 4.3495 (0.8); 4.3427 (0.8); 4.3324 (1.1); 4.3258 (0.9); 4.3111 (1.2); 4.2965 (1.6); 4.2837 (1.0); 4.2683 (0.5); 3.7022 (0.6); 3.6932 (1.0); 3.6737 (2.0); 3.6649 (15.7); 3.6635 (16.0); 3.6403 (0.5); 3.5503 (8.5); 2.5888 (1.5); 2.5804 (1.3); 2.5732 (2.7); 2.5645 (2.0); 2.5576 (1.6); 2.5478 (1.0); 1.9955 (0.5); 1.9763 (0.7); 1.9604 (1.0); 1.9422 (1.2); 1.9226 (0.8); 1.8682 (0.8); 1.8515 (1.2); 1.8332 (1.4); 1.8150 (1.0); 1.7986 (0.6); 1.6535 (1.3); 1.0666 (4.9); 1.0482 (10.7); 1.0298 (4.6); 0.0080 (2.5); -0.0002 (74.9); -0.0085 (2.3)
Beispiel Nr. I.2-32:
   ¹H-NMR (400 MHz, CDCl₃ δ, ppm) 7.5180 (7.0); 7.4695 (2.1); 7.4534 (2.4); 7.4346 (2.0); 7.3794 (1.0); 7.3549 (2.3); 7.3511 (2.6); 7.3322 (3.0); 7.3287 (2.9); 7.2998 (2.8); 7.2966 (3.0); 7.2918 (4.6); 7.2871 (4.9); 7.2839 (5.8); 7.2831 (5.7); 7.2814 (6.8); 7.2807 (7.0); 7.2799 (7.2); 7.2791 (7.5); 7.2783 (8.0); 7.2775 (8.2); 7.2767 (8.8); 7.2759 (9.8); 7.2751 (10.3); 7.2743 (10.8); 7.2735 (11.5); 7.2727 (12.4); 7.2719 (13.3); 7.2711 (14.6); 7.2703 (16.0); 7.2695 (17.6); 7.2687 (19.6); 7.2679 (21.6); 7.2671 (24.0); 7.2663 (27.3); 7.2591 (1212.4); 7.2558 (43.1); 7.2550 (33.0); 7.2542 (26.8); 7.2533 (23.0); 7.2525 (19.8); 7.2517 (18.2); 7.2509 (16.2); 7.2501 (14.8); 7.2493 (13.7); 7.2485 (12.7); 7.2477 (12.1); 7.2469 (11.5); 7.2462 (10.9); 7.2454 (10.1); 7.2446 (9.6); 7.2437 (9.1); 7.2430 (8.8); 7.2421 (8.4); 7.2414 (8.5); 7.2406 (7.9); 7.2398 (7.6); 7.2390 (7.4); 7.2382 (7.2); 7.2374 (6.9); 7.2366 (6.8); 7.2358 (6.8); 7.2350 (6.8); 7.2342 (6.4); 7.2334 (6.4); 7.2318 (6.4); 7.2295 (5.8); 7.2247 (6.6); 7.2152 (5.8); 7.2087 (6.5); 7.1400 (2.6); 7.1170 (1.5); 6.9951 (6.9); 6.3501 (3.5); 6.2796 (0.8); 4.1771 (1.4); 4.1667 (1.6); 4.1503 (2.2); 4.1399 (2.4); 4.0479 (2.4); 4.0268 (2.3); 4.0212 (1.7); 4.0001 (1.8); 3.6845 (1.1); 3.6678 (2.1); 3.6382 (15.7); 3.6327 (16.0); 3.5539 (6.0); 3.5505 (6.1); 3.5192 (1.5); 2.0048 (2.0); 1.9486 (1.0); 1.9295 (1.2); 1.9091 (0.8); 1.8721 (0.8); 1.8539 (1.2); 1.8377 (1.5); 1.8191 (1.0); 1.5275 (180.9); 1.2545 (1.0); 1.1668 (11.1); 1.1643 (10.5); 1.1383 (10.9); 1.1361 (10.9); 1.0508 (4.6); 1.0325 (9.5); 1.0141 (4.1); 0.1459 (1.6); 0.0365 (1.0); 0.0142 (4.2); 0.0079 (19.7); 0.0062 (12.4); 0.0046 (20.3); -0.0002 (470.3); - 0.0052 (13.0); -0.0060 (11.4); -0.0068 (10.5); -0.0085 (18.6); -0.0116 (5.8); -0.0123 (5.6); -0.0179 (3.9); -0.0235 (3.2); -0.0275 (2.9); -0.0346 (2.9); -0.0498 (2.8); -0.1495 (2.0)
Beispiel Nr. I.2-46:
   ¹H-NMR (400 MHz, CDCl₃ δ, ppm) δ= 7.3818 (0.6); 7.3633 (0.8); 7.3519 (0.6); 7.3461 (1.8); 7.3427 (1.9); 7.3378 (0.8); 7.3236 (1.8); 7.3214 (1.5); 7.2593 (69.0); 6.3157 (0.8); 6.2865 (0.8); 5.1265 (2.2); 4.0533 (0.5); 4.0400 (0.6); 3.5182 (1.2); 3.5154 (1.3); 3.4994 (1.2); 3.4965 (1.2); 2.0046 (0.8); 1.5337 (16.0); 1.1242 (1.3); 1.1057 (2.7); 1.0872 (1.2); 0.0079 (1.2); -0.0002 (34.5); -0.0085 (1.3)
Beispiel Nr. I.4-43:
   ¹H-NMR (400 MHz, CDCl₃ δ, ppm) 7.5932 (0.5); 7.3734 (0.5); 7.3507 (0.5); 7.2646 (0.6); 7.2597 (46.5); 6.3405 (1.1); 4.5843 (0.7); 4.5710 (0.8); 4.5270 (0.8); 4.5203 (0.7); 4.1612 (0.8); 4.1433 (0.8); 3.5481 (1.8); 3.5444 (1.4); 1.5388 (16.0); 1.2627 (1.2); 1.2613 (1.3); 1.2448 (2.4); 1.2434 (2.6); 1.2269 (1.1); 1.2255 (1.2); 0.9781 (1.2); 0.9754 (1.4); 0.9678 (2.3); 0.9619 (1.4); 0.9591 (1.4); 0.9518 (2.0); 0.0079 (0.6); -0.0002 (19.5); -0.0085 (0.6)
Beispiel Nr. I.5-2:
   ¹H-NMR (400 MHz, CDCl₃ δ, ppm) δ= 7.5185 (1.0); 7.3596 (0.7); 7.3566 (0.5); 7.3370 (0.6); 7.3340 (0.5); 7.2742 (0.5); 7.2734 (0.5); 7.2726 (0.6); 7.2718 (0.6); 7.2710 (0.7); 7.2702 (0.8); 7.2694 (0.9); 7.2686 (1.1); 7.2678 (1.2); 7.2670 (1.4); 7.2662 (1.6); 7.2654 (2.0); 7.2645 (2.4); 7.2597 (179.3); 7.2524 (1.0); 7.2516 (0.7); 7.2508 (0.6); 7.2500 (0.6); 7.2492 (0.6); 6.9956 (1.0); 6.3475 (1.2); 6.3434 (1.2); 3.6602 (6.4); 3.6584 (4.5); 3.6438 (0.5); 3.6403 (0.5); 3.5469 (3.0); 2.5634 (0.8); 2.5557 (0.6); 1.5338 (16.0); 1.1284 (2.2); 1.1114 (2.1); 1.0391 (1.2); 1.0222 (1.2); 0.9467 (0.7); 0.9362 (1.1); 0.9281 (1.4); 0.9177 (2.4); 0.9096 (0.6); 0.8991 (0.9); 0.1263 (0.5); 0.0080 (2.3); -0.0002 (79.6); -0.0085 (2.2)
Beispiel Nr. I.5-3:
   ¹H-NMR (400 MHz, CDCl₃ δ, ppm) δ= 7.2694 (0.5); 7.2686 (0.6); 7.2678 (0.7); 7.2670 (0.7); 7.2662 (0.9); 7.2654 (1.0); 7.2646 (1.2); 7.2638 (1.6); 7.2597 (83.0); 7.2557 (1.6); 7.2549 (1.2); 7.2540 (0.9); 7.2532 (0.7); 7.2524 (0.6); 6.3458 (0.6); 6.3403 (0.6); 5.2988 (16.0); 4.1258 (0.6); 4.1080 (0.6); 3.5457 (1.4); 2.0439 (0.8); 1.5355 (12.5); 1.2591 (1.2); 1.2429 (1.6); 1.2414 (1.8); 1.2238 (0.8); 1.1266 (1.0); 1.1097 (1.0); 1.0394 (0.6); 1.0227 (0.6); 0.9344 (0.6); 0.9260 (0.7); 0.9159 (1.1); 0.8973 (0.5); 0.0080 (1.0); -0.0002 (36.9); -0.0085 (1.0)
Beispiel Nr. I.8-2:
   ¹H-NMR (400 MHz, CDCl₃ δ, ppm) 7.5184 (1.0); 7.4121 (2.2); 7.3938 (2.2); 7.3592 (2.3); 7.3367 (2.3); 7.3105 (0.8); 7.2596 (173.6); 6.9956 (1.0); 6.3545 (3.5); 5.2986 (1.2); 4.3769 (2.3); 4.3612 (4.6); 4.3456 (2.4); 3.6752 (16.0); 3.6351 (9.4); 3.5541 (5.3); 3.5510 (5.6); 2.6266 (2.1); 2.6109 (4.1); 2.5952 (2.0); 1.5836 (5.4); 0.0079 (2.0); -0.0002 (64.9); -0.0085 (2.1)
Beispiel Nr. I.8-3:
   ¹H-NMR (400 MHz, CDCl₃ δ, ppm) δ= 7.4172 (3.9); 7.3989 (3.9); 7.3582 (4.0); 7.3356 (4.0); 7.2616 (8.1); 6.3525 (6.8); 4.3777 (3.8); 4.3620 (7.9); 4.3462 (4.1); 4.1567 (2.1); 4.1389 (6.4); 4.1210 (6.5); 4.1032 (2.2); 3.6349 (16.0); 3.5526 (10.3); 3.5498 (11.0); 2.6123 (3.9); 2.5966 (7.7); 2.5809 (3.8); 1.5637 (0.6); 1.2689 (7.0); 1.2511 (14.2); 1.2332 (7.1); -0.0002 (10.4); -0.0083 (0.6)
Beispiel Nr. I.8-23:
   ¹H-NMR (400 MHz, CDCl₃ δ, ppm) 7.5182 (2.3); 7.4130 (1.7); 7.4059 (1.8); 7.3948 (1.9); 7.3878 (1.8); 7.3489 (2.8); 7.3262 (2.9); 7.2969 (0.5); 7.2929 (0.7); 7.2825 (0.7); 7.2802 (0.9); 7.2785 (0.9); 7.2778 (0.9); 7.2762 (1.1); 7.2754 (1.3); 7.2746 (1.3); 7.2738 (1.4); 7.2730 (1.5); 7.2722 (1.6); 7.2714 (1.8); 7.2706 (1.9); 7.2698 (2.2); 7.2690 (2.5); 7.2682 (2.7); 7.2674 (3.2); 7.2666 (3.6); 7.2657 (4.3); 7.2594 (419.5); 7.2544 (10.8); 7.2536 (9.1); 7.2528 (7.8); 7.2520 (6.7); 7.2512 (5.9); 7.2504 (5.3); 7.2496 (4.8); 7.2488 (4.5); 7.2480 (4.2); 7.2472 (4.0); 7.2464 (3.7); 7.2456 (3.6); 7.2448 (3.4); 7.2440 (3.3); 7.2432 (3.0); 7.2424 (2.9); 7.2416 (3.0); 7.2408 (2.8); 7.2400 (2.8); 7.2393 (2.6); 7.2384 (2.5); 7.2376 (2.5); 7.2369 (2.4); 7.2360 (2.3); 7.2352 (2.3); 7.2345 (2.2); 7.2337 (2.0); 7.2329 (1.9); 7.2321 (2.0); 7.2313 (1.9); 7.2305 (1.8); 7.2297 (1.8); 7.2289 (1.8); 7.2273 (1.7); 7.2249 (1.6); 7.2233 (1.6); 7.2209 (1.6); 7.2091 (1.4); 7.1971 (1.0); 7.1875 (0.8); 6.9953 (2.4); 6.3494 (3.8); 5.3010 (0.6); 5.2853 (1.0); 5.2666 (1.0); 5.2512 (0.6); 4.1250 (1.4); 4.1070 (4.3); 4.0898 (4.4); 4.0719 (1.5); 3.6193 (4.9); 3.6167 (8.6); 3.6149 (5.4); 3.5518 (8.0); 3.5487 (8.3); 2.6314 (0.9); 2.6123 (1.0); 2.5923 (1.6); 2.5735 (1.5); 2.4920 (1.3); 2.4887 (1.3); 2.4781 (1.4); 2.4746 (1.4); 2.4530 (0.8); 2.4497 (0.8); 2.4390 (0.8); 2.4356 (0.8); 1.5403 (24.9); 1.2712 (7.0); 1.2696 (7.2); 1.2554 (7.2); 1.2537 (7.5); 1.2493 (8.4); 1.2314 (16.0); 1.2135 (7.6); 0.0080 (4.6); -0.0002 (161.4); -0.0085 (6.1); -0.1496 (0.6)
Beispiel Nr. I.8-42:
   ¹H-NMR (400 MHz, CDCl₃ δ, ppm) 7.5102 (2.3); 7.4919 (2.3); 7.3576 (2.4); 7.3350 (2.4); 7.2601 (38.9); 6.3459 (3.9); 4.7512 (1.0); 4.7495 (0.9); 4.6549 (1.3); 4.6400 (8.5); 3.7478 (2.6); 3.7399 (9.6); 3.7086 (16.0); 3.5515 (5.3); 3.5484 (5.5); 3.5451 (2.6); 3.5406 (1.0); -0.0002 (14.5)
Beispiel Nr. I.8-43:
   ¹H-NMR (400 MHz, CDCl₃ δ, ppm) δ= 7.5170 (2.5); 7.4983 (2.1); 7.3530 (2.3); 7.3303 (2.3); 7.2929 (0.6); 7.2594 (225.1); 7.2091 (1.2); 6.9954 (1.3); 6.3409 (3.7); 5.2985 (0.7); 4.6242 (8.9); 4.1858 (1.0); 4.1679 (3.2); 4.1500 (3.3); 4.1321 (1.2); 3.7409 (8.7); 3.5498 (5.6); 3.5469 (5.6); 1.5667 (16.0); 1.2715 (3.8); 1.2536 (7.9); 1.2358 (3.8); 0.0080 (2.7); -0.0002 (81.2); -0.0084 (3.3)
Beispiel Nr. I.8-127:
   ¹H-NMR (400 MHz, CDCl₃ δ, ppm) 7.3850 (1.1); 7.3669 (1.1); 7.3494 (1.2); 7.3269 (1.2); 7.2604 (21.7); 6.3480 (1.8); 5.5276 (0.5); 5.5117 (0.8); 5.4959 (0.5); 3.6477 (16.0); 3.6211 (4.4); 3.5497 (2.6); 3.5467 (2.7); 2.6947 (3.5); 2.6786 (3.4); 1.5467 (6.4); -0.0002 (7.9)
Beispiel Nr. I.11-3:
   ¹H-NMR (400 MHz, CDCl₃ δ, ppm) 7.5184 (1.8); 7.4851 (2.1); 7.4820 (2.1); 7.4664 (2.1); 7.4632 (2.1); 7.3719 (3.5); 7.3490 (3.6); 7.2854 (0.6); 7.2781 (0.8); 7.2773 (0.8); 7.2741 (1.1); 7.2717 (1.5); 7.2710 (1.5); 7.2701 (1.7); 7.2693 (1.9); 7.2686 (2.1); 7.2669 (2.7); 7.2595 (328.4); 6.9955 (1.8); 4.3477 (0.6); 4.3375 (1.4); 4.3323 (0.6); 4.3220 (1.7); 4.3195 (1.7); 4.3086 (1.5); 4.3046 (1.5); 4.2928 (2.7); 4.2810 (0.6); 4.2771 (1.5); 4.2652 (0.9); 4.2495 (0.5); 4.1496 (1.6); 4.1319 (4.8); 4.1140 (4.8); 4.0964 (1.6); 3.8979 (0.6); 3.8815 (2.0); 3.8799 (2.0); 3.8636 (2.1); 3.8620 (2.0); 3.8457 (0.6); 3.5516 (2.9); 3.5463 (8.0); 3.5409 (8.2); 3.5354 (2.9); 2.5736 (1.2); 2.5580 (3.2); 2.5429 (2.8); 2.5280 (0.9); 2.5249 (0.9); 2.2496 (1.7); 2.2404 (4.6); 2.2381 (4.9); 2.2289 (4.9); 2.2266 (4.9); 2.2174 (1.7); 2.2150 (1.7); 2.0243 (0.7); 1.5864 (8.3); 1.5167 (10.9); 1.4989 (10.7); 1.2713 (0.6); 1.2634 (7.8); 1.2567 (0.9); 1.2455 (16.0); 1.2277 (7.6); 0.0080 (4.5); 0.0057 (1.8); 0.0048 (2.1); -0.0002 (147.0); -0.0085 (3.9)
Beispiel Nr. 1.46-2:
   ¹H-NMR (400 MHz, CDCl₃ δ, ppm) 7.5387 (2.2); 7.5193 (2.4); 7.4067 (2.2); 7.3836 (2.3); 7.2596 (84.2); 6.3496 (3.7); 4.3413 (2.0); 4.3253 (4.5); 4.3094 (2.2); 3.6677 (16.0); 3.5559 (5.4); 3.5530 (5.6); 2.6364 (2.0); 2.6204 (4.2); 2.6044 (2.0); 1.5380 (2.6); 1.5245 (11.2); 1.5202 (11.2); 0.0080 (1.0); - 0.0002 (31.3); -0.0084 (0.9)

Gegenstand der vorliegenden Erfindung ist weiterhin die Verwendung einer oder mehrerer erfindungsgemäßer Verbindungen der Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (1.1) bis (I.50) und/oder deren Salze, jeweils wie oben definiert,
als Herbizid und/oder Pflanzenwachstumsregulator, vorzugsweise in Kulturen von Nutz- und/oder Zierpflanzen.

Gegenstand der vorliegenden Erfindung ist ferner ein Verfahren zur Bekämpfung von Schadpflanzen und/oder zur Wachstumsregulierung von Pflanzen, dadurch gekennzeichnet, dass eine wirksame Menge
- einer oder mehrerer erfindungsgemäßer Verbindungen der Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (1.1) bis (I.50) und/oder deren Salze, jeweils wie oben definiert, oder
- eines erfindungsgemäßen Mittels, wie nachstehend definiert,
auf die (Schad)Pflanzen, (Schad)Pflanzensamen, den Boden, in dem oder auf dem die (Schad)Pflanzen wachsen, oder die Anbaufläche appliziert wird.

Gegenstand der vorliegenden Erfindung ist auch ein Verfahren zur Bekämpfung von unerwünschten Pflanzen, vorzugsweise in Nutzpflanzenkulturen, dadurch gekennzeichnet, dass eine wirksame Menge
- einer oder mehrerer Verbindungen der Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (1.1) bis (I.50) und/oder deren Salze, jeweils wie oben definiert, oder
- eines erfindungsgemäßen Mittels, wie nachstehend definiert,
auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschte Pflanzen wachsen werden) appliziert wird.

Gegenstand der vorliegenden Erfindung ist ferner auch Verfahren zur Bekämpfung zur Wachstumsregulierung von Pflanzen, vorzugsweise von Nutzpflanzen, dadurch gekennzeichnet, dass eine wirksame Menge
- einer oder mehrerer Verbindungen der Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1.1) bis (I.50) und/oder deren Salze, jeweils wie oben definiert, oder
- eines erfindungsgemäßen Mittels, wie nachstehend definiert,
die Pflanze, das Saatgut der Pflanze (d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Dabei können die erfindungsgemäßen Verbindungen bzw. die erfindungsgemäßen Mittel z.B. im Vorsaat- (ggf. auch durch Einarbeitung in den Boden), Vorauflauf- und/oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Vorzugsweise werden in einem erfindungsgemäßen Verfahren zur Bekämpfung von Schadpflanzen oder zur Wachstumsregulierung von Pflanzen eine oder mehrere Verbindungen der Formel (I) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Die erfindungsgemäßen Verbindungen Formel (I) und/oder deren Salze eignen sich zur Bekämpfung der folgenden Gattungen von monokotylen und dikotylen Schadpflanzen:
Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Getreide, dabei vorzugsweise Mais, Weizen, Gerste, Roggen, Hafer, Hirse, oder Reis, im Vor- oder Nachauflauf.

Bevorzugt ist auch die Verwendung in Soja im Vor- oder Nachauflauf.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, dass man eine wirksame Menge einer oder mehreren Verbindungen der Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Gegenstand der Erfindung ist auch ein herbizides und/oder pflanzenwachstumsregulierendes Mittel, dadurch gekennzeichnet, dass das Mittel
(a) eine oder mehrere Verbindungen der Formel (I) und/oder deren Salze enthält wie oben definiert, vorzugsweise in einer der als bevorzugt bzw. besonders bevorzugt gekennzeichneten Ausgestaltung, insbesondere eine oder mehrere Verbindungen der Formeln (1.1) bis (I.XX) und/oder deren Salze, jeweils wie oben definiert, und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Die weiteren agrochemischen wirksamen Stoffe des Bestandteils (i) eines erfindungsgemäßen Mittels sind dabei vorzugsweise ausgewählt aus der Gruppe der Stoffe, die in "The Pesticide Manual", 16th edition, The British Crop Protection Council und the Royal Soc. of Chemistry, 2012 genannt sind.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) und/oder deren Salze enthalten.

Die Verbindungen der Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder "The Pesticide Manual", 16th edition, The British Crop Protection Council and the Royal Soc. of Chemistry, 2012 und der dort zitierten Literatur beschrieben sind.

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (1) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der Verbindungen der Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an Verbindungen der Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf.

Bei der Anwendung von Verbindungen der Formel (I) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Als Kombinationspartner für die erfindungsgemäßen Verbindungen der Formel (I) in erfindungsgemäßen Mitteln (z.B. Mischungsformulierungen oder im Tank-Mix) sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2012 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

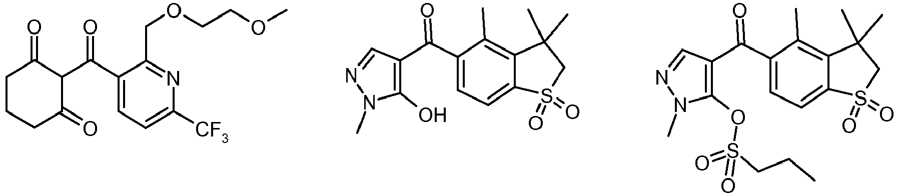

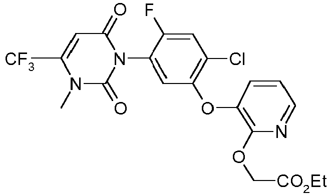

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2-naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Ebenfalls als Kombinationspartner für die erfindungsgemäßen Verbindungen der Formel (I) kommen beispielsweise die folgenden Safener in Frage:
S1) Verbindungen aus der Gruppe heterocyclischer Carbonsäurederivate:
S1^{a}) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
S1^{b}) Derivate der Dichlorphenylpyrazolcarbonsäure (S1^{b}), vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1 -dimethyl-ethyl)pyrazol-3-carbonsäureethylester (S1-4) und verwandte Verbindungen, wie sie in EP-A-333131 und EP-A-269806 beschrieben sind;
S1^{c} Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
S1^{d}) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen, wie sie in EP-A-174562 und EP-A-346620 beschrieben sind;
S1^{e}) Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-3-carbonsäureethylester (S1-11) ("Isoxadifen-ethyl") oder -n-propylester (S1-12) oder 5-(4-Fluorphenyl)-5-phenyl-2-isoxazolin-3-carbon-säureethylester (S1-13), wie sie in der Patentanmeldung WO-A-95/07897 beschrieben sind.
S2) Verbindungen aus der Gruppe der 8-Chinolinoxyderivate (S2):
S2^{a}) Verbindungen vom Typ der 8-Chinolinoxyessigsäure (S2^{a}), vorzugsweise (5-Chlor-8-chinolinoxy)essigsäure-(1-methylhexyl)-ester ("Cloquintocet-mexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86750, EP-A-94349 und EP-A-191736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Wirkstoffe vom Typ der Dichloracetamide (S3), die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF,
   "Furilazol" oder "MON 13900" ((RS)-3-Dichloracetyl-5-(2-füryl)-2,2-dimethyloxazolidin) (S3-10), sowie dessen (R)-Isomer (S3-11).
S4) Verbindungen aus der Klasse der Acylsulfonamide (S4):
S4^{a}) N-Acylsulfonamide der Formel (S4^{a}) und deren Salze wie sie in der WO-A-97/45016 beschrieben sind, 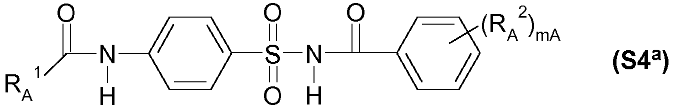 worin
   - R_{A}¹: (C₁-C₆)Alkyl, (C₃-C₆)Cycloalkyl, wobei die 2 letztgenannten Reste durch V_{A} Substituenten aus der Gruppe Halogen, (C₁-C₄)Alkoxy, (C₁-C₆)Haloalkoxy und (C₁-C₄)Alkylthio und im Falle cyclischer Reste auch durch (C₁-C₄)Alkyl und (C₁-C₄)Haloalkyl substituiert sind;
   - R_{A}²: Halogen, (C₁-C₄)Alkyl, (C₁-C₄)Alkoxy, CF₃;
   - m_{A}: 1 oder 2;
   - V_{A}: ist 0, 1, 2 oder 3 bedeuten;
S4^{b}) Verbindungen vom Typ der 4-(Benzoylsulfamoyl)benzamide der Formel (S4^{b}) und deren Salze, wie sie in der WO-A-99/16744 beschrieben sind, 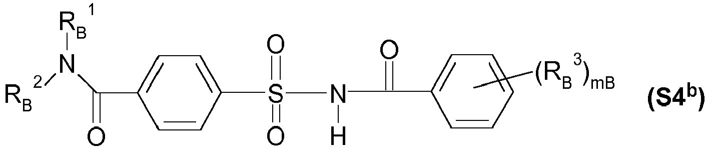 worin
   - R_{B}¹, R_{B}²: unabhängig voneinander Wasserstoff, (C₁-C₆)Alkyl, (C₃-C₆)Cycloalkyl, (C₃-C₆)Alkenyl, (C₃-C₆)Alkinyl,
   - R_{B}³: Halogen, (C₁-C₄)Alkyl, (C₁-C₄)Haloalkyl oder (C₁-C₄)Alkoxy und
   - m_{B}: 1 oder 2 bedeuten,

   z.B. solche worin
   R_{B}¹ = Cyclopropyl, R_{B}² = Wasserstoff und (R_{B}³) = 2-OMe ist ("Cyprosulfamide", S4-1),
   R_{B}¹ = Cyclopropyl, R_{B}² = Wasserstoff und (R_{B}³) = 5-Cl-2-OMe ist (S4-2),
   R_{B}¹ = Ethyl, R_{B}² = Wasserstoff und (R_{B}³) = 2-OMe ist (S4-3),
   R_{B}¹ = Isopropyl, R_{B}² = Wasserstoff und (R_{B}³) = 5-Cl-2-OMe ist (S4-4) und
   R_{B}¹ = Isopropyl, R_{B}² = Wasserstoff und (R_{B}³) = 2-OMe ist (S4-5);
S4^{c}) Verbindungen aus der Klasse der Benzoylsulfamoylphenylharnstoffe der Formel (S4^{c}), wie sie in der EP-A-365484 beschrieben sind, 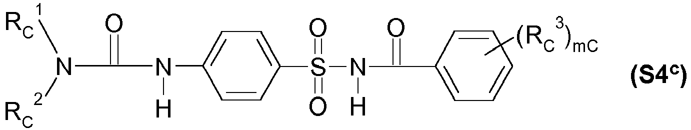 worin
   - R_{C}¹, R_{C}²: unabhängig voneinander Wasserstoff, (C₁-C₈)Alkyl, (C₃-C₈)Cycloalkyl, (C₃-C₆)Alkenyl, (C₃-C₆)Alkinyl,
   - R_{C}³: Halogen, (C₁-C₄)Alkyl, (C₁-C₄)Alkoxy, CF₃ und
   - m_{C}: 1 oder 2 bedeuten;
   beispielsweise
   1-[4-(N-2-Methoxybenzoylsulfamoyl)phenyl]-3-methylhanstoff,
   1-[4-(N-2-Methoxybenzoylsulfamoyl)phenyl]-3,3-dimethylhanstoff,
   1-[4-(N-4,5-Dimethylbenzoylsulfamoyl)phenyl]-3-methylhanstoff,
S4^{d}) Verbindungen vom Typ der N-Phenylsulfonylterephthalamide der Formel (S4^{d}) und deren Salze, die z.B. bekannt sind aus CN 101838227, 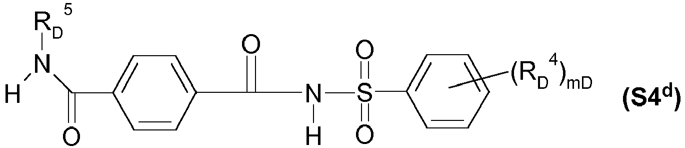 worin
   - R_{D}⁴: Halogen, (C₁-C₄)Alkyl, (C₁-C₄)Alkoxy, CF_{3;}
   - m_{D}: 1 oder 2;
   - R_{D}⁵: Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydro-chinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8), wie sie in der WO-A-98/27049 beschrieben sind, 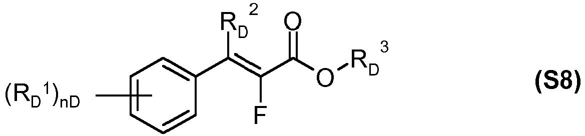 worin die Symbole und Indizes folgende Bedeutungen haben:
   - R_{D}¹: ist Halogen, (C₁-C₄)Alkyl, (C₁-C₄)Haloalkyl, (C₁-C₄)Alkoxy, (C₁-C₄)Haloalkoxy,
   - R_{D}²: ist Wasserstoff oder (C₁-C₄)Alkyl,
   - R_{D}³: ist Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze,
   - n_{D}: ist eine ganze Zahl von 0 bis 2.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr.: 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}),
   wie sie in der WO-A-2007/023719 und WO-A-2007/023764 beschrieben sind, 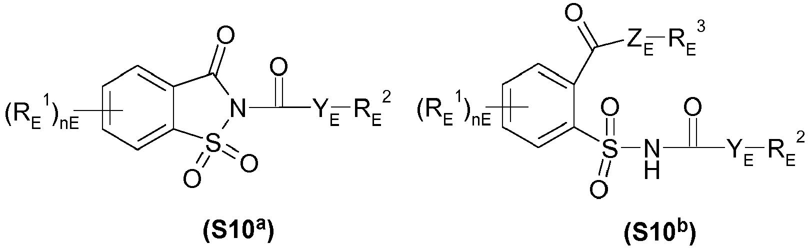 worin
   - R_{E}¹: Halogen, (C₁-C₄)Alkyl, Methoxy, Nitro, Cyano, CF₃, OCF₃
   - Y_{E}, Z_{E}: unabhängig voneinander O oder S,
   - n_{E}: eine ganze Zahl von 0 bis 4,
   - R_{E}²: (C₁-C₁₆)Alkyl, (C₂-C₆)Alkenyl, (C₃-C₆)Cycloalkyl, Aryl; Benzyl, Halogenbenzyl,
   - R_{E}³: Wasserstoff oder (C₁-C₆)Alkyl bedeuten.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5) (2-propenyl 1-oxa-4-azaspiro[4.5]decan-4-carbodithioat) (S13-6) der Firma Nitrokemia
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (*S*-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere, 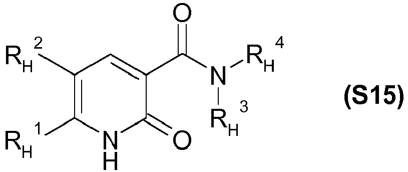 wie sie in der WO-A-2008/131861 und WO-A-2008/131860 beschrieben sind,
   worin
   - R_{H}¹: einen (C₁-C₆)Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet und
   - R_{H}⁴: Wasserstoff oder (C₁-C₄)-Alkyl bedeutet oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z. B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Bevorzugte Safener in Kombination mit den erfindungsgemäßen Verbindungend der Formel (I) und/oder deren Salze, insbesondere mit den Verbindungen der Formeln (I.1) bis (I.50) und/oder deren Salze sind: Cloquintocet-mexyl, Cyprosulfamid, Fenchlorazol-ethylester, Isoxadifen-ethyl, Mefenpyr-diethyl, Fenclorim, Cumyluron, S4-1 und S4-5, und besonders bevorzugte Safener sind: Cloquintocet-mexyl, Cyprosulfamid, Isoxadifen-ethyl und Mefenpyr-diethyl.

Biologische Beispiele:

### A. Herbizide Wirkung und Kulturverträglichkeit im Nachauflauf

Samen von mono- bzw. dikotylen Unkraut- bzw. Kulturpflanzen wurden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die grünen Pflanzenteile gesprüht. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen. Entsprechend wurden auch die Kulturpflanzenverträglichkeiten bonitiert.

In den nachstehenden Tabllen A1 bis A16 sind die Wirkungen ausgewählter Verbindungen der allgemeinen Formel (I) gemäß der Tabellen I.1 bis I.50 auf verschiedene Schadpflanzen und einer Aufwandmenge entsprechend 80 g/ha und niedriger, die gemäß zuvor genannter Versuchvorschrift erhalten wurden, dargestellt.

**Tabelle A1**

| Verbindung Beispiel Nr. | Alopecurus myosuroides (Wirkung in %) | Aufwandmenge [g/ha] |
|---|---|---|
| I.1-2 | 100 | 80 |
| I.1-3 | 100 | 80 |
| I.1-22 | 80 | 80 |
| I.1-23 | 90 | 80 |
| I.1-127 | 90 | 80 |
| I.1-132 | 80 | 80 |
| I.1-141 | 90 | 80 |
| I.1-151 | 90 | 80 |
| I.1-182 | 80 | 80 |
| I.1-183 | 80 | 80 |
| I.1-184 | 80 | 80 |
| I.1-242 | 90 | 20 |
| I.1-251 | 100 | 80 |
| I.1-255 | 90 | 80 |
| I.2-2 | 100 | 80 |
| I.8-2 | 80 | 80 |
| I.8-127 | 80 | 80 |
| I.1-32 | 80 | 80 |
| I.1-142 | 80 | 20 |
| I.1-2-3 | 90 | 20 |
| I.1-144 | 80 | 20 |
| I.1-8 | 90 | 80 |
| I.1-43 | 80 | 20 |
| I.1-46 | 80 | 20 |
| I.2-6 | 80 | 20 |
| I.2-32 | 80 | 20 |
| I.5-3 | 80 | 20 |
| I.5-2 | 80 | 20 |
| I.1-233 | 90 | 20 |
| I.1-153 | 90 | 20 |
| I.1-152 | 80 | 20 |

**Tabelle A2**

| Verbindung Beispiel Nr. | Echinochloa crus-galli (Wirkung in %) | Aufwandmenge [g/ha] |
|---|---|---|
| I.1-1 | 100 | 80 |
| I.1-2 | 100 | 80 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 80 |
| I.1-127 | 100 | 80 |
| I.1-132 | 100 | 80 |
| I.1-141 | 100 | 80 |
| I.1-151 | 100 | 80 |
| I.1-182 | 100 | 80 |
| I.1-183 | 100 | 80 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 80 |
| I.8-2 | 100 | 80 |
| I.8-23 | 100 | 80 |
| I.8-42 | 100 | 80 |
| I.8-127 | 100 | 20 |
| I.46-2 | 100 | 80 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-8 | 100 | 80 |
| I.11-3 | 100 | 20 |
| I.1-43 | 100 | 20 |
| I.8-43 | 90 | 20 |
| I.1-51 | 100 | 20 |
| I.8-46 | 90 | 20 |
| I.1-46 | 100 | 20 |
| I.8-51 | 90 | 20 |
| I.47-3 | 80 | 20 |
| I.2-6 | 100 | 20 |
| I.2-8 | 100 | 20 |
| I.2-32 | 100 | 20 |
| I.2-43 | 100 | 20 |
| I.2-145 | 100 | 20 |
| I.5-3 | 100 | 20 |
| I.5-2 | 100 | 20 |
| I.12-3 | 100 | 20 |
| I.1-293 | 90 | 20 |
| I.1-233 | 100 | 20 |
| I.1-231 | 100 | 20 |
| I.1-153 | 100 | 20 |
| I.1-152 | 100 | 20 |

**Tabelle A3**

| Verbindung Beispiel Nr. | Setaria viridis (Wirkung in %) | Aufwandmenge [g/ha] |
|---|---|---|
| I.1-1 | 100 | 80 |
| I.1-2 | 100 | 80 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 20 |
| I.1-127 | 100 | 80 |
| I.1-132 | 100 | 80 |
| I.1-141 | 100 | 20 |
| I.1-151 | 100 | 80 |
| I.1-183 | 100 | 80 |
| I.1-184 | 100 | 20 |
| I.1-241 | 100 | 80 |
| I.1-242 | 100 | 80 |
| I.1-251 | 100 | 20 |
| I.1-254 | 100 | 80 |
| I.1-255 | 100 | 80 |
| I.2-2 | 100 | 80 |
| I.8-2 | 100 | 80 |
| I.8-23 | 100 | 80 |
| I.8-42 | 100 | 80 |
| I.8-127 | 100 | 20 |
| I.46-2 | 100 | 80 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-8 | 100 | 80 |
| I.1-43 | 100 | 20 |
| I.8-43 | 100 | 20 |
| I.1-51 | 100 | 20 |
| I.8-46 | 100 | 20 |
| I.1-46 | 100 | 20 |
| I.8-51 | 100 | 20 |
| I.8-44 | 100 | 20 |
| I.47-6 | 100 | 20 |
| I.47-3 | 100 | 20 |
| I.2-6 | 100 | 20 |
| I.2-8 | 100 | 20 |
| I.2-32 | 100 | 20 |
| I.2-43 | 100 | 20 |
| I.6-3 | 100 | 20 |
| I.2-145 | 100 | 20 |
| I.4-3 | 90 | 20 |
| I.5-3 | 100 | 20 |
| I.5-2 | 100 | 20 |
| I.12-3 | 100 | 20 |
| I.14-3 | 90 | 20 |
| I.1-293 | 90 | 20 |
| I.1-233 | 100 | 20 |
| I.1-231 | 100 | 20 |
| I.1-153 | 100 | 20 |
| I.1-152 | 100 | 20 |

**Tabelle A4**

| Verbindung Beispiel Nr. | Abutilon theophrasti (Wirkung in %) | Aufwandmenge [g/ha] |
|---|---|---|
| I.1-1 | 100 | 80 |
| I.1-2 | 100 | 80 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 20 |
| I.1-127 | 100 | 80 |
| I.1-132 | 100 | 80 |
| I.1-141 | 100 | 80 |
| I.1-151 | 100 | 80 |
| I.1-182 | 100 | 80 |
| I.1-183 | 100 | 80 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 80 |
| I.8-2 | 100 | 80 |
| I.8-23 | 100 | 80 |
| I.8-42 | 100 | 80 |
| I.8-127 | 100 | 20 |
| I.46-2 | 100 | 80 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-6 | 100 | 20 |
| I.1-8 | 100 | 80 |
| I.11-3 | 100 | 20 |
| I.1-43 | 100 | 20 |
| I.8-43 | 100 | 20 |
| I.1-51 | 100 | 20 |
| I.8-46 | 100 | 20 |
| I.1-46 | 100 | 20 |
| I.8-51 | 100 | 20 |
| I.8-44 | 100 | 20 |
| I.47-6 | 100 | 20 |
| I.47-3 | 100 | 20 |
| I.2-6 | 100 | 20 |
| I.2-8 | 100 | 20 |
| I.2-32 | 100 | 20 |
| I.2-43 | 100 | 20 |
| I.6-3 | 100 | 20 |
| I.6-43 | 100 | 20 |
| I.2-145 | 100 | 20 |
| I.4-3 | 100 | 20 |
| I.4-43 | 100 | 20 |
| I.5-3 | 100 | 20 |
| I.5-2 | 100 | 20 |
| I.12-3 | 100 | 20 |
| I.14-3 | 100 | 20 |
| I.1-293 | 100 | 20 |
| I.1-233 | 100 | 20 |
| I.1-231 | 100 | 20 |
| I.1-153 | 100 | 20 |
| I.1-152 | 100 | 20 |

**Tabelle A5**

| Verbindung Beispiel Nr. | Amaranthus retroflexus (Wirkung in %) | Aufwandmenge [g/ha] |
|---|---|---|
| I.1-1 | 100 | 20 |
| I.1-2 | 100 | 80 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 20 |
| I.1-127 | 100 | 20 |
| I.1-132 | 100 | 80 |
| I.1-141 | 100 | 80 |
| I.1-151 | 100 | 80 |
| I.1-182 | 100 | 80 |
| I.1-183 | 100 | 80 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 80 |
| I.8-2 | 100 | 20 |
| I.8-23 | 100 | 80 |
| I.8-42 | 100 | 20 |
| I.8-127 | 100 | 20 |
| I.46-2 | 100 | 80 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-6 | 100 | 20 |
| I.1-8 | 100 | 80 |
| I.1-43 | 100 | 20 |
| I.8-43 | 100 | 20 |
| I.1-51 | 100 | 20 |
| I.8-46 | 100 | 20 |
| I.1-46 | 100 | 20 |
| I.8-51 | 100 | 20 |
| I.8-44 | 100 | 20 |
| I.47-6 | 100 | 20 |
| I.47-3 | 100 | 20 |
| I.2-6 | 100 | 20 |
| I.2-8 | 100 | 20 |
| I.2-32 | 100 | 20 |
| I.2-43 | 100 | 20 |
| I.6-3 | 100 | 20 |
| I.2-145 | 100 | 20 |
| I.4-3 | 100 | 20 |
| I.4-43 | 100 | 20 |
| I.5-3 | 100 | 20 |
| I.5-2 | 100 | 20 |
| I.12-3 | 100 | 20 |
| I.14-3 | 100 | 20 |
| I.1-293 | 100 | 20 |
| I.1-233 | 100 | 20 |
| I.1-231 | 100 | 20 |
| I.1-153 | 100 | 20 |
| I.1-152 | 100 | 20 |

**Tabelle A6**

| Verbindung Beispiel Nr. | Matricaria inodora (Wirkung in %) | Aufwandmenge [g/ha] |
|---|---|---|
| I.1-1 | 100 | 80 |
| I.1-2 | 100 | 80 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 80 |
| I.1-127 | 100 | 80 |
| I.1-132 | 90 | 80 |
| I.1-141 | 100 | 80 |
| I.1-151 | 100 | 80 |
| I.1-182 | 100 | 80 |
| I.1-183 | 100 | 80 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 80 |
| I.8-2 | 100 | 20 |
| I.8-23 | 100 | 20 |
| I.8-42 | 100 | 20 |
| I.8-127 | 100 | 20 |
| I.46-2 | 100 | 20 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-8 | 100 | 80 |
| I.11-3 | 90 | 20 |
| I.1-43 | 100 | 20 |
| I.8-43 | 100 | 20 |
| I.1-51 | 100 | 20 |
| I.8-46 | 100 | 20 |
| I.1-46 | 100 | 20 |
| I.8-51 | 100 | 20 |
| I.8-44 | 100 | 20 |
| I.47-3 | 100 | 20 |
| I.2-6 | 100 | 20 |
| I.2-8 | 100 | 20 |
| I.2-32 | 100 | 20 |
| I.2-43 | 100 | 20 |
| I.6-3 | 100 | 20 |
| I.2-145 | 100 | 20 |
| I.4-3 | 80 | 20 |
| I.4-43 | 90 | 20 |
| I.5-3 | 100 | 20 |
| I.5-2 | 100 | 20 |
| I.1-293 | 100 | 20 |
| I.1-233 | 100 | 20 |
| I.1-231 | 100 | 20 |
| I.1-153 | 100 | 20 |
| I.1-152 | 100 | 20 |

**Tabelle A7**

| Verbindung Beispiel Nr. | Polygonum convolvulus (Wirkung in %) | Aufwandmenge [g/ha] |
|---|---|---|
| I.1-1 | 100 | 80 |
| I.1-2 | 100 | 20 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 80 |
| I.1-127 | 100 | 80 |
| I.1-132 | 100 | 80 |
| I.1-141 | 100 | 80 |
| I.1-151 | 100 | 80 |
| I.1-182 | 100 | 80 |
| I.1-183 | 100 | 80 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 80 |
| I.8-2 | 100 | 20 |
| I.8-23 | 100 | 20 |
| I.8-42 | 100 | 80 |
| I.8-127 | 100 | 80 |
| I.46-2 | 100 | 20 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-8 | 100 | 80 |
| I.1-43 | 100 | 20 |
| I.8-43 | 100 | 20 |
| I.1-51 | 100 | 20 |
| I.8-46 | 100 | 20 |
| I.1-46 | 100 | 20 |
| I.8-51 | 100 | 20 |
| I.8-44 | 100 | 5 |
| I.47-6 | 100 | 20 |
| I.47-3 | 100 | 20 |
| I.2-6 | 100 | 20 |
| I.2-8 | 100 | 20 |
| I.2-32 | 100 | 20 |
| I.2-43 | 100 | 20 |
| I.2-145 | 100 | 20 |
| I.4-43 | 100 | 20 |
| I.5-3 | 100 | 20 |
| I.5-2 | 100 | 20 |
| I.12-3 | 100 | 20 |
| I.14-3 | 100 | 20 |
| I.1-233 | 100 | 20 |
| I.1-231 | 100 | 20 |
| I.1-153 | 100 | 20 |
| I.1-152 | 100 | 20 |

**Tabelle A8**

| Verbindung Beispiel Nr. | Stellaria media (Wirkung in %) | Aufwandmenge [g/ha] |
|---|---|---|
| I.1-1 | 100 | 80 |
| I.1-2 | 100 | 20 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 80 |
| I.1-127 | 100 | 20 |
| I.1-132 | 100 | 80 |
| I.1-141 | 100 | 80 |
| I.1-151 | 90 | 80 |
| I.1-182 | 100 | 80 |
| I.1-183 | 100 | 20 |
| I.1-184 | 100 | 80 |
| I.1-241 | 100 | 80 |
| I.1-242 | 100 | 80 |
| I.1-251 | 100 | 80 |
| I.1-254 | 90 | 80 |
| I.1-255 | 100 | 80 |
| I.2-2 | 100 | 80 |
| I.8-2 | 100 | 20 |
| I.8-23 | 100 | 80 |
| I.8-42 | 100 | 80 |
| I.8-127 | 100 | 20 |
| I.46-2 | 80 | 80 |
| I.1-32 | 100 | 80 |
| I.1-142 | 100 | 20 |
| I.1-102 | 80 | 20 |
| I.1-2-3 | 100 | 20 |
| I.1-144 | 80 | 20 |
| I.1-8 | 100 | 80 |
| I.11-3 | 100 | 20 |
| I.1-43 | 80 | 20 |
| I.8-43 | 80 | 20 |
| I.1-51 | 100 | 20 |
| I.8-46 | 100 | 20 |
| I.1-46 | 100 | 20 |
| I.8-44 | 100 | 20 |
| I.47-6 | 80 | 20 |
| I.2-6 | 100 | 20 |
| I.2-8 | 100 | 20 |
| I.2-43 | 100 | 20 |
| I.6-3 | 100 | 20 |
| I.6-43 | 90 | 20 |
| I.2-145 | 100 | 20 |
| I.4-43 | 80 | 20 |
| I.5-3 | 100 | 20 |
| I.5-2 | 100 | 20 |
| I.12-3 | 100 | 20 |
| I.1-293 | 100 | 20 |
| I.1-233 | 100 | 20 |
| I.1-231 | 100 | 20 |
| I.1-153 | 100 | 20 |
| I.1-152 | 100 | 20 |

**Tabelle A9**

| Verbindung Beispiel Nr. | Viola tricolor (Wirkung in %) | Aufwandmenge [g/ha] |
|---|---|---|
| I.1-1 | 100 | 80 |
| I.1-2 | 100 | 80 |
| I.1-3 | 100 | 80 |
| I.1-5 | 100 | 20 |
| I.1-22 | 100 | 80 |
| I.1-23 | 100 | 20 |
| I.1-42 | 100 | 20 |
| I.1-72 | 100 | 80 |
| I.1-127 | 100 | 80 |
| I.1-151 | 100 | 80 |
| I.1-182 | 100 | 20 |
| I.1-183 | 100 | 20 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 80 |
| I.8-2 | 100 | 80 |
| I.8-23 | 100 | 20 |
| I.8-42 | 100 | 80 |
| I.8-127 | 100 | 80 |
| I.46-2 | 80 | 80 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-6 | 100 | 20 |
| I.1-8 | 100 | 80 |
| I.11-3 | 90 | 20 |
| I.1-43 | 100 | 20 |
| I.8-43 | 100 | 20 |
| I.1-51 | 100 | 20 |
| I.8-46 | 100 | 20 |
| I.1-46 | 100 | 20 |
| I.8-51 | 100 | 20 |
| I.8-44 | 100 | 20 |
| I.47-6 | 100 | 20 |
| I.47-3 | 100 | 20 |
| I.2-6 | 100 | 20 |
| I.2-8 | 100 | 20 |
| I.2-32 | 100 | 20 |
| I.2-43 | 100 | 20 |
| I.6-3 | 100 | 20 |
| I.6-43 | 100 | 20 |
| I.2-145 | 100 | 20 |
| I.4-3 | 100 | 20 |
| I.4-43 | 100 | 20 |
| I.5-3 | 100 | 20 |
| I.5-2 | 100 | 20 |
| I.12-3 | 100 | 20 |
| I.14-3 | 100 | 20 |
| I.1-293 | 100 | 20 |
| I.1-233 | 100 | 20 |
| I.1-231 | 100 | 20 |
| I.1-153 | 100 | 20 |
| I.1-152 | 100 | 20 |

**Tabelle A10**

| Verbindung Beispiel Nr. | Veronica persica (Wirkung in %) | Aufwandmenge [g/ha] |
|---|---|---|
| I.1-1 | 100 | 80 |
| I.1-2 | 100 | 20 |
| I.1-3 | 100 | 80 |
| I.1-5 | 100 | 80 |
| I.1-22 | 100 | 80 |
| I.1-23 | 100 | 80 |
| I.1-42 | 100 | 80 |
| I.1-72 | 100 | 80 |
| I.1-127 | 100 | 20 |
| I.1-132 | 100 | 80 |
| I.1-141 | 100 | 80 |
| I.1-151 | 100 | 80 |
| I.1-182 | 100 | 80 |
| I.1-183 | 100 | 20 |
| I.1-184 | 100 | 80 |
| I.1-241 | 100 | 80 |
| I.1-242 | 100 | 80 |
| I.1-251 | 100 | 80 |
| I.1-254 | 90 | 80 |
| I.1-255 | 100 | 80 |
| I.2-2 | 100 | 20 |
| I.8-2 | 100 | 80 |
| I.8-23 | 100 | 80 |
| I.8-42 | 100 | 80 |
| 1.8-127 | 100 | 20 |
| I.46-2 | 80 | 80 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-6 | 100 | 20 |
| I.1-8 | 100 | 80 |
| I.1-43 | 100 | 20 |
| I.8-43 | 100 | 20 |
| I.1-51 | 100 | 20 |
| I.8-46 | 100 | 20 |
| 1.1-46 | 100 | 20 |
| I.8-51 | 100 | 20 |
| I.8-44 | 100 | 20 |
| I.47-6 | 100 | 20 |
| I.47-3 | 100 | 20 |
| 1.2-6 | 100 | 20 |
| 1.2-8 | 100 | 20 |
| I.2-32 | 100 | 20 |
| I.2-43 | 100 | 20 |
| I.6-3 | 100 | 20 |
| I.6-43 | 100 | 5 |
| 1.2-145 | 100 | 20 |
| I.4-3 | 80 | 20 |
| I.4-43 | 100 | 20 |
| I.5-3 | 100 | 20 |
| I.5-2 | 100 | 20 |
| I.12-3 | 100 | 20 |
| I.14-3 | 100 | 20 |
| I.1-293 | 100 | 20 |
| I.1-233 | 100 | 20 |
| I.1-231 | 100 | 20 |
| I.1-153 | 100 | 20 |
| I.1-152 | 100 | 20 |

**Tabelle A11**

| Verbindung Beispiel Nr. | Pharbitis purpurea (Wirkung in %) | Aufwandmenge [g/ha] |
|---|---|---|
| I.1-1 | 100 | 80 |
| I.1-2 | 100 | 20 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 20 |
| I.1-127 | 100 | 20 |
| I.1-132 | 100 | 80 |
| I.1-141 | 100 | 80 |
| I.1-151 | 100 | 80 |
| I.1-182 | 100 | 80 |
| I.1-183 | 100 | 80 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 20 |
| I.8-2 | 100 | 80 |
| I.8-23 | 100 | 20 |
| I.8-42 | 100 | 80 |
| 1.8-127 | 100 | 20 |
| I.46-2 | 100 | 80 |
| I.1-32 | 100 | 80 |
| I.1-142 | 100 | 20 |
| I.1-102 | 100 | 20 |
| I.1-2-3 | 100 | 20 |
| I.1-144 | 100 | 20 |
| I.1-8 | 100 | 80 |
| I.1-43 | 100 | 20 |
| I.8-43 | 100 | 20 |
| I.1-51 | 100 | 20 |
| I.8-46 | 100 | 20 |
| 1.1-46 | 100 | 20 |
| I.8-51 | 100 | 20 |
| I.8-44 | 100 | 20 |
| I.47-6 | 100 | 20 |
| I.47-3 | 100 | 20 |
| 1.2-6 | 100 | 20 |
| 1.2-8 | 100 | 20 |
| I.2-32 | 100 | 20 |
| I.2-43 | 100 | 20 |
| I.5-3 | 100 | 20 |
| I.5-2 | 100 | 20 |
| I.12-3 | 100 | 5 |
| I.14-3 | 100 | 20 |
| I.1-233 | 100 | 20 |
| I.1-231 | 100 | 20 |
| I.1-153 | 100 | 20 |
| I.1-152 | 100 | 20 |

**Tabelle A12**

| Verbindung Beispiel Nr. | Hordeum murinum (Wirkung in %) | Aufwandmenge [g/ha] |
|---|---|---|
| I.1-2 | 100 | 20 |
| I.1-5 | 80 | 80 |
| I.1-127 | 90 | 80 |
| I.1-184 | 80 | 80 |
| I.1-242 | 90 | 80 |
| I.1-254 | 100 | 80 |
| I.1-254 | 80 | 80 |
| I.8-2 | 80 | 20 |
| I.2-2 | 80 | 80 |
| I.8-42 | 80 | 80 |
| I.1-102 | 100 | 20 |
| I.1-144 | 80 | 20 |
| I.1-8 | 90 | 80 |
| 1.1-46 | 100 | 20 |

**Tabelle A13**

| Verbindung Beispiel Nr. | Avena fatua (Wirkung in %) | Aufwandmenge [g/ha] |
|---|---|---|
| I.1-2 | 80 | 80 |
| I.1-3 | 80 | 80 |
| I.1-127 | 80 | 80 |
| I.1-182 | 90 | 80 |
| I.1-242 | 80 | 80 |
| I.1-251 | 80 | 80 |
| I.1-254 | 80 | 80 |
| I.1-255 | 80 | 80 |
| I.2-2 | 80 | 80 |
| 1.8-127 | 80 | 80 |
| I.1-32 | 80 | 80 |
| I.2-3 | 100 | 20 |
| I.1-144 | 100 | 20 |
| I.1-8 | 80 | 80 |

**Tabelle A14**

| Verbindung Beispiel Nr. | Lolium multiflorum (Wirkung in %) | Aufwandmenge [g/ha] |
|---|---|---|
| I.1-3 | 100 | 80 |

**Tabelle A15**

| Verbindung Beispiel Nr. | Lolium rigidum (Wirkung in %) | Aufwandmenge [g/ha] |
|---|---|---|
| I.1-1 | 80 | 80 |
| I.1-2 | 90 | 80 |
| I.1-5 | 80 | 80 |
| I.1-22 | 90 | 80 |
| I.1-23 | 90 | 80 |
| I.1-42 | 90 | 80 |
| I.1-127 | 100 | 80 |
| I.1-182 | 100 | 80 |
| I.1-183 | 100 | 80 |
| I.1-184 | 100 | 80 |
| I.1-241 | 90 | 80 |
| I.1-242 | 100 | 80 |
| I.1-251 | 100 | 80 |
| I.1-255 | 100 | 80 |
| I.2-2 | 80 | 80 |
| I.8-2 | 90 | 80 |
| 1.8-127 | 90 | 80 |
| I.1-32 | 100 | 80 |
| I.1-102 | 80 | 20 |
| I.1-144 | 100 | 20 |
| I.1-8 | 90 | 80 |
| 1.1-46 | 80 | 20 |
| I.2-32 | 80 | 20 |
| I.1-233 | 90 | 20 |
| I.1-153 | 80 | 20 |
| I.1-152 | 80 | 20 |

**Tabelle A16**

| Verbindung Beispiel Nr. | Digitaria sanguinalis (Wirkung in %) | Aufwandmenge [g/ha] |
|---|---|---|
| I.1-32 | 100 | 80 |
| I.1-142 | 100 | 20 |
| I.1-102 | 100 | 20 |
| I.1-2-3 | 100 | 20 |
| I.1-144 | 80 | 20 |
| I.1-8 | 100 | 80 |
| I.1-43 | 100 | 20 |
| I.1-51 | 90 | 20 |
| 1.1-46 | 100 | 20 |
| I.8-51 | 100 | 20 |
| I.47-6 | 80 | 20 |
| I.47-3 | 100 | 20 |
| 1.2-6 | 80 | 20 |
| 1.2-8 | 100 | 20 |
| I.2-32 | 100 | 20 |
| I.2-43 | 100 | 20 |
| I.6-3 | 100 | 20 |
| 1.2-145 | 100 | 20 |
| I.5-3 | 80 | 20 |
| I.5-2 | 80 | 20 |
| I.12-3 | 100 | 20 |
| I.1-293 | 100 | 20 |
| I.1-233 | 100 | 20 |
| I.1-231 | 100 | 20 |
| I.1-153 | 100 | 20 |
| I.1-152 | 100 | 20 |

In den nachstehenden Tabllen A17 bis A21 sind die Kulturverträglichkeiten ausgewählter Verbindungen der allgemeinen Formel (I) gemäß der Tabellen 1.1 bis 1.50 bei einer Aufwandmenge entsprechend 80 g/ha oder niedriger, die bei Versuchen gemäß zuvor genannter Versuchvorschrift beobachtet wurden, dargestellt. Es werden dabei die beobachteten Effekte an ausgewählten Kulturpflanzen im Vergleich zu den unbehandelten Kontrollen angegeben (Werte in %).

**Tabelle A17**

| Verbindung Beispiel Nr. | Oryza sativa (Wirkung in %) | Aufwandmenge [g/ha] |
|---|---|---|
| I.1-32 | 20 | 20 |
| I.1-102 | 0 | 5 |
| I.1-2-3 | 0 | 20 |
| I.1-144 | 20 | 5 |
| I.1-6 | 0 | 20 |
| I.1-8 | 20 | 5 |
| I.11-3 | 10 | 20 |
| I.8-51 | 0 | 20 |
| I.47-6 | 20 | 20 |
| I.47-3 | 20 | 20 |
| 1.2-6 | 10 | 20 |
| 1.2-8 | 0 | 20 |
| I.2-32 | 0 | 20 |
| I.2-43 | 20 | 20 |
| I.6-3 | 20 | 20 |
| I.6-43 | 0 | 20 |
| 1.2-145 | 0 | 20 |
| I.4-43 | 10 | 20 |
| I.12-3 | 0 | 20 |
| I.14-3 | 0 | 20 |
| I.1-233 | 20 | 20 |
| I.1-231 | 10 | 5 |
| I.1-153 | 10 | 5 |
| I.1-152 | 10 | 5 |
| I.1-3 | 20 | 20 |
| I.1-72 | 10 | 20 |
| 1.8-42 | 0 | 20 |
| I.1-42 | 10 | 20 |
| I.1-22 | 0 | 5 |
| I.2-2 | 20 | 5 |
| I.46-2 | 20 | 80 |
| I.8-23 | 20 | 5 |
| I.1-241 | 10 | 5 |
| I.1-242 | 20 | 5 |
| I.1-183 | 20 | 20 |
| I.1-254 | 0 | 80 |
| I.1-255 | 0 | 80 |
| I.1-251 | 20 | 20 |
| I.1-182 | 20 | 20 |
| I.1-145 | 20 | 5 |
| I.1-151 | 10 | 20 |
| I.1-141 | 0 | 20 |
| I.1-132 | 20 | 80 |

**Tabelle A18**

| Verbindung Beispiel Nr. | Zea mays (Wirkung in %) | Aufwandmenge [g/ha] |
|---|---|---|
| I.1-32 | 20 | 20 |
| I.1-102 | 0 | 5 |
| I.1-2-3 | 0 | 20 |
| I.1-144 | 10 | 5 |
| I.1-6 | 10 | 20 |
| I.47-6 | 20 | 20 |
| 1.2-6 | 0 | 5 |
| 1.2-8 | 0 | 20 |
| I.2-32 | 20 | 5 |
| I.2-43 | 20 | 20 |
| I.1-231 | 20 | 5 |
| I.1-127 | 20 | 5 |
| I.46-2 | 20 | 20 |
| I.1-1 | 10 | 5 |
| I.1-242 | 20 | 80 |
| I.1-183 | 10 | 20 |
| I.1-254 | 0 | 80 |
| I.1-184 | 20 | 20 |
| I.1-255 | 10 | 80 |
| I.1-251 | 10 | 20 |
| I.1-182 | 20 | 20 |
| I.1-151 | 20 | 5 |
| I.1-141 | 10 | 5 |
| I.1-132 | 20 | 20 |

**Tabelle A19**

| Verbindung Beispiel Nr. | Brassica napis (Wirkung in %) | Aufwandmenge [g/ha] |
|---|---|---|
| I.1-6 | 0 | 5 |
| I.1-3 | 0 | 20 |
| I.46-2 | 20 | 5 |
| I.1-254 | 20 | 20 |
| I.1-255 | 10 | 20 |

**Tabelle A20**

| Verbindung Beispiel Nr. | Glycine max (Wirkung in %) | Aufwandmenge [g/ha] |
|---|---|---|
| 1.47-6 | 20 | 5 |
| 1.2-6 | 20 | 5 |
| I.2-43 | 20 | 5 |
| I.14-3 | 20 | 20 |
| I.1-152 | 20 | 5 |
| I.46-2 | 20 | 5 |
| I.1-241 | 10 | 5 |
| I.1-254 | 20 | 20 |
| I.1-255 | 20 | 20 |

**Tabelle A21**

| Verbindung Beispiel Nr. | Triticum aestivum (Wirkung in %) | Aufwandmenge [g/ha] |
|---|---|---|
| I.1-32 | 20 | 5 |
| I.1-142 | 20 | 20 |
| I.1-6 | 0 | 20 |
| I.1-8 | 20 | 20 |
| I.11-3 | 10 | 5 |
| I.1-43 | 10 | 5 |
| I.8-43 | 10 | 5 |
| I.1-51 | 0 | 5 |
| I.8-46 | 20 | 5 |
| 1.1-46 | 20 | 20 |
| I.8-51 | 20 | 20 |
| I.8-44 | 20 | 5 |
| I.47-6 | 20 | 5 |
| I.47-3 | 20 | 20 |
| 1.2-6 | 20 | 20 |
| 1.2-8 | 0 | 20 |
| I.2-32 | 20 | 5 |
| I.2-43 | 10 | 20 |
| 1.2-145 | 20 | 20 |
| I.4-43 | 10 | 20 |
| I.5-3 | 20 | 5 |
| I.5-2 | 20 | 5 |
| I.12-3 | 20 | 20 |
| I.14-3 | 20 | 20 |
| I.1-231 | 20 | 5 |
| I.1-152 | 20 | 5 |
| I.1-3 | 10 | 20 |
| I.1-72 | 20 | 20 |
| I.8-42 | 20 | 5 |
| I.1-42 | 20 | 20 |
| I.1-22 | 20 | 20 |
| I.2-2 | 20 | 5 |
| I.46-2 | 20 | 20 |
| I.1-5 | 20 | 80 |
| I.1-23 | 20 | 20 |
| I.8-23 | 20 | 20 |
| I.1-241 | 10 | 5 |
| I.1-1 | 20 | 80 |
| I.1-242 | 20 | 5 |
| I.1-254 | 10 | 20 |
| I.1-255 | 10 | 20 |
| I.1-251 | 10 | 20 |
| I.1-182 | 10 | 5 |
| I.1-151 | 20 | 80 |
| I.1-141 | 20 | 20 |
| I.1-132 | 10 | 20 |

Wie die Ergebnisse zeigen, weisen erfindungsgemäße Verbindungen der allgemeinen Formel (I) bei Behandlung im Nachauflauf eine gute herbizide Wirksamkeit gegen Schadpflanzen auf wie z. B. *Abutilon theophrasti, Alopecurus myosuroides, Amaranthus retroflexus, Avena fatua, Echinochloa crus-galli, Hordeum murinum, Lolium multiflorum, Lolium rigidum, Matricaria inodora, Pharbitis purpurea, Polygonum convolvulus, Setaria viridis, Stellaria media, Veronica persica* und *Viola tricolor* bei einer Aufwandmenge von 0,08 kg Aktivsubstanz oder weniger pro Hektar.

Die beprobten Kulturpflanzen Brassica napus, Glycine max, Oryza sativa, Triticum aestivum und Zea mays werden nach Applikation erfindungsgemäßer Verbindungen der allgemeinen Formel (I) bei einer Aufwandmenge von 0.005 bis 0.08 kg Aktivdsubstanz pro Hektar nicht oder nur gerinfügig beieinträchtigt.

### B. Herbizide Wirkung und Kulturverträglichkeit im Vorauflauf

Samen von mono- bzw. dikotylen Unkraut und Kulturpflanzen wurden in Kunststoff- oder organischen Pflanztöpfen ausgelegt und mit Erde abgedeckt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

Entsprechend wurden auch die Kulturpflanzenverträglichkeiten bonitiert.

In den nachstehenden Tabllen B1 bis B17 sind die Wirkungen ausgewählter Verbindungen der allgemeinen Formel (I) gemäß der Tabellen 1.1 bis 1.50 auf verschiedene Schadpflanzen und einer Aufwandmenge entsprechend 320 g/ha oder niedriger, die gemäß zuvor genannter Versuchvorschrift erhalten wurden, dargestellt.

**Tabelle B1**

| Verbindung Beispiel Nr. | Alopecurus myosuroides (Wirkung in %) | Aufwandmenge [g/ha] |
|---|---|---|
| I.1-1 | 100 | 320 |
| I.1-2 | 90 | 320 |
| I.1-3 | 90 | 80 |
| I.1-5 | 100 | 320 |
| I.1-22 | 80 | 320 |
| I.1-23 | 100 | 320 |
| I.1-42 | 90 | 320 |
| I.1-127 | 90 | 320 |
| I.1-132 | 100 | 320 |
| I.1-141 | 80 | 320 |
| I.1-182 | 80 | 320 |
| I.1-183 | 90 | 320 |
| I.1-184 | 90 | 320 |
| I.1-241 | 100 | 320 |
| I.1-242 | 90 | 320 |
| I.1-251 | 100 | 320 |
| I.1-254 | 100 | 320 |
| I.1-255 | 90 | 320 |
| I.2-2 | 100 | 320 |
| I.8-2 | 80 | 320 |
| I.46-2 | 90 | 320 |
| I.1-32 | 90 | 320 |
| I.1-8 | 80 | 320 |

**Tabelle B2**

| Verbindung Beispiel Nr. | Echinochloa crus-galli (Wirkung in %) | Aufwandmenge [g/ha] |
|---|---|---|
| I.1-1 | 100 | 320 |
| I.1-2 | 100 | 320 |
| I.1-3 | 100 | 320 |
| I.1-5 | 100 | 320 |
| I.1-22 | 100 | 320 |
| I.1-23 | 100 | 320 |
| I.1-42 | 100 | 320 |
| I.1-72 | 100 | 320 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 320 |
| I.1-241 | 100 | 320 |
| I.1-242 | 100 | 320 |
| I.1-251 | 100 | 320 |
| I.1-254 | 100 | 320 |
| I.1-255 | 100 | 320 |
| I.2-2 | 100 | 320 |
| I.8-2 | 100 | 320 |
| I.8-23 | 100 | 320 |
| I.8-42 | 100 | 320 |
| 1.8-127 | 100 | 320 |
| I.46-2 | 100 | 320 |
| I.1-32 | 100 | 320 |
| I.1-142 | 90 | 80 |
| I.1-8 | 100 | 320 |

**Tabelle B3**

| Verbindung Beispiel Nr. | Setaria viridis (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 320 |
| I.1-22 | 100 | 80 |
| I.1-23 | 100 | 80 |
| I.1-42 | 100 | 80 |
| I.1-72 | 100 | 80 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-251 | 100 | 320 |
| I.1-254 | 100 | 320 |
| I.1-255 | 100 | 320 |
| I.2-2 | 100 | 320 |
| I.8-2 | 100 | 320 |
| I.8-23 | 100 | 320 |
| I.8-42 | 100 | 320 |
| I.8-127 | 100 | 320 |
| I.46-2 | 100 | 320 |
| I.1-32 | 100 | 320 |
| I.1-142 | 100 | 80 |
| I.1-8 | 100 | 320 |

**Tabelle B4**

| Verbindung Beispiel Nr. | Abutilon theophrasti (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 320 |
| I.1-22 | 100 | 80 |
| I.1-23 | 100 | 80 |
| I.1-42 | 100 | 80 |
| I.1-72 | 100 | 80 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-251 | 100 | 320 |
| I.1-254 | 100 | 320 |
| I.1-255 | 100 | 320 |
| I.2-2 | 100 | 320 |
| I.8-2 | 100 | 320 |
| I.8-23 | 100 | 320 |
| I.8-42 | 100 | 320 |
| I.8-127 | 100 | 320 |
| I.46-2 | 100 | 320 |
| I.1-32 | 100 | 320 |
| I.1-142 | 100 | 80 |
| I.1-8 | 100 | 320 |

**Tabelle B5**

| Verbindung Beispiel Nr. | Amaranthus retroflexus (Wirkung in %) | Aufwandmenge [g/ha] |
|---|---|---|
| I.1-1 | 100 | 20 |
| I.1-2 | 100 | 80 |
| I.1-3 | 100 | 80 |
| I.1-5 | 100 | 80 |
| I.1-22 | 100 | 80 |
| I.1-23 | 100 | 80 |
| I.1-42 | 100 | 20 |
| I.1-72 | 100 | 20 |
| I.1-127 | 100 | 20 |
| I.1-132 | 100 | 80 |
| I.1-141 | 100 | 80 |
| I.1-151 | 100 | 80 |
| I.1-182 | 100 | 80 |
| I.1-183 | 100 | 80 |
| I.1-184 | 100 | 80 |
| I.1-241 | 100 | 80 |
| I.1-242 | 100 | 80 |
| I.1-251 | 100 | 80 |
| I.1-254 | 100 | 80 |
| I.1-255 | 100 | 80 |
| I.2-2 | 100 | 80 |
| I.8-2 | 100 | 20 |
| I.8-23 | 100 | 80 |
| I.8-42 | 100 | 20 |
| I.8-127 | 100 | 20 |
| I.46-2 | 100 | 80 |
| I.1-32 | 100 | 320 |
| I.1-142 | 100 | 80 |
| I.1-8 | 100 | 320 |

**Tabelle B6**

| Verbindung Beispiel Nr. | Matricaria inodora (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 80 |
| I.1-22 | 100 | 80 |
| I.1-23 | 100 | 800 |
| I.1-42 | 100 | 80 |
| I.1-72 | 100 | 80 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-251 | 100 | 320 |
| I.1-254 | 100 | 320 |
| I.1-255 | 100 | 320 |
| I.2-2 | 100 | 320 |
| I.8-2 | 100 | 320 |
| I.8-23 | 100 | 80 |
| I.8-42 | 100 | 320 |
| I.8-127 | 100 | 320 |
| I.46-2 | 100 | 320 |
| I.1-32 | 100 | 320 |
| I.1-142 | 100 | 80 |
| I.1-8 | 100 | 320 |

**Tabelle B7**

| Verbindung Beispiel Nr. | Polygonum convolvulus (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 320 |
| I.1-22 | 100 | 80 |
| I.1-23 | 100 | 80 |
| I.1-42 | 100 | 80 |
| I.1-72 | 100 | 80 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-251 | 100 | 320 |
| I.1-254 | 100 | 320 |
| I.1-255 | 100 | 320 |
| I.2-2 | 100 | 320 |
| I.8-2 | 100 | 320 |
| I.8-23 | 100 | 320 |
| I.8-42 | 100 | 320 |
| I.8-127 | 100 | 320 |
| I.46-2 | 100 | 320 |
| I.1-32 | 100 | 320 |
| I.1-142 | 100 | 80 |
| I.1-8 | 100 | 320 |

**Tabelle B8**

| Verbindung Beispiel Nr. | Stellaria media (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 80 |
| I.1-22 | 100 | 80 |
| I.1-23 | 100 | 80 |
| I.1-42 | 100 | 80 |
| I.1-72 | 100 | 80 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-254 | 100 | 320 |
| I.1-255 | 100 | 320 |
| I.2-2 | 100 | 320 |
| I.8-2 | 100 | 320 |
| I.8-23 | 100 | 80 |
| I.8-42 | 100 | 320 |
| I.8-127 | 100 | 320 |
| I.46-2 | 100 | 320 |
| I.1-32 | 100 | 320 |
| I.1-142 | 100 | 80 |
| I.1-8 | 100 | 320 |

**Tabelle B9**

| Verbindung Beispiel Nr. | Viola tricolor (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 320 |
| I.1-22 | 100 | 80 |
| I.1-23 | 100 | 80 |
| I.1-42 | 100 | 80 |
| I.1-72 | 100 | 80 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-251 | 100 | 320 |
| I.1-254 | 100 | 320 |
| I.1-255 | 100 | 320 |
| I.2-2 | 100 | 320 |
| I.8-2 | 100 | 320 |
| I.8-23 | 100 | 320 |
| I.8-42 | 100 | 320 |
| I.1-32 | 100 | 320 |
| I.1-142 | 100 | 80 |
| I.1-8 | 100 | 320 |

**Tabelle B10**

| Verbindung Beispiel Nr. | Veronica persica (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 320 |
| I.1-22 | 100 | 320 |
| I.1-23 | 100 | 80 |
| I.1-42 | 100 | 80 |
| I.1-72 | 100 | 80 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-251 | 100 | 320 |
| I.1-254 | 100 | 320 |
| I.1-255 | 100 | 320 |
| I.2-2 | 100 | 80 |
| I.8-2 | 100 | 80 |
| I.8-23 | 100 | 320 |
| I.8-42 | 100 | 320 |
| I.8-127 | 100 | 320 |
| I.46-2 | 100 | 320 |
| I.1-32 | 100 | 320 |
| I.1-142 | 100 | 80 |
| I.1-8 | 100 | 320 |

**Tabelle B11**

| Verbindung Beispiel Nr. | Pharbitis purpurea (Wirkung in %) | Aufwandmenge [g/ha] |
|---|---|---|
| I.1-1 | 100 | 320 |
| I.1-2 | 100 | 80 |
| I.1-3 | 100 | 320 |
| I.1-5 | 100 | 320 |
| I.1-22 | 100 | 320 |
| I.1-23 | 100 | 80 |
| I.1-42 | 100 | 80 |
| I.1-72 | 100 | 320 |
| I.1-127 | 100 | 320 |
| I.1-132 | 100 | 320 |
| I.1-141 | 100 | 320 |
| I.1-151 | 100 | 320 |
| I.1-182 | 100 | 320 |
| I.1-183 | 100 | 320 |
| I.1-184 | 100 | 80 |
| I.1-241 | 100 | 320 |
| I.1-242 | 100 | 80 |
| I.1-251 | 100 | 320 |
| I.1-254 | 100 | 320 |
| I.1-255 | 100 | 320 |
| I.2-2 | 100 | 320 |
| I.8-2 | 100 | 320 |
| I.8-23 | 100 | 320 |
| I.8-42 | 100 | 320 |
| I.8-127 | 100 | 320 |
| I.46-2 | 100 | 320 |
| I.1-32 | 100 | 320 |
| I.1-142 | 90 | 80 |
| I.1-8 | 100 | 320 |

**Tabelle B12**

| Verbindung Beispiel Nr. | Hordeum murinum (Wirkung in %) | Aufwandmenge [g/ha] |
|---|---|---|
| I.1-1 | 90 | 320 |
| I.1-2 | 90 | 320 |
| I.1-5 | 100 | 320 |
| I.1-22 | 100 | 320 |
| I.1-23 | 100 | 320 |
| I.1-42 | 100 | 320 |
| I.1-72 | 80 | 320 |
| I.1-127 | 100 | 320 |
| I.1-132 | 80 | 320 |
| I.1-141 | 100 | 320 |
| I.1-151 | 80 | 320 |
| I.1-182 | 90 | 320 |
| I.1-183 | 90 | 320 |
| I.1-184 | 90 | 320 |
| I.1-241 | 100 | 320 |
| I.1-242 | 90 | 320 |
| I.1-251 | 90 | 320 |
| I.1-254 | 90 | 320 |
| I.1-255 | 100 | 320 |
| I.2-2 | 100 | 320 |
| I.8-2 | 80 | 320 |
| I.8-23 | 80 | 320 |
| I.8-42 | 80 | 320 |
| I.8-127 | 90 | 320 |
| I.1-32 | 90 | 320 |

**Tabelle B13**

| Verbindung Beispiel Nr. | Avena fatua (Wirkung in %) | Aufwandmenge [g/ha] |
|---|---|---|
| I.1-2 | 90 | 320 |
| I.1-5 | 100 | 320 |
| I.1-23 | 100 | 320 |
| I.1-42 | 90 | 320 |
| I.1-72 | 90 | 320 |
| I.1-127 | 90 | 320 |
| I.1-132 | 90 | 320 |
| I.1-141 | 80 | 320 |
| I.1-151 | 90 | 320 |
| I.1-182 | 90 | 320 |
| I.1-183 | 90 | 320 |
| I.1-184 | 90 | 320 |
| I.1-241 | 100 | 320 |
| I.1-242 | 80 | 320 |
| I.1-251 | 90 | 320 |
| I.1-254 | 90 | 320 |
| I.1-255 | 90 | 320 |
| I.8-2 | 80 | 320 |
| I.8-42 | 80 | 320 |

**Tabelle B14**

| Verbindung Beispiel Nr. | Lolium multiflorum (Wirkung in %) | Aufwandmenge [g/ha] |
|---|---|---|
| I.1-3 | 100 | 80 |

**Tabelle B15**

| Verbindung Beispiel Nr. | Lolium rigidum (Wirkung in %) | Aufwandmenge [g/ha] |
|---|---|---|
| I.1-1 | 100 | 320 |
| I.1-2 | 100 | 320 |
| I.1-5 | 100 | 320 |
| I.1-22 | 100 | 320 |
| I.1-23 | 100 | 80 |
| I.1-42 | 100 | 320 |
| I.1-72 | 90 | 320 |
| I.1-127 | 100 | 320 |
| I.1-132 | 100 | 320 |
| I.1-141 | 100 | 320 |
| I.1-151 | 90 | 320 |
| I.1-182 | 100 | 320 |
| I.1-183 | 90 | 320 |
| I.1-184 | 100 | 320 |
| I.1-241 | 100 | 320 |
| I.1-242 | 100 | 320 |
| I.1-254 | 100 | 320 |
| I.1-255 | 100 | 320 |
| I.2-2 | 100 | 320 |
| I.8-2 | 90 | 80 |
| I.8-23 | 90 | 320 |
| I.8-42 | 80 | 320 |
| I.8-127 | 90 | 320 |
| I.46-2 | 100 | 320 |
| I.1-32 | 90 | 320 |
| I.1-8 | 90 | 320 |

**Tabelle B16**

| Verbindung Beispiel Nr. | Cyperus esculentus (Wirkung in %) | Aufwandmenge [g/ha] |
|---|---|---|
| I.1-3 | 100 | 320 |
| I.1-5 | 80 | 320 |
| I.1-22 | 90 | 320 |
| I.1-42 | 100 | 320 |
| I.1-72 | 100 | 320 |
| I.1-182 | 80 | 320 |
| I.1-183 | 80 | 320 |
| I.1-241 | 100 | 320 |
| I.1-242 | 80 | 80 |
| I.1-251 | 80 | 320 |
| I.1-254 | 80 | 320 |
| I.2-2 | 90 | 320 |
| I.8-2 | 90 | 320 |
| I.8-23 | 90 | 320 |
| I.8-42 | 100 | 320 |
| I.8-127 | 90 | 320 |

**Tabelle B17**

| Verbindung Beispiel Nr. | Digitaria sanguinalis (Wirkung in %) | Aufwandmenge [g/ha] |
|---|---|---|
| I.1-32 | 100 | 320 |
| I.1-142 | 100 | 80 |
| 1.1- | 100 | 320 |

Wie die Ergebnisse zeigen, weisen erfindungsgemäße Verbindungen der allgemeinen Formel (I) bei Behandlung im Vorauflauf eine gute herbizide Wirksamkeit gegen Schadpflanzen auf, z. B. gegen Schadpflanzen wie *Abutilon theophrasti*, *Alopecurus myosuroides*, *Amaranthus retroflexus, Avena fatua, Cyperus esculentus, Echinochloa crus-galli, Hordeum murinum, Lolium rigidum*, *Matricaria inodora*, *Pharbitis purpurea*, *Polygonum convolvulus, Setaria viridis, Stellaria media, Veronica persica* und *Viola tricolor* bei einer Aufwandmenge von 0.32 kg Aktivsubstanz oder weniger pro Hektar.

## Patentansprüche

1. Substituierte Thiophenyluracile der allgemeinen Formel (I) oder deren Salze 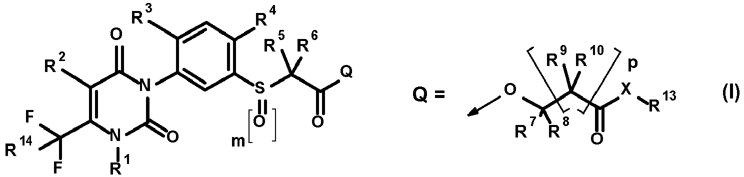 worin
R¹ für (C₁-C₈)-Alkyl, Amino, Bis-[(C₁-C₈)-alkyl]amino steht,
R² für Wasserstoff, (C₁-C₈)-Alkyl steht,
R³ für Wasserstoff, Halogen, (C₁-C₈)-Alkoxy steht,
R⁴ für Halogen, Cyano, NO₂, C(O)NH₂, C(S)NH₂, (C₁-C₈)-Haloalkyl, (C₂-C₈)-Alkinyl steht,
R⁵ und R⁶ unabhängig voneinander für Wasserstoff, Halogen, (C₁-C₈)-Alkyl, (C₃-C₈)-Cycloalkyl, (C₃-C₈)-Cycloalkyl-(C₁-C₈)-alkyl, (C₂-C₈)-Alkenyl, (C₂-C₈)-Alkinyl, (C₁-C₁₀)-Haloalkyl, (C₂-C₈)-Haloalkenyl, (C₂-C₈)-Haloalkinyl, (C₃-C₁₀)-Halocycloalkyl, (C₄-C₁₀)-Cycloalkenyl, (C₄-C₁₀)-Halocycloalkenyl, (C₁-C₈)-Alkoxy, (C₁-C₈)-Alkoxy-(C₁-C₈)-alkyl, (C₁-C₈)-Alkoxy-(C₁-C₈)-haloalkyl, (C₁-C₈)-Haloalkoxy-(C₁-C₈)-haloalkyl, (C₁-C₈)-Haloalkoxy-(C₁-C₈)-alkyl, Aryl, Aryl-(C₁-C₈)-alkyl, Heteroaryl, Heteroaryl-(C₁-C₈)-alkyl, (C₄-C₁₀)-Cycloalkenyl-(C₁-C₈)-alkyl, Heterocyclyl, Heterocyclyl-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkylcarbonyl-(C₁-C₈)-alkyl, C(O)OR¹⁷, C(O)NR¹⁵R¹⁶, C(O)R¹⁷, R¹⁷O(O)C-(C₁-C₈)-alkyl, R¹⁵R¹⁶N(O)C-(C₁-C₈)-alkyl, R¹⁵R¹⁶N-(C₁-C₈)-alkyl stehen, oder
R⁵ und R⁶ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁵ und R⁶ mit dem Kohlenstoffatom, an das sie gebunden sind, eine gegebenenfalls weiter durch R¹⁹ und R²⁰ substituierte Doppelbindung, gemäß nachfolgender Formel (I'), bilden 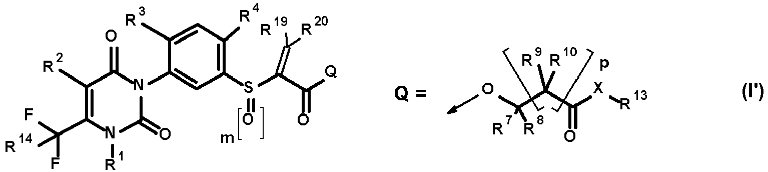
m für 0, 1, 2 steht,
p für 0, 1, 2, 3, 4, 5 steht,
X für O (Sauerstoff) oder die Gruppierungen N-R¹¹ oder N-O-R¹² steht und wobei R¹¹ und R¹² in den Gruppierung N-R¹¹ und N-O-R¹² unabhängig voneinander die Bedeutungen gemäß der nachfolgenden Definitionen haben,
R⁷, R⁸, R⁹ und R¹⁰ unabhängig voneinander für Wasserstoff, Fluor, (C₁-C₈)-Alkyl, (C₃-C₈)-Cycloalkyl, (C₃-C₈)-Cycloalkyl-(C₁-C₈)-alkyl, (C₂-C₈)-Alkenyl, (C₂-C₈)-Alkinyl, (C₁-C₁₀)-Haloalkyl, (C₂-C₈)-Haloalkenyl, (C₂-C₈)-Haloalkinyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-haloalkyl, (C₁-C₈)-Haloalkoxy-(C₁-C₈)-haloalkyl, Aryl, Aryl-(C₁-C₈)-alkyl, Heteroaryl, Heteroaryl-(C₁-C₈)-alkyl, (C₄-C₁₀)-Cycloalkenyl-(C₁-C₈)-alkyl, Heterocyclyl, Heterocyclyl-(C₁-C₈)-alkyl, R¹⁷O-(C₁-C₈)-alkyl, R¹⁸S-(C₁-C₈)-alkyl, (C₁-C₈)-Alkylcarbonyl-(C₁-C₈)-alkyl, C(O)OR¹⁷, C(O)NR¹⁵R¹⁶, C(O)R¹⁷, R¹⁷O(O)C-(C₁-C₈)-alkyl, R¹⁵R¹⁶N(O)C-(C₁-C₈)-alkyl, R¹⁵R¹⁶N-(C₁-C₈)-alkyl stehen, oder
R⁵ und R⁷ mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 8-gliedrigen monocyclischen oder bicyclischen Ring bilden, oder
R⁷ und R⁸ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹⁰ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹⁰ mit dem Kohlenstoffatom, an das sie gebunden sind, eine Exomethylengruppe bilden oder
R⁷ und R⁹ mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹¹ für Wasserstoff, (C₁-C₈)-Alkyl, (C₃-C₈)-Cycloalkyl, Cyano-(C₁-C₈)-alkyl, (C₃-C₈)-Cycloalkyl-(C₁-C₈)-alkyl, (C₁-C₈)-Alkylsulfonyl, Arylsulfonyl, Heteroarylsulfonyl, (C₃-C₈)-Cycloalkylsulfonyl, Heterocyclylsulfonyl, Aryl-(C₁-C₈)-alkylsulfonyl, (C₁-C₈)-Alkylcarbonyl, Arylcarbonyl, Heteroarylcarbonyl, (C₃-C₈)-Cycloalkylcarbonyl, Heterocyclylcarbonyl, (C₁-C₈)-Alkoxycarbonyl, (C₁-C₈)-Alkoxy, (C₂-C₈)-Alkenyloxy, Aryl-(C₁-C₈)-alkoxycarbonyl, (C₁-C₈)-Haloalkylcarbonyl, (C₂-C₈)-Alkenyl, (C₂-C₈)-Alkinyl, (C₁-C₈)-Haloalkyl, Halo-(C₂-C₈)-alkinyl, Halo-(C₂-C₈)-alkenyl, (C₁-C₈)-Alkoxy-(C₁-C₈)-alkyl, Amino, (C₁-C₈)-Alkylamino, Bis[(C₁-C₈)-alkyl]amino, (C₁-C₈)-Alkoxy-(C₁-C₈)-alkoxy-(C₁-C₈)-alkyl, Heteroaryl-(C₁-C₈)-alkylsulfonyl, Heterocyclyl-(C₁-C₈)-alkylsulfonyl, (C₂-C₈)-Alkenyloxycarbonyl, (C₂-C₈)-Alkinyloxycarbonyl, (C₁-C₈)-Alkylaminocarbonyl, (C₃-C₈)-Cycloalkylaminocarbonyl, Bis-[(C₁-C₈)-alkyl]aminocarbonyl steht,
R¹² für Wasserstoff, (C₁-C₈)-Alkyl, (C₃-C₈)-Cycloalkyl-(C₁-C₈)-alkyl, (C₂-C₈)-Alkenyl, (C₂-C₈)-Alkinyl, (C₁-C₈)-Alkoxy-(C₁-C₈)-alkyl, Aryl, Aryl-(C₁-C₈)-alkyl, R¹⁷O(O)C-(C₁-C₈)-alkyl, R¹⁵R¹⁶N(O)C-(C₁-C₈)-alkyl stehen,
R¹³ für Wasserstoff, (C₁-C₈)-Alkyl, (C₁-C₈)-Haloalkyl, (C₃-C₈)-Cycloalkyl, (C₃-C₈)-Cycloalkyl-(C₁-C₈)-alkyl, (C₂-C₈)-Alkenyl, (C₂-C₈)-Alkinyl, (C₁-C₈)-Alkoxy-(C₁-C₈)-alkyl, (C₁-C₈)-Haloalkoxy-(C₁-C₈)-alkyl, Aryl, Aryl-(C₁-C₈)-alkyl, Heteroaryl, Heteroaryl-(C₁-C₈)-alkyl, Heterocyclyl, Heterocyclyl-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkylcarbonyl-(C₁-C₈)-alkyl, R¹⁷O(O)C-(C₁-C₈)-alkyl, R¹⁵R¹⁶N(O)C-(C₁-C₈)-alkyl, R¹⁵R¹⁶N-(C₁-C₈)-alkyl stehen, oder
R⁷ und R¹³ mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹³ mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R¹¹ und R¹³ in dem Fall, daß X für N-R¹¹ steht, mit dem Stickstoffatom, an das sie gebunden sind, einen vollständig gesättigten oder gegebenenfalls durch Heteroatome unterbrochenen und gegebenenfalls weiter substituierten 4 bis 10-gliedrigen monocyclischen oder bicyclischen Ring bilden, oder
R¹² und R¹³ in dem Fall, daß X für N-O-R¹² steht, mit dem Stickstoffatom, an das sie gebunden sind, einen vollständig gesättigten und gegebenenfalls weiter substituierten 4 bis 10-gliedrigen monocyclischen oder bicyclischen Ring bilden,
R¹⁴ für Wasserstoff, Fluor, Chlor, Brom, (C₁-C₅)-Haloalkyl, (C₁-C₈)-Alkoxy steht,
R¹⁵ und R¹⁶ gleich oder verschieden sind und unabhängig voneinander für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₂-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, COR¹⁷, SO₂R¹⁸, (C₁-C₈)-Alkyl-HNO₂S-, (C₃-C₈)-Cycloalkyl-HNO₂S-, Heterocyclyl, (C₁-C₈)-Alkoxycarbonyl-(C₁-C₈)-alkyl, (C₁-C₈)-Alkoxycarbonyl, Aryl-(C₁-C₈)-Alkoxycarbonyl-(C₁-C₈)-alkyl, Aryl-(C₁-C₈)-Alkoxycarbonyl, Heteroaryl-(C₁-C₈)-Alkoxycarbonyl, (C₂-C₈)-Alkenyloxycarbonyl, (C₂-C₈)-Alkinyloxycarbonyl, Heterocyclyl-(C₁-C₈)-alkyl stehen,
R¹⁷ für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₂-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, (C₁-C₈)-Alkoxycarbonyl-(C₁-C₈)-alkyl, (C₂-C₈)-Alkenyloxycarbonyl-(C₁-C₈)-alkyl, Aryl-(C₁-C₈)-Alkoxycarbonyl-(C₁-C₈)-alkyl, Hydroxycarbonyl-(C₁-C₈)-alkyl, Heterocyclyl, Heterocyclyl-(C₁-C₈)-alkyl steht,
R¹⁸ für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₂-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₁₀)-Cycloalkenyl-(C₁-C₈)-alkyl, NR¹⁵R¹⁶ steht
und
R¹⁹ und R²⁰ unabhängig voneinander für Wasserstoff, Halogen, (C₁-C₈)-Alkyl, (C₃-C₈)-Cycloalkyl, (C₂-C₈)-Alkenyl, (C₂-C₈)-Alkinyl, (C₁-C₁₀)-Haloalkyl, Aryl stehen, oder
R¹⁹ und R²⁰ mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden.

2. Verbindung der allgemeinen Formel (I) gemäß Anspruch 1und/oder deren Salz, **dadurch gekennzeichnet, dass**
R¹ für (C₁-C₇)-Alkyl, Amino, Bis-[(C₁-C₇)-alkyl]amino steht,
R² für Wasserstoff, (C₁-C₇)-Alkyl steht,
R³ für Wasserstoff, Halogen, (C₁-C₇)-Alkoxy steht,
R⁴ für Halogen, Cyano, NO₂, C(O)NH₂, C(S)NH₂, (C₁-C₇)-Haloalkyl, (C₂-C₇)-Alkinyl steht,
R⁵ und R⁶ unabhängig voneinander für Wasserstoff, Halogen, (C₁-C₇)-Alkyl, (C₃-C₇)-Cycloalkyl, (C₃-C₇)-Cycloalkyl-(C₁-C₇)-alkyl, (C₂-C₇)-Alkenyl, (C₂-C₇)-Alkinyl, (C₁-C₁₀)-Haloalkyl, (C₂-C₇)-Haloalkenyl, (C₂-C₇)-Haloalkinyl, (C₃-C₁₀)-Halocycloalkyl, (C₄-C₁₀)-Cycloalkenyl, (C₄-C₁₀)-Halocycloalkenyl, (C₁-C₇)-Alkoxy, (C₁-C₇)-Alkoxy-(C₁-C₇)-alkyl, (C₁-C₇)-Alkoxy-(C₁-C₇)-haloalkyl, (C₁-C₇)-Haloalkoxy-(C₁-C₇)-haloalkyl, (C₁-C₇)-Haloalkoxy-(C₁-C₇)-alkyl, Aryl, Aryl-(C₁-C₇)-alkyl, Heteroaryl, Heteroaryl-(C₁-C₇)-alkyl, (C₄-C₁₀)-Cycloalkenyl-(C₁-C₇)-alkyl, Heterocyclyl, Heterocyclyl-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkylcarbonyl-(C₁-C₇)-alkyl, C(O)OR¹⁷, C(O)NR¹⁵R¹⁶, C(O)R¹⁷, R¹⁷O(O)C-(C₁-C₇)-alkyl, R¹⁵R¹⁶N(O)C-(C₁-C₇)-alkyl, R¹⁵R¹⁶N-(C₁-C₇)-alkyl stehen, oder
R⁵ und R⁶ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁵ und R⁶ mit dem Kohlenstoffatom, an das sie gebunden sind, eine gegebenenfalls weiter durch R¹⁹ und R²⁰ substituierte Doppelbindung, gemäß nachfolgender Formel (I'), bilden 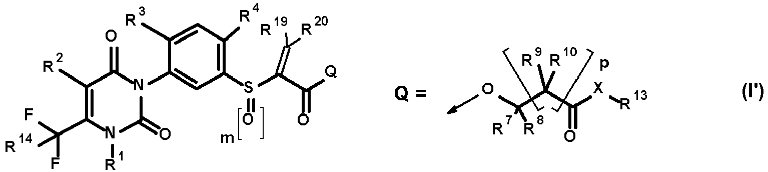
m für 0, 1, 2 steht,
p für 0, 1, 2, 3 steht,
X für O (Sauerstoff), N (Stickstoff) oder die Gruppierungen N-R¹¹ oder N-O-R¹² steht und wobei R¹¹ und R¹² in den Gruppierung N-R¹¹ und N-O-R¹² unabhängig voneinander die Bedeutungen gemäß der nachfolgenden Definitionen haben,
R⁷, R⁸, R⁹ und R¹⁰ unabhängig voneinander für Wasserstoff, Fluor, (C₁-C₇)-Alkyl, (C₃-C₇)-Cycloalkyl, (C₃-C₇)-Cycloalkyl-(C₁-C₇)-alkyl, (C₂-C₇)-Alkenyl, (C₂-C₇)-Alkinyl, (C₁-C₁₀)-Haloalkyl, (C₂-C₇)-Haloalkenyl, (C₂-C₇)-Haloalkinyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-haloalkyl, (C₁-C₇)-Haloalkoxy-(C₁-C₇)-haloalkyl, Aryl, Aryl-(C₁-C₇)-alkyl, Heteroaryl, Heteroaryl-(C₁-C₇)-alkyl, (C₄-C₁₀)-Cycloalkenyl-(C₁-C₇)-alkyl, Heterocyclyl, Heterocyclyl-(C₁-C₇)-alkyl, R¹⁷O-(C₁-C₇)-alkyl, R¹⁸S-(C₁-C₇)-alkyl, (C₁-C₇)-Alkylcarbonyl-(C₁-C₇)-alkyl, C(O)OR¹⁷, C(O)NR¹⁵R¹⁶, C(O)R¹⁷, R¹⁷O(O)C-(C₁-C₇)-alkyl, R¹⁵R¹⁶N(O)C-(C₁-C₇)-alkyl, R¹⁵R¹⁶N-(C₁-C₇)-alkyl stehen, oder
R⁵ und R⁷ mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 8-gliedrigen monocyclischen oder bicyclischen Ring bilden, oder
R⁷ und R⁸ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹⁰ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹⁰ mit dem Kohlenstoffatom, an das sie gebunden sind, eine Exomethylengruppe bilden oder
R⁷ und R⁹ mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹¹ für Wasserstoff, (C₁-C₇)-Alkyl, (C₃-C₇)-Cycloalkyl, Cyano-(C₁-C₇)-alkyl, (C₃-C₇)-Cycloalkyl-(C₁-C₇)-alkyl, (C₁-C₇)-Alkylsulfonyl, Arylsulfonyl, Heteroarylsulfonyl, (C₃-C₇)-Cycloalkylsulfonyl, Heterocyclylsulfonyl, Aryl-(C₁-C₇)-alkylsulfonyl, (C₁-C₇)-Alkylcarbonyl, Arylcarbonyl, Heteroarylcarbonyl, (C₃-C₇)-Cycloalkylcarbonyl, Heterocyclylcarbonyl, (C₁-C₇)-Alkoxycarbonyl, (C₁-C₇)-Alkoxy, (C₂-C₇)-Alkenyloxy, Aryl-(C₁-C₇)-alkoxycarbonyl, (C₁-C₇)-Haloalkylcarbonyl, (C₂-C₇)-Alkenyl, (C₂-C₇)-Alkinyl, (C₁-C₇)-Haloalkyl, Halo-(C₂-C₇)-alkinyl, Halo-(C₂-C₇)-alkenyl, (C₁-C₇)-Alkoxy-(C₁-C₇)-alkyl, Amino, (C₁-C₇)-Alkylamino, Bis[(C₁-C₇)-alkyl]amino, (C₁-C₇)-Alkoxy-(C₁-C₇)-alkoxy-(C₁-C₇)-alkyl, Heteroaryl-(C₁-C₇)-alkylsulfonyl, Heterocyclyl-(C₁-C₇)-alkylsulfonyl, (C₂-C₇)-Alkenyloxycarbonyl, (C₂-C₇)-Alkinyloxycarbonyl, (C₁-C₇)-Alkylaminocarbonyl, (C₃-C₇)-Cycloalkylaminocarbonyl, Bis-[(C₁-C₇)-alkyl]aminocarbonyl steht,
R¹² für Wasserstoff, (C₁-C₇)-Alkyl, (C₃-C₇)-Cycloalkyl-(C₁-C₇)-alkyl, (C₂-C₇)-Alkenyl, (C₂-C₇)-Alkinyl, (C₁-C₇)-Alkoxy-(C₁-C₇)-alkyl, Aryl, Aryl-(C₁-C₇)-alkyl, R¹⁷O(O)C-(C₁-C₇)-alkyl, R¹⁵R¹⁶N(O)C-(C₁-C₇)-alkyl stehen,
R¹³ für Wasserstoff, (C₁-C₇)-Alkyl, (C₁-C₇)-Haloalkyl, (C₃-C₇)-Cycloalkyl, (C₃-C₇)-Cycloalkyl-(C₁-C₇)-alkyl, (C₂-C₇)-Alkenyl, (C₂-C₇)-Alkinyl, (C₁-C₇)-Alkoxy-(C₁-C₇)-alkyl, (C₁-C₇)-Haloalkoxy-(C₁-C₇)-alkyl, Aryl, Aryl-(C₁-C₇)-alkyl, Heteroaryl, Heteroaryl-(C₁-C₇)-alkyl, Heterocyclyl, Heterocyclyl-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkylcarbonyl-(C₁-C₇)-alkyl, R¹⁷O(O)C-(C₁-C₇)-alkyl, R¹⁵R¹⁶N(O)C-(C₁-C₇)-alkyl, R¹⁵R¹⁶N-(C₁-C₇)-alkyl stehen, oder
R⁷ und R¹³ mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹³ mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R¹¹ und R¹³ in dem Fall, daß X für N-R¹¹ steht, mit dem Stickstoffatom, an das sie gebunden sind, einen vollständig gesättigten oder gegebenenfalls durch Heteroatome unterbrochenen und gegebenenfalls weiter substituierten 4 bis 10-gliedrigen monocyclischen oder bicyclischen Ring bilden, oder
R¹² und R¹³ in dem Fall, daß X für N-O-R¹² steht, mit dem Stickstoffatom, an das sie gebunden sind, einen vollständig gesättigten und gegebenenfalls weiter substituierten 4 bis 10-gliedrigen monocyclischen oder bicyclischen Ring bilden,
R¹⁴ für Wasserstoff, Fluor, Chlor, Brom, (C₁-C₅)-Haloalkyl, (C₁-C₇)-Alkoxy steht,
R¹⁵ und R¹⁶ gleich oder verschieden sind und unabhängig voneinander für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₂-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Haloalkoxy-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, COR¹⁷, SO₂R¹⁸, (C₁-C₇)-Alkyl-HNO₂S-, (C₃-C₇)-Cycloalkyl-HNO₂S-, Heterocyclyl, (C₁-C₇)-Alkoxycarbonyl-(C₁-C₇)-alkyl, (C₁-C₇)-Alkoxycarbonyl, Aryl-(C₁-C₇)-Alkoxycarbonyl-(C₁-C₇)-alkyl, Aryl-(C₁-C₇)-Alkoxycarbonyl, Heteroaryl-(C₁-C₇)-Alkoxycarbonyl, (C₂-C₇)-Alkenyloxycarbonyl, (C₂-C₇)-Alkinyloxycarbonyl, Heterocyclyl-(C₁-C₇)-alkyl stehen,
R¹⁷ für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₂-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, (C₁-C₇)-Alkoxycarbonyl-(C₁-C₇)-alkyl, (C₂-C₇)-Alkenyloxycarbonyl-(C₁-C₇)-alkyl, Aryl-(C₁-C₇)-Alkoxycarbonyl-(C₁-C₇)-alkyl, Hydroxycarbonyl-(C₁-C₇)-alkyl, Heterocyclyl, Heterocyclyl-(C₁-C₇)-alkyl steht
R¹⁸ für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₂-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, Heterocyclyl-(C₁-C₇)-alkyl, (C₃-C₇)-Cycloalkyl-(C₁-C₇)-alkyl, (C₄-C₁₀)-Cycloalkenyl-(C₁-C₇)-alkyl, NR¹⁵R¹⁶ steht
und
R¹⁹ und R²⁰ unabhängig voneinander für Wasserstoff, Halogen, (C₁-C₇)-Alkyl, (C₃-C₇)-Cycloalkyl, (C₂-C₇)-Alkenyl, (C₂-C₇)-Alkinyl, (C₁-C₇)-Haloalkyl, Aryl stehen, oder
R¹⁹ und R²⁰ mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden.

3. Verbindungen der allgemeinen Formel (I) gemäß Anspruch 1und/oder deren Salz, **dadurch gekennzeichnet, dass**
R¹ für (C₁-C₆)-Alkyl, Amino, Bis-[(C₁-C₆)-alkyl]amino steht,
R² für Wasserstoff, (C₁-C₆)-Alkyl steht,
R³ für Wasserstoff, Halogen, (C₁-C₆)-Alkoxy steht,
R⁴ für Halogen, Cyano, NO₂, C(O)NH₂, C(S)NH₂, (C₁-C₆)-Haloalkyl, (C₂-C₆)-Alkinyl steht,
R⁵ und R⁶ unabhängig voneinander für Wasserstoff, Halogen, (C₁-C₆)-Alkyl, (C₃-C₆)-Cycloalkyl, (C₃-C₆)-Cycloalkyl-(C₁-C₆)-alkyl, (C₂-C₆)-Alkenyl, (C₂-C₆)-Alkinyl, (C₁-C₁₀)-Haloalkyl, (C₂-C₆)-Haloalkenyl, (C₂-C₆)-Haloalkinyl, (C₃-C₁₀)-Halocycloalkyl, (C₄-C₁₀)-Cycloalkenyl, (C₄-C₁₀)-Halocycloalkenyl, (C₁-C₆)-Alkoxy, (C₁-C₆)-Alkoxy-(C₁-C₆)-alkyl, (C₁-C₆)-Alkoxy-(C₁-C₆)-haloalkyl, (C₁-C₆)-Haloalkoxy-(C₁-C₆)-haloalkyl, (C₁-C₆)-Haloalkoxy-(C₁-C₆)-alkyl, Aryl, Aryl-(C₁-C₆)-alkyl, Heteroaryl, Heteroaryl-(C₁-C₆)-alkyl, (C₄-C₁₀)-Cycloalkenyl-(C₁-C₆)-alkyl, Heterocyclyl, Heterocyclyl-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkylcarbonyl-(C₁-C₆)-alkyl, C(O)OR¹⁷, C(O)NR¹⁵R¹⁶, C(O)R¹⁷, R¹⁷O(O)C-(C₁-C₆)-alkyl, R¹⁵R¹⁶N(O)C-(C₁-C₆)-alkyl, R¹⁵R¹⁶N-(C₁-C₆)-alkyl stehen, oder
R⁵ und R⁶ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁵ und R⁶ mit dem Kohlenstoffatom, an das sie gebunden sind, eine gegebenenfalls weiter durch
R¹⁹ und R²⁰ substituierte Doppelbindung, gemäß nachfolgender Formel (I'), bilden 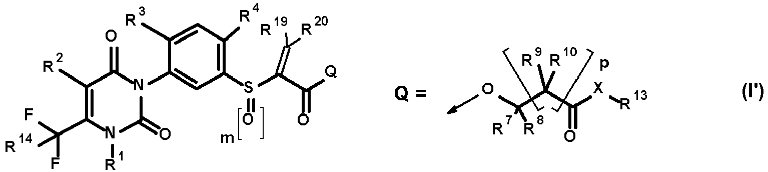
m für 0, 1, 2 steht,
p für 0, 1, 2, 3 steht,
X für O (Sauerstoff), N (Stickstoff) oder die Gruppierungen N-R¹¹ oder N-O-R¹² steht und wobei R¹¹ und R¹² in den Gruppierung N-R¹¹ und N-O-R¹² unabhängig voneinander die Bedeutungen gemäß der nachfolgenden Definitionen haben,
R⁷ R⁸, R⁹ und R¹⁰ unabhängig voneinander für Wasserstoff, Fluor, (C₁-C₆)-Alkyl, (C₃-C₆)-Cycloalkyl, (C₃-C₆)-Cycloalkyl-(C₁-C₆)-alkyl, (C₂-C₆)-Alkenyl, (C₂-C₆)-Alkinyl, (C₁-C₁₀)-Haloalkyl, (C₂-C₆)-Haloalkenyl, (C₂-C₆)-Haloalkinyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-haloalkyl, (C₁-C₆)-Haloalkoxy-(C₁-C₆)-haloalkyl, Aryl, Aryl-(C₁-C₆)-alkyl, Heteroaryl, Heteroaryl-(C₁-C₆)-alkyl, (C₄-C₁₀)-Cycloalkenyl-(C₁-C₆)-alkyl, Heterocyclyl, Heterocyclyl-(C₁-C₆)-alkyl, R¹⁷O-(C₁-C₆)-alkyl, R¹⁸S-(C₁-C₆)-alkyl, (C₁-C₆)-Alkylcarbonyl-(C₁-C₆)-alkyl, C(O)OR¹⁷, C(O)NR¹⁵R¹⁶, C(O)R¹⁷, R¹⁷O(O)C-(C₁-C₆)-alkyl, R¹⁵R¹⁶N(O)C-(C₁-C₆)-alkyl, R¹⁵R¹⁶N-(C₁-C₆)-alkyl stehen, oder
R⁵ und R⁷ mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 8-gliedrigen monocyclischen oder bicyclischen Ring bilden, oder
R⁷ und R⁸ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹⁰ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹⁰ mit dem Kohlenstoffatom, an das sie gebunden sind, eine Exomethylengruppe bilden oder
R⁷ und R⁹ mit den Kohlenstoffatomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹¹ für Wasserstoff, (C₁-C₆)-Alkyl, (C₃-C₆)-Cycloalkyl, Cyano-(C₁-C₆)-alkyl, (C₃-C₆)-Cycloalkyl-(C₁-C₆)-alkyl, (C₁-C₆)-Alkylsulfonyl, Arylsulfonyl, Heteroarylsulfonyl, (C₃-C₆)-Cycloalkylsulfonyl, Heterocyclylsulfonyl, Aryl-(C₁-C₆)-alkylsulfonyl, (C₁-C₆)-Alkylcarbonyl, Arylcarbonyl, Heteroarylcarbonyl, (C₃-C₆)-Cycloalkylcarbonyl, Heterocyclylcarbonyl, (C₁-C₆)-Alkoxycarbonyl, (C₁-C₆)-Alkoxy, (C₂-C₆)-Alkenyloxy, Aryl-(C₁-C₆)-alkoxycarbonyl, (C₁-C₆)-Haloalkylcarbonyl, (C₂-C₆)-Alkenyl, (C₂-C₆)-Alkinyl, (C₁-C₆)-Haloalkyl, Halo-(C₂-C₆)-alkinyl, Halo-(C₂-C₆)-alkenyl, (C₁-C₆)-Alkoxy-(C₁-C₆)-alkyl, Amino, (C₁-C₆)-Alkylamino, Bis[(C₁-C₆)-alkyl]amino, (C₁-C₆)-Alkoxy-(C₁-C₆)-alkoxy-(C₁-C₆)-alkyl, Heteroaryl-(C₁-C₆)-alkylsulfonyl, Heterocyclyl-(C₁-C₆)-alkylsulfonyl, (C₂-C₆)-Alkenyloxycarbonyl, (C₂-C₆)-Alkinyloxycarbonyl, (C₁-C₆)-Alkylaminocarbonyl, (C₃-C₆)-Cycloalkylaminocarbonyl, Bis-[(C₁-C₆)-alkyl]aminocarbonyl steht,
R¹² für Wasserstoff, (C₁-C₆)-Alkyl, (C₃-C₆)-Cycloalkyl-(C₁-C₆)-alkyl, (C₂-C₆)-Alkenyl, (C₂-C₆)-Alkinyl, (C₁-C₆)-Alkoxy-(C₁-C₆)-alkyl, Aryl, Aryl-(C₁-C₆)-alkyl, R¹⁷O(O)C-(C₁-C₆)-alkyl, R¹⁵R¹⁶N(O)C-(C₁-C₆)-alkyl stehen,
R¹³ für Wasserstoff, (C₁-C₆)-Alkyl, (C₁-C₆)-Haloalkyl, (C₃-C₆)-Cycloalkyl, (C₃-C₆)-Cycloalkyl-(C₁-C₆)-alkyl, (C₂-C₆)-Alkenyl, (C₂-C₆)-Alkinyl, (C₁-C₆)-Alkoxy-(C₁-C₆)-alkyl, (C₁-C₆)-Haloalkoxy-(C₁-C₆)-alkyl, Aryl, Aryl-(C₁-C₆)-alkyl, Heteroaryl, Heteroaryl-(C₁-C₆)-alkyl, Heterocyclyl, Heterocyclyl-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkylcarbonyl-(C₁-C₆)-alkyl, R¹⁷O(O)C-(C₁-C₆)-alkyl, R¹⁵R¹⁶N(O)C-(C₁-C₆)-alkyl, R¹⁵R¹⁶N-(C₁-C₆)-alkyl stehen, oder
R⁷ und R¹³ mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁹ und R¹³ mit den Atomen, an die sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R¹¹ und R¹³ in dem Fall, daß X für N-R¹¹ steht, mit dem Stickstoffatom, an das sie gebunden sind, einen vollständig gesättigten oder gegebenenfalls durch Heteroatome unterbrochenen und gegebenenfalls weiter substituierten 4 bis 10-gliedrigen monocyclischen oder bicyclischen Ring bilden, oder
R¹² und R¹³ in dem Fall, daß X für N-O-R¹² steht, mit dem Stickstoffatom, an das sie gebunden sind, einen vollständig gesättigten und gegebenenfalls weiter substituierten 4 bis 10-gliedrigen monocyclischen oder bicyclischen Ring bilden,
R¹⁴ für Wasserstoff, Fluor, Chlor, Brom, (C₁-C₅)-Haloalkyl, (C₁-C₆)-Alkoxy steht,
R¹⁵ und R¹⁶ gleich oder verschieden sind und unabhängig voneinander für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₂-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, COR¹⁷, SO₂R¹⁸, (C₁-C₆)-Alkyl-HNO₂S-, (C₃-C₆)-Cycloalkyl-HNO₂S-, Heterocyclyl, (C₁-C₆)-Alkoxycarbonyl-(C₁-C₆)-alkyl, (C₁-C₆)-Alkoxycarbonyl, Aryl-(C₁-C₆)-Alkoxycarbonyl-(C₁-C₆)-alkyl, Aryl-(C₁-C₆)-Alkoxycarbonyl, Heteroaryl-(C₁-C₆)-Alkoxycarbonyl, (C₂-C₆)-Alkenyloxycarbonyl, (C₂-C₆)-Alkinyloxycarbonyl, Heterocyclyl-(C₁-C₆)-alkyl stehen,
R¹⁷ für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₂-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, (C₁-C₆)-Alkoxycarbonyl-(C₁-C₆)-alkyl, (C₂-C₆)-Alkenyloxycarbonyl-(C₁-C₆)-alkyl, Aryl-(C₁-C₆)-Alkoxycarbonyl-(C₁-C₆)-alkyl, Hydroxycarbonyl-(C₁-C₆)-alkyl, Heterocyclyl, Heterocyclyl-(C₁-C₆)-alkyl steht,
R¹⁸ für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₂-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₁₀)-Cycloalkenyl-(C₁-C₆)-alkyl, NR¹⁵R¹⁶ steht
und
R¹⁹ und R²⁰ unabhängig voneinander für Wasserstoff, Halogen, (C₁-C₆)-Alkyl, (C₃-C₆)-Cycloalkyl, (C₂-C₆)-Alkenyl, (C₂-C₆)-Alkinyl, (C₁-C₆)-Haloalkyl, Aryl stehen, oder
R¹⁹ und R²⁰ mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden.

4. Verbindungen der allgemeinen Formel (I) gemäß Anspruch 1und/oder deren Salz, **dadurch gekennzeichnet, dass**
R¹ für Methyl, Ethyl, n-Propyl, 1 -Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbuty1, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Amino, Dimethylamino, Diethylamino, Methyl(ethyl)amino, Methyl(n-propyl)amino steht,
R² für Wasserstoff, Methyl, Ethyl, n-Propyl, iso-Propyl steht,
R³ für Wasserstoff, Fluor, Chlor, Brom, Methoxy, Ethoxy steht,
R⁴ für Halogen, Cyano, NO₂, C(O)NH₂, C(S)NH₂, Difluormethyl, Trifluormethyl, Ethinyl, Propin-1-yl, 1-Butin-1-yl, Pentin-1-yl, Hexin-1-yl steht,
R⁵ und R⁶ unabhängig voneinander für Wasserstoff, Fluor, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexy1, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl,Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanocyclopropyl, 2-Cyanocyclopropyl, 1-Methylcyclobutyl, 2-Methylcyclobutyl, 3-Methylcyclobutyl, 3,3-Dimethylcyclobut-1-yl, 1-Cyanocyclobutyl, 2-Cyanocyclobutyl, 3-Cyanocyclobutyl, 3,3-Difluorcyclobut-1-yl, 3-Fluorcyclobut-1-yl, 2,2-Difluorcycloprop-1-yl, 1-Fluorcycloprop-1-yl, 2-Fluorcycloprop-1-yl, 1-Allylcyclopropyl, 1-Vinylcyclobutyl, 1-Vinylcyclopropyl, 1-Ethylcyclopropyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, 2-Fluorcycloprop-1-yl, 4-Fluorcyclohexyl, 4,4-Difluorcyclohexyl, Cyclopropylmethyl, Cyclobutylmethyl, Cyclopentylmethyl, Cyclohexylmethyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Ioddifluormethyl, Bromfluormethyl, 1-Fluorethyl, 2-Fluorethyl, Fluormethyl, Difluormethyl, 2,2-Difluorethyl, 2,2,2-Trifluorethyl, Difluor-tert.-butyl, Chlormethyl, Brommethyl, Methoxy, Ethoxy, n-Propyloxy, iso-Propyloxy, n-Butyloxy, tert-Butyloxy, Methoxymethyl, Ethoxymethyl, n-Propyloxymethyl, iso-Propyloxymethyl, Methoxyethyl, Ethoxyethyl, n-Propyloxyethyl, iso-Propyloxyethyl, Methoxy-n-propyl, Methoxydifluormethyl, Ethoxydifluormethyl, n-propyloxydifluormethyl, n-Butyloxydifluormethyl, Trifluormethoxymethyl, Trifluormethoxyethyl, Trifluormethoxy-n-propyl, Phenyl, 2-Fluor-Phenyl, 3-Fluor-Phenyl, 4-Fluor-Phenyl, 2,4-Difluor-Phenyl, 2,5-Difluor-Phenyl, 2,6-Difluor-Phenyl, 2,3-Difluor-Phenyl, 3,4-Difluor-Phenyl, 3,5-Difluor-Phenyl, 2,4,5-Trifluor-Phenyl, 3,4,5-Trifluor-Phenyl, 2-Chlor-Phenyl, 3-Chlor-Phenyl, 4-Chlor-Phenyl, 2,4-Dichlor-Phenyl, 2,5-Dichlor-Phenyl, 2,6-Dichlor-Phenyl, 2,3-Dichlor-Phenyl, 3,4-Dichlor-Phenyl, 3,5-Dichlor-Phenyl, 2,4,5-Trichlor-Phenyl, 3,4,5-Trichlor-Phenyl, 2,4,6-Trichlor-Phenyl, 2-Brom-Phenyl, 3-Brom-Phenyl, 4-Brom-Phenyl, 2-Iod-Phenyl, 3-Iod-Phenyl, 4-Iod-Phenyl, 2-Brom-4-Fluor-Phenyl, 2-Brom-4-Chlor-Phenyl, 3-Brom-4-Fluor-Phenyl, 3-Brom-4-Chlor-Phenyl, 3-Brom-5-Fluor-Phenyl, 3-Brom-5-Chlor-Phenyl, 2-Fluor-4-Brom-Phenyl, 2-Chlor-4-Brom-Phenyl, 3-Fluor-4-Brom-Phenyl, 3-Chlor-4-Brom-Phenyl, 2-Chlor-4-Fluor-Phenyl, 3-Chlor-4-Fluor-Phenyl, 2-Fluor-3-Chlor-Phenyl, 2-Fluor-4-Chlor-Phenyl, 2-Fluor-5-Chlor-Phenyl, 3-Fluor-4-Chlor-Phenyl, 3-Fluor-5-Chlor-Phenyl, 2-Fluor-6-Chlor-Phenyl, 2-Methyl-Phenyl, 3-Methyl-Phenyl, 4-Methyl-Phenyl, 2,4-Dimethyl-Phenyl, 2,5-Dimethyl-Phenyl, 2,6-Dimethyl-Phenyl, 2,3-Dimethyl-Phenyl, 3,4-Dimethyl-Phenyl, 3,5-Dimethyl-Phenyl, 2,4,5-Trimethyl-Phenyl, 3,4,5-Trimethyl-Phenyl, 2,4,6-Trimethyl-Phenyl, 2-Methoxy-Phenyl, 3-Methoxy-Phenyl, 4-Methoxy-Phenyl, 2,4-Dimethoxy-Phenyl, 2,5-Dimethoxy-Phenyl, 2,6-Dimethoxy-Phenyl, 2,3-Dimethoxy-Phenyl, 3,4-Dimethoxy-Phenyl, 3,5-Dimethoxy-Phenyl, 2,4,5-Trimethoxy-Phenyl, 3,4,5-Trimethoxy-Phenyl, 2,4,6-Trimethoxy-Phenyl, 2-Trifluormethoxy-Phenyl, 3-Trifluormethoxy-Phenyl, 4-Trifluormethoxy-Phenyl, 2-Difluormethoxy-Phenyl, 3-Difluormethoxy-Phenyl, 4-Difluormethoxy-Phenyl, 2-Trifluormethyl-Phenyl, 3-Trifluormethyl-Phenyl, 4-Trifluormethyl-Phenyl, 2-Difluormethyl-Phenyl, 3-Difluormethyl-Phenyl, 4-Difluormethyl-Phenyl, 3,5-Bis(Trifluormethyl)-Phenyl, 3-Trifluormethyl-5-Fluor-Phenyl, 3-Trifluormethyl-5-Chlor-Phenyl, 3-Methyl-5-Fluor-Phenyl, 3-Methyl-5-Chlor-Phenyl, 3-Methoxy-5-Fluor-Phenyl, 3-Methoxy-5-Chlor-Phenyl, 3-Trifluormethoxy-5-Chlor-Phenyl, 2-Ethoxy-Phenyl, 3-Ethoxy-Phenyl, 4-Ethoxy-Phenyl, 2-Methylthio-Phenyl, 3-Methylthio-Phenyl, 4-Methylthio-Phenyl, 2-Trifluormethylthio-Phenyl, 3-Trifluormethylthio-Phenyl, 4-Trifluormethylthio-Phenyl, 2-Ethyl-Phenyl, 3-Ethyl-Phenyl, 4-Ethyl-Phenyl, 2-Methoxycarbonyl-Phenyl, 3-Methoxycarbonyl-Phenyl, 4-Methoxycarbonyl-Phenyl, 2-Ethoxycarbonyl-Phenyl, 3-Ethoxycarbonyl-Phenyl, 4-Ethoxycarbonyl-Phenyl, Pyridin-2-yl, Pyridin-3-yl, Pyridin-4-yl, Pyrazin-2-yl, Pyridazin-3-yl, Pyridazin-4-yl, Pyrimidin-2-yl, Pyrimidin-5-yl, Pyrimidin-4-yl, Pyridazin-3-ylmethyl, Pyridazin-4-ylmethyl, Pyrimidin-2-ylmethyl, Pyrimidin-5-ylmethyl, Pyrimidin-4-ylmethyl, Pyrazin-2-ylmethyl, 3-Chlor-Pyrazin-2-yl, 3-Brom-Pyrazin-2-yl, 3-Methoxy-Pyrazin-2-yl, 3-Ethoxy-Pyrazin-2-yl, 3-Trifluormethylpyrazin-2-yl, 3-Cyanopyrazin-2-yl, Naphth-2-yl, Naphth-1-yl, Chinolin-4-yl, Chinolin-6-yl, Chinolin-8-yl, Chinolin-2-yl, Chinoxalin-2-yl, 2-Naphthylmethyl, 1-Naphthylmethyl, Chinolin-4-ylmethyl, Chinolin-6-ylmethyl, Chinolin-8-ylmethyl, Chinolin-2-ylmethyl, Chinoxalin-2-ylmethyl, Pyrazin-2-ylmethyl, 4-Chloropyridin-2-yl, 3-Chloropyridin-4-yl, 2-Chloropyridin-3-yl, 2-Chloropyridin-4-yl, 2-Chlorpyridin-5-yl, 2,6-Dichlorpyridin-4-yl, 3-Chlorpyridin-5-yl, 3,5-Dichlorpyridin-2-yl, 3-Chlor-5-Trifluormethylpyridin-2-yl, (4-Chloropyridin-2-yl)methyl, (3-Chloropyridin-4-yl)methyl, (2-Chloropyridin-3-yl)methyl, (2-Chloropyridin-4-yl)methyl, (2-Chlorpyridin-5-yl)methyl, (2,6-Dichlorpyridin-4-yl)methyl, (3-Chlorpyridin-5-yl)methyl, (3,5-Dichlorpyridin-2-yl)methyl, Thiophen-2-yl, Thiophen-3-yl, 5-Methylthiophen-2-yl, 5-Ethylthiophen-2-yl, 5-Chlorthiophen-2-yl, 5-Bromthiophen-2-yl, 4-Methylthiophen-2-yl, 3-Methylthiophen-2-yl, 5-Fluorthiophen-3-yl, 3,5-Dimethylthiophen-2-yl, 3-Ethylthiophen-2-yl, 4,5-Dimethylthiophen-2-yl, 3,4-Dimethylthiophen-2-yl, 4-Chlorthiophen-2-yl, Furan-2-yl, 5-Methylfuran-2-yl, 5-Ethylfuran-2-yl, 5-Methoxycarbonylfuran-2-yl, 5-Chlorfuran-2-yl, 5-Bromfuran-2-yl, Thiophan-2-yl, Thiophan-3-yl, Sulfolan-2-yl, Sulfolan-3-yl, Tetrahydrothiopyran-4-yl, Tetrahydropyran-4-yl, Tetrahydrofuran-2-yl, Tetrahydrofuran-3-yl, 1-(4-Methylphenyl)ethyl, 1-(3-Methylphenyl)ethyl, 1-(2-Methylphenyl)ethyl, 1-(4-Chlorphenyl)ethyl, 1-(3-Chlorphenyl)ethyl, 1-(2-Chlorphenyl)ethyl, Benzyl, (4-Fluorphenyl)methyl, (3-Fluorphenyl)methyl, (2-Fluorphenyl)methyl, (2,4-Difluorphenyl)methyl, (3,5-Difluorphenyl)methyl, (2,5-Difluorphenyl)methyl, (2,6-Difluorphenyl)methyl, (2,4,5-Trifluorphenyl)methyl, (2,4,6-Trifluorphenyl)methyl, (4-Chlorphenyl)methyl, (3-Chlorphenyl)methyl, (2-Chlorphenyl)methyl, (2,4-Dichlorphenyl)methyl, (3,5-Dichlorphenyl)methyl, (2,5-Dichlorphenyl)methyl, (2,6-Dichlorphenyl)methyl, (2,4,5-Trichlorphenyl)methyl, (2,4,6-Trichlorphenyl)methyl, (4-Bromphenyl)methyl, (3-Bromphenyl)methyl, (2-Bromphenyl)methyl, (4-Iodphenyl)methyl, (3-Iodphenyl)methyl, (2-Iodphenyl)methyl, (3-Chlor-5-Trifluormethyl-pyridin-2-yl)methyl, (2-Brom-4-Fluorphenl)methyl, (2-Brom-4-Chlorphenyl)methyl, (3-Brom-4-Fluorphenyl)methyl, (3-Brom-4-Chlorphenyl)methyl, (3-Brom-5-Fluorphenyl)methyl, (3-Brom-5-Chlorphenyl)methyl, (2-Fluor-4-Bromphenyl)methyl, (2-Chlor-4-Bromphenyl)methyl, (3 -Fluor-4-Bromphenyl)methyl, (3-Chlor-4-Bromphenyl)methyl, (2-Chlor-4-Fluorphenyl)methyl, (3-Chlor-4-Fluorphenyl)methyl, (2-Fluor-3-Chlorphenyl)methyl, (2-Fluor-4-Chlorphenyl)methyl, (2-Fluor-5-Chlorphenyl)methyl, (3-Fluor-4-Chlorphenyl)methyl, (3-Fluor-5-Chlorphenyl)methyl, (2-Fluor-6-Chlorphenyl)methyl, 2-Phenyleth-1-yl, 3-Trifluormethyl-4-Chlorphenyl, 3-Chlor-4-Trifluormethylphenyl, 2-Chlor-4-Trifluormethylphenyl, 3,5-Dfluorpyridm-2-yl, (3,6-Dichlor-pyridin-2-yl)methyl, (4-Trifluormethylphenyl)methyl, (3-Trifluormethylphenyl)methyl, (2-Trifluormethylphenyl)methyl, (4-Trifluormethoxyphenyl)methyl, (3-Trifluormethoxyphenyl)methyl, (2-Trifluormethoxyphenyl)methyl, (4-Methoxyphenyl)methyl, (3-Methoxyphenyl)methyl, (2-Methoxyphenyl)methyl, (4-Methylphenyl)methyl, (3-Methylphenyl)methyl, (2-Methylphenyl)methyl, (4-Cyanophenyl)methyl, (3-Cyanophenyl)methyl, (2-Cyanophenyl)methyl, (2,4-Diethylphenyl)methyl, (3,5-Diethylphenyl)methyl, (3,4-Dimethylphenyl)methyl, (3,5-Dimethoxyphenyl)methyl, 1-Phenyleth-1-yl, 1-(o-Chlorphenyl)eth-1-yl, 1,3-Thiazol-2-yl, 4-Methyl-1,3-thiazol-2-yl, 1,3-Thiazol-2-yl, Methylthiomethyl, Ethylthiomethyl, Ethylthioethyl, Methylthioethyl, n-Propylthiomethyl, iso-Propylthiomethyl, Trifluormethylthiomethyl, trifluormethylthioethyl, Methylcarbonylmethyl, Ethylcarbonylmethyl, n-Propylcarbonylmethyl, iso-Propylcarbonylmethyl, Methylcarbonylethyl, Hydroxycarbonyl, Methoxycarbonyl, Ethoxycarbonyl, n-Propyloxycarbonyl, iso-Propyloxycarbonyl, n-Butyloxycarbonyl, tert-Butyloxycarbonyl, Allyloxycarbonyl, Benzyloxycarbonyl, Aminocarbonyl, Methylaminocarbonyl, Ethylaminocarbonyl, n-Propylaminocarbonyl, iso-Propylaminocarbonyl, Dimethylaminocarbonyl, Diethylaminocarbonyl, Methyl(ethyl)aminocarbonyl, Cyclopropylaminocarbonyl, Cyclobutylaminocarbonyl, Cyclopentylaminocarbonyl, Cyclohexylaminocarbonyl, Allylaminocarbonyl, benzylaminocarbonyl, tert-Butyloxycarbonylaminocarbonyl, Hydroxycarbonylmethyl, Methoxycarbonylmethyl, Ethoxycarbonylmethyl, n-Propyloxycarbonylmethyl, iso-Propyloxycarbonylmethyl, n-Butyloxycarbonylmethyl, tert-Butyloxycarbonylmethyl, Allyloxycarbonylmethyl, Benzyloxycarbonylmethyl, Aminocarbonylmethyl, Methylaminocarbonylmethyl, Ethylaminocarbonylmethyl, n-Propylaminocarbonylmethyl, iso-Propylaminocarbonylmethyl, Dimethylaminocarbonylmethyl, Diethylaminocarbonylmethyl, Methyl(ethyl)aminocarbonylmethyl, Cyclopropylaminocarbonylmethyl, Cyclobutylaminocarbonylmethyl, Cyclopentylaminocarbonylmethyl, Cyclohexylaminocarbonylmethyl, Allylaminocarbonylmethyl, Benzylaminocarbonylmethyl, 1-(Hydroxycarbonyl)eth-1-yl, 1-(Methoxycarbonyl)eth-1-yl, 1-(Ethoxycarbonyl)eth-1-yl, 1-(tert-Butyloxycarbonyl)eth-1-yl, 1-(Benzyloxycarbonyl)eth-1-yl, 2-(Hydroxycarbonyl)eth-1-yl, 2-(Methoxycarbonyl)eth-1-yl, 2-(Ethoxycarbonyl)eth-1-yl, 2-(tert-Butyloxycarbonyl)eth-1 -yl, 2-(Benzyloxycarbonyl)eth-1-yl, 1-(Hydroxycarbonyl)prop-1-yl, 1-(Methoxycarbonyl)prop-1-yl, 1-(Ethoxycarbonyl)prop-1-yl, 1-(tert-Butyloxycarbonyl)prop-1-yl, 1-(Benzyloxycarbonyl)prop-1-yl, 2-(Hydroxycarbonyl)prop-1-yl, 2-(Methoxycarbonyl)prop-1-yl, 2-(Ethoxycarbonyl)prop-1-yl, 2-(tert-Butyloxycarbonyl)prop-1-yl, 2-(Benzyloxycarbonyl)prop-1-yl, 1-(Hydroxycarbonyl)prop-2-yl, 1-(Methoxycarbonyl)prop-2-yl, 1-(Ethoxycarbonyl)prop-2-yl, 1-(tert-Butyloxycarbonyl)prop-2-yl, 1-(Benzyloxycarbonyl)prop-2-yl, 3-(Hydroxycarbonyl)prop-1-yl, 3-(Methoxycarbonyl)prop-1-yl, 3-(Ethoxycarbonyl)prop-1-yl, 3-(tert-Butyloxycarbonyl)prop-1-yl, 3-(Benzyloxycarbonyl)prop-1-yl, Aminomethyl, 2-Aminoeth-1-yl, 1-Aminoeth1-yl, 1-Amino-prop-1-yl, 3-Amino-prop-1-yl, Methylaminomethyl, Dimethylaminomethyl, Diethylaminomethyl, Ethylaminomethyl, iso-Propylaminomethyl, Cyclopropylaminomethyl, Cyclobutylaminomethyl, Cyclopentylaminomethyl, Cyclohexylaminomethyl, Methoxycarbonylaminomethyl, Ethoxycarbonylaminomethyl tert-Butyloxycarbonylaminomethyl, Methylcarbonylaminomethyl, Ethylcarbonylaminomethyl, n-propylcarbonylaminomethyl, iso-Propylcarbonylaminomethyl, 2-(Methylamino)eth-1-yl, 2-(Dieth-1-ylamino)eth-1-yl, 2-(Diethylamino)eth-1-yl, 2-(Ethylamino)eth-1-yl, 2-(iso-Propylamino)eth-1-yl, 2-(Cyclopropylamino)eth-1-yl, 2-(Cyclobutylamino)eth-1-yl, 2-(Cyclopentylamino)eth-1-yl, 2-(Cyclohexylamino)eth-1-yl, 2-(Methoxycarbonylamino)eth-1-yl, 2-(Ethoxycarbonylamino)eth-1 -yl, 2-(tert-Butyloxycarbonylamino)eth-1 -yl, 2-(Methylcarbonylamino)eth-1 -yl, 2-(Ethylcarbonylamino)eth-1 -yl, 2-(n-Propylcarbonylamino)eth-1 -yl, 2-(iso-Propylcarbonylamino)eth-1 -yl, 1-(Methylamino)eth-1-yl, 1-(Dieth-1-ylamino)eth-1-yl, 1-(Diethylamino)eth-1-yl, 1-(Ethylamino)eth-1-yl, 1-(iso-Propylamino)eth-1-yl, 1-(Cyclopropylamino)eth-1-yl, 1-(Cyclobutylamino)eth-1-yl, 1-(Cyclopentylamino)eth-1 -yl, 1-(Cyclohexylamino)eth-1-yl, 1-(Methoxycarbonylamino)eth-1-yl, 1-(Ethoxycarbonylamino)eth-1-yl, 1-(tert-Butyloxycarbonylamino)eth-1-yl, 1-(Methylcarbonylamino)eth-1-yl, 1-(Ethylcarbonylamino)eth-1-yl, 1-(n-Propylcarbonylamino)eth-1-yl, 1-(iso-Propylcarbonylamino)eth-1-yl stehen, oder
R⁵ und R⁶ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁵ und R⁶ mit dem Kohlenstoffatom, an das sie gebunden sind, eine gegebenenfalls weiter durch R¹⁹ und R²⁰ substituierte Doppelbindung, gemäß nachfolgender Formel (I'), bilden 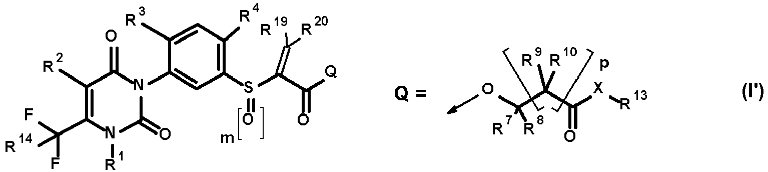
R¹⁴ für Wasserstoff, Fluor, Chlor, Brom, Trifluormethyl, Methoxy, Ethoxy, n-Propyloxy, n-Butyloxy steht,
m für 0, 1, 2 steht,
R¹⁹ und R²⁰ unabhängig voneinander für Wasserstoff, Fluor, Chlor, Brom, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, Cyclopropyl, Cyclobutyl, Cyclopentyl, Cyclohexyl, Trifluormethyl, Difluormethyl, Pentafluorethyl, Ethenyl, 1-Propenyl, 1-Methyl-ethenyl, 1-Butenyl, Phenyl stehen, oder
R¹⁹ und R²⁰ mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden,
und Q für eine der nachfolgend spezifisch genannten Gruppierungen Q-1 bis Q-300 steht:
| | | | | |
|---|---|---|---|---|
| 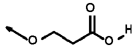 | 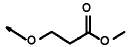 | 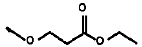 | 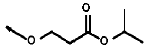 | 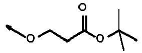 |
| Q-1 | Q-2 | Q-3 | Q-4 | Q-5 |
| | | | | |
| 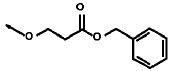 | 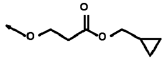 | 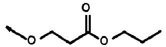 | 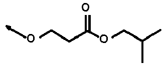 | 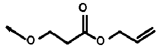 |
| Q-6 | Q-7 | Q-8 | Q-9 | Q-10 |
| | | | | |
| 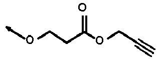 | 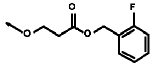 | 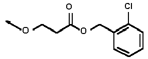 | 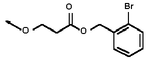 | 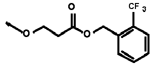 |
| Q-11 | Q-12 | Q-13 | Q-14 | Q-15 |
| | | | | |
| 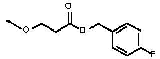 | 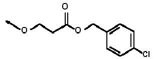 | 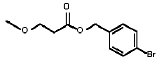 | 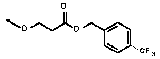 | 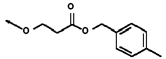 |
| Q-16 | Q-17 | Q-18 | Q-19 | Q-20 |
| | | | | |
| 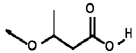 | 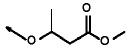 | 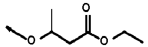 | 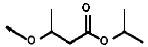 | 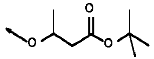 |
| Q-21 | Q-22 | Q-23 | Q-24 | Q-25 |
| | | | | |
| 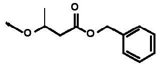 | 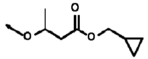 | 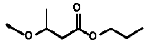 | 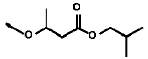 | 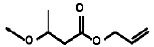 |
| Q-26 | Q-27 | Q-28 | Q-29 | Q-30 |
| 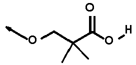 | 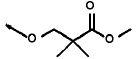 | 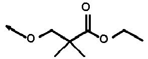 | 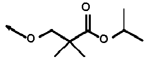 | 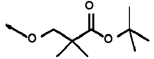 |
| Q-31 | Q-32 | Q-33 | Q-34 | Q-35 |
| | | | | |
| 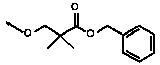 | 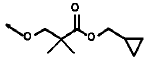 | 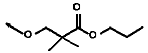 | 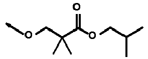 | 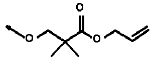 |
| Q-36 | Q-37 | Q-38 | Q-39 | Q-40 |
| | | | | |
| 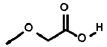 | 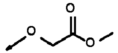 | 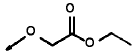 | 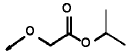 | 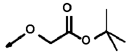 |
| Q-41 | Q-42 | Q-43 | Q-44 | Q-45 |
| | | | | |
| 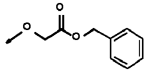 | 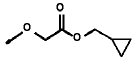 | 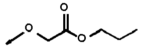 | 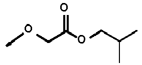 | 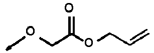 |
| Q-46 | Q-47 | Q-48 | Q-49 | Q-50 |
| | | | | |
| 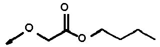 | 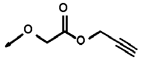 | 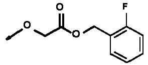 | 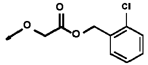 | 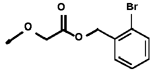 |
| Q-51 | Q-52 | Q-53 | Q-54 | Q-55 |
| | | | | |
| 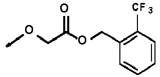 | 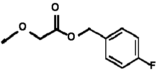 | 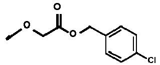 | 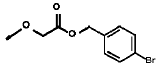 | 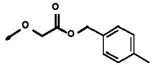 |
| Q-56 | Q-57 | Q-58 | Q-59 | Q-50 |
| | | | | |
| 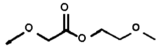 | 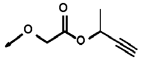 | 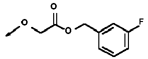 | 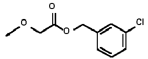 | 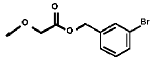 |
| Q-61 | Q-62 | Q-63 | Q-64 | Q-65 |
| | | | | |
| 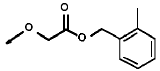 | 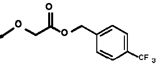 | 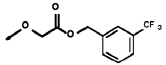 | 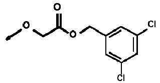 | 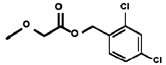 |
| Q-66 | Q-67 | Q-68 | Q-69 | Q-70 |
| 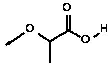 | 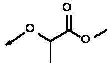 | 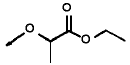 | 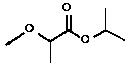 | 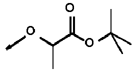 |
| Q-71 | Q-72 | Q-73 | Q-74 | Q-75 |
| | | | | |
| 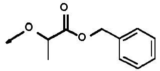 | 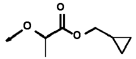 | 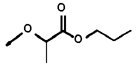 | 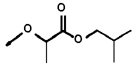 | 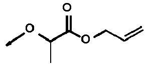 |
| Q-76 | Q-77 | Q-78 | Q-79 | Q-80 |
| | | | | |
| 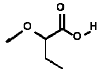 | 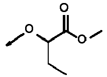 | 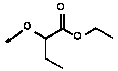 | 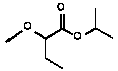 | 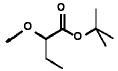 |
| Q-81 | Q-82 | Q-83 | Q-84 | Q-85 |
| | | | | |
| 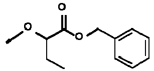 | 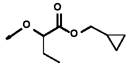 | 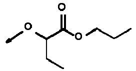 | 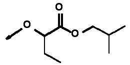 | 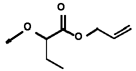 |
| Q-86 | Q-87 | Q-88 | Q-89 | Q-90 |
| | | | | |
| 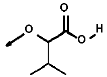 | 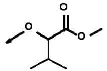 | 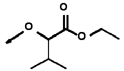 | 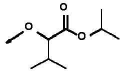 | 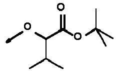 |
| Q-91 | Q-92 | Q-93 | Q-94 | Q-95 |
| | | | | |
| 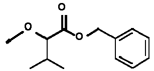 | 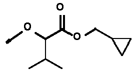 | 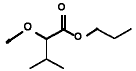 | 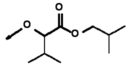 | 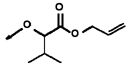 |
| Q-96 | Q-97 | Q-98 | Q-99 | Q-100 |
| | | | | |
| 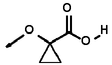 | 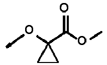 | 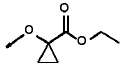 | 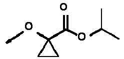 | 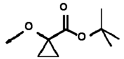 |
| Q-101 | Q-102 | Q-103 | Q-104 | Q-105 |
| | | | | |
| 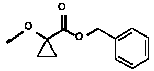 | 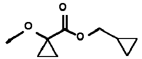 | 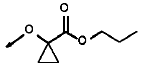 | 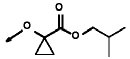 | 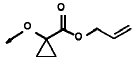 |
| Q-106 | Q-107 | Q-108 | Q-109 | Q-110 |
| 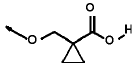 | 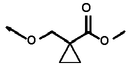 | 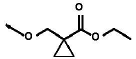 | 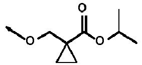 | 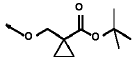 |
| Q-111 | Q-112 | Q-113 | Q-114 | Q-115 |
| | | | | |
| 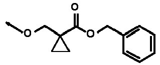 | 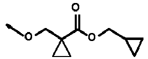 | 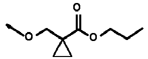 | 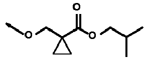 | 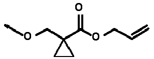 |
| Q-116 | Q-117 | Q-118 | Q-119 | Q-120 |
| | | | | |
| 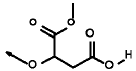 | 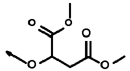 | 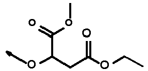 | 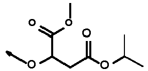 | 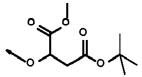 |
| Q-121 | Q-122 | Q-123 | Q-124 | Q-125 |
| | | | | |
| 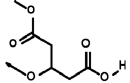 | 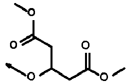 | 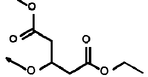 | 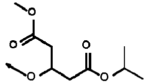 | 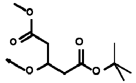 |
| Q-126 | Q-127 | Q-128 | Q-129 | Q-130 |
| | | | | |
| 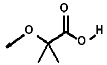 | 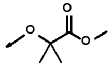 | 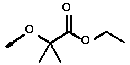 | 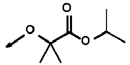 | 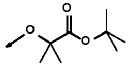 |
| Q-131 | Q-132 | Q-133 | Q-134 | Q-135 |
| | | | | |
| 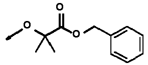 | 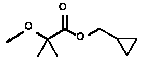 | 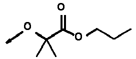 | 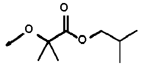 | 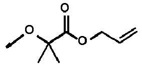 |
| Q-136 | Q-137 | Q-138 | Q-139 | Q-140 |
| | | | | |
| 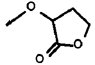 | 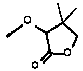 | 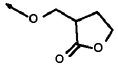 | 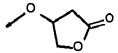 | 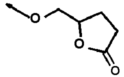 |
| Q-141 | Q-142 | Q-143 | Q-144 | Q-145 |
| | | | | |
| 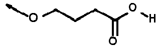 | 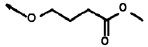 | 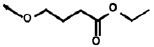 | 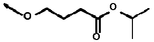 | 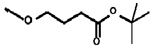 |
| Q-146 | Q-147 | Q-148 | Q-149 | Q-150 |
| 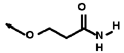 | 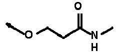 | 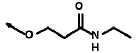 | 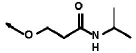 | 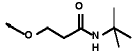 |
| Q-151 | Q-152 | Q-153 | Q-154 | Q-155 |
| | | | | |
| 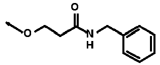 | 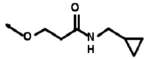 | 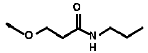 | 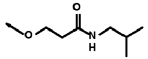 | 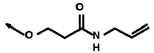 |
| Q-156 | Q-157 | Q-158 | Q-159 | Q-160 |
| | | | | |
| 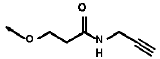 | 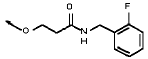 | 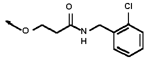 | 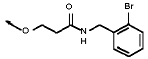 | 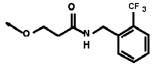 |
| Q-161 | Q-162 | Q-163 | Q-164 | Q-165 |
| | | | | |
| 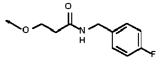 | 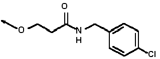 | 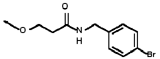 | 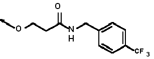 | 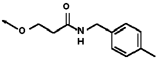 |
| Q-166 | Q-167 | Q-168 | Q-169 | Q-170 |
| | | | | |
| 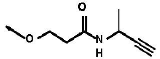 | 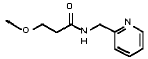 | 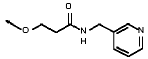 | 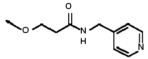 | 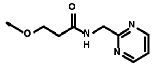 |
| Q-171 | Q-172 | Q-173 | Q-174 | Q-175 |
| | | | | |
| 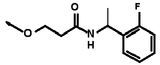 | 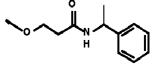 | 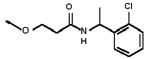 | 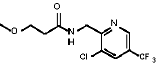 | 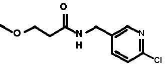 |
| Q-176 | Q-177 | Q-178 | Q-179 | Q-180 |
| | | | | |
| 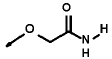 | 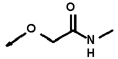 | 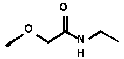 | 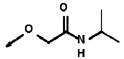 | 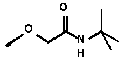 |
| Q-181 | Q-182 | Q-183 | Q-184 | Q-185 |
| | | | | |
| 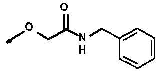 | 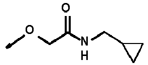 | 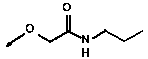 | 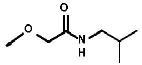 | 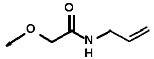 |
| Q-186 | Q-187 | Q-188 | Q-189 | Q-190 |
| 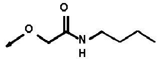 | 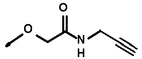 | 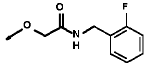 | 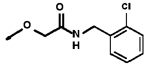 | 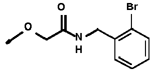 |
| Q-191 | Q-192 | Q-193 | Q-194 | Q-195 |
| | | | | |
| 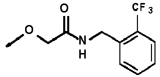 | 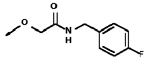 | 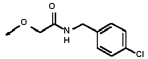 | 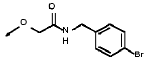 | 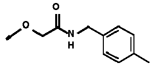 |
| Q-196 | Q-197 | Q-198 | Q-199 | Q-200 |
| | | | | |
| 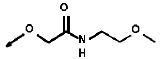 | 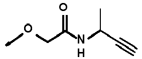 | 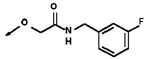 | 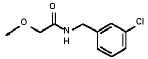 | 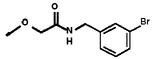 |
| Q-201 | Q-202 | Q-203 | Q-204 | Q-205 |
| | | | | |
| 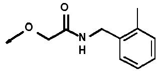 | 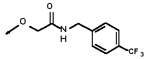 | 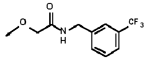 | 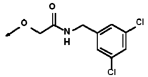 | 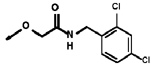 |
| Q-206 | Q-207 | Q-208 | Q-209 | Q-210 |
| | | | | |
| 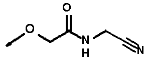 | 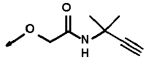 | 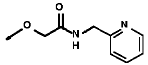 | 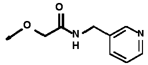 | 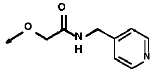 |
| Q-211 | Q-212 | Q-213 | Q-214 | Q-215 |
| | | | | |
| 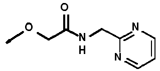 | 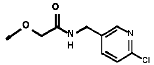 | 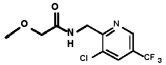 | 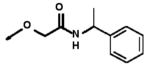 | 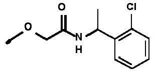 |
| Q-216 | Q-217 | Q-218 | Q-219 | Q-220 |
| | | | | |
| 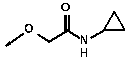 | 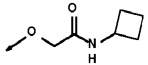 | 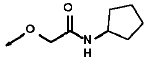 | 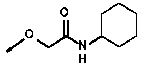 | 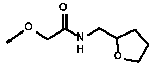 |
| Q-221 | Q-222 | Q-223 | Q-224 | Q-225 |
| | | | | |
| 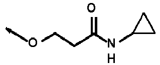 | 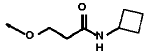 | 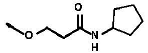 | 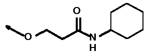 | 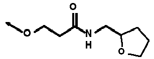 |
| Q-226 | Q-227 | Q-228 | Q-229 | Q-230 |
| 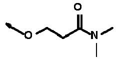 | 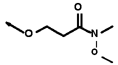 | 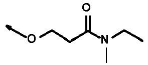 | 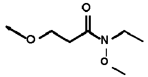 | 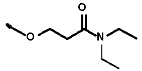 |
| Q-231 | Q-232 | Q-233 | Q-234 | Q-235 |
| | | | | |
| 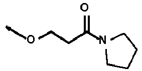 | 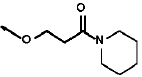 | 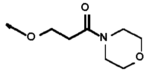 | 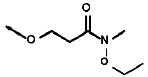 | 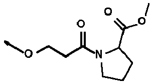 |
| Q-236 | Q-237 | Q-238 | Q-239 | Q-240 |
| | | | | |
| 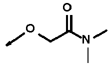 | 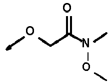 | 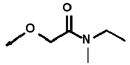 | 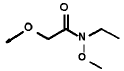 | 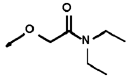 |
| Q-241 | Q-242 | Q-243 | Q-244 | Q-245 |
| | | | | |
| 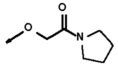 | 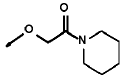 | 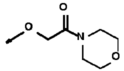 | 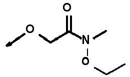 | 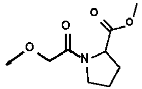 |
| Q-246 | Q-247 | Q-248 | Q-249 | Q-250 |
| | | | | |
| 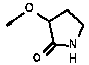 | 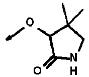 | 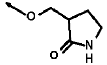 | 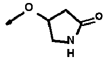 | 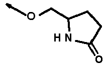 |
| Q-251 | Q-252 | Q-253 | Q-254 | Q-255 |
| | | | | |
| 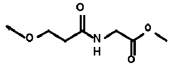 | 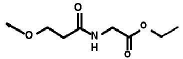 | 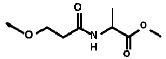 | 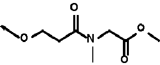 | 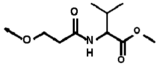 |
| Q-256 | Q-257 | Q-258 | Q-259 | Q-260 |
| | | | | |
| 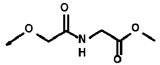 | 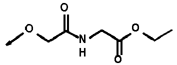 | 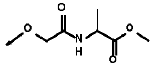 | 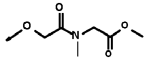 | 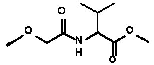 |
| Q-261 | Q-262 | Q-263 | Q-264 | Q-265 |
| | | | | |
| 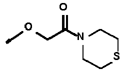 | 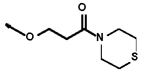 | 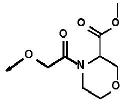 | 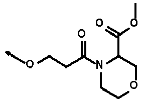 | 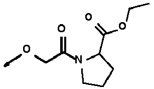 |
| Q-266 | Q-267 | Q-268 | Q-269 | Q-270 |
| 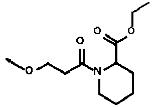 | 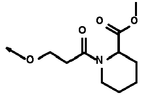 | 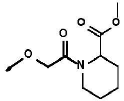 | 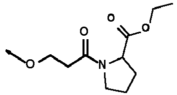 | 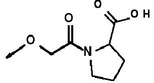 |
| Q-271 | Q-272 | Q-273 | Q-274 | Q-275 |
| | | | | |
| 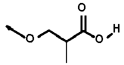 | 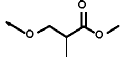 | 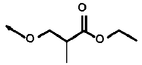 | 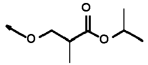 | 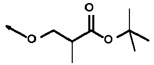 |
| Q-276 | Q-277 | Q-278 | Q-279 | Q-280 |
| | | | | |
| 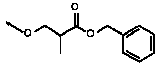 | 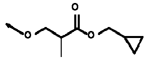 | 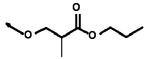 | 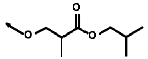 | 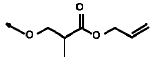 |
| Q-281 | Q-282 | Q-283 | Q-284 | Q-285 |
| | | | | |
| 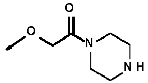 | 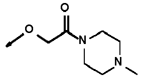 | 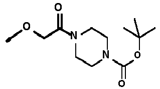 | 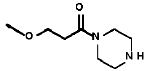 | 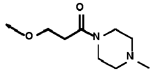 |
| Q-286 | Q-287 | Q-288 | Q-289 | Q-290 |
| | | | | |
| 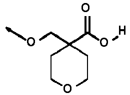 | 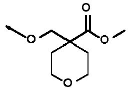 | 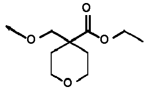 | 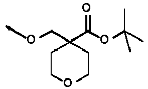 | 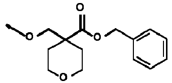 |
| Q-291 | Q-292 | Q-293 | Q-294 | Q-295 |
| | | | | |
| 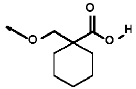 | 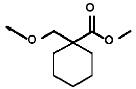 | 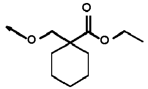 | 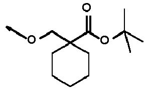 | 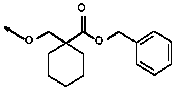 |
| Q-296 | Q-297 | Q-298 | Q-299 | Q-300 |

5. Verbindungen der allgemeinen Formel (I) gemäß Anspruch 1und/oder deren Salz, **dadurch gekennzeichnet, dass**
R¹ für Methyl, Ethyl, n-Propyl, 1-Methylethyl, n-Butyl, 1-Methylpropyl, 2-Methylpropyl, 1,1-Dimethylethyl, n-Pentyl, Amino, Dimethylamino, Diethylamino steht,
R² für Wasserstoff, Methyl, Ethyl, n-Propyl, iso-Propyl steht,
R³ für Wasserstoff, Fluor, Chlor, Brom, Methoxy, Ethoxy steht,
R⁴ für Halogen, Cyano, C(O)NH₂, C(S)NH₂, Difluormethyl, Trifluormethyl, Ethinyl, Propin-1-yl steht,
R⁵ und R⁶ unabhängig voneinander für Wasserstoff, Fluor, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropyl, Cyclobutyl, Cyclopentyl, Cyclohexyl, Cyclopropylmethyl, Cyclobutylmethyl, Cyclopentylmethyl, Cyclohexylmethyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Ioddifluormethyl, Bromfluormethyl, 1-Fluorethyl, 2-Fluorethyl, Fluormethyl, Difluormethyl, 2,2-Difluorethyl, 2,2,2-Trifluorethyl, Difluor-tert.-butyl, Methoxy, Ethoxy, n-Propyloxy, iso-Propyloxy, n-Butyloxy, tert-Butyloxy, Methoxymethyl, Ethoxymethyl, n-Propyloxymethyl, iso-Propyloxymethyl, Methoxyethyl, Ethoxyethyl, n-Propyloxyethyl, iso-Propyloxyethyl, Methoxy-n-propyl, Methoxydifluormethyl, Ethoxydifluormethyl, n-propyloxydifluormethyl, n-Butyloxydifluormethyl, Trifluormethoxymethyl, Trifluormethoxyethyl, Trifluormethoxy-n-propyl, Phenyl, 2-Fluor-Phenyl, 3-Fluor-Phenyl, 4-Fluor-Phenyl, 2,4-Difluor-Phenyl, 2,5-Difluor-Phenyl, 2,6-Difluor-Phenyl, 2,3-Difluor-Phenyl, 3,4-Difluor-Phenyl, 3,5-Difluor-Phenyl, 2,4,5-Trifluor-Phenyl, 3,4,5-Trifluor-Phenyl, 2-Chlor-Phenyl, 3-Chlor-Phenyl, 4-Chlor-Phenyl, 2,4-Dichlor-Phenyl, 2,5-Dichlor-Phenyl, 2,6-Dichlor-Phenyl, 2,3-Dichlor-Phenyl, 3,4-Dichlor-Phenyl, 3,5-Dichlor-Phenyl, 2,4,5-Trichlor-Phenyl, 3,4,5-Trichlor-Phenyl, 2,4,6-Trichlor-Phenyl, Pyridin-2-yl, Pyridin-3-yl, Pyridin-4-yl, Thiophen-2-yl, Thiophen-3-yl, Furan-2-yl, Tetrahydrofuran-2-yl, Tetrahydrofuran-3-yl, 1-(4-Methylphenyl)ethyl, 1-(3-Methylphenyl)ethyl, 1-(2-Methylphenyl)ethyl, 1-(4-Chlorphenyl)ethyl, 1-(3-Chlorphenyl)ethyl, 1-(2-Chlorphenyl)ethyl, Benzyl, (4-Fluorphenyl)methyl, (3-Fluorphenyl)methyl, (2-Fluorphenyl)methyl, (2,4-Difluorphenyl)methyl, (3,5-Difluorphenyl)methyl, (2,5-Difluorphenyl)methyl, (2,6-Difluorphenyl)methyl, (2,4,5-Trifluorphenyl)methyl, (2,4,6-Trifluorphenyl)methyl, (4-Chlorphenyl)methyl, (3-Chlorphenyl)methyl, (2-Chlorphenyl)methyl, (2,4-Dichlorphenyl)methyl, (3,5-Dichlorphenyl)methyl, (2,5-Dichlorphenyl)methyl, (2,6-Dichlorphenyl)methyl, (2,4,5-Trichlorphenyl)methyl, (2,4,6-Trichlorphenyl)methyl, Methylthiomethyl, Ethylthiomethyl, Ethylthioethyl, Methylthioethyl, n-Propylthiomethyl, iso-Propylthiomethyl, Trifluormethylthiomethyl, trifluormethylthioethyl, Methylcarbonylmethyl, Ethylcarbonylmethyl, n-Propylcarbonylmethyl, iso-Propylcarbonylmethyl, Methylcarbonylethyl, Hydroxycarbonyl, Methoxycarbonyl, Ethoxycarbonyl, n-Propyloxycarbonyl, iso-Propyloxycarbonyl, n-Butyloxycarbonyl, tert-Butyloxycarbonyl, Allyloxycarbonyl, Benzyloxycarbonyl, Aminocarbonyl, Methylaminocarbonyl, Ethylaminocarbonyl, n-Propylaminocarbonyl, iso-Propylaminocarbonyl, Dimethylaminocarbonyl, Diethylaminocarbonyl, Methyl(ethyl)aminocarbonyl, Cyclopropylaminocarbonyl, Cyclobutylaminocarbonyl, Cyclopentylaminocarbonyl, Cyclohexylaminocarbonyl, Allylaminocarbonyl, benzylaminocarbonyl, tert-Butyloxycarbonylaminocarbonyl, Hydroxycarbonylmethyl, Methoxycarbonylmethyl, Ethoxycarbonylmethyl, n-Propyloxycarbonylmethyl, iso-Propyloxycarbonylmethyl, n-Butyloxycarbonylmethyl, tert-Butyloxycarbonylmethyl, Allyloxycarbonylmethyl, Benzyloxycarbonylmethyl, Aminocarbonylmethyl, Methylaminocarbonylmethyl, Ethylaminocarbonylmethyl, n-Propylaminocarbonylmethyl, iso-Propylaminocarbonylmethyl, Dimethylaminocarbonylmethyl, Diethylaminocarbonylmethyl, Methyl(ethyl)aminocarbonylmethyl, Cyclopropylaminocarbonylmethyl, Cyclobutylaminocarbonylmethyl, Cyclopentylaminocarbonylmethyl, Cyclohexylaminocarbonylmethyl, Allylaminocarbonylmethyl, Benzylaminocarbonylmethyl, 1-(Hydroxycarbonyl)eth-1-yl, 1-(Methoxycarbonyl)eth-1-yl, 1-(Ethoxycarbonyl)eth-1-yl, 1-(tert-Butyloxycarbonyl)eth-1-yl, 1-(Benzyloxycarbonyl)eth-1-yl, 2-(Hydroxycarbonyl)eth-1-yl, 2-(Methoxycarbonyl)eth-1-yl, 2-(Ethoxycarbonyl)eth-1-yl, 2-(tert-Butyloxycarbonyl)eth-1-yl, 2-(Benzyloxycarbonyl)eth-1-yl, 1-(Hydroxycarbonyl)prop-1-yl, 1-(Methoxycarbonyl)prop-1-yl, 1-(Ethoxycarbonyl)prop-1-yl, 1-(tert-Butyloxycarbonyl)prop-1-yl, 1-(Benzyloxycarbonyl)prop-1-yl, 2-(Hydroxycarbonyl)prop-1-yl, 2-(Methoxycarbonyl)prop-1-yl, 2-(Ethoxycarbonyl)prop-1-yl, 2-(tert-Butyloxycarbonyl)prop-1-yl, 2-(Benzyloxycarbonyl)prop-1-yl, 1-(Hydroxycarbonyl)prop-2-yl, 1-(Methoxycarbonyl)prop-2-yl, 1-(Ethoxycarbonyl)prop-2-yl, 1-(tert-Butyloxycarbonyl)prop-2-yl, 1-(Benzyloxycarbonyl)prop-2-yl, 3-(Hydroxycarbonyl)prop-1-yl, 3-(Methoxycarbonyl)prop-1-yl, 3-(Ethoxycarbonyl)prop-1-yl, 3-(tert-Butyloxycarbonyl)prop-1-yl, 3-(Benzyloxycarbonyl)prop-1-yl, Aminomethyl, 2-Aminoeth-1-yl, 1-Aminoeth1-yl, 1-Amino-prop-1-yl, 3-Amino-prop-1-yl, Methylaminomethyl, Dimethylaminomethyl, Diethylaminomethyl, Ethylaminomethyl, iso-Propylaminomethyl, Cyclopropylaminomethyl, Cyclobutylaminomethyl, Cyclopentylaminomethyl, Cyclohexylaminomethyl, Methoxycarbonylaminomethyl, Ethoxycarbonylaminomethyl tert-Butyloxycarbonylaminomethyl, Methylcarbonylaminomethyl, Ethylcarbonylaminomethyl, n-propylcarbonylaminomethyl, iso-Propylcarbonylaminomethyl, 2-(Methylamino)eth-1-yl, 2-(Dieth-1-ylamino)eth-1-yl, 2-(Diethylamino)eth-1-yl, 2-(Ethylamino)eth-1-yl, 2-(iso-Propylamino)eth-1-yl, 2-(Cyclopropylamino)eth-1-yl, 2-(Cyclobutylammo)eth-1-yl, 2-(Cyclopentylamino)eth-1-yl, 2-(Cyclohexylamino)eth-1-yl, 2-(Methoxycarbonylamino)eth-1-yl, 2-(Ethoxycarbonylamino)eth-1-yl, 2-(tert-Butyloxycarbonylamino)eth-1-yl, 2-(Methylcarbonylamino)eth-1 -yl, 2-(Ethylcarbonylamino)eth-1-yl, 2-(n-Propylcarbonylamino)eth-1 -yl, 2-(iso-Propylcarbonylamino)eth-1-yl, 1-(Methylamino)eth-1-yl, 1-(Dieth-1-ylamino)eth-1-yl, 1-(Diethylamino)eth-1-yl, 1-(Ethylamino)eth-1-yl, 1-(iso-Propylamino)eth-1-yl, 1-(Cyclopropylamino)eth-1-yl, 1-(Cyclobutylamino)eth-1-yl, 1-(Cyclopentylamino)eth-1-yl, 1-(Cyclohexylamino)eth-1-yl, 1-(Methoxycarbonylamino)eth-1-yl, 1-(Ethoxycarbonylamino)eth-1-yl, 1-(tert-Butyloxycarbonylamino)eth-1-yl, 1-(Methylcarbonylamino)eth-1-yl, 1-(Ethylcarbonylamino)eth-1-yl, 1-(n-Propylcarbonylamino)eth-1-yl, 1-(iso-Propylcarbonylamino)eth-1-yl stehen, oder
R⁵ und R⁶ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden, oder
R⁵ und R⁶ mit dem Kohlenstoffatom, an das sie gebunden sind, eine gegebenenfalls weiter durch
R¹⁹ und R²⁰ substituierte Doppelbindung, gemäß nachfolgender Formel (I'), bilden 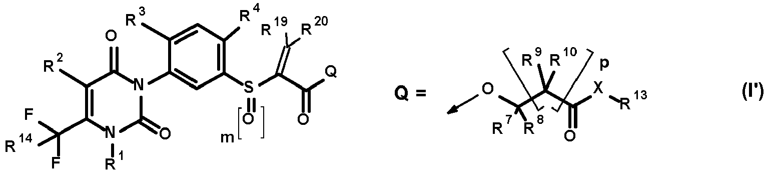
R¹⁴ für Wasserstoff, Fluor, Chlor, Brom, Trifluormethyl, Methoxy, Ethoxy, n-Propyloxy, n-Butyloxy steht,
m für 0, 1, 2 steht,
R¹⁹ und R²⁰ unabhängig voneinander für Wasserstoff, Fluor, Chlor, Brom, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, Cyclopropyl, Cyclobutyl, Cyclopentyl, Cyclohexyl, Trifluormethyl, Difluormethyl, Pentafluorethyl, Ethenyl, 1-Propenyl, 1-Methyl-ethenyl, 1-Butenyl, Phenyl stehen oder
R¹⁹ und R²⁰ mit dem Kohlenstoffatom, an das sie gebunden sind, einen gesättigten oder gegebenenfalls durch Heteroatome unterbrochenen und gegebenenfalls weiter substituierten 3 bis 10-gliedrigen monocyclischen oder bicyclischen Ring bilden
und
Q für eine der in der in Anspruch 4 genannten Gruppierungen Q-1 bis Q-300 steht.

6. Verwendung einer oder mehrere Verbindungen der allgemeinen Formel (I) und/oder deren Salze wie in einem der Ansprüche 1 bis 5 definiert, als Herbizid und/oder Pflanzenwachstumsregulator, vorzugsweise in Kulturen von Nutz- und/oder Zierpflanzen.

7. Herbizides und/oder pflanzenwachstumsregulierendes Mittel, **dadurch gekennzeichnet, dass** das Mittel eine oder mehrere Verbindungen der Formel (I) und/oder deren Salze enthält wie in einem der Ansprüche 1 bis 5 definiert, und ein oder mehrere weitere Stoffe ausgewählt aus den Gruppen (i) und/oder (ii), mit
(i) ein oder mehrere weitere agrochemisch wirksame Stoffe, vorzugsweise ausgewählt aus der Gruppe bestehend aus Insektiziden, Akariziden, Nematiziden, weiteren Herbiziden, Fungiziden, Safenern, Düngemitteln und/oder weiteren Wachstumsregulatoren,
(ii) ein oder mehrere im Pflanzenschutz übliche Formulierungshilfsmittel.

8. Verfahren zur Bekämpfung von Schadpflanzen oder zur Wachstumsregulierung von Pflanzen, **dadurch gekennzeichnet, dass** eine wirksame Menge
- einer oder mehrerer Verbindungen der Formel (I) und/oder deren Salze, wie in einem der Ansprüche 1 bis 5 definiert, oder
- eines Mittels nach Anspruch 7,
auf die Pflanzen, Pflanzensamen, den Boden, in dem oder auf dem die Pflanzen wachsen, oder die Anbaufläche appliziert wird.

## Claims

1. Substituted thiophenyluracils of the general formula (I) or salts thereof 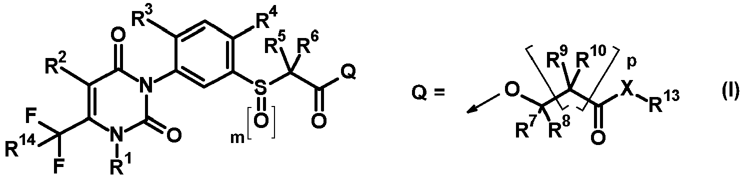 in which
R¹ is (C₁-C₈) -alkyl, amino, bis[(C₁-C₈)-alkyl]amino,
R² is hydrogen, (C₁-C₈) -alkyl,
R³ is hydrogen, halogen, (C₁-C₈) -alkoxy,
R⁴ is halogen, cyano, NO₂, C(O)NH₂, C(S)NH₂, (C₁-C₈) -haloalkyl, (C₂-C₈) -alkynyl,
R⁵ and R⁶ are independently hydrogen, halogen, (C₁-C₈)-alkyl, (C₃-C₈)-cycloalkyl, (C₃-C₈)-cycloalkyl- (C₁-C₈) -alkyl, (C₂-C₈) -alkenyl, (C₂-C₈)-alkynyl, (C₁-C₁₀)-haloalkyl, (C₂-C₈)-haloalkenyl, (C₂-C₈) -haloalkynyl, (C₃-C₁₀)-halocycloalkyl, (C₄-C₁₀)-cycloalkenyl, (C₄-C₁₀)-halocycloalkenyl, (C₁-C₈) -alkoxy, (C₁-C₈)-alkoxy- (C₁-C₈)-alkyl, (C₁-C₈)-alkoxy- (C₁-C₈)-haloalkyl, (C₁-C₈)-haloalkoxy-(C₁-C₈)-haloalkyl, (C₁-C₈)-haloalkoxy-(C₁-C₈)-alkyl, aryl, aryl- (C₁-C₈)-alkyl, heteroaryl, heteroaryl- (C₁-C₈)-alkyl, (C₄-C₁₀)-cycloalkenyl-(C₁-C₈)-alkyl, heterocyclyl, heterocyclyl- (C₁-C₈)-alkyl, (C₁-C₈)-alkylthio-(C₁-C₈)-alkyl, (C₁-C₈)-haloalkylthio- (C₁-C₈)-alkyl, (C₁-C₈)-alkylcarbonyl-(C₁-C₈)-alkyl, C(O)OR¹⁷, C(O)NR¹⁵R¹⁶, C(O)R¹⁷ , R¹⁷O(O)C-(C₁-C₈)-alkyl, R¹⁵R¹⁶N(O)C-(C₁-C₈) -alkyl, R¹⁵R¹⁶N-(C₁-C₈)-alkyl, or
R⁵ and R⁶ together with the carbon atom to which they are bonded form a fully saturated or partly saturated 3- to 10-membered monocyclic or bicyclic ring optionally interrupted by heteroatoms and optionally having further substitution, or
R⁵ and R⁶ together with the carbon atom to which they are bonded form a double bond optionally further substituted by R¹⁹ and R²⁰, according to formula (I') below 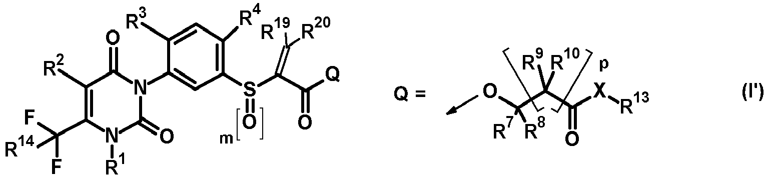
m is 0, 1, 2,
p is 0, 1, 2, 3, 4, 5,
X is O (oxygen) or the N-R¹¹ or N-O-R¹² moieties, and where R¹¹ and R¹² in the N-R¹¹ and N-O-R¹² moiety independently have the meanings according to the definitions below,
R⁷, R⁸, R⁹ and R¹⁰ are independently hydrogen, fluorine, (C₁-C₈) -alkyl, (C₃-C₈) -cycloalkyl, (C₃-C₈) -cycloalkyl- (C₁-C₈) -alkyl, (C₂-C₈)-alkenyl, (C₂-C₈) -alkynyl, (C₁-C₁₀)-haloalkyl, (C₂-C₈) -haloalkenyl, (C₂-C₈) -haloalkynyl, (C₃-C₁₀)-halocycloalkyl, (C₄-C₁₀)-cycloalkenyl, (C₄-C₁₀)-halocycloalkenyl, (C₁-C₈) -alkoxy- (C₁-C₈)-haloalkyl, (C₁-C₈)-haloalkoxy-(C₁-C₈)-haloalkyl, aryl, aryl-(C₁-C₈)-alkyl, heteroaryl, heteroaryl- (C₁-C₈) -alkyl, (C₄-C₁₀)-cycloalkenyl-(C₁-C₈)-alkyl, heterocyclyl, heterocyclyl- (C₁-C₈) -alkyl, R¹⁷O-(C₁-C₈)-alkyl, R¹⁸S-(C₁-C₈) -alkyl, (C₁-C₈) -alkylcarbonyl-(C₁-C₈)-alkyl, C(O)OR¹⁷, C(O)NR¹⁵R¹⁶, C(O)R¹⁷, R¹⁷O(O)C-(C₁-C₈)-alkyl, R¹⁵R¹⁶N(O)C-(C₁-C₈)-alkyl, R¹⁵R¹⁶N-(C₁-C₈) -alkyl, or
R⁵ and R⁷ together with the carbon atoms to which they are bonded form a fully saturated or partly saturated 3- to 8-membered monocyclic or bicyclic ring optionally interrupted by heteroatoms and optionally having further substitution, or
R⁷ and R⁸ together with the carbon atom to which they are bonded form a fully saturated or partly saturated 3- to 10-membered monocyclic or bicyclic ring optionally interrupted by heteroatoms and optionally having further substitution, or
R⁹ and R¹⁰ together with the carbon atom to which they are bonded form a fully saturated or partly saturated 3- to 10-membered monocyclic or bicyclic ring optionally interrupted by heteroatoms and optionally having further substitution, or
R⁹ and R¹⁰ together with the carbon atom to which they are bonded form an exomethylene group or
R⁷ and R⁹ together with the carbon atoms to which they are bonded form a fully saturated or partly saturated 3- to 10-membered monocyclic or bicyclic ring optionally interrupted by heteroatoms and optionally having further substitution,
R¹¹ is hydrogen, (C₁-C₈) -alkyl, (C₃-C₈) -cycloalkyl, cyano- (C₁-C₈) -alkyl, (C₃-C₈) -cycloalkyl- (C₁-C₈)-alkyl, (C₁-C₈)-alkylsulfonyl, arylsulfonyl, heteroarylsulfonyl, (C₃-C₈)-cycloalkylsulfonyl, heterocyclylsulfonyl, aryl- (C₁-C₈) -alkylsulfonyl, (C₁-C₈)-alkylcarbonyl, arylcarbonyl, heteroarylcarbonyl, (C₃-C₈)-cycloalkylcarbonyl, heterocyclylcarbonyl, (C₁-C₈) -alkoxycarbonyl, (C₁-C₈) -alkoxy, (C₂-C₈)-alkenyloxy, aryl- (C₁-C₈) -alkoxycarbonyl, (C₁-C₈) -haloalkylcarbonyl, (C₂-C₈) -alkenyl, (C₂-C₈)-alkynyl, (C₁-C₈) -haloalkyl, halo- (C₂-C₈)-alkynyl, halo- (C₂-C₈) -alkenyl, (C₁-C₈) -alkoxy-(C₁-C₈) -alkyl, amino, (C₁-C₈) -alkylamino, bis [(C₁-C₈)-alkyl]amino, (C₁-C₈) -alkoxy- (C₁-C₈)-alkoxy-(C₁-C₈)-alkyl, heteroaryl-(C₁-C₈)-alkylsulfonyl, heterocyclyl-(C₁-C₈)-alkylsulfonyl, (C₂-C₈)-alkenyloxycarbonyl, (C₂-C₈)-alkynyloxycarbonyl, (C₁-C₈)-alkylaminocarbonyl, (C₃-C₈)-cycloalkylaminocarbonyl, bis-[(C₁-C₈)-alkyl]aminocarbonyl,
R¹² is hydrogen, (C₁-C₈) -alkyl, (C₃-C₈) -cycloalkyl-(C₁-C₈) -alkyl, (C₂-C₈) -alkenyl, (C₂-C₈) -alkynyl, (C₁-C₈) -alkoxy- (C₁-C₈) -alkyl, aryl, aryl-(C₁-C₈)-alkyl, R¹⁷O(O)C-(C₁-C₈) -alkyl, R¹⁵R¹⁶N(O)C-(C₁-C₈) -alkyl,
R¹³ is hydrogen, (C₁-C₈) -alkyl, (C₁-C₈)-haloalkyl, (C₃-C₈) -cycloalkyl, (C₃-C₈) -cycloalkyl- (C₁-C₈)-alkyl, (C₂-C₈) -alkenyl, (C₂-C₈) -alkynyl, (C₁-C₈) -alkoxy- (C₁-C₈) -alkyl, (C₁-C₈)-haloalkoxy-(C₁-C₈) -alkyl, aryl, aryl- (C₁-C₈)-alkyl, heteroaryl, heteroaryl-(C₁-C₈)-alkyl, heterocyclyl, heterocyclyl- (C₁-C₈)-alkyl, (C₁-C₈)-alkylthio- (C₁-C₈) -alkyl, (C₁-C₈)-haloalkylthio- (C₁-C₈) -alkyl, (C₁-C₈)-alkylcarbonyl- (C₁-C₈) -alkyl, R¹⁷O(O)C-(C₁-C₈)-alkyl, R¹⁵R¹⁶N(O)C-(C₁-C₈) -alkyl, R¹⁵R¹⁶N-(C₁-C₈)-alkyl, or
R⁷ and R¹³ together with the atoms to which they are bonded form a fully saturated or partly saturated 3- to 10-membered monocyclic or bicyclic ring optionally interrupted by heteroatoms and optionally having further substitution, or
R⁹ and R¹³ together with the atoms to which they are bonded form a fully saturated or partly saturated 3- to 10-membered monocyclic or bicyclic ring optionally interrupted by heteroatoms and optionally having further substitution, or
R¹¹ and R¹³, if X is N-R¹¹, together with the nitrogen atom to which they are bonded form a 4- to 10-membered monocyclic or bicyclic ring which is fully saturated or optionally interrupted by heteroatoms and optionally has further substitution, or
R¹² and R¹³ , if X is N-O-R¹², together with the nitrogen atom to which they are bonded form a fully saturated and optionally further-substituted 4- to 10-membered monocyclic or bicyclic ring,
R¹⁴ is hydrogen, fluorine, chlorine, bromine, (C₁-C₅) -haloalkyl, (C₁-C₈) -alkoxy,
R¹⁵ and R¹⁶ are the same or different and are independently hydrogen, (C₁-C₈) -alkyl, (C₂-C₈)-alkenyl, (C₂-C₈) -alkynyl, (C₁-C₈) -cyanoalkyl, (C₁-C₁₀)-haloalkyl, (C₂-C₈) -haloalkenyl, (C₂-C₈) -haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈) -alkoxy- (C₁-C₈) -alkyl, (C₁-C₈) -haloalkoxy- (C₁-C₈) -alkyl, (C₁-C₈)-alkylthio- (C₁-C₈) -alkyl, (C₁-C₈) -haloalkylthio-(C₁-C₈) -alkyl, (C₁-C₈) -alkoxy- (C₁-C₈) -haloalkyl, aryl, aryl-(C₁-C₈)-alkyl, heteroaryl, heteroaryl- (C₁-C₈) -alkyl, (C₃-C₈) -cycloalkyl-(C₁-C₈) -alkyl, (C₄-C₁₀)-cycloalkenyl- (C₁-C₈)-alkyl, COR¹⁷, SO₂R¹⁸, (C₁-C₈)-alkyl-HNO₂S-, (C₃-C₈)-cycloalkyl-HNO₂S-, heterocyclyl, (C₁-C₈)-alkoxycarbonyl-(C₁-C₈)-alkyl, (C₁-C₈)-alkoxycarbonyl, aryl-(C₁-C₈)-alkoxycarbonyl-(C₁-C₈) -alkyl, aryl- (C₁-C₈) -alkoxycarbonyl, heteroaryl- (C₁-C₈) -alkoxycarbonyl, (C₂-C₈)-alkenyloxycarbonyl, (C₂-C₈)-alkynyloxycarbonyl, heterocyclyl-(C₁-C₈)-alkyl,
R¹⁷ is hydrogen, (C₁-C₈) -alkyl, (C₂-C₈) -alkenyl, (C₂-C₈) -alkynyl, (C₁-C₈) -cyanoalkyl, (C₁-C₁₀)-haloalkyl, (C₂-C₈)-haloalkenyl, (C₂-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, (C₁-C₈)-alkoxycarbonyl-(C₁-C₈)-alkyl, (C₂-C₈)-alkenyloxycarbonyl-(C₁-C₈)-alkyl, aryl-(C₁-C₈)-alkoxycarbonyl-(C₁-C₈)-alkyl, hydroxycarbonyl-(C₁-C₈)-alkyl, heterocyclyl, heterocyclyl-(C₁-C₈)-alkyl,
R¹⁸ is hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₂-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, heterocyclyl-(C₁-C₈)-alkyl, (C₃-C₈)-cycloalkyl-(C₁-C₈)-alkyl, (C₄-C₁₀)-cycloalkenyl-(C₁-C₈)-alkyl, NR¹⁵R¹⁶
and
R¹⁹ and R²⁰ are independently hydrogen, halogen, (C₁-C₈)-alkyl, (C₃-C₈)-cycloalkyl, (C₂-C₈)-alkenyl, (C₂-C₈)-alkynyl, (C₁-C₁₀)-haloalkyl, aryl, or
R¹⁹ and R²⁰ together with the carbon atom to which they are bonded form a 3- to 10-membered monocyclic or bicyclic ring which is saturated or optionally interrupted by heteroatoms and optionally has further substitution.

2. Compound of the general formula (I) according to Claim 1 and/or salt thereof, **characterized in that**
R¹ is (C₁-C₇)-alkyl, amino, bis[(C₁-C₇)-alkyl]amino,
R² is hydrogen, (C₁-C₇)-alkyl,
R³ is hydrogen, halogen, (C₁-C₇)-alkoxy,
R⁴ is halogen, cyano, NO₂, C(O)NH₂, C(S)NH₂, (C₁-C₇)-haloalkyl, (C₂-C₇)-alkynyl,
R⁵ and R⁶ are independently hydrogen, halogen, (C₁-C₇)-alkyl, (C₃-C₇)-cycloalkyl, (C₃-C₇)-cycloalkyl-(C₁-C₇)-alkyl, (C₂-C₇)-alkenyl, (C₂-C₇)-alkynyl, (C₁-C₁₀)-haloalkyl, (C₂-C₇)-haloalkenyl, (C₂-C₇)-haloalkynyl, (C₃-C₁₀)-halocycloalkyl, (C₄-C₁₀)-cycloalkenyl, (C₄-C₁₀)-halocycloalkenyl, (C₁-C₇)-alkoxy, (C₁-C₇)-alkoxy-(C₁-C₇)-alkyl, (C₁-C₇)-alkoxy-(C₁-C₇)-haloalkyl, (C₁-C₇)-haloalkoxy-(C₁-C₇)-haloalkyl, (C₁-C₇)-haloalkoxy-(C₁-C₇)-alkyl, aryl, aryl-(C₁-C₇)-alkyl, heteroaryl, heteroaryl-(C₁-C₇)-alkyl, (C₄-C₁₀)-cycloalkenyl-(C₁-C₇)-alkyl, heterocyclyl, heterocyclyl-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkylcarbonyl-(C₁-C₇)-alkyl, C(O)OR¹⁷, C(O)NR¹⁵R¹⁶, C(O)R¹⁷ , R¹⁷O(O)C-(C₁-C₇)-alkyl, R¹⁵R¹⁶N(O)C-(C₁-C₇)-alkyl, R¹⁵R¹⁶N-(C₁-C₇)-alkyl, or
R⁵ and R⁶ together with the carbon atom to which they are bonded form a fully saturated or partly saturated 3- to 10-membered monocyclic or bicyclic ring optionally interrupted by heteroatoms and optionally having further substitution, or
R⁵ and R⁶ together with the carbon atom to which they are bonded form a double bond optionally further substituted by R¹⁹ and R²⁰, according to formula (I') below 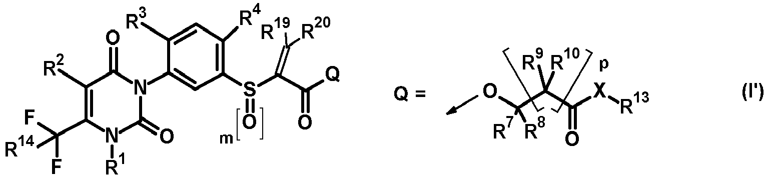
m is 0, 1, 2,
p is 0, 1, 2, 3,
X is O (oxygen), N (nitrogen) or the N-R¹¹ or N-O-R¹² moieties, and where R¹¹ and R¹² in the N-R¹¹ and N-O-R¹² moiety independently have the meanings according to the definitions below,
R⁷, R⁸, R⁹ and R¹⁰ are independently hydrogen, fluorine, (C₁-C₇)-alkyl, (C₃-C₇)-cycloalkyl, (C₃-C₇)-cycloalkyl-(C₁-C₇)-alkyl, (C₂-C₇)-alkenyl, (C₂-C₇)-alkynyl, (C₁-C₁₀)-haloalkyl, (C₂-C₇)-haloalkenyl, (C₂-C₇)-haloalkynyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-haloalkyl, (C₁-C₇)-haloalkoxy-(C₁-C₇)-haloalkyl, aryl, aryl-(C₁-C₇)-alkyl, heteroaryl, heteroaryl-(C₁-C₇)-alkyl, (C₄-C₁₀)-cycloalkenyl-(C₁-C₇)-alkyl, heterocyclyl, heterocyclyl-(C₁-C₇)-alkyl, R¹⁷O-(C₁-C₇)-alkyl, R¹⁸S-(C₁-C₇)-alkyl, (C₁-C₇)-alkylcarbonyl-(C₁-C₇)-alkyl, C(O)OR¹⁷, C(O)NR¹⁵R¹⁶, C(O)R¹⁷ , R¹⁷O(O)C-(C₁-C₇)-alkyl, R¹⁵R¹⁶N(O)C-(C₁-C₇)-alkyl, R¹⁵R¹⁶N-(C₁-C₇)-alkyl, or
R⁵ and R⁷ together with the carbon atoms to which they are bonded form a fully saturated or partly saturated 3- to 8-membered monocyclic or bicyclic ring optionally interrupted by heteroatoms and optionally having further substitution, or
R⁷ and R⁸ together with the carbon atom to which they are bonded form a fully saturated or partly saturated 3- to 10-membered monocyclic or bicyclic ring optionally interrupted by heteroatoms and optionally having further substitution, or
R⁹ and R¹⁰ together with the carbon atom to which they are bonded form a fully saturated or partly saturated 3- to 10-membered monocyclic or bicyclic ring optionally interrupted by heteroatoms and optionally having further substitution, or
R⁹ and R¹⁰ together with the carbon atom to which they are bonded form an exomethylene group or
R⁷ and R⁹ together with the carbon atoms to which they are bonded form a fully saturated or partly saturated 3- to 10-membered monocyclic or bicyclic ring optionally interrupted by heteroatoms and optionally having further substitution,
R¹¹ is hydrogen, (C₁-C₇)-alkyl, (C₃-C₇)-cycloalkyl, cyano-(C₁-C₇)-alkyl, (C₃-C₇)-cycloalkyl-(C₁-C₇)-alkyl, (C₁-C₇)-alkylsulfonyl, arylsulfonyl, heteroarylsulfonyl, (C₃-C₇)-cycloalkylsulfonyl, heterocyclylsulfonyl, aryl-(C₁-C₇)-alkylsulfonyl, (C₁-C₇)-alkylcarbonyl, arylcarbonyl, heteroarylcarbonyl, (C₃-C₇)-cycloalkylcarbonyl, heterocyclylcarbonyl, (C₁-C₇)-alkoxycarbonyl, (C₁-C₇)-alkoxy, (C₂-C₇)-alkenyloxy, aryl-(C₁-C₇)-alkoxycarbonyl, (C₁-C₇)-haloalkylcarbonyl, (C₂-C₇)-alkenyl, (C₂-C₇)-alkynyl, (C₁-C₇)-haloalkyl, halo-(C₂-C₇)-alkynyl, halo-(C₂-C₇)-alkenyl, (C₁-C₇)-alkoxy-(C₁-C₇)-alkyl, amino, (C₁-C₇)-alkylamino, bis[(C₁-C₇)-alkyl]amino, (C₁-C₇)-alkoxy-(C₁-C₇)-alkoxy-(C₁-C₇)-alkyl, heteroaryl-(C₁-C₇)-alkylsulfonyl, heterocyclyl-(C₁-C₇)-alkylsulfonyl, (C₂-C₇)-alkenyloxycarbonyl, (C₂-C₇)-alkynyloxycarbonyl, (C₁-C₇)-alkylaminocarbonyl, (C₃-C₇)-cycloalkylaminocarbonyl, bis-[(C₁-C₇)-alkyl]aminocarbonyl,
R¹² is hydrogen, (C₁-C₇)-alkyl, (C₃-C₇)-cycloalkyl-(C₁-C₇)-alkyl, (C₂-C₇)-alkenyl, (C₂-C₇)-alkynyl, (C₁-C₇)-alkoxy-(C₁-C₇)-alkyl, aryl, aryl-(C₁-C₇)-alkyl, R¹⁷O(O)C-(C₁-C₇)-alkyl, R¹⁵R¹⁶N(O)C-(C₁-C₇)-alkyl,
R¹³ is hydrogen, (C₁-C₇)-alkyl, (C₁-C₇)-haloalkyl, (C₃-C₇)-cycloalkyl, (C₃-C₇)-cycloalkyl-(C₁-C₇)-alkyl, (C₂-C₇)-alkenyl, (C₂-C₇)-alkynyl, (C₁-C₇)-alkoxy-(C₁-C₇)-alkyl, (C₁-C₇)-haloalkoxy-(C₁-C₇)-alkyl, aryl, aryl-(C₁-C₇)-alkyl, heteroaryl, heteroaryl-(C₁-C₇)-alkyl, heterocyclyl, heterocyclyl-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkylcarbonyl-(C₁-C₇)-alkyl, R¹⁷O(O)C-(C₁-C₇)-alkyl, R¹⁵R¹⁶N(O)C-(C₁-C₇)-alkyl, R¹⁵R¹⁶N-(C₁-C₇)-alkyl, or
R⁷ and R¹³ together with the atoms to which they are bonded form a fully saturated or partly saturated 3- to 10-membered monocyclic or bicyclic ring optionally interrupted by heteroatoms and optionally having further substitution, or
R⁹ and R¹³ together with the atoms to which they are bonded form a fully saturated or partly saturated 3- to 10-membered monocyclic or bicyclic ring optionally interrupted by heteroatoms and optionally having further substitution, or
R¹¹ and R¹³, if X is N-R¹¹, together with the nitrogen atom to which they are bonded form a 4- to 10-membered monocyclic or bicyclic ring which is fully saturated or optionally interrupted by heteroatoms and optionally has further substitution, or
R¹² and R¹³, if X is N-O-R¹², together with the nitrogen atom to which they are bonded form a fully saturated and optionally further-substituted 4- to 10-membered monocyclic or bicyclic ring,
R¹⁴ is hydrogen, fluorine, chlorine, bromine, (C₁-C₅)-haloalkyl, (C₁-C₇)-alkoxy,
R¹⁵ and R¹⁶ are the same or different and are independently hydrogen, (C₁-C₇)-alkyl, (C₂-C₇)-alkenyl, (C₂-C₇)-alkynyl, (C₁-C₇)-cyanoalkyl, (C₁-C₁₀)-haloalkyl, (C₂-C₇)-haloalkenyl, (C₂-C₇)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-haloalkoxy-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, (C₃-C₇)-cycloalkyl-(C₁-C₇)-alkyl, (C₄-C₁₀)-cycloalkenyl-(C₁-C₇)-alkyl, COR¹⁷, SO₂R¹⁸, (C₁-C₇)-alkyl-HNO₂S-, (C₃-C₇)-cycloalkyl-HNO₂S-, heterocyclyl, (C₁-C₇)-alkoxycarbonyl-(C₁-C₇)-alkyl, (C₁-C₇)-alkoxycarbonyl, aryl-(C₁-C₇)-alkoxycarbonyl-(C₁-C₇)-alkyl, aryl-(C₁-C₇)-alkoxycarbonyl, heteroaryl-(C₁-C₇)-alkoxycarbonyl, (C₂-C₇)-alkenyloxycarbonyl, (C₂-C₇)-alkynyloxycarbonyl, heterocyclyl-(C₁-C₇)-alkyl,
R¹⁷ is hydrogen, (C₁-C₇)-alkyl, (C₂-C₇)-alkenyl, (C₂-C₇)-alkynyl, (C₁-C₇)-cyanoalkyl, (C₁-C₁₀)-haloalkyl, (C₂-C₇)-haloalkenyl, (C₂-C₇)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, (C₃-C₇)-cycloalkyl-(C₁-C₇)-alkyl, (C₄-C₁₀)-cycloalkenyl-(C₁-C₇)-alkyl, (C₁-C₇)-alkoxycarbonyl-(C₁-C₇)-alkyl, (C₂-C₇)-alkenyloxycarbonyl-(C₁-C₇)-alkyl, aryl-(C₁-C₇)-alkoxycarbonyl-(C₁-C₇)-alkyl, hydroxycarbonyl-(C₁-C₇)-alkyl, heterocyclyl, heterocyclyl- (C₁-C₇)-alkyl
R¹⁸ is hydrogen, (C₁-C₇)-alkyl, (C₂-C₇)-alkenyl, (C₂-C₇)-alkynyl, (C₁-C₇)-cyanoalkyl, (C₁-C₁₀)-haloalkyl, (C₂-C₇)-haloalkenyl, (C₂-C₇)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, heterocyclyl-(C₁-C₇)-alkyl, (C₃-C₇)-cycloalkyl-(C₁-C₇)-alkyl, (C₄-C₁₀)-cycloalkenyl-(C₁-C₇)-alkyl, NR¹⁵R¹⁶
and
R¹⁹ and R²⁰ are independently hydrogen, halogen, (C₁-C₇)-alkyl, (C₃-C₇)-cycloalkyl, (C₂-C₇)-alkenyl, (C₂-C₇)-alkynyl, (C₁-C₇)-haloalkyl, aryl, or
R¹⁹ and R²⁰ together with the carbon atom to which they are bonded form a 3- to 10-membered monocyclic or bicyclic ring which is saturated or optionally interrupted by heteroatoms and optionally has further substitution.

3. Compounds of the general formula (I) according to Claim 1 and/or salt thereof, **characterized in that**
R¹ is (C₁-C₆)-alkyl, amino, bis[(C₁-C₆)-alkyl]amino,
R² is hydrogen, (C₁-C₆)-alkyl,
R³ is hydrogen, halogen, (C₁-C₆)-alkoxy,
R⁴ is halogen, cyano, NO₂, C(O)NH₂, C(S)NH₂, (C₁-C₆)-haloalkyl, (C₂-C₆)-alkynyl,
R⁵ and R⁶ are independently hydrogen, halogen, (C₁-C₆)-alkyl, (C₃-C₆)-cycloalkyl, (C₃-C₆)-cycloalkyl-(C₁-C₆)-alkyl, (C₂-C₆)-alkenyl, (C₂-C₆)-alkynyl, (C₁-C₁₀)-haloalkyl, (C₂-C₆)-haloalkenyl, (C₂-C₆)-haloalkynyl, (C₃-C₁₀)-halocycloalkyl, (C₄-C₁₀)-cycloalkenyl, (C₄-C₁₀)-halocycloalkenyl, (C₁-C₆)-alkoxy, (C₁-C₆)-alkoxy-(C₁-C₆)-alkyl, (C₁-C₆)-alkoxy-(C₁-C₆)-haloalkyl, (C₁-C₆)-haloalkoxy- (C₁-C₆)-haloalkyl, (C₁-C₆)-haloalkoxy- (C₁-C₆)-alkyl, aryl, aryl- (C₁-C₆)-alkyl, heteroaryl, heteroaryl- (C₁-C₆)-alkyl, (C₄-C₁₀)-cycloalkenyl-(C₁-C₆)-alkyl, heterocyclyl, heterocyclyl- (C₁-C₆)-alkyl, (C₁-C₆)-alkylthio-(C₁-C₆)-alkyl, (C₁-C₆)-haloalkylthio- (C₁-C₆)-alkyl, (C₁-C₆)-alkylcarbonyl- (C₁-C₆)-alkyl, C(O)OR¹⁷, C(O)NR¹⁵R¹⁶, C(O)R¹⁷, R¹⁷O(O)C-(C₁-C₆)-alkyl, R¹⁵R¹⁶N(O)C-(C₁-C₆)-alkyl, R¹⁵R¹⁶N-(C₁-C₆)-alkyl, or
R⁵ and R⁶ together with the carbon atom to which they are bonded form a fully saturated or partly saturated 3- to 10-membered monocyclic or bicyclic ring optionally interrupted by heteroatoms and optionally having further substitution, or
R⁵ and R⁶ together with the carbon atom to which they are bonded form a double bond optionally further substituted by R¹⁹ and R²⁰, according to formula (I') below 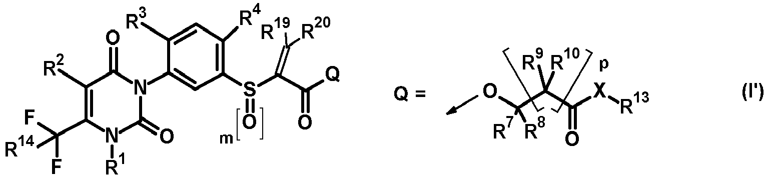
m is 0, 1, 2,
p is 0, 1, 2, 3,
X is O (oxygen), N (nitrogen) or the N-R¹¹ or N-O-R¹² moieties, and where R¹¹ and R¹² in the N-R¹¹ and N-O-R¹² moiety independently have the meanings according to the definitions below,
R⁷, R⁸, R⁹ and R¹⁰ are independently hydrogen, fluorine, (C₁-C₆)-alkyl, (C₃-C₆)-cycloalkyl, (C₃-C₆)-cycloalkyl-(C₁-C₆)-alkyl, (C₂-C₆)-alkenyl, (C₂-C₆)-alkynyl, (C₁-C₁₀)-haloalkyl, (C₂-C₆)-haloalkenyl, (C₂-C₆)-haloalkynyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-haloalkyl, (C₁-C₆)-haloalkoxy-(C₁-C₆)-haloalkyl, aryl, aryl-(C₁-C₆)-alkyl, heteroaryl, heteroaryl-(C₁-C₆)-alkyl, (C₄-C₁₀)-cycloalkenyl-(C₁-C₆)-alkyl, heterocyclyl, heterocyclyl-(C₁-C₆)-alkyl, R¹⁷O-(C₁-C₆)-alkyl, R¹⁸S-(C₁-C₆)-alkyl, (C₁-C₆)-alkylcarbonyl-(C₁-C₆)-alkyl, C(O)OR¹⁷, C(O)NR¹⁵R¹⁶, C(O)R¹⁷, R¹⁷O(O)C-(C₁-C₆)-alkyl, R¹⁵R¹⁶N(O)C-(C₁-C₆)-alkyl, R¹⁵R¹⁶N-(C₁-C₆)-alkyl, or
R⁵ and R⁷ together with the carbon atoms to which they are bonded form a fully saturated or partly saturated 3- to 8-membered monocyclic or bicyclic ring optionally interrupted by heteroatoms and optionally having further substitution, or
R⁷ and R⁸ together with the carbon atom to which they are bonded form a fully saturated or partly saturated 3- to 10-membered monocyclic or bicyclic ring optionally interrupted by heteroatoms and optionally having further substitution, or
R⁹ and R¹⁰ together with the carbon atom to which they are bonded form a fully saturated or partly saturated 3- to 10-membered monocyclic or bicyclic ring optionally interrupted by heteroatoms and optionally having further substitution, or
R⁹ and R¹⁰ together with the carbon atom to which they are bonded form an exomethylene group or
R⁷ and R⁹ together with the carbon atoms to which they are bonded form a fully saturated or partly saturated 3- to 10-membered monocyclic or bicyclic ring optionally interrupted by heteroatoms and optionally having further substitution,
R¹¹ is hydrogen, (C₁-C₆)-alkyl, (C₃-C₆)-cycloalkyl, cyano-(C₁-C₆)-alkyl, (C₃-C₆)-cycloalkyl-(C₁-C₆)-alkyl, (C₁-C₆)-alkylsulfonyl, arylsulfonyl, heteroarylsulfonyl, (C₃-C₆)-cycloalkylsulfonyl, heterocyclylsulfonyl, aryl-(C₁-C₆)-alkylsulfonyl, (C₁-C₆)-alkylcarbonyl, arylcarbonyl, heteroarylcarbonyl, (C₃-C₆)-cycloalkylcarbonyl, heterocyclylcarbonyl, (C₁-C₆)-alkoxycarbonyl, (C₁-C₆)-alkoxy, (C₂-C₆)-alkenyloxy, aryl-(C₁-C₆)-alkoxycarbonyl, (C₁-C₆)-haloalkylcarbonyl, (C₂-C₆)-alkenyl, (C₂-C₆)-alkynyl, (C₁-C₆)-haloalkyl, halo-(C₂-C₆)-alkynyl, halo-(C₂-C₆)-alkenyl, (C₁-C₆)-alkoxy-(C₁-C₆)-alkyl, amino, (C₁-C₆)-alkylamino, bis[(C₁-C₆)-alkyl]amino, (C₁-C₆)-alkoxy-(C₁-C₆)-alkoxy-(C₁-C₆)-alkyl, heteroaryl-(C₁-C₆)-alkylsulfonyl, heterocyclyl-(C₁-C₆)-alkylsulfonyl, (C₂-C₆)-alkenyloxycarbonyl, (C₂-C₆)-alkynyloxycarbonyl, (C₁-C₆)-alkylaminocarbonyl, (C₃-C₆)-cycloalkylaminocarbonyl, bis-[(C₁-C₆)-alkyl]aminocarbonyl,
R¹² is hydrogen, (C₁-C₆)-alkyl, (C₃-C₆)-cycloalkyl-(C₁-C₆)-alkyl, (C₂-C₆)-alkenyl, (C₂-C₆)-alkynyl, (C₁-C₆)-alkoxy-(C₁-C₆)-alkyl, aryl, aryl- (C₁-C₆)-alkyl, R¹⁷O(O)C-(C₁-C₆)-alkyl, R¹⁵R¹⁶N(O)C-(C₁-C₆)-alkyl,
R¹³ is hydrogen, (C₁-C₆)-alkyl, (C₁-C₆)-haloalkyl, (C₃-C₆)-cycloalkyl, (C₃-C₆)-cycloalkyl-(C₁-C₆)-alkyl, (C₂-C₆)-alkenyl, (C₂-C₆)-alkynyl, (C₁-C₆)-alkoxy-(C₁-C₆)-alkyl, (C₁-C₆)-haloalkoxy-(C₁-C₆)-alkyl, aryl, aryl-(C₁-C₆)-alkyl, heteroaryl, heteroaryl-(C₁-C₆)-alkyl, heterocyclyl, heterocyclyl-(C₁-C₆)-alkyl, (C₁-C₆)-alkylthio-(C₁-C₆)-alkyl, (C₁-C₆)-haloalkylthio-(C₁-C₆)-alkyl, (C₁-C₆)-alkylcarbonyl-(C₁-C₆)-alkyl, R¹⁷O(O)C-(C₁-C₆)-alkyl, R¹⁵R¹⁶N(O)C-(C₁-C₆)-alkyl, R¹⁵R¹⁶N-(C₁-C₆)-alkyl, or
R⁷ and R¹³ together with the atoms to which they are bonded form a fully saturated or partly saturated 3- to 10-membered monocyclic or bicyclic ring optionally interrupted by heteroatoms and optionally having further substitution, or
R⁹ and R¹³ together with the atoms to which they are bonded form a fully saturated or partly saturated 3- to 10-membered monocyclic or bicyclic ring optionally interrupted by heteroatoms and optionally having further substitution, or
R¹¹ and R¹³ if X is N-R¹¹, together with the nitrogen atom to which they are bonded form a 4- to 10-membered monocyclic or bicyclic ring which is fully saturated or optionally interrupted by heteroatoms and optionally has further substitution, or
R¹² and R¹³, if X is N-O-R¹², together with the nitrogen atom to which they are bonded form a fully saturated and optionally further-substituted 4- to 10-membered monocyclic or bicyclic ring,
R¹⁴ is hydrogen, fluorine, chlorine, bromine, (C₁-C₅)-haloalkyl, (C₁-C₆)-alkoxy,
R¹⁵ and R¹⁶ are the same or different and are independently hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₂-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-haloalkoxy-(C₁-C₆)-alkyl, (C₁-C₆)-alkylthio-(C₁-C₆)-alkyl, (C₁-C₆)-haloalkylthio-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, COR¹⁷, SO₂R¹⁸, (C₁-C₆)-alkyl-HNO₂S-, (C₃-C₆)-cycloalkyl-HNO₂S-, heterocyclyl, (C₁-C₆)-alkoxycarbonyl-(C₁-C₆)-alkyl, (C₁-C₆)-alkoxycarbonyl, aryl-(C₁-C₆)-alkoxycarbonyl-(C₁-C₆)-alkyl, aryl-(C₁-C₆)-alkoxycarbonyl, heteroaryl-(C₁-C₆)-alkoxycarbonyl, (C₂-C₆)-alkenyloxycarbonyl, (C₂-C₆)-alkynyloxycarbonyl, heterocyclyl-(C₁-C₆)-alkyl,
R¹⁷ is hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₂-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, (C₁-C₆)-alkoxycarbonyl-(C₁-C₆)-alkyl, (C₂-C₆)-alkenyloxycarbonyl-(C₁-C₆)-alkyl, aryl-(C₁-C₆)-alkoxycarbonyl-(C₁-C₆)-alkyl, hydroxycarbonyl-(C₁-C₆)-alkyl, heterocyclyl, heterocyclyl-(C₁-C₆)-alkyl,
R¹⁸ is hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₂-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, heterocyclyl-(C₁-C₆)-alkyl, (C₃-C₆)-cycloalkyl-(C₁-C₆)-alkyl, (C₄-C₁₀)-cycloalkenyl-(C₁-C₆)-alkyl, NR¹⁵R¹⁶
and
R¹⁹ and R²⁰ are independently hydrogen, halogen, (C₁-C₆)-alkyl, (C₃-C₆)-cycloalkyl, (C₂-C₆)-alkenyl, (C₂-C₆)-alkynyl, (C₁-C₆)-haloalkyl, aryl, or
R¹⁹ and R²⁰ together with the carbon atom to which they are bonded form a 3- to 10-membered monocyclic or bicyclic ring which is saturated or optionally interrupted by heteroatoms and optionally has further substitution.

4. Compounds of the general formula (I) according to Claim 1 and/or salt thereof, **characterized in that**
R¹ is methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, amino, dimethylamino, diethylamino, methyl(ethyl)amino, methyl(n-propyl)amino,
R² is hydrogen, methyl, ethyl, n-propyl, isopropyl,
R³ is hydrogen, fluorine, chlorine, bromine, methoxy, ethoxy,
R⁴ is halogen, cyano, NO₂, C(O)NH₂, C(S)NH₂, difluoromethyl, trifluoromethyl, ethynyl, propyn-1-yl, 1-butyn-1-yl, pentyn-1-yl, hexyn-1-yl,
R⁵ and R⁶ are independently hydrogen, fluorine, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, spiro[2.2]pent-1-yl, spiro[2.3]hex-1-yl, spiro[2.3]hex-4-yl, 3-spiro[2.3]hex-5-yl, bicyclo[1.1.0]butan-1-yl, bicyclo[1.1.0]butan-2-yl, bicyclo[2.1.0]pentan-1-yl, bicyclo[1.1.1]pentan-1-yl, bicyclo[2.1.0]pentan-2-yl, bicyclo[2.1.0]pentan-5-yl, bicyclo[2.1.1]hexyl, 1-methylcyclopropyl, 2-methylcyclopropyl, 2,2-dimethylcyclopropyl, 2,3-dimethylcyclopropyl, 1,1'-bi(cyclopropyl)-1-yl, 1,1'-bi(cyclopropyl)-2-yl, 2'-methyl-1,1'-bi(cyclopropyl)-2-yl, 1-cyanocyclopropyl, 2-cyanocyclopropyl, 1-methylcyclobutyl, 2-methylcyclobutyl, 3-methylcyclobutyl, 3,3-dimethylcyclobut-1-yl, 1-cyanocyclobutyl, 2-cyanocyclobutyl, 3-cyanocyclobutyl, 3,3-difluorocyclobut-1-yl, 3-fluorocyclobut-1-yl, 2,2-difluorocycloprop-1-yl, 1-fluorocycloprop-1-yl, 2-fluorocycloprop-1-yl, 1-allylcyclopropyl, 1-vinylcyclobutyl, 1-vinylcyclopropyl, 1-ethylcyclopropyl, 1-methylcyclohexyl, 2-methylcyclohexyl, 3-methylcyclohexyl, 1-methoxycyclohexyl, 2-methoxycyclohexyl, 3-methoxycyclohexyl, 2-fluorocycloprop-1-yl, 4-fluorocyclohexyl, 4,4-difluorocyclohexyl, cyclopropylmethyl, cyclobutylmethyl, cyclopentylmethyl, cyclohexylmethyl, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl, trifluoromethyl, pentafluoroethyl, 1,1,2,2-tetrafluoroethyl, heptafluoropropyl, nonafluorobutyl, chlorodifluoromethyl, bromodifluoromethyl, dichlorofluoromethyl, iododifluoromethyl, bromofluoromethyl, 1-fluoroethyl, 2-fluoroethyl, fluoromethyl, difluoromethyl, 2,2-difluoroethyl, 2,2,2-trifluoroethyl, difluoro-tert-butyl, chloromethyl, bromomethyl, methoxy, ethoxy, n-propyloxy, isopropyloxy, n-butyloxy, tert-butyloxy, methoxymethyl, ethoxymethyl, n-propyloxymethyl, isopropyloxymethyl, methoxyethyl, ethoxyethyl, n-propyloxyethyl, isopropyloxyethyl, methoxy-n-propyl, methoxydifluoromethyl, ethoxydifluoromethyl, n-propyloxydifluoromethyl, n-butyloxydifluoromethyl, trifluoromethoxymethyl, trifluoromethoxyethyl, trifluoromethoxy-n-propyl, phenyl, 2-fluorophenyl, 3-fluorophenyl, 4-fluorophenyl, 2,4-difluorophenyl, 2,5-difluorophenyl, 2,6-difluorophenyl, 2,3-difluorophenyl, 3,4-difluorophenyl, 3,5-difluorophenyl, 2,4,5-trifluorophenyl, 3,4,5-trifluorophenyl, 2-chlorophenyl, 3-chlorophenyl, 4-chlorophenyl, 2,4-dichlorophenyl, 2,5-dichlorophenyl, 2,6-dichlorophenyl, 2,3-dichlorophenyl, 3,4-dichlorophenyl, 3,5-dichlorophenyl, 2,4,5-trichlorophenyl, 3,4,5-trichlorophenyl, 2,4,6-trichlorophenyl, 2-bromophenyl, 3-bromophenyl, 4-bromophenyl, 2-iodophenyl, 3-iodophenyl, 4-iodophenyl, 2-bromo-4-fluorophenyl, 2-bromo-4-chlorophenyl, 3-bromo-4-fluorophenyl, 3-bromo-4-chlorophenyl, 3-bromo-5-fluorophenyl, 3-bromo-5-chlorophenyl, 2-fluoro-4-bromophenyl, 2-chloro-4-bromophenyl, 3-fluoro-4-bromophenyl, 3-chloro-4-bromophenyl, 2-chloro-4-fluorophenyl, 3-chloro-4-fluorophenyl, 2-fluoro-3-chlorophenyl, 2-fluoro-4-chlorophenyl, 2-fluoro-5-chlorophenyl, 3-fluoro-4-chlorophenyl, 3-fluoro-5-chlorophenyl, 2-fluoro-6-chlorophenyl, 2-methylphenyl, 3-methylphenyl, 4-methylphenyl, 2,4-dimethylphenyl, 2,5-dimethylphenyl, 2,6-dimethylphenyl, 2,3-dimethylphenyl, 3,4-dimethylphenyl, 3,5-dimethylphenyl, 2,4,5-trimethylphenyl, 3,4,5-trimethylphenyl, 2,4,6-trimethylphenyl, 2-methoxyphenyl, 3-methoxyphenyl, 4-methoxyphenyl, 2,4-dimethoxyphenyl, 2,5-dimethoxyphenyl, 2,6-dimethoxyphenyl, 2,3-dimethoxyphenyl, 3,4-dimethoxyphenyl, 3,5-dimethoxyphenyl, 2,4,5-trimethoxyphenyl, 3,4,5-trimethoxyphenyl, 2,4,6-trimethoxyphenyl, 2-trifluoromethoxyphenyl, 3-trifluoromethoxyphenyl, 4-trifluoromethoxyphenyl, 2-difluoromethoxyphenyl, 3-difluoromethoxyphenyl, 4-difluoromethoxyphenyl, 2-trifluoromethylphenyl, 3-trifluoromethylphenyl, 4-trifluoromethylphenyl, 2-difluoromethylphenyl, 3-difluoromethylphenyl, 4-difluoromethylphenyl, 3,5-bis(trifluoromethyl)phenyl, 3-trifluoromethyl-5-fluorophenyl, 3-trifluoromethyl-5-chlorophenyl, 3-methyl-5-fluorophenyl, 3-methyl-5-chlorophenyl, 3-methoxy-5-fluorophenyl, 3-methoxy-5-chlorophenyl, 3-trifluoromethoxy-5-chlorophenyl, 2-ethoxyphenyl, 3-ethoxyphenyl, 4-ethoxyphenyl, 2-methylthiophenyl, 3-methylthiophenyl, 4-methylthiophenyl, 2-trifluoromethylthiophenyl, 3-trifluoromethylthiophenyl, 4-trifluoromethylthiophenyl, 2-ethylphenyl, 3-ethylphenyl, 4-ethylphenyl, 2-methoxycarbonylphenyl, 3-methoxycarbonylphenyl, 4-methoxycarbonylphenyl, 2-ethoxycarbonylphenyl, 3-ethoxycarbonylphenyl, 4-ethoxycarbonylphenyl, pyridin-2-yl, pyridin-3-yl, pyridin-4-yl, pyrazin-2-yl, pyridazin-3-yl, pyridazin-4-yl, pyrimidin-2-yl, pyrimidin-5-yl, pyrimidin-4-yl, pyridazin-3-ylmethyl, pyridazin-4-ylmethyl, pyrimidin-2-ylmethyl, pyrimidin-5-ylmethyl, pyrimidin-4-ylmethyl, pyrazin-2-ylmethyl, 3-chloropyrazin-2-yl, 3-bromopyrazin-2-yl, 3-methoxypyrazin-2-yl, 3-ethoxypyrazin-2-yl, 3-trifluoromethylpyrazin-2-yl, 3-cyanopyrazin-2-yl, naphth-2-yl, naphth-1-yl, quinolin-4-yl, quinolin-6-yl, quinolin-8-yl, quinolin-2-yl, quinoxalin-2-yl, 2-naphthylmethyl, 1-naphthylmethyl, quinolin-4-ylmethyl, quinolin-6-ylmethyl, quinolin-8-ylmethyl, quinolin-2-ylmethyl, quinoxalin-2-ylmethyl, pyrazin-2-ylmethyl, 4-chloropyridin-2-yl, 3-chloropyridin-4-yl, 2-chloropyridin-3-yl, 2-chloropyridin-4-yl, 2-chloropyridin-5-yl, 2,6-dichloropyridin-4-yl, 3-chloropyridin-5-yl, 3,5-dichloropyridin-2-yl, 3-chloro-5-trifluoromethylpyridin-2-yl, (4-chloropyridin-2-yl)methyl, (3-chloropyridin-4-yl)methyl, (2-chloropyridin-3-yl)methyl, (2-chloropyridin-4-yl)methyl, (2-chloropyridin-5-yl)methyl, (2,6-dichloropyridin-4-yl)methyl, (3-chloropyridin-5-yl)methyl, (3,5-dichloropyridin-2-yl)methyl, thiophen-2-yl, thiophen-3-yl, 5-methylthiophen-2-yl, 5-ethylthiophen-2-yl, 5-chlorothiophen-2-yl, 5-bromothiophen-2-yl, 4-methylthiophen-2-yl, 3-methylthiophen-2-yl, 5-fluorothiophen-3-yl, 3,5-dimethylthiophen-2-yl, 3-ethylthiophen-2-yl, 4,5-dimethylthiophen-2-yl, 3,4-dimethylthiophen-2-yl, 4-chlorothiophen-2-yl, furan-2-yl, 5-methylfuran-2-yl, 5-ethylfuran-2-yl, 5-methoxycarbonylfuran-2-yl, 5-chlorofuran-2-yl, 5-bromofuran-2-yl, thiophan-2-yl, thiophan-3-yl, sulfolan-2-yl, sulfolan-3-yl, tetrahydrothiopyran-4-yl, tetrahydropyran-4-yl, tetrahydrofuran-2-yl, tetrahydrofuran-3-yl, 1-(4-methylphenyl)ethyl, 1-(3-methylphenyl)ethyl, 1-(2-methylphenyl)ethyl, 1-(4-chlorophenyl)ethyl, 1-(3-chlorophenyl)ethyl, 1-(2-chlorophenyl)ethyl, benzyl, (4-fluorophenyl)methyl, (3-fluorophenyl)methyl, (2-fluorophenyl)methyl, (2,4-difluorophenyl)methyl, (3,5-difluorophenyl)methyl, (2,5-difluorophenyl)methyl, (2,6-difluorophenyl)methyl, (2,4,5-trifluorophenyl)methyl, (2,4,6-trifluorophenyl)methyl, (4-chlorophenyl)methyl, (3-chlorophenyl)methyl, (2-chlorophenyl)methyl, (2,4-dichlorophenyl)methyl, (3,5-dichlorophenyl)methyl, (2,5-dichlorophenyl)methyl, (2,6-dichlorophenyl)methyl, (2,4,5-trichlorophenyl)methyl, (2,4,6-trichlorophenyl)methyl, (4-bromophenyl)methyl, (3-bromophenyl)methyl, (2-bromophenyl)methyl, (4-iodophenyl)methyl, (3-iodophenyl)methyl, (2-iodophenyl)methyl, (3-chloro-5-trifluoromethylpyridin-2-yl)methyl, (2-bromo-4-fluorophenyl)methyl, (2-bromo-4-chlorophenyl)methyl, (3-bromo-4-fluorophenyl)methyl, (3-bromo-4-chlorophenyl)methyl, (3-bromo-5-fluorophenyl)methyl, (3-bromo-5-chlorophenyl)methyl, (2-fluoro-4-bromophenyl)methyl, (2-chloro-4-bromophenyl)methyl, (3-fluoro-4-bromophenyl)methyl, (3-chloro-4-bromophenyl)methyl, (2-chloro-4-fluorophenyl)methyl, (3-chloro-4-fluorophenyl)methyl, (2-fluoro-3-chlorophenyl)methyl, (2-fluoro-4-chlorophenyl)methyl, (2-fluoro-5-chlorophenyl)methyl, (3-fluoro-4-chlorophenyl)methyl, (3-fluoro-5-chlorophenyl)methyl, (2-fluoro-6-chlorophenyl)methyl, 2-phenyleth-1-yl, 3-trifluoromethyl-4-chlorophenyl, 3-chloro-4-trifluoromethylphenyl, 2-chloro-4-trifluoromethylphenyl, 3,5-difluoropyridin-2-yl, (3,6-dichloropyridin-2-yl)methyl, (4-trifluoromethylphenyl)methyl, (3-trifluoromethylphenyl)methyl, (2-trifluoromethylphenyl)methyl, (4-trifluoromethoxyphenyl)methyl, (3-trifluoromethoxyphenyl)methyl, (2-trifluoromethoxyphenyl)methyl, (4-methoxyphenyl)methyl, (3-methoxyphenyl)methyl, (2-methoxyphenyl)methyl, (4-methylphenyl)methyl, (3-methylphenyl)methyl, (2-methylphenyl)methyl, (4-cyanophenyl)methyl, (3-cyanophenyl)methyl, (2-cyanophenyl)methyl, (2,4-diethylphenyl)methyl, (3,5-diethylphenyl)methyl, (3,4-dimethylphenyl)methyl, (3,5-dimethoxyphenyl)methyl, 1-phenyleth-1-yl, 1-(o-chlorophenyl)eth-1-yl, 1,3-thiazol-2-yl, 4-methyl-1,3-thiazol-2-yl, 1,3-thiazol-2-yl, methylthiomethyl, ethylthiomethyl, ethylthioethyl, methylthioethyl, n-propylthiomethyl, isopropylthiomethyl, trifluoromethylthiomethyl, trifluoromethylthioethyl, methylcarbonylmethyl, ethylcarbonylmethyl, n-propylcarbonylmethyl, isopropylcarbonylmethyl, methylcarbonylethyl, hydroxycarbonyl, methoxycarbonyl, ethoxycarbonyl, n-propyloxycarbonyl, isopropyloxycarbonyl, n-butyloxycarbonyl, tert-butyloxycarbonyl, allyloxycarbonyl, benzyloxycarbonyl, aminocarbonyl, methylaminocarbonyl, ethylaminocarbonyl, n-propylaminocarbonyl, isopropylaminocarbonyl, dimethylaminocarbonyl, diethylaminocarbonyl, methyl(ethyl)aminocarbonyl, cyclopropylaminocarbonyl, cyclobutylaminocarbonyl, cyclopentylaminocarbonyl, cyclohexylaminocarbonyl, allylaminocarbonyl, benzylaminocarbonyl, tert-butyloxycarbonylaminocarbonyl, hydroxycarbonylmethyl, methoxycarbonylmethyl, ethoxycarbonylmethyl, n-propyloxycarbonylmethyl, isopropyloxycarbonylmethyl, n-butyloxycarbonylmethyl, tert-butyloxycarbonylmethyl, allyloxycarbonylmethyl, benzyloxycarbonylmethyl, aminocarbonylmethyl, methylaminocarbonylmethyl, ethylaminocarbonylmethyl, n-propylaminocarbonylmethyl, isopropylaminocarbonylmethyl, dimethylaminocarbonylmethyl, diethylaminocarbonylmethyl, methyl(ethyl)aminocarbonylmethyl, cyclopropylaminocarbonylmethyl, cyclobutylaminocarbonylmethyl, cyclopentylaminocarbonylmethyl, cyclohexylaminocarbonylmethyl, allylaminocarbonylmethyl, benzylaminocarbonylmethyl, 1-(hydroxycarbonyl)eth-1-yl, 1-(methoxycarbonyl)eth-1-yl, 1-(ethoxycarbonyl)eth-1-yl, 1-(tert-butyloxycarbonyl)eth-1-yl, 1-(benzyloxycarbonyl)eth-1-yl, 2-(hydroxycarbonyl)eth-1-yl, 2-(methoxycarbonyl)eth-1-yl, 2-(ethoxycarbonyl)eth-1-yl, 2-(tert-butyloxycarbonyl)eth-1-yl, 2-(benzyloxycarbonyl)eth-1-yl, 1-(hydroxycarbonyl)prop-1-yl, 1-(methoxycarbonyl)prop-1-yl, 1-(ethoxycarbonyl)prop-1-yl, 1-(tert-butyloxycarbonyl)prop-1-yl, 1-(benzyloxycarbonyl)prop-1-yl, 2-(hydroxycarbonyl)prop-1-yl, 2-(methoxycarbonyl)prop-1-yl, 2-(ethoxycarbonyl)prop-1-yl, 2-(tert-butyloxycarbonyl)prop-1-yl, 2-(benzyloxycarbonyl)prop-1-yl, 1-(hydroxycarbonyl)prop-2-yl, 1-(methoxycarbonyl)prop-2-yl, 1-(ethoxycarbonyl)prop-2-yl, 1-(tert-butyloxycarbonyl)prop-2-yl, 1-(benzyloxycarbonyl)prop-2-yl, 3-(hydroxycarbonyl)prop-1-yl, 3-(methoxycarbonyl)prop-1-yl, 3-(ethoxycarbonyl)prop-1-yl, 3-(tert-butyloxycarbonyl)prop-1-yl, 3-(benzyloxycarbonyl)prop-1-yl, aminomethyl, 2-aminoeth-1-yl, 1-aminoeth-1-yl, 1-aminoprop-1-yl, 3-aminoprop-1-yl, methylaminomethyl, dimethylaminomethyl, diethylaminomethyl, ethylaminomethyl, isopropylaminomethyl, cyclopropylaminomethyl, cyclobutylaminomethyl, cyclopentylaminomethyl, cyclohexylaminomethyl, methoxycarbonylaminomethyl, ethoxycarbonylaminomethyl, tert-butyloxycarbonylaminomethyl, methylcarbonylaminomethyl, ethylcarbonylaminomethyl, n-propylcarbonylaminomethyl, isopropylcarbonylaminomethyl, 2-(methylamino)eth-1-yl, 2-(dieth-1-ylamino)eth-1-yl, 2-(diethylamino)eth-1-yl, 2-(ethylamino)eth-1-yl, 2-(isopropylamino)eth-1-yl, 2-(cyclopropylamino)eth-1-yl, 2-(cyclobutylamino)eth-1-yl, 2-(cyclopentylamino)eth-1-yl, 2-(cyclohexylamino)eth-1-yl, 2-(methoxycarbonylamino)eth-1-yl, 2-(ethoxycarbonylamino)eth-1-yl, 2-(tert-butyloxycarbonylamino)eth-1-yl, 2-(methylcarbonylamino)eth-1-yl, 2-(ethylcarbonylamino)eth-1-yl, 2-(n-propylcarbonylamino)eth-1-yl, 2-(isopropylcarbonylamino)eth-1-yl, 1-(methylamino)eth-1-yl, 1-(dieth-1-ylamino)eth-1-yl, 1-(diethylamino)eth-1-yl, 1-(ethylamino)eth-1-yl, 1-(isopropylamino)eth-1-yl, 1-(cyclopropylamino)eth-1-yl, 1-(cyclobutylamino)eth-1-yl, 1-(cyclopentylamino)eth-1-yl, 1-(cyclohexylamino)eth-1-yl, 1-(methoxycarbonylamino)eth-1-yl, 1-(ethoxycarbonylamino)eth-1-yl, 1-(tert-butyloxycarbonylamino)eth-1-yl, 1-(methylcarbonylamino)eth-1-yl, 1-(ethylcarbonylamino)eth-1-yl, 1-(n-propylcarbonylamino)eth-1-yl, 1-(isopropylcarbonylamino)eth-1-yl, or
R⁵ and R⁶ together with the carbon atom to which they are bonded form a fully saturated or partly saturated 3- to 10-membered monocyclic or bicyclic ring optionally interrupted by heteroatoms and optionally having further substitution, or
R⁵ and R⁶ together with the carbon atom to which they are bonded form a double bond optionally further substituted by R¹⁹ and R²⁰, according to formula (I') below 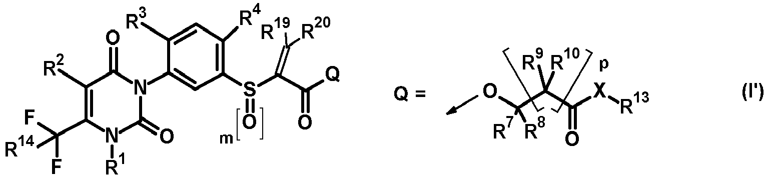
R¹⁴ is hydrogen, fluorine, chlorine, bromine, trifluoromethyl, methoxy, ethoxy, n-propyloxy, n-butyloxy,
m is 0, 1, 2,
R¹⁹ and R²⁰ are independently hydrogen, fluorine, chlorine, bromine, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, cyclopropyl, cyclobutyl, cyclopentyl, cyclohexyl, trifluoromethyl, difluoromethyl, pentafluoroethyl, ethenyl, 1-propenyl, 1-methylethenyl, 1-butenyl, phenyl, or
R¹⁹ and R²⁰ together with the carbon atom to which they are bonded form a 3- to 10-membered monocyclic or bicyclic ring which is saturated or optionally interrupted by heteroatoms and optionally has further substitution,
and Q is one of the moieties Q-1 to Q-300 specified below:
| | | | | |
|---|---|---|---|---|
| 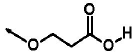 | 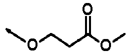 | 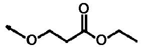 | 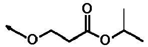 | 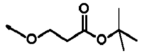 |
| Q-1 | Q-2 | Q-3 | Q-4 | Q-5 |
| | | | | |
| 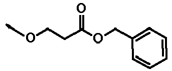 | 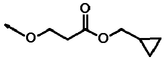 | 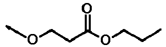 | 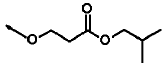 | 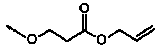 |
| Q-6 | Q-7 | Q-8 | Q-9 | Q-10 |
| | | | | |
| 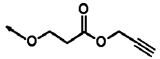 | 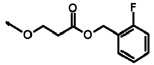 | 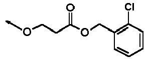 | 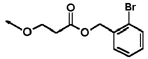 | 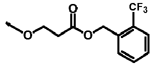 |
| Q-11 | Q-12 | Q-13 | Q-14 | Q-15 |
| | | | | |
| 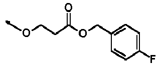 | 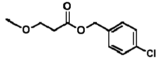 | 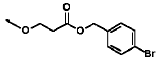 | 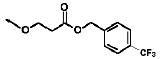 | 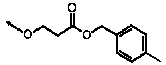 |
| Q-16 | Q-17 | Q-18 | Q-19 | Q-20 |
| | | | | |
| 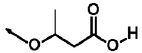 | 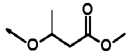 | 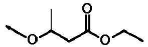 | 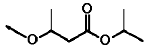 | 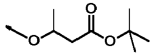 |
| Q-21 | Q-22 | Q-23 | Q-24 | Q-25 |
| | | | | |
| 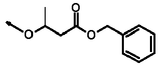 | 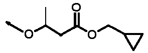 | 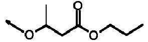 | 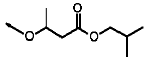 | 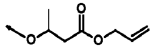 |
| Q-26 | Q-27 | Q-28 | Q-29 | Q-30 |
| 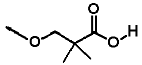 | 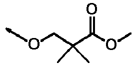 | 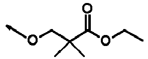 | 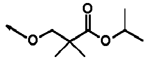 | 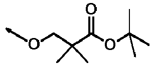 |
| Q-31 | Q-32 | Q-33 | Q-34 | Q-35 |
| | | | | |
| 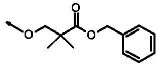 | 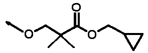 | 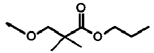 | 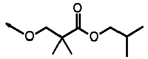 | 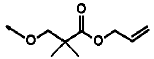 |
| Q-36 | Q-37 | Q-38 | Q-39 | Q-40 |
| | | | | |
| 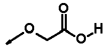 | 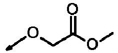 | 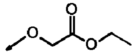 | 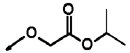 | 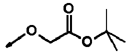 |
| Q-41 | Q-42 | Q-43 | Q-44 | Q-45 |
| | | | | |
| 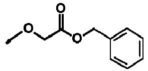 | 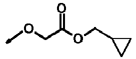 | 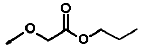 | 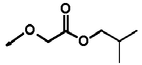 | 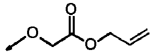 |
| Q-46 | Q-47 | Q-48 | Q-49 | Q-50 |
| | | | | |
| 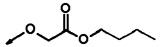 | 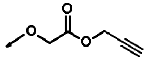 | 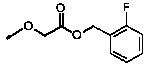 | 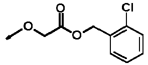 | 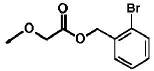 |
| Q-51 | Q-52 | Q-53 | Q-54 | Q-55 |
| 5 | | | | |
| 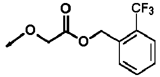 | 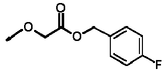 | 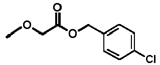 | 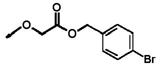 | 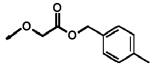 |
| Q-56 | Q-57 | Q-58 | Q-59 | Q-50 |
| | | | | |
| 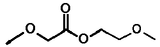 | 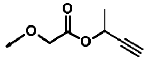 | 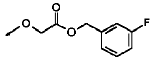 | 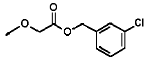 | 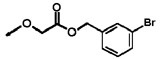 |
| Q-61 | Q-62 | Q-63 | Q-64 | Q-65 |
| | | | | |
| 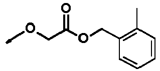 | 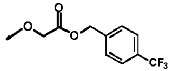 | 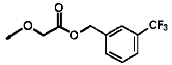 | 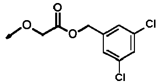 | 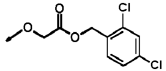 |
| Q-66 | Q-67 | Q-68 | Q-69 | Q-70 |
| | | | | |
| 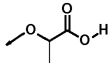 | 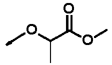 | 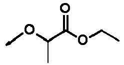 | 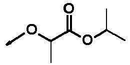 | 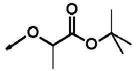 |
| Q-71 | Q-72 | Q-73 | Q-74 | Q-75 |
| 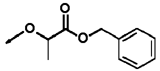 | 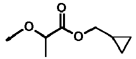 | 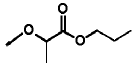 | 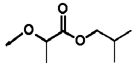 | 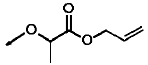 |
| Q-76 | Q-77 | Q-78 | Q-79 | Q-80 |
| | | | | |
| 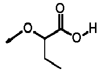 | 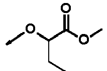 | 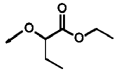 | 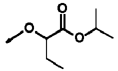 | 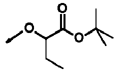 |
| Q-81 | Q-82 | Q-83 | Q-84 | Q-85 |
| | | | | |
| 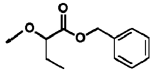 | 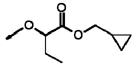 | 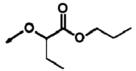 | 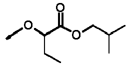 | 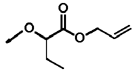 |
| Q-86 | Q-87 | Q-88 | Q-89 | Q-90 |
| | | | | |
| 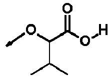 | 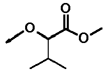 | 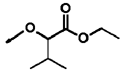 | 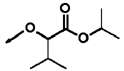 | 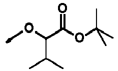 |
| Q-91 | Q-92 | Q-93 | Q-94 | Q-95 |
| | | | | |
| 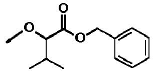 | 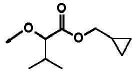 | 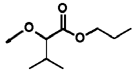 | 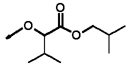 | 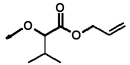 |
| Q-96 | Q-97 | Q-98 | Q-99 | Q-100 |
| | | | | |
| 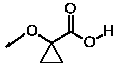 | 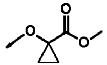 | 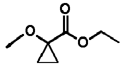 | 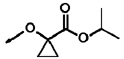 | 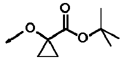 |
| Q-101 | Q-102 | Q-103 | Q-104 | Q-105 |
| | | | | |
| 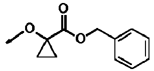 | 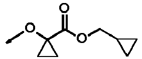 | 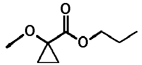 | 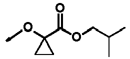 | 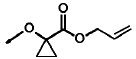 |
| Q-106 | Q-107 | Q-108 | Q-109 | Q-110 |
| | | | | |
| 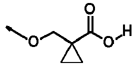 | 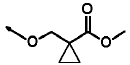 | 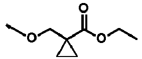 | 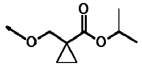 | 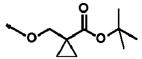 |
| Q-111 | Q-112 | Q-113 | Q-114 | Q-115 |
| | | | | |
| 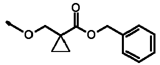 | 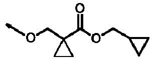 | 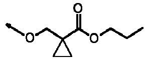 | 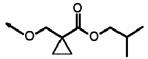 | 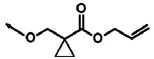 |
| Q-116 | Q-117 | Q-118 | Q-119 | Q-120 |
| 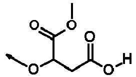 | 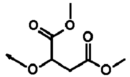 | 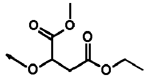 | 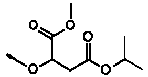 | 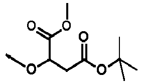 |
| Q-121 | Q-122 | Q-123 | Q-124 | Q-125 |
| | | | | |
| 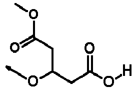 | 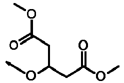 | 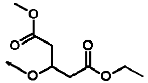 | 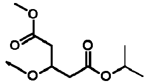 | 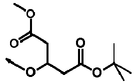 |
| Q-126 | Q-127 | Q-128 | Q-129 | Q-130 |
| | | | | |
| 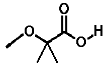 | 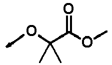 | 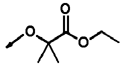 | 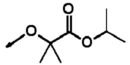 | 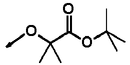 |
| Q-131 | Q-132 | Q-133 | Q-134 | Q-135 |
| | | | | |
| 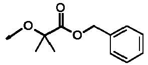 | 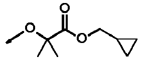 | 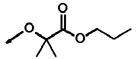 | 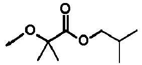 | 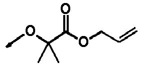 |
| Q-136 | Q-137 | Q-138 | Q-139 | Q-140 |
| | | | | |
| 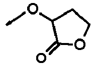 | 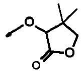 | 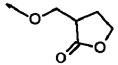 | 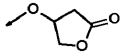 | 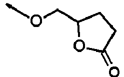 |
| Q-141 | Q-142 | Q-143 | Q-144 | Q-145 |
| | | | | |
| 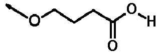 | 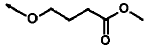 | 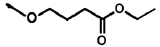 | 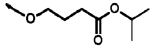 | 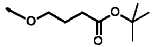 |
| Q-146 | Q-147 | Q-148 | Q-149 | Q-150 |
| | | | | |
| 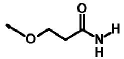 | 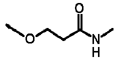 | 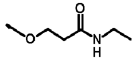 | 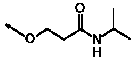 | 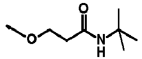 |
| Q-151 | Q-152 | Q-153 | Q-154 | Q-155 |
| | | | | |
| 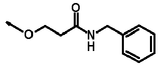 | 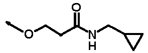 | 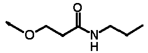 | 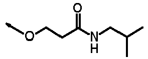 | 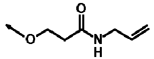 |
| Q-156 | Q-157 | Q-158 | Q-159 | Q-160 |
| | | | | |
| 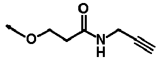 | 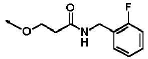 | 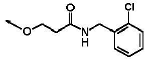 | 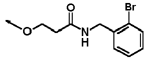 | 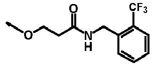 |
| Q-161 | Q-162 | Q-163 | Q-164 | Q-165 |
| | | | | |
| 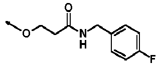 | 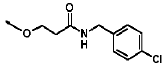 | 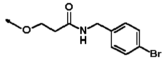 | 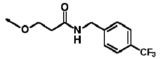 | 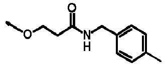 |
| Q-166 | Q-167 | Q-168 | Q-169 | Q-170 |
| | | | | |
| 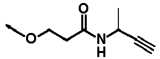 | 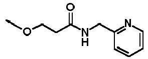 | 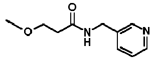 | 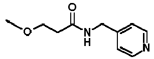 | 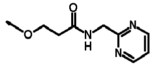 |
| Q-171 | Q-172 | Q-173 | Q-174 | Q-175 |
| | | | | |
| 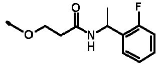 | 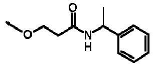 | 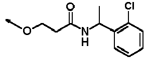 | 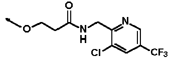 | 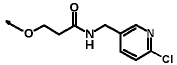 |
| Q-176 | Q-177 | Q-178 | Q-179 | Q-180 |
| | | | | |
| 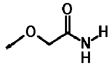 | 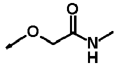 | 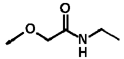 | 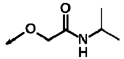 | 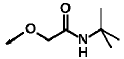 |
| Q-181 | Q-182 | Q-183 | Q-184 | Q-185 |
| | | | | |
| 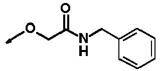 | 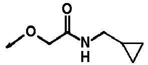 | 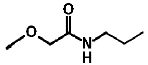 | 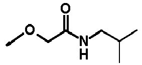 | 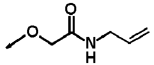 |
| Q-186 | Q-187 | Q-188 | Q-189 | Q-190 |
| | | | | |
| 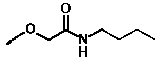 | 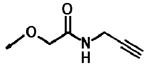 | 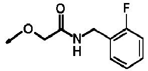 | 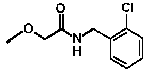 | 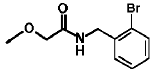 |
| Q-191 | Q-192 | Q-193 | Q-194 | Q-195 |
| | | | | |
| 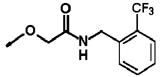 | 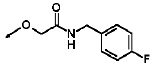 | 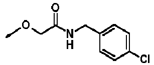 | 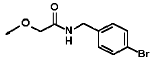 | 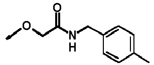 |
| Q-196 | Q-197 | Q-198 | Q-199 | Q-200 |
| | | | | |
| 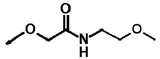 | 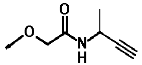 | 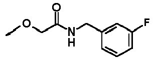 | 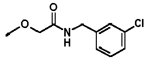 | 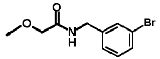 |
| Q-201 | Q-202 | Q-203 | Q-204 | Q-205 |
| | | | | |
| 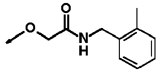 | 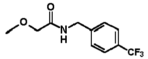 | 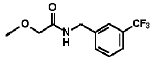 | 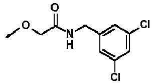 | 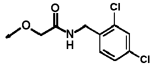 |
| Q-206 | Q-207 | Q-208 | Q-209 | Q-210 |
| | | | | |
| 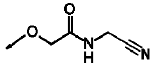 | 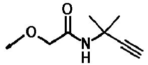 | 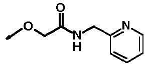 | 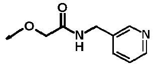 | 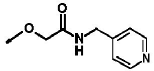 |
| Q-211 | Q-212 | Q-213 | Q-214 | Q-215 |
| | | | | |
| 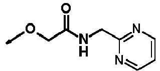 | 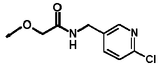 | 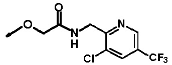 | 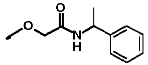 | 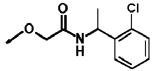 |
| Q-216 | Q-217 | Q-218 | Q-219 | Q-220 |
| | | | | |
| 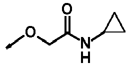 | 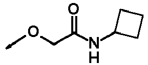 | 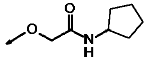 | 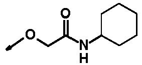 | 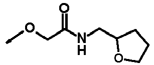 |
| Q-221 | Q-222 | Q-223 | Q-224 | Q-225 |
| | | | | |
| 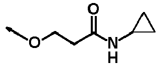 | 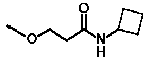 | 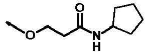 | 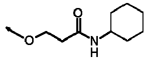 | 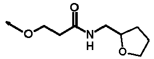 |
| Q-226 | Q-227 | Q-228 | Q-229 | Q-230 |
| | | | | |
| 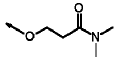 | 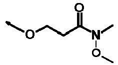 | 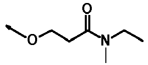 | 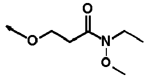 | 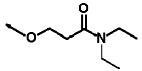 |
| Q-231 | Q-232 | Q-233 | Q-234 | Q-235 |
| | | | | |
| 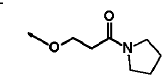 | 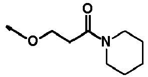 | 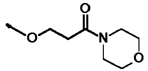 | 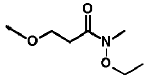 | 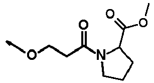 |
| Q-236 | Q-237 | Q-238 | Q-239 | Q-240 |
| | | | | |
| 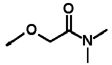 | 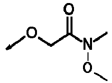 | 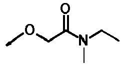 | 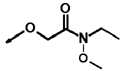 | 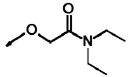 |
| Q-241 | Q-242 | Q-243 | Q-244 | Q-245 |
| | | | | |
| 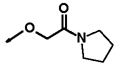 | 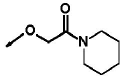 | 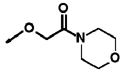 | 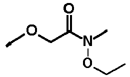 | 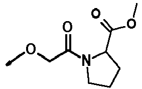 |
| Q-246 | Q-247 | Q-248 | Q-249 | Q-250 |
| | | | | |
| 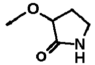 | 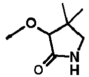 | 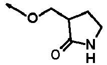 | 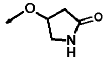 | 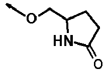 |
| Q-251 | Q-252 | Q-253 | Q-254 | Q-255 |
| | | | | |
| 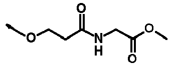 | 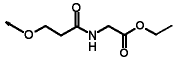 | 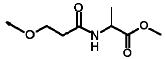 | 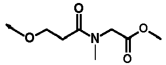 | 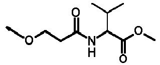 |
| Q-256 | Q-257 | Q-258 | Q-259 | Q-260 |
| | | | | |
| 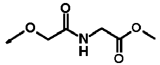 | 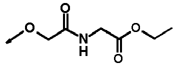 | 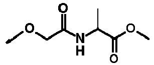 | 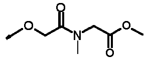 | 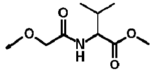 |
| Q-261 | Q-262 | Q-263 | Q-264 | Q-265 |
| | | | | |
| 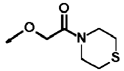 | 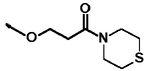 | 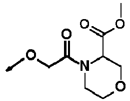 | 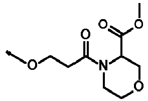 | 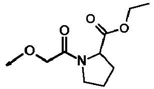 |
| Q-266 | Q-267 | Q-268 | Q-269 | Q-270 |
| | | | | |
| 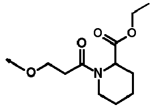 | 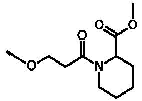 | 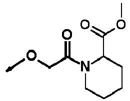 | 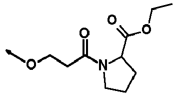 | 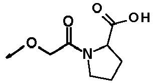 |
| Q-271 | Q-272 | Q-273 | Q-274 | Q-275 |
| | | | | |
| 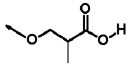 | 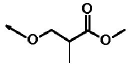 | 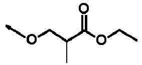 | 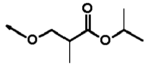 | 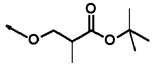 |
| Q-276 | Q-277 | Q-278 | Q-279 | Q-280 |
| | | | | |
| 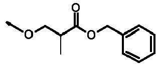 | 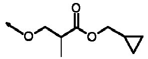 | 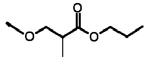 | 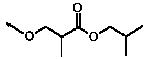 | 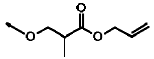 |
| Q-281 | Q-282 | Q-283 | Q-284 | Q-285 |
| | | | | |
| 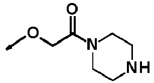 | 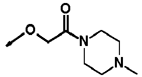 | 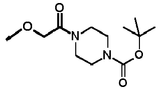 | 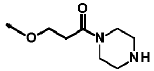 | 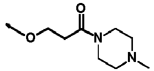 |
| Q-286 | Q-287 | Q-288 | Q-289 | Q-290 |
| | | | | |
| 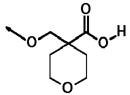 | 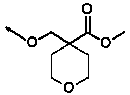 | 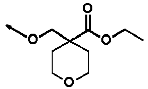 | 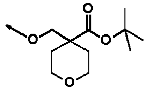 | 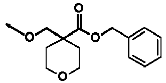 |
| Q-291 | Q-292 | Q-293 | Q-294 | Q-295 |
| | | | | |
| 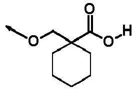 | 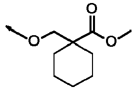 | 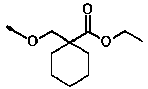 | 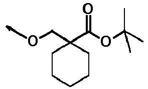 | 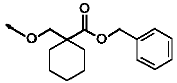 |
| Q-296 | Q-297 | Q-298 | Q-299 | Q-300 |

5. Compounds of the general formula (I) according to Claim 1 and/or salt thereof, **characterized in that**
R¹ is methyl, ethyl, n-propyl, 1-methylethyl, n-butyl, 1-methylpropyl, 2-methylpropyl, 1,1-dimethylethyl, n-pentyl, amino, dimethylamino, diethylamino,
R² is hydrogen, methyl, ethyl, n-propyl, isopropyl,
R³ is hydrogen, fluorine, chlorine, bromine, methoxy, ethoxy,
R⁴ is halogen, cyano, C(O)NH₂, C(S)NH₂, difluoromethyl, trifluoromethyl, ethynyl, propyn-1-yl,
R⁵ and R⁶ are independently hydrogen, fluorine, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, cyclopropylmethyl, cyclobutylmethyl, cyclopentylmethyl, cyclohexylmethyl, ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, trifluoromethyl, pentafluoroethyl, 1,1,2,2-tetrafluoroethyl, heptafluoropropyl, nonafluorobutyl, chlorodifluoromethyl, bromodifluoromethyl, dichlorofluoromethyl, iododifluoromethyl, bromofluoromethyl, 1-fluoroethyl, 2-fluoroethyl, fluoromethyl, difluoromethyl, 2,2-difluoroethyl, 2,2,2-trifluoroethyl, difluoro-tert-butyl, methoxy, ethoxy, n-propyloxy, isopropyloxy, n-butyloxy, tert-butyloxy, methoxymethyl, ethoxymethyl, n-propyloxymethyl, isopropyloxymethyl, methoxyethyl, ethoxyethyl, n-propyloxyethyl, isopropyloxyethyl, methoxy-n-propyl, methoxydifluoromethyl, ethoxydifluoromethyl, n-propyloxydifluoromethyl, n-butyloxydifluoromethyl, trifluoromethoxymethyl, trifluoromethoxyethyl, trifluoromethoxy-n-propyl, phenyl, 2-fluorophenyl, 3-fluorophenyl, 4-fluorophenyl, 2,4-difluorophenyl, 2,5-difluorophenyl, 2,6-difluorophenyl, 2,3-difluorophenyl, 3,4-difluorophenyl, 3,5-difluorophenyl, 2,4,5-trifluorophenyl, 3,4,5-trifluorophenyl, 2-chlorophenyl, 3-chlorophenyl, 4-chlorophenyl, 2,4-dichlorophenyl, 2,5-dichlorophenyl, 2,6-dichlorophenyl, 2,3-dichlorophenyl, 3,4-dichlorophenyl, 3,5-dichlorophenyl, 2,4,5-trichlorophenyl, 3,4,5-trichlorophenyl, 2,4,6-trichlorophenyl, pyridin-2-yl, pyridin-3-yl, pyridin-4-yl, thiophen-2-yl, thiophen-3-yl, furan-2-yl, tetrahydrofuran-2-yl, tetrahydrofuran-3-yl, 1-(4-methylphenyl)ethyl, 1-(3-methylphenyl)ethyl, 1-(2-methylphenyl)ethyl, 1-(4-chlorophenyl)ethyl, 1-(3-chlorophenyl)ethyl, 1-(2-chlorophenyl)ethyl, benzyl, (4-fluorophenyl)methyl, (3-fluorophenyl)methyl, (2-fluorophenyl)methyl, (2,4-difluorophenyl)methyl, (3,5-difluorophenyl)methyl, (2,5-difluorophenyl)methyl, (2,6-difluorophenyl)methyl, (2,4,5-trifluorophenyl)methyl, (2,4,6-trifluorophenyl)methyl, (4-chlorophenyl)methyl, (3-chlorophenyl)methyl, (2-chlorophenyl)methyl, (2,4-dichlorophenyl)methyl, (3,5-dichlorophenyl)methyl, (2,5-dichlorophenyl)methyl, (2,6-dichlorophenyl)methyl, (2,4,5-trichlorophenyl)methyl, (2,4,6-trichlorophenyl)methyl, methylthiomethyl, ethylthiomethyl, ethylthioethyl, methylthioethyl, n-propylthiomethyl, isopropylthiomethyl, trifluoromethylthiomethyl, trifluoromethylthioethyl, methylcarbonylmethyl, ethylcarbonylmethyl, n-propylcarbonylmethyl, isopropylcarbonylmethyl, methylcarbonylethyl, hydroxycarbonyl, methoxycarbonyl, ethoxycarbonyl, n-propyloxycarbonyl, isopropyloxycarbonyl, n-butyloxycarbonyl, tert-butyloxycarbonyl, allyloxycarbonyl, benzyloxycarbonyl, aminocarbonyl, methylaminocarbonyl, ethylaminocarbonyl, n-propylaminocarbonyl, isopropylaminocarbonyl, dimethylaminocarbonyl, diethylaminocarbonyl, methyl(ethyl)aminocarbonyl, cyclopropylaminocarbonyl, cyclobutylaminocarbonyl, cyclopentylaminocarbonyl, cyclohexylaminocarbonyl, allylaminocarbonyl, benzylaminocarbonyl, tert-butyloxycarbonylaminocarbonyl, hydroxycarbonylmethyl, methoxycarbonylmethyl, ethoxycarbonylmethyl, n-propyloxycarbonylmethyl, isopropyloxycarbonylmethyl, n-butyloxycarbonylmethyl, tert-butyloxycarbonylmethyl, allyloxycarbonylmethyl, benzyloxycarbonylmethyl, aminocarbonylmethyl, methylaminocarbonylmethyl, ethylaminocarbonylmethyl, n-propylaminocarbonylmethyl, isopropylaminocarbonylmethyl, dimethylaminocarbonylmethyl, diethylaminocarbonylmethyl, methyl(ethyl)aminocarbonylmethyl, cyclopropylaminocarbonylmethyl, cyclobutylaminocarbonylmethyl, cyclopentylaminocarbonylmethyl, cyclohexylaminocarbonylmethyl, allylaminocarbonylmethyl, benzylaminocarbonylmethyl, 1-(hydroxycarbonyl)eth-1-yl, 1-(methoxycarbonyl)eth-1-yl, 1-(ethoxycarbonyl)eth-1-yl, 1-(tert-butyloxycarbonyl)eth-1-yl, 1-(benzyloxycarbonyl)eth-1-yl, 2-(hydroxycarbonyl)eth-1-yl, 2-(methoxycarbonyl)eth-1-yl, 2-(ethoxycarbonyl)eth-1-yl, 2-(tert-butyloxycarbonyl)eth-1-yl, 2-(benzyloxycarbonyl)eth-1-yl, 1-(hydroxycarbonyl)prop-1-yl, 1-(methoxycarbonyl)prop-1-yl, 1-(ethoxycarbonyl)prop-1-yl, 1-(tert-butyloxycarbonyl)prop-1-yl, 1-(benzyloxycarbonyl)prop-1-yl, 2-(hydroxycarbonyl)prop-1-yl, 2-(methoxycarbonyl)prop-1-yl, 2-(ethoxycarbonyl)prop-1-yl, 2-(tert-butyloxycarbonyl)prop-1-yl, 2-(benzyloxycarbonyl)prop-1-yl, 1-(hydroxycarbonyl)prop-2-yl, 1-(methoxycarbonyl)prop-2-yl, 1-(ethoxycarbonyl)prop-2-yl, 1-(tert-butyloxycarbonyl)prop-2-yl, 1-(benzyloxycarbonyl)prop-2-yl, 3-(hydroxycarbonyl)prop-1-yl, 3-(methoxycarbonyl)prop-1-yl, 3-(ethoxycarbonyl)prop-1-yl, 3-(tert-butyloxycarbonyl)prop-1-yl, 3-(benzyloxycarbonyl)prop-1-yl, aminomethyl, 2-aminoeth-1-yl, 1-aminoeth-1-yl, 1-aminoprop-1-yl, 3-aminoprop-1-yl, methylaminomethyl, dimethylaminomethyl, diethylaminomethyl, ethylaminomethyl, isopropylaminomethyl, cyclopropylaminomethyl, cyclobutylaminomethyl, cyclopentylaminomethyl, cyclohexylaminomethyl, methoxycarbonylaminomethyl, ethoxycarbonylaminomethyl, tert-butyloxycarbonylaminomethyl, methylcarbonylaminomethyl, ethylcarbonylaminomethyl, n-propylcarbonylaminomethyl, isopropylcarbonylaminomethyl, 2-(methylamino)eth-1-yl, 2-(dieth-1-ylamino)eth-1-yl, 2-(diethylamino)eth-1-yl, 2-(ethylamino)eth-1-yl, 2-(isopropylamino)eth-1-yl, 2-(cyclopropylamino)eth-1-yl, 2-(cyclobutylamino)eth-1-yl, 2-(cyclopentylamino)eth-1-yl, 2-(cyclohexylamino)eth-1-yl, 2-(methoxycarbonylamino)eth-1-yl, 2-(ethoxycarbonylamino)eth-1-yl, 2-(tert-butyloxycarbonylamino)eth-1-yl, 2-(methylcarbonylamino)eth-1-yl, 2-(ethylcarbonylamino)eth-1-yl, 2-(n-propylcarbonylamino)eth-1-yl, 2-(isopropylcarbonylamino)eth-1-yl, 1-(methylamino)eth-1-yl, 1-(dieth-1-ylamino)eth-1-yl, 1-(diethylamino)eth-1-yl, 1-(ethylamino)eth-1-yl, 1-(isopropylamino)eth-1-yl, 1-(cyclopropylamino)eth-1-yl, 1-(cyclobutylamino)eth-1-yl, 1-(cyclopentylamino)eth-1-yl, 1-(cyclohexylamino)eth-l-yl, 1-(methoxycarbonylamino)eth-1-yl, 1-(ethoxycarbonylamino)eth-1-yl, 1-(tert-butyloxycarbonylamino)eth-1-yl, 1-(methylcarbonylamino)eth-1-yl, 1-(ethylcarbonylamino)eth-1-yl, 1-(n-propylcarbonylamino)eth-1-yl, 1-(isopropylcarbonylamino)eth-1-yl, or
R⁵ and R⁶ together with the carbon atom to which they are bonded form a fully saturated or partly saturated 3- to 10-membered monocyclic or bicyclic ring optionally interrupted by heteroatoms and optionally having further substitution, or
R⁵ and R⁶ together with the carbon atom to which they are bonded form a double bond optionally further substituted by R¹⁹ and R²⁰, according to formula (I') below 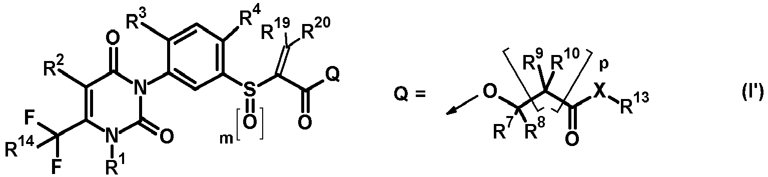
R¹⁴ is hydrogen, fluorine, chlorine, bromine, trifluoromethyl, methoxy, ethoxy, n-propyloxy, n-butyloxy,
m is 0, 1, 2,
R¹⁹ and R²⁰ are independently hydrogen, fluorine, chlorine, bromine, methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, n-hexyl, cyclopropyl, cyclobutyl, cyclopentyl, cyclohexyl, trifluoromethyl, difluoromethyl, pentafluoroethyl, ethenyl, 1-propenyl, 1-methylethenyl, 1-butenyl, phenyl or
R¹⁹ and R²⁰ together with the carbon atom to which they are bonded form a 3- to 10-membered monocyclic or bicyclic ring which is saturated or optionally interrupted by heteroatoms and optionally has further substitution
and Q is one of the moieties Q-1 to Q-300 specified in the in Claim 4.

6. Use of one or more compounds of the general formula (I) and/or salts thereof as defined in any of Claims 1 to 5 as herbicide and/or plant growth regulator, preferably in crops of useful plants and/or ornamentals.

7. Herbicidal and/or plant growth-regulating composition, **characterized in that** the composition contains one or more compounds of the formula (I) and/or salts thereof as defined in any of Claims 1 to 5, and one or more further substances selected from groups (i) and/or (ii):
(i) one or more further active agrochemical ingredients, preferably selected from the group consisting of insecticides, acaricides, nematicides, further herbicides, fungicides, safeners, fertilizers and/or further growth regulators,
(ii) one or more formulation auxiliaries customary in crop protection.

8. Method of controlling harmful plants or of regulating the growth of plants, **characterized in that** an effective amount of
- one or more compounds of the formula (I) and/or salts thereof as defined in any of Claims 1 to 5 or
- a composition according to Claim 7 is applied to the plants, plant seeds, the soil in or on which the plants grow, or the cultivation area.

## Revendications

1. Thiophényluraciles substitués de formule générale (I) ou leurs sels 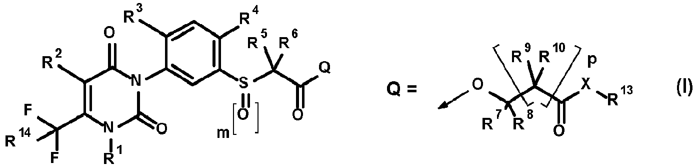 dans laquelle
R¹ représente (C₁-C₈) -alkyle, amino, bis-[(C₁-C₈)-alkyl]amino,
R² représente hydrogène, (C₁-C₈)-alkyle,
R³ représente hydrogène, halogène, (C₁-C₈)-alcoxy,
R⁴ représente halogène, cyano, NO₂, C (O) NH₂, C (S) NH₂, (C₁-C₈)-halogénoalkyle, (C₂-C₈)-alcynyle,
R⁵ et R⁶ représentent, indépendamment l'un de l'autre, hydrogène, halogène, (C₁-C₈)-alkyle, (C₃-C₈)-cycloalkyle, (C₃-C₈)-cycloalkyl-(C₁-C₈)-alkyle, (C₂-C₈)-alcényle, (C₂-C₈)-alcynyle, (C₁-C₁₀)-halogénoalkyle, (C₂-C₈)-halogénoalcényle, (C₂-C₈)-halogénoalcynyle, (C₃-C₁₀)-halogénocycloalkyle, (C₄-C₁₀)-cycloalcényle, (C₄-C₁₀)-halogénocycloalcényle, (C₁-C₈)-alcoxy, (C₁-C₈)-alcoxy-(C₁-C₈)-alkyle, (C₁-C₈)-alcoxy-(C₁-C₈)-halogénoalkyle, (C₁-C₈)-halogénoalcoxy-(C₁-C₈)-halogénoalkyle, (C₁-C₈)-halogénoalcoxy-(C₁-C₈)-alkyle, aryle, aryl-(C₁-C₈)-alkyle, hétéroaryle, hétéroaryl-(C₁-C₈)-alkyle, (C₄-C₁₀)-cycloalcényl-(C₁-C₈)-alkyle, hétérocyclyle, hétérocyclyl-(C₁-C₈)-alkyle, (C₁-C₈)-alkylthio-(C₁-C₈)-alkyle, (C₁-C₈)-halogénoalkylthio-(C₁-C₈)-alkyle, (C₁-C₈)-alkylcarbonyl-(C₁-C₈)-alkyle, C(O)OR¹⁷, C(O)NR¹⁵R¹⁶, C(O)R¹⁷, R¹⁷O(O)C-(C₁-C₈)-alkyle, R¹⁵R¹⁶N(O)C-(C₁-C₈)-alkyle, R¹⁵R¹⁶N-(C₁-C₈)-alkyle ou
R⁵ et R⁶ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁵ et R⁶ forment, avec l'atome de carbone auquel ils sont liés, une double liaison le cas échéant substituée davantage par R¹⁹ et R²⁰, selon la formule suivante (I') 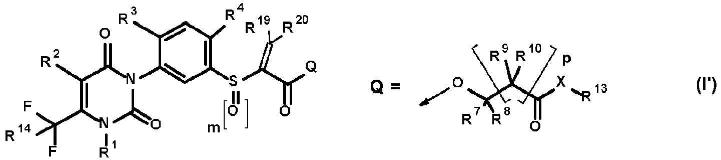
m représente 0, 1, 2,
p représente 0, 1, 2, 3, 4, 5,
X représente O (oxygène) ou les groupements N-R¹¹ ou N-O-R¹² et R¹¹ et R¹² dans les groupements N-R¹¹ et N-O-R¹² présentant, indépendamment l'un de l'autre, les significations selon les définitions suivantes,
R⁷, R⁸, R⁹ et R¹⁰ représentent, indépendamment les uns des autres, hydrogène, fluor, (C₁-C₈)-alkyle, (C₃-C₈)-cycloalkyle, (C₃-C₈)-cycloalkyl-(C₁-C₈)-alkyle, (C₂-C₈)-alcényle, (C₂-C₈)-alcynyle, (C₁-C₁₀)-halogénoalkyle, (C₂-C₈)-halogénoalcényle, (C₂-C₈)-halogénoalcynyle, (C₃-C₁₀)-halogénocycloalkyle, (C₄-C₁₀)-cycloalcényle, (C₄-C₁₀)-halogénocycloalcényle, (C₁-C₈)-alcoxy-(C₁-C₈)-halogénoalkyle, (C₁-C₈)-halogénoalcoxy-(C₁-C₈)-halogénoalkyle, aryle, aryl-(C₁-C₈)-alkyle, hétéroaryle, hétéroaryl-(C₁-C₈)-alkyle, (C₄-C₁₀)-cycloalcényl-(C₁-C₈)-alkyle, hétérocyclyle, hétérocyclyl-(C₁-C₈)-alkyle, R¹⁷O-(C₁-C₈)-alkyle, R¹⁸S-(C₁-C₈)-alkyle, (C₁-C₈)-alkylcarbonyl-(C₁-C₈)-alkyle, C(O)OR¹⁷, C(O)NR¹⁵R¹⁶, C(O)R¹⁷, R¹⁷O(O)C-(C₁-C₈)-alkyle, R¹⁵R¹⁶N(O)C-(C₁-C₈)-alkyle, R¹⁵R¹⁶N-(C₁-C₈)-alkyle ou
R⁵ et R⁷ forment, avec les atomes de carbone auxquels ils sont liés, un cycle monocyclique ou bicyclique de 3 à 8 chaînons, complètement saturé ou partiellement saturé, le cas échéant interrompu par des hétéroatomes et le cas échéant substitué davantage ou
R⁷ et R⁸ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹⁰ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹⁰ forment avec l'atome de carbone auquel ils sont liés, un groupe exométhylène ou
R⁷ et R⁹ forment, avec les atomes de carbone auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R¹¹ représente hydrogène, (C₁-C₈)-alkyle, (C₃-C₈)-cycloalkyle, cyano-(C₁-C₈)-alkyle, (C₃-C₈)-cycloalkyl-(C₁-C₈)-alkyle, (C₁-C₈)-alkylsulfonyle, arylsulfonyle, hétéroarylsulfonyle, (C₃-C₈)-cycloalkylsulfonyle, hétérocyclylsulfonyle, aryl-(C₁-C₈)-alkylsulfonyle, (C₁-C₈)-alkylcarbonyle, arylcarbonyle, hétéroarylcarbonyle, (C₃-C₈)-cycloalkylcarbonyle, hétérocyclylcarbonyle, (C₁-C₈)-alcoxycarbonyle, (C₁-C₈)-alcoxy, (C₂-C₈)-
R¹² alcényloxy, aryl-(C₁-C₈)-alcoxycarbonyle, (C₁-C₈)-halogénoalkylcarbonyle, (C₂-C₈)-alcényle, (C₂-C₈)-alcynyle, (C₁-C₈)-halogénoalkyle, halogéno-(C₂-C₈)-alcynyle, halogéno-(C₂-C₈)-alcényle, (C₁-C₈)-alcoxy-(C₁-C₈)-alkyle, amino, (C₁-C₈)-alkylamino, bis[(C₁-C₈)-alkyl]amino, (C₁-C₈)-alcoxy-(C₁-C₈)-alcoxy-(C₁-C₈)-alkyle, hétéroaryl-(C₁-C₈)-alkylsulfonyle, hétérocyclyl-(C₁-C₈)-alkylsulfonyle, (C₂-C₈)-alcényloxycarbonyle, (C₂-C₈)-alcynyloxycarbonyle, (C₁-C₈)-alkylaminocarbonyle, (C₃-C₈)-cycloalkylaminocarbonyle, bis-[(C₁-C₈)-alkyl]aminocarbonyle, représente hydrogène, (C₁-C₈)-alkyle, (C₃-C₈)-cycloalkyl-(C₁-C₈)-alkyle, (C₂-C₈)-alcényle, (C₂-C₈)-alcynyle, (C₁-C₈)-alcoxy-(C₁-C₈)-alkyle, aryle, aryl-(C₁-C₈)-alkyle, R¹⁷O(O)C-(C₁-C₈)-alkyle, R¹⁵R¹⁶N(O)C-(C₁-C₈)-alkyle,
R¹³ représente hydrogène, (C₁-C₈)-alkyle, (C₁-C₈)-halogénoalkyle, (C₃-C₈)-cycloalkyle, (C₃-C₈)-cycloalkyl-(C₁-C₈)-alkyle, (C₂-C₈)-alcényle, (C₂-C₈)-alcynyle, (C₁-C₈)-alcoxy-(C₁-C₈)-alkyle, (C₁-C₈)-halogénoalcoxy-(C₁-C₈)-alkyle, aryle, aryl-(C₁-C₈)-alkyle, hétéroaryle, hétéroaryl-(C₁-C₈)-alkyle, hétérocyclyle, hétérocyclyl-(C₁-C₈)-alkyle, (C₁-C₈)-alkylthio-(C₁-C₈)-alkyle, (C₁-C₈)-halogénoalkylthio-(C₁-C₈)-alkyle, (C₁-C₈)-alkylcarbonyl-(C₁-C₈)-alkyle, R¹⁷O(O)C-(C₁-C₈)-alkyle, R¹⁵R¹⁶N(O)C-(C₁-C₈)-alkyle, R¹⁵R¹⁶N-(C₁-C₈)-alkyle ou
R⁷ et R¹³ forment, avec les atomes auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹³ forment, avec les atomes auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R¹¹ et R¹³ forment, dans le cas où X représente N-R¹¹, avec l'atome d'azote auquel ils sont liés, un cycle monocyclique ou bicyclique de 4 à 10 chaînons, complètement saturé ou le cas échéant interrompu par des hétéroatomes et le cas échéant substitué davantage ou
R¹² et R¹³ forment, dans le cas où X représente N-O-R¹², avec l'atome d'azote auquel ils sont liés, un cycle monocyclique ou bicyclique de 4 à 10 chaînons, complètement saturé et le cas échéant substitué davantage,
R¹⁴ représente hydrogène, fluor, chlore, brome, (C₁-C₅)-halogénoalkyle, (C₁-C₈)-alcoxy,
R¹⁵ et R¹⁶ sont identiques ou différents et représentent, indépendamment l'un de l'autre, hydrogène, (C₁-C₈)-alkyle, (C₂-C₈)-alcényle, (C₂-C₈)-alcynyle, (C₁-C₈)-cyanoalkyle, (C₁-C₁₀)-halogénoalkyle, (C₂-C₈)-halogénoalcényle, (C₂-C₈)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₈)-alcoxy-(C₁-C₈)-alkyle, (C₁-C₈)-halogénoalcoxy-(C₁-C₈)-alkyle, (C₁-C₈)-alkylthio-(C₁-C₈)-alkyle, (C₁-C₈)-halogénoalkylthio-(C₁-C₈)-alkyle, (C₁-C₈)-alcoxy-(C₁-C₈)-halogénoalkyle, aryle, aryl-(C₁-C₈)-alkyle, hétéroaryle, hétéroaryl-(C₁-C₈)-alkyle, (C₃-C₈)-cycloalkyl-(C₁-C₈)-alkyle, (C₄-C₁₀)-cycloalcényl-(C₁-C₈)-alkyle, COR¹⁷, SO₂R¹⁸, (C₁-C₈)-alkyl-HNO₂S-, (C₃-C₈)-cycloalkyl-HNO₂S-, hétérocyclyle, (C₁-C₈)-alcoxycarbonyl-(C₁-C₈)-alkyle, (C₁-C₈)-alcoxycarbonyle, aryl-(C₁-C₈)-alcoxycarbonyl-(C₁-C₈)-alkyle, aryl-(C₁-C₈)-alcoxycarbonyle, hétéroaryl-(C₁-C₈)-alcoxycarbonyle, (C₂-C₈)-alcényloxycarbonyle, (C₂-C₈)-alcynyloxycarbonyle, hétérocyclyl-(C₁-C₈)-alkyle,
R¹⁷ représente hydrogène, (C₁-C₈)-alkyle, (C₂-C₈)-alcényle, (C₂-C₈)-alcynyle, (C₁-C₈)-cyanoalkyle, (C₁-C₁₀)-halogénoalkyle, (C₂-C₈)-halogénoalcényle, (C₂-C₈)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₈)-alcoxy-(C₁-C₈)-alkyle, (C₁-C₈)-alcoxy-(C₁-C₈)-halogénoalkyle, aryle, aryl-(C₁-C₈)-alkyle, hétéroaryle, hétéroaryl-(C₁-C₈)-alkyle, (C₃-C₈)-cycloalkyl-(C₁-C₈)-alkyle, (C₄-C₁₀)-cycloalcényl-(C₁-C₈)-alkyle, (C₁-C₈)-alcoxycarbonyl-(C₁-C₈)-alkyle, (C₂-C₈)-alcényloxycarbonyl-(C₁-C₈)-alkyle, aryl-(C₁-C₈)-alcoxycarbonyl-(C₁-C₈)-alkyle, hydroxycarbonyl-(C₁-C₈)-alkyle, hétérocyclyle, hétérocyclyl-(C₁-C₈)-alkyle,
R¹⁸ représente hydrogène, (C₁-C₈)-alkyle, (C₂-C₈)-alcényle, (C₂-C₈)-alcynyle, (C₁-C₈)-cyanoalkyle, (C₁-C₁₀)-halogénoalkyle, (C₂-C₈)-halogénoalcényle, (C₂-C₈)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₈)-alcoxy-(C₁-C₈)-alkyle, (C₁-C₈)-alcoxy-(C₁-C₈)-halogénoalkyle, aryle, aryl-(C₁-C₈)-alkyle, hétéroaryle, hétéroaryl-(C₁-C₈)-alkyle, hétérocyclyl-(C₁-C₈)-alkyle, (C₃-C₈)-cycloalkyl-(C₁-C₈)-alkyle, (C₄-C₁₀)-cycloalcényl-(C₁-C₈)-alkyle, NR¹⁵R¹⁶
et
R¹⁹ et R²⁰ représentent, indépendamment l'un de l'autre, hydrogène, halogène, (C₁-C₈)-alkyle, (C₃-C₈)-cycloalkyle, (C₂-C₈)-alcényle, (C₂-C₈)-alcynyle, (C₁-C₁₀)-halogénoalkyle, aryle ou
R¹⁹ et R²⁰ forment, avec l'atome de carbone auquel ils sont liés, un cycle monocyclique ou bicyclique de 3 à 10 chaînons, complètement saturé ou le cas échéant interrompu par des hétéroatomes et le cas échéant substitué davantage.

2. Composé de formule générale (I) selon la revendication 1 et/ou son sel, **caractérisé en ce que**
R¹ représente (C₁-C₇)-alkyle, amino, bis-[(C₁-C₇)-alkyl]amino,
R² représente hydrogène, (C₁-C₇)-alkyle,
R³ représente hydrogène, halogène, (C₁-C₇)-alcoxy,
R⁴ représente halogène, cyano, NO₂, C(O)NH₂, C(S)NH₂, (C₁-C₇)-halogénoalkyle, (C₂-C₇)-alcynyle,
R⁵ et R⁶ représentent, indépendamment l'un de l'autre, hydrogène, halogène, (C₁-C₇)-alkyle, (C₃-C₇)-cycloalkyle, (C₃-C₇)-cycloalkyl-(C₁-C₇)-alkyle, (C₂-C₇)-alcényle, (C₂-C₇)-alcynyle, (C₁-C₁₀)-halogénoalkyle, (C₂-C₇)-halogénoalcényle, (C₂-C₇)-halogénoalcynyle, (C₃-C₁₀)-halogénocycloalkyle, (C₄-C₁₀)-cycloalcényle, (C₄-C₁₀)-halogénocycloalcényle, (C₁-C₇)-alcoxy, (C₁-C₇)-alcoxy-(C₁-C₇)-alkyle, (C₁-C₇)-alcoxy-(C₁-C₇)-halogénoalkyle, (C₁-C₇)-halogénoalcoxy-(C₁-C₇)-halogénoalkyle, (C₁-C₇)-halogénoalcoxy-(C₁-C₇)-alkyle, aryle, aryl-(C₁-C₇)-alkyle, hétéroaryle, hétéroaryl-(C₁-C₇)-alkyle, (C₄-C₁₀)-cycloalcényl-(C₁-C₇)-alkyle, hétérocyclyle, hétérocyclyl-(C₁-C₇)-alkyle, (C₁-C₇)-alkylthio-(C₁-C₇)-alkyle, (C₁-C₇)-halogénoalkylthio-(C₁-C₇)-alkyle, (C₁-C₇)-alkylcarbonyl-(C₁-C₇)-alkyle, C(O)OR¹⁷, C(O)NR¹⁵R¹⁶, C(O)R¹⁷, R¹⁷O(O)C-(C₁-C₇)-alkyle, R¹⁵R¹⁶N(O)C-(C₁-C₇)-alkyle, R¹⁵R¹⁶N-(C₁-C₇)-alkyle ou
R⁵ et R⁶ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁵ et R⁶ forment, avec l'atome de carbone auquel ils sont liés, une double liaison le cas échéant substituée davantage par R¹⁹ et R²⁰, selon la formule suivante (I') 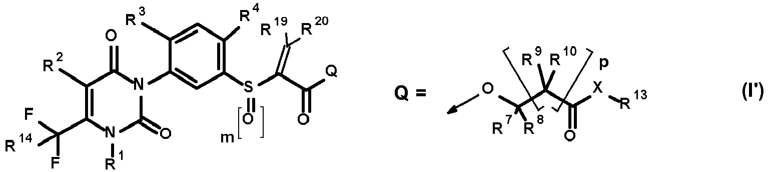
m représente 0, 1, 2,
p représente 0, 1, 2, 3,
X représente O (oxygène), N (azote) ou les groupements N-R¹¹ ou N-O-R¹² et R¹¹ et R¹² dans les groupements N-R¹¹ et N-O-R¹² présentant, indépendamment l'un de l'autre, les significations selon les définitions suivantes,
R⁷, R⁸, R⁹ et R¹⁰ représentent, indépendamment les uns des autres, hydrogène, fluor, (C₁-C₇)-alkyle, (C₃-C₇)-cycloalkyle, (C₃-C₇)-cycloalkyl-(C₁-C₇)-alkyle, (C₂-C₇)-alcényle, (C₂-C₇)-alcynyle, (C₁-C₁₀)-halogénoalkyle, (C₂-C₇)-halogénoalcényle, (C₂-C₇)-halogénoalcynyle, (C₃-C₁₀)-halogénocycloalkyle, (C₄-C₁₀)-cycloalcényle, (C₄-C₁₀)-halogénocycloalcényle, (C₁-C₇)-alcoxy-(C₁-C₇)-halogénoalkyle, (C₁-C₇)-halogénoalcoxy-(C₁-C₇)-halogénoalkyle, aryle, aryl-(C₁-C₇)-alkyle, hétéroaryle, hétéroaryl-(C₁-C₇)-alkyle, (C₄-C₁₀)-cycloalcényl-(C₁-C₇)-alkyle, hétérocyclyle, hétérocyclyl-(C₁-C₇)-alkyle, R¹⁷O-(C₁-C₇)-alkyle, R¹⁸S-(C₁-C₇)-alkyle, (C₁-C₇)-alkylcarbonyl-(C₁-C₇)-alkyle, C(O)OR¹⁷, C(O)NR¹⁵R¹⁶, C(O)R¹⁷, R¹⁷O(O)C-(C₁-C₇) -alkyle, R¹⁵R¹⁶N(O)C-(C₁-C₇)-alkyle, R¹⁵R¹⁶N-(C₁-C₇)-alkyle ou
R⁵ et R⁷ forment, avec les atomes de carbone auxquels ils sont liés, un cycle monocyclique ou bicyclique de 3 à 8 chaînons, complètement saturé ou partiellement saturé, le cas échéant interrompu par des hétéroatomes et le cas échéant substitué davantage ou
R⁷ et R⁸ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹⁰ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹⁰ forment avec l'atome de carbone auquel ils sont liés, un groupe exométhylène ou
R⁷ et R⁹ forment, avec les atomes de carbone auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R¹¹ représente hydrogène, (C₁-C₇)-alkyle, (C₃-C₇)-cycloalkyle, cyano-(C₁-C₇)-alkyle, (C₃-C₇)-cycloalkyl-(C₁-C₇)-alkyle, (C₁-C₇)-alkylsulfonyle, arylsulfonyle, hétéroarylsulfonyle, (C₃-C₇)-cycloalkylsulfonyle, hétérocyclylsulfonyle, aryl-(C₁-C₇)-alkylsulfonyle, (C₁-C₇)-alkylcarbonyle, arylcarbonyle, hétéroarylcarbonyle, (C₃-C₇)-cycloalkylcarbonyle, hétérocyclylcarbonyle, (C₁-C₇)-alcoxycarbonyle, (C₁-C₇)-alcoxy, (C₂-C₇)-alcényloxy, aryl-(C₁-C₇) -alcoxycarbonyle, (C₁-C₇)-halogénoalkylcarbonyle, (C₂-C₇)-alcényle, (C₂-C₇)-alcynyle, (C₁-C₇)-halogénoalkyle, halogéno-(C₂-C₇)-alcynyle, halogéno-(C₂-C₇)-alcényle, (C₁-C₇)-alcoxy-(C₁-C₇)-alkyle, amino, (C₁-C₇)-alkylamino, bis[(C₁-C₇)-alkyl]amino, (C₁-C₇)-alcoxy-(C₁-C₇)-alcoxy-(C₁-C₇)-alkyle, hétéroaryl-(C₁-C₇)-alkylsulfonyle, hétérocyclyl-(C₁-C₇)-alkylsulfonyle, (C₂-C₇)-alcényloxycarbonyle, (C₂-C₇)-alcynyloxycarbonyle, (C₁-C₇)-alkylaminocarbonyle, (C₃-C₇)-cycloalkylaminocarbonyle, bis-[(C₁-C₇)-alkyl]aminocarbonyle,
R¹² représente hydrogène, (C₁-C₇)-alkyle, (C₃-C₇)-cycloalkyl-(C₁-C₇)-alkyle, (C₂-C₇)-alcényle, (C₂-C₇)-alcynyle, (C₁-C₇)-alcoxy-(C₁-C₇)-alkyle, aryle, aryl-(C₁-C₇)-alkyle, R¹⁷O(O)C-(C₁-C₇)-alkyle, R¹⁵R¹⁶N(O)C-(C₁-C₇)-alkyle,
R¹³ représente hydrogène, (C₁-C₇)-alkyle, (C₁-C₇)-halogénoalkyle, (C₃-C₇)-cycloalkyle, (C₃-C₇)-cycloalkyl-(C₁-C₇)-alkyle, (C₂-C₇)-alcényle, (C₂-C₇)-alcynyle, (C₁-C₇)-alcoxy-(C-C₇)-alkyle, (C₁-C₇)-halogénoalcoxy-(C₁-C₇)-alkyle, aryle, aryl-(C₁-C₇)-alkyle, hétéroaryle, hétéroaryl-(C₁-C₇)-alkyle, hétérocyclyle, hétérocyclyl-(C₁-C₇)-alkyle, (C₁-C₇)-alkylthio-(C₁-C₇)-alkyle, (C₁-C₇)-halogénoalkylthio-(C₁-C₇)-alkyle, (C₁-C₇)-alkylcarbonyl-(C₁-C₇)-alkyle, R¹⁷O(O)C-(C₁-C₇)-alkyle, R¹⁵R¹⁶N(O)C-(C₁-C₇)-alkyle, R¹⁵R¹⁶N-(C₁-C₇)-alkyle ou
R⁷ et R¹³ forment, avec les atomes auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹³ forment, avec les atomes auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R¹¹ et R¹³ forment, dans le cas où X représente N-R¹¹, avec l'atome d'azote auquel ils sont liés, un cycle monocyclique ou bicyclique de 4 à 10 chaînons, complètement saturé ou le cas échéant interrompu par des hétéroatomes et le cas échéant substitué davantage ou
R¹² et R¹³ forment, dans le cas où X représente N-O-R¹², avec l'atome d'azote auquel ils sont liés, un cycle monocyclique ou bicyclique de 4 à 10 chaînons, complètement saturé et le cas échéant substitué davantage,
R¹⁴ représente hydrogène, fluor, chlore, brome, (C₁-C₅)-halogénoalkyle, (C₁-C₇)-alcoxy,
R¹⁵ et R¹⁶ sont identiques ou différents et représentent, indépendamment l'un de l'autre, hydrogène, (C₁-C₇)-alkyle, (C₂-C₇)-alcényle, (C₂-C₇)-alcynyle, (C₁-C₇)-cyanoalkyle, (C₁-C₁₀)-halogénoalkyle, (C₂-C₇)-halogénoalcényle, (C₂-C₇)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₇)-alcoxy-(C₁-C₇)-alkyle, (C₁-C₇)-halogénoalcoxy-(C₁-C₇)-alkyle, (C₁-C₇)-alkylthio-(C₁-C₇)-alkyle, (C₁-C₇)-halogénoalkylthio-(C₁-C₇)-alkyle, (C₁-C₇)-alcoxy-(C₁-C₇)-halogénoalkyle, aryle, aryl-(C₁-C₇)-alkyle,hétéroaryle, hétéroaryl-(C₁-C₇)-alkyle, (C₃-C₇)-cycloalkyl-(C₁-C₇)-alkyle, (C₄-C₁₀)-cycloalcényl-(C₁-C₇)-alkyle, COR¹⁷, SO₂R¹⁸, (C₁-C₇)-alkyl-HNO₂S-, (C₃-C₇)-cycloalkyl-HNO₂S-, hétérocyclyle, (C₁-C₇)-alcoxycarbonyl-(C₁-C₇)-alkyle, (C₁-C₇)-alcoxycarbonyle, aryl-(C₁-C₇)-alcoxycarbonyl-(C₁-C₇)-alkyle, aryl-(C₁-C₇)-alcoxycarbonyle, hétéroaryl-(C₁-C₇)-alcoxycarbonyle, (C₂-C₇)-alcényloxycarbonyle, (C₂-C₇)-alcynyloxycarbonyle, hétérocyclyl-(C₁-C₇)-alkyle,
R¹⁷ représente hydrogène, (C₁-C₇)-alkyle, (C₂-C₇)-alcényle, (C₂-C₇)-alcynyle, (C₁-C₇)-cyanoalkyle, (C₁-C₁₀)-halogénoalkyle, (C₂-C₇)-halogénoalcényle, (C₂-C₇)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₇)-alcoxy-(C₁-C₇)-alkyle, (C₁-C₇)-alcoxy-(C₁-C₇)-halogénoalkyle, aryle, aryl-(C₁-C₇)-alkyle,hétéroaryle, hétéroaryl-(C₁-C₇)-alkyle, (C₃-C₇)-cycloalkyl-(C₁-C₇)-alkyle, (C₄-C₁₀)-cycloalcényl-(C₁-C₇)-alkyle, (C₁-C₇)-alcoxycarbonyl-(C₁-C₇)-alkyle, (C₂-C₇)-alcényloxycarbonyl-(C₁-C₇)-alkyle, aryl-(C₁-C₇)-alcoxycarbonyl-(C₁-C₇)-alkyle, hydroxycarbonyl-(C₁-C₇)-alkyle, hétérocyclyle, hétérocyclyl-(C₁-C₇)-alkyle
R¹⁸ représente hydrogène, (C₁-C₇)-alkyle, (C₂-C₇)-alcényle, (C₂-C₇)-alcynyle, (C₁-C₇)-cyanoalkyle, (C₁-C₁₀)-halogénoalkyle, (C₂-C₇)-halogénoalcényle, (C₂-C₇)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₇)-alcoxy-(C₁-C₇)-alkyle, (C₁-C₇)-alcoxy-(C₁-C₇)-halogénoalkyle, aryle, aryl-(C₁-C₇)-alkyle,hétéroaryle, hétéroaryl-(C₁-C₇)-alkyle, hétérocyclyl-(C₁-C₇)-alkyle, (C₃-C₇)-cycloalkyl-(C₁-C₇)-alkyle, (C₄-C₁₀)-cycloalcényl-(C₁-C₇)-alkyle, NR¹⁵R¹⁶
et
R¹⁹ et R²⁰ représentent, indépendamment l'un de l'autre, hydrogène, halogène, (C₁-C₇)-alkyle, (C₃-C₇)-cycloalkyle, (C₂-C₇)-alcényle, (C₂-C₇)-alcynyle, (C₁-C₇)-halogénoalkyle, aryle ou
R¹⁹ et R²⁰ forment, avec l'atome de carbone auquel ils sont liés, un cycle monocyclique ou bicyclique de 3 à 10 chaînons, complètement saturé ou le cas échéant interrompu par des hétéroatomes et le cas échéant substitué davantage.

3. Composés de formule générale (I) selon la revendication 1 et/ou leur sel, **caractérisé en ce que**
R¹ représente (C₁-C₆)-alkyle, amino, bis- [(C₁-C₆)-alkyl]amino,
R² représente hydrogène, (C₁-C₆)-alkyle,
R³ représente hydrogène, halogène, (C₁-C₆)-alcoxy,
R⁴ représente halogène, cyano, NO₂, C(O)NH₂, C(S)NH₂, (C₁-C₆)-halogénoalkyle, (C₂-C₆)-alcynyle,
R⁵ et R⁶ représentent, indépendamment l'un de l'autre, hydrogène, halogène, (C₁-C₆)-alkyle, (C₃-C₆)-cycloalkyle, (C₃-C₆)-cycloalkyl-(C₁-C₆)-alkyle, (C₂-C₆)-alcényle, (C₂-C₆)-alcynyle, (C₁-C₁₀)-halogénoalkyle, (C₂-C₆)-halogénoalcényle, (C₂-C₆)-halogénoalcynyle, (C₃-C₁₀)-halogénocycloalkyle, (C₄-C₁₀)-cycloalcényle, (C₄-C₁₀)-halogénocycloalcényle, (C₁-C₆)-alcoxy, (C₁-C₆)-alcoxy-(C₁-C₆)-alkyle, (C₁-C₆)-alcoxy-(C₁-C₆)-halogénoalkyle, (C₁-C₆)-halogénoalcoxy-(C₁-C₆)-halogénoalkyle, (C₁-C₆)-halogénoalcoxy-(C₁-C₆)-alkyle, aryle, aryl-(C₁-C₆)-alkyle, hétéroaryle, hétéroaryl-(C₁-C₆)-alkyle, (C₄-C₁₀)-cycloalcényl-(C₁-C₆)-alkyle, hétérocyclyle, hétérocyclyl-(C₁-C₆)-alkyle, (C₁-C₆)-alkylthio-(C₁-C₆)-alkyle, (C₁-C₆)-halogénoalkylthio-(C₁-C₆)-alkyle, (C₁-C₆)-alkylcarbonyl-(C₁-C₆)-alkyle, C(O)OR¹⁷, C(O)NR¹⁵R¹⁶, C(O)R¹⁷, R¹⁷O(O)C-(C₁-C₆)-alkyle, R¹⁵R¹⁶N(O)C-(C₁-C₆)-alkyle, R¹⁵R¹⁶N-(C₁-C₆)-alkyle ou
R⁵ et R⁶ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁵ et R⁶ forment, avec l'atome de carbone auquel ils sont liés, une double liaison le cas échéant substituée davantage par R¹⁹ et R²⁰, selon la formule suivante (I') 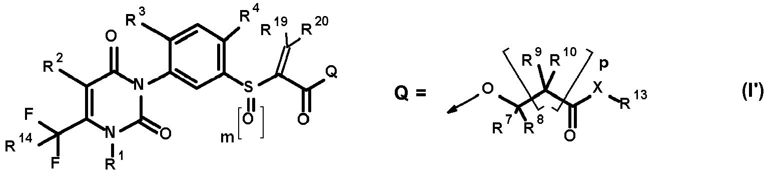
m représente 0, 1, 2,
p représente 0, 1, 2, 3,
X représente O (oxygène), N (azote) ou les groupements N-R¹¹ ou N-O-R¹² et R¹¹ et R¹² dans les groupements N-R¹¹ et N-O-R¹² présentant, indépendamment l'un de l'autre, les significations selon les définitions suivantes,
R⁷, R⁸, R⁹ et R¹⁰ représentent, indépendamment les uns des autres, hydrogène, fluor, (C₁-C₆)-alkyle, (C₃-C₆)-cycloalkyle, (C₃-C₆)-cycloalkyl-(C₁-C₆)-alkyle, (C₂-C₆)-alcényle, (C₂-C₆)-alcynyle, (C₁-C₁₀)-halogénoalkyle, (C₂-C₆)-halogénoalcényle, (C₂-C₆)-halogénoalcynyle, (C₃-C₁₀)-halogénocycloalkyle, (C₄-C₁₀)-cycloalcényle, (C₄-C₁₀)-halogénocycloalcényle, (C₁-C₆)-alcoxy-(C₁-C₆)-halogénoalkyle, (C₁-C₆)-halogénoalcoxy-(C₁-C₆)-halogénoalkyle, aryle, aryl-(C₁-C₆)-alkyle, hétéroaryle, hétéroaryl-(C₁-C₆)-alkyle, (C₄-C₁₀)-cycloalcényl-(C₁-C₆)-alkyle, hétérocyclyle, hétérocyclyl-(C₁-C₆)-alkyle, R¹⁷O-(C₁-C₆)-alkyle, R¹⁸S-(C₁-C₆)-alkyle, (C₁-C₆)-alkylcarbonyl-(C₁-C₆)-alkyle, C(O)OR¹⁷, C(O)NR¹⁵R¹⁶, C(O)R¹⁷, R¹⁷O(O)C-(C₁-C₆)-alkyle, R¹⁵R¹⁶N(O)C-(C₁-C₆)-alkyle, R¹⁵R¹⁶N-(C₁-C₆)-alkyle ou
R⁵ et R⁷ forment, avec les atomes de carbone auxquels ils sont liés, un cycle monocyclique ou bicyclique de 3 à 8 chaînons, complètement saturé ou partiellement saturé, le cas échéant interrompu par des hétéroatomes et le cas échéant substitué davantage ou
R⁷ et R⁸ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹⁰ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹⁰ forment avec l'atome de carbone auquel ils sont liés, un groupe exométhylène ou
R⁷ et R⁹ forment, avec les atomes de carbone auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R¹¹ représente hydrogène, (C₁-C₆)-alkyle, (C₃-C₆)-cycloalkyle, cyano-(C₁-C₆)-alkyle, (C₃-C₆)-cycloalkyl-(C₁-C₆)-alkyle, (C₁-C₆)-alkylsulfonyle, arylsulfonyle, hétéroarylsulfonyle, (C₃-C₆)-cycloalkylsulfonyle, hétérocyclylsulfonyle, aryl-(C₁-C₆)-alkylsulfonyle, (C₁-C₆)-alkylcarbonyle, arylcarbonyle, hétéroarylcarbonyle, (C₃-C₆)-cycloalkylcarbonyle, hétérocyclylcarbonyle, (C₁-C₆)-alcoxycarbonyle, (C₁-C₆)-alcoxy, (C₂-C₆)-alcényloxy, aryl-(C₁-C₆)-alcoxycarbonyle, (C₁-C₆)-halogénoalkylcarbonyle, (C₂-C₆)-alcényle, (C₂-C₆)-alcynyle, (C₁-C₆)-halogénoalkyle, halogéno-(C₂-C₆)-alcynyle, halogéno-(C₂-C₆)-alcényle, (C₁-C₆)-alcoxy-(C₁-C₆)-alkyle, amino, (C₁-C₆)-alkylamino, bis[(C₁-C₆)-alkyl]amino, (C₁-C₆)-alcoxy-(C₁-C₆)-alcoxy-(C₁-C₆)-alkyle, hétéroaryl-(C₁-C₆)-alkylsulfonyle, hétérocyclyl- (C₁-C₆)-alkylsulfonyle, (C₂-C₆)-alcényloxycarbonyle, (C₂-C₆)-alcynyloxycarbonyle, (C₁-C₆)-alkylaminocarbonyle, (C₃-C₆)-cycloalkylaminocarbonyle, bis-[(C₁-C₆)-alkyl]aminocarbonyle,
R¹² représente hydrogène, (C₁-C₆)-alkyle,(C₃-C₆)-cycloalkyl- (C₁-C₆)-alkyle, (C₂-C₆)-alcényle, (C₂-C₆)-alcynyle, (C₁-C₆)-alcoxy- (C₁-C₆)-alkyle, aryle, aryl- (C₁-C₆)-alkyle, R¹⁷O(O)C-(C₁-C₆)-alkyle, R¹⁵R¹⁶N(O)C-(C₁-C₆)-alkyle,
R¹³ représente hydrogène, (C₁-C₆)-alkyle, (C₁-C₆)-halogénoalkyle, (C₃-C₆)-cycloalkyle, (C₃-C₆)-cycloalkyl- (C₁-C₆)-alkyle, (C₂-C₆)-alcényle, (C₂-C₆)-alcynyle, (C₁-C₆)-alcoxy-(C-C₆)-alkyle, (C₁-C₆)-halogénoalcoxy-(C₁-C₆)-alkyle, aryle, aryl- (C₁-C₆)-alkyle, hétéroaryle, hétéroaryl-(C₁-C₆)-alkyle, hétérocyclyle, hétérocyclyl- (C₁-C₆)-alkyle, (C₁-C₆)-alkylthio-(C₁-C₆)-alkyle, (C₁-C₆)-halogénoalkylthio-(C₁-C₆)-alkyle, (C₁-C₆)-alkylcarbonyl- (C₁-C₆)-alkyle, R¹⁷O(O)C-(C₁-C₆)-alkyle, R¹⁵R¹⁶N(O)C-(C₁-C₆)-alkyle, R¹⁵R¹⁶N-(C₁-C₆)-alkyle ou
R⁷ et R¹³ forment, avec les atomes auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁹ et R¹³ forment, avec les atomes auxquels ils sont liés, un cycle monocyclique ou bicyclique de 3 à 10 chaînons, complètement saturé ou partiellement saturé, le cas échéant interrompu par des hétéroatomes et le cas échéant substitué davantage ou
R¹¹ et R¹³ forment, dans le cas où X représente N-R¹¹, avec l'atome d'azote auquel ils sont liés, un cycle monocyclique ou bicyclique de 4 à 10 chaînons, complètement saturé ou le cas échéant interrompu par des hétéroatomes et le cas échéant substitué davantage ou
R¹² et R¹³ forment, dans le cas où X représente N-O-R¹², avec l'atome d'azote auquel ils sont liés, un cycle monocyclique ou bicyclique de 4 à 10 chaînons, complètement saturé et le cas échéant substitué davantage,
R¹⁴ représente hydrogène, fluor, chlore, brome, (C₁-C₅)-halogénoalkyle, (C₁-C₆)-alcoxy,
R¹⁵ et R¹⁶ sont identiques ou différents et représentent, indépendamment l'un de l'autre, hydrogène, (C₁-C₆)-alkyle, (C₂-C₆)-alcényle, (C₂-C₆)-alcynyle, (C₁-C₆)-cyanoalkyle, (C₁-C₁₀)-halogénoalkyle, (C₂-C₆)-halogénoalcényle, (C₂-C₆)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₆)-alcoxy- (C₁-C₆)-alkyle, (C₁-C₆)-halogénoalcoxy-(C₁-C₆)-alkyle, (C₁-C₆)-alkylthio-(C₁-C₆)-alkyle, (C₁-C₆)-halogénoalkylthio-(C₁-C₆)-alkyle, (C₁-C₆)-alcoxy- (C₁-C₆)-halogénoalkyle, aryle, aryl-(C₁-C₆)-alkyle, hétéroaryle, hétéroaryl-(C₁-C₆)-alkyle, (C₃-C₆)-cycloalkyl-(C₁-C₆)-alkyle, (C₄-C₁₀)-cycloalcényl-(C₁-C₆)-alkyle, COR¹⁷, SO₂R¹⁸, (C₁-C₆)-alkyl-HNO₂S-, (C₃-C₆)-cycloalkyl-HNO₂S-, hétérocyclyle, (C₁-C₆)-alcoxycarbonyl-(C₁-C₆)-alkyle, (C₁-C₆)-alcoxycarbonyle, aryl- (C₁-C₆)-alcoxycarbonyl-(C₁-C₆)-alkyle, aryl- (C₁-C₆)-alcoxycarbonyle, hétéroaryl-(C₁-C₆)-alcoxycarbonyle, (C₂-C₆)-alcényloxycarbonyle, (C₂-C₆)-alcynyloxycarbonyle, hétérocyclyl- (C₁-C₆)-alkyle,
R¹⁷ représente hydrogène, (C₁-C₆)-alkyle, (C₂-C₆)-alcényle, (C₂-C₆)-alcynyle, (C₁-C₆)-cyanoalkyle, (C₁-C₁₀)-halogénoalkyle, (C₂-C₆)-halogénoalcényle, (C₂-C₆)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₆)-alcoxy- (C₁-C₆)-alkyle, (C₁-C₆)-alcoxy- (C₁-C₆)-halogénoalkyle, aryle, aryl-(C₁-C₆)-alkyle, hétéroaryle, hétéroaryl-(C₁-C₆)-alkyle, (C₃-C₆)-cycloalkyl-(C₁-C₆)-alkyle, (C₄-C₁₀)-cycloalcényl- (C₁-C₆)-alkyle, (C₁-C₆)-alcoxycarbonyl-(C₁-C₆)-alkyle, (C₂-C₆)-alcényloxycarbonyl-(C₁-C₆)-alkyle, aryl- (C₁-C₆)-alcoxycarbonyl-(C₁-C₆)-alkyle, hydroxycarbonyl-(C₁-C₆)-alkyle, hétérocyclyle, hétérocyclyl- (C₁-C₆)-alkyle,
R¹⁸ représente hydrogène, (C₁-C₆)-alkyle, (C₂-C₆)-alcényle, (C₂-C₆)-alcynyle, (C₁-C₆)-cyanoalkyle, (C₁-C₁₀)-halogénoalkyle, (C₂-C₆)-halogénoalcényle, (C₂-C₆)-halogénoalcynyle, (C₃-C₁₀)-cycloalkyle, (C₃-C₁₀)-halogénocycloalkyle, (C₄-C₁₀)-cycloalcényle, (C₄-C₁₀)-halogénocycloalcényle, (C₁-C₆)-alcoxy- (C₁-C₆)-alkyle, (C₁-C₆)-alcoxy- (C₁-C₆)-halogénoalkyle, aryle, aryl-(C₁-C₆)-alkyle, hétéroaryle, hétéroaryl-(C₁-C₆)-alkyle, hétérocyclyl- (C₁-C₆)-alkyle, (C₃-C₆)-cycloalkyl-(C₁-C₆)-alkyle, (C₄-C₁₀)-cycloalcényl-(C₁-C₆)-alkyle, NR¹⁵R¹⁶
et
R¹⁹ et R²⁰ représentent, indépendamment l'un de l'autre, hydrogène, halogène, (C₁-C₆)-alkyle, (C₃-C₆)-cycloalkyle, (C₂-C₆)-alcényle, (C₂-C₆)-alcynyle, (C₁-C₆)-halogénoalkyle, aryle ou
R¹⁹ et R²⁰ forment, avec l'atome de carbone auquel ils sont liés, un cycle monocyclique ou bicyclique de 3 à 10 chaînons, complètement saturé ou le cas échéant interrompu par des hétéroatomes et le cas échéant substitué davantage.

4. Composés de formule générale (I) selon la revendication 1 et/ou leur sel, **caractérisé en ce que**
R¹ représente méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1, 1,2-triméthylpropyle, 1,2,2-triméthylpropyle, 1-éthyl-1-méthylpropyle, 1-éthyl-2-méthylpropyle, amino, diméthylamino, diéthylamino, méthyl(éthyl)amino, méthyl(n-propyl)amino,
R² représente hydrogène, méthyle, éthyle, n-propyle, iso-propyle,
R³ représente hydrogène, fluor, chlore, brome, méthoxy, éthoxy,
R⁴ représente halogène, cyano, NO₂, C(O)NH₂, C(S)NH₂, difluorométhyle, trifluorométhyle, éthynyle, propyn-1-yle, 1-butyn-1-yle, pentyn-1-yle, hexyn-1-yle,
R⁵ et R⁶ représentent, indépendamment l'un de l'autre, hydrogène, fluor, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, cyclopropyle, cyclobutyle, cyclopentyle, cyclohexyle, spiro[2.2]pent-1-yle, spiro[2.3]hex-1-yle, spiro[2.3]hex-4-yle, 3-spiro[2.3]hex-5-yle, bicyclo[1.1.0]butan-1-yle, bicyclo[1.1.0]butan-2-yle, bicyclo[2.1.0]pentan-1-yle, bicyclo[1.1.1]pentan-1-yle, bicyclo[2.1.0]pentan-2-yle, bicyclo[2.1.0]pentan-5-yle, bicyclo[2.1.1]hexyle, 1-méthylcyclopropyle, 2-méthylcyclopropyle, 2,2-diméthylcyclopropyle, 2,3-diméthylcyclopropyle, 1,1'-bi(cyclopropyl)-1-yle, 1,1'-bi(cyclopropyl)-2-yle, 2'-méthyl-1,1'-bi(cyclopropyl)-2-yle, 1-cyanocyclopropyle, 2-cyanocyclopropyle, 1-méthylcyclobutyle, 2-méthylcyclobutyle, 3-méthylcyclobutyle, 3,3-diméthylcyclobut-1-yle, 1-cyanocyclobutyle, 2-cyanocyclobutyle, 3-cyanocyclobutyle, 3,3-difluorocyclobut-1-yle, 3-fluorocyclobut-1-yle, 2,2-difluorocycloprop-1-yle, 1-fluorocycloprop-1-yle, 2-fluorocycloprop-1-yle, 1-allylcyclopropyle, 1-vinylcyclobutyle, 1-vinylcyclopropyle, 1-éthylcyclopropyle, 1-méthylcyclohexyle, 2-méthylcyclohexyle, 3-méthylcyclohexyle, 1-méthoxycyclohexyle, 2-méthoxycyclohexyle, 3-méthoxycyclohexyle, 2-fluorocycloprop-1-yle, 4-fluorocyclohexyle, 4,4-difluorocyclohexyle, cyclopropylméthyle, cyclobutylméthyle, cyclopentylméthyle, cyclohexylméthyle, éthényle, 1-propényle, 2-propényle, 1-méthyléthényle, 1-butényle, 2-butényle, 3-butényle, 1-méthyl-1-propényle, 2-méthyl-1-propényle, 1-méthyl-2-propényle, 2-méthyl-2-propényle, 1-pentényle, 2-pentényle, 3-pentényle, 4-pentényle, 1-méthyl-1-butényle, 2-méthyl-1-butényle, 3-méthyl-1-butényle, 1-méthyl-2-butényle, 2-méthyl-2-butényle, 3-méthyl-2-butényle, 1-méthyl-3-butényle, 2-méthyl-3-butényle, 3-méthyl-3-butényle, 1,1-diméthyl-2-propényle, 1,2-diméthyl-1-propényle, 1,2-diméthyl-2-propényle, 1-éthyl-1-propényle, 1-éthyl-2-propényle, 1-hexényle, 2-hexényle, 3-hexényle, 4-hexényle, 5-hexényle, 1-méthyl-1-pentényle, 2-méthyl-1-pentényle, 3-méthyl-1-pentényle, 4-méthyl-1-pentényle, 1-méthyl-2-pentényle, 2-méthyl-2-pentényle, 3-méthyl-2-pentényle, 4-méthyl-2-pentényle, 1-méthyl-3-pentényle, 2-méthyl-3-pentényle, 3-méthyl-3-pentényle, 4-méthyl-3-pentényle, 1-méthyl-4-pentényle, 2-méthyl-4-pentényle, 3-méthyl-4-pentényle, 4-méthyl-4-pentényle, 1,1-diméthyl-2-butényle, 1,1-diméthyl-3-butényle, 1,2-diméthyl-1-butényle, 1,2-diméthyl-2-butényle, 1,2-diméthyl-3-butényle, 1,3-diméthyl-1-butényle, 1,3-diméthyl-2-butényle, 1,3-diméthyl-3-butényle, 2,2-diméthyl-3-butényle, 2,3-diméthyl-1-butényle, 2,3-diméthyl-2-butényle, 2,3-diméthyl-3-butényle, 3,3-diméthyl-1-butényle, 3,3-diméthyl-2-butényle, 1-éthyl-1-butényle, 1-éthyl-2-butényle, 1-éthyl-3-butényle, 2-éthyl-1-butényle, 2-éthyl-2-butényle, 2-éthyl-3-butényle, 1,1,2-triméthyl-2-propényle, 1-éthyl-1-méthyl-2-propényle, 1-éthyl-2-méthyl-1-propényle, 1-éthyl-2-méthyl-2-propényle, éthynyle, 1-propynyle, 2-propynyle, 1-butynyle, 2-butynyle, 3-butynyle, 1-méthyl-2-propynyle, 1-pentynyle, 2-pentynyle, 3-pentynyle, 4-pentynyle, 1-méthyl-2-butynyle, 1-méthyl-3-butynyle, 2-méthyl-3-butynyle, 3-méthyl-1-butynyle, 1,1-diméthyl-2-propynyle, 1-éthyl-2-propynyle, 1-hexynyle, 2-hexynyle, 3-hexynyle, 4-hexynyle, 5-hexynyle, 1-méthyl-2-pentynyle, 1-méthyl-3-pentynyle, 1-méthyl-4-pentynyle, 2-méthyl-3-pentynyle, 2-méthyl-4-pentynyle, 3-méthyl-1-pentynyle, 3-méthyl-4-pentynyle, 4-méthyl-1-pentynyle, 4-méthyl-2-pentynyle, 1,1-diméthyl-2-butynyle, 1,1-diméthyl-3-butynyle, 1,2-diméthyl-3-butynyle, 2,2-diméthyl-3-butynyle, 3,3-diméthyl-1-butynyle, 1-éthyl-2-butynyle, 1-éthyl-3-butynyle, 2-éthyl-3-butynyle, 1-éthyl-1-méthyl-2-propynyle, trifluorométhyle, pentafluoroéthyle, 1,1,2,2-tétrafluoroéthyle, heptafluoropropyle, nonafluorobutyle, chlorodifluorométhyle, bromodifluorométhyle, dichlorofluorométhyle, iododifluorométhyle, bromofluorométhyle, 1-fluoroéthyle, 2-fluoroéthyle, fluorométhyle, difluorométhyle, 2,2-difluoroéthyle, 2,2,2-trifluoroéthyle, difluoro-tert-butyle, chlorométhyle, bromométhyle, méthoxy, éthoxy, n-propyloxy, iso-propyloxy, n-butyloxy, tert-butyloxy, méthoxyméthyle, éthoxyméthyle, n-propyloxyméthyle, iso-propyloxyméthyle, méthoxyéthyle, éthoxyéthyle, n-propyloxyéthyle, iso-propyloxyéthyle, méthoxy-n-propyle, méthoxydifluorométhyle, éthoxydifluorométhyle, n-propyloxydifluorométhyle, n-butyloxydifluorométhyle, trifluorométhoxyméthyle, trifluorométhoxyéthyle, trifluorométhoxy-n-propyle, phényle, 2-fluoro-phényle, 3-fluoro-phényle, 4-fluoro-phényle, 2,4-difluoro-phényle, 2,5-difluoro-phényle, 2,6-difluoro-phényle, 2,3-difluoro-phényle, 3,4-difluoro-phényle, 3,5-difluoro-phényle, 2,4,5-trifluoro-phényle, 3,4,5-trifluoro-phényle, 2-chloro-phényle, 3-chloro-phényle, 4-chloro-phényle, 2,4-dichloro-phényle, 2,5-dichloro-phényle, 2,6-dichloro-phényle, 2,3-dichloro-phényle, 3,4-dichloro-phényle, 3,5-dichloro-phényle, 2,4,5-trichloro-phényle, 3,4,5-trichloro-phényle, 2,4,6-trichloro-phényle, 2-bromo-phényle, 3-bromo-phényle, 4-bromo-phényle, 2-iodo-phényle, 3-iodo-phényle, 4-iodo-phényle, 2-bromo-4-fluoro-phényle, 2-bromo-4-chloro-phényle, 3-bromo-4-fluoro-phényle, 3-bromo-4-chlorophényle, 3-bromo-5-fluoro-phényle, 3-bromo-5-chloro-phényle, 2-fluoro-4-bromo-phényle, 2-chloro-4-bromo-phényle, 3-fluoro-4-bromo-phényle, 3-chloro-4-bromo-phényle, 2-chloro-4-fluoro-phényle, 3-chloro-4-fluoro-phényle, 2-fluoro-3-chloro-phényle, 2-fluoro-4-chloro-phényle, 2-fluoro-5-chloro-phényle, 3-fluoro-4-chloro-phényle, 3-fluoro-5-chloro-phényle, 2-fluoro-6-chloro-phényle, 2-méthyl-phényle, 3-méthyl-phényle, 4-méthyl-phényle, 2,4-diméthyl-phényle, 2,5-diméthyl-phényle, 2,6-diméthyl-phényle, 2,3-diméthyl-phényle, 3,4-diméthyl-phényle, 3,5-diméthyl-phényle, 2,4,5-triméthyl-phényle, 3,4,5-triméthyl-phényle, 2,4,6-triméthyl-phényle, 2-méthoxy-phényle, 3-méthoxy-phényle, 4-méthoxy-phényle, 2,4-diméthoxy-phényle, 2,5-diméthoxy-phényle, 2,6-diméthoxy-phényle, 2,3-diméthoxy-phényle, 3,4-diméthoxy-phényle, 3,5-diméthoxy-phényle, 2,4,5-triméthoxy-phényle, 3,4,5-triméthoxy-phényle, 2,4,6-triméthoxy-phényle, 2-trifluorométhoxy-phényle, 3-trifluorométhoxy-phényle, 4-trifluorométhoxy-phényle, 2-difluorométhoxy-phényle, 3-difluorométhoxy-phényle, 4-difluorométhoxy-phényle, 2-trifluorométhyl-phényle, 3-trifluorométhyl-phényle, 4-trifluorométhyl-phényle, 2-difluorométhyl-phényle, 3-difluorométhyl-phényle, 4-difluorométhyl-phényle, 3,5-bis(trifluorométhyl)-phényle, 3-trifluorométhyl-5-fluoro-phényle, 3-trifluorométhyl-5-chloro-phényle, 3-méthyl-5-fluoro-phényle, 3-méthyl-5-chlorophényle, 3-méthoxy-5-fluoro-phényle, 3-méthoxy-5-chloro-phényle, 3-trifluorométhoxy-5-chloro-phényle, 2-éthoxy-phényle, 3-éthoxy-phényle, 4-éthoxy-phényle, 2-méthylthio-phényle, 3-méthylthio-phényle, 4-méthylthio-phényle, 2-trifluorométhylthio-phényle, 3-trifluorométhylthio-phényle, 4-trifluorométhylthio-phényle, 2-éthyl-phényle, 3-éthyl-phényle, 4-éthyl-phényle, 2-méthoxycarbonyl-phényle, 3-méthoxycarbonyl-phényle, 4-méthoxycarbonyl-phényle, 2-éthoxycarbonyl-phényle, 3-éthoxycarbonyl-phényle, 4-éthoxycarbonyl-phényle, pyridin-2-yle, pyridin-3-yle, pyridin-4-yle, pyrazin-2-yle, pyridazin-3-yle, pyridazin-4-yle, pyrimidine-2-yle, pyrimidine-5-yle, pyrimidine-4-yle, pyridazin-3-ylméthyle, pyridazin-4-ylméthyle, pyrimidine-2-ylméthyle, pyrimidine-5-ylméthyle, pyrimidine-4-ylméthyle, pyrazin-2-ylméthyle, 3-chloropyrazin-2-yle, 3-bromopyrazin-2-yle, 3-méthoxy-pyrazin-2-yle, 3-éthoxy-pyrazin-2-yle, 3-trifluorométhylpyrazin-2-yle, 3-cyanopyrazin-2-yle, napht-2-yle, napht-1-yle, quinoléin-4-yle, quinoléin-6-yle, quinoléin-8-yle, quinoléin-2-yle, quinoxalin-2-yle, 2-naphtylméthyle, 1-naphtylméthyle, quinoléin-4-ylméthyle, quinoléin-6-ylméthyle, quinoléin-8-ylméthyle, quinoléin-2-ylméthyle, quinoxalin-2-ylméthyle, pyrazin-2-ylméthyle, 4-chloropyridin-2-yle, 3-chloropyridin-4-yle, 2-chloropyridin-3-yle, 2-chloropyridin-4-yle, 2-chloropyridin-5-yle, 2,6-dichloropyridin-4-yle, 3-chloropyridin-5-yle, 3,5-dichloropyridin-2-yle, 3-chloro-5-trifluorométhylpyridin-2-yle, (4-chloropyridin-2-yl)méthyle, (3-chloropyridin-4-yl)méthyle, (2-chloropyridin-3-yl)méthyle, (2-chloropyridin-4-yl)méthyle, (2-chloropyridin-5-yl)méthyle, (2,6-dichloropyridin-4-yl)méthyle, (3-chloropyridin-5-yl)méthyle, (3,5-dichloropyridin-2-yl)méthyle, thiophén-2-yle, thiophén-3-yle, 5-méthylthiophén-2-yle, 5-éthylthiophén-2-yle, 5-chlorothiophén-2-yle, 5-bromothiophén-2-yle, 4-méthylthiophén-2-yle, 3-méthylthiophén-2-yle, 5-fluorothiophén-3-yle, 3,5-diméthylthiophén-2-yle, 3-éthylthiophén-2-yle, 4,5-diméthylthiophén-2-yle, 3,4-diméthylthiophén-2-yle, 4-chlorothiophén-2-yle, furann-2-yle, 5-méthylfurann-2-yle, 5-éthylfurann-2-yle, 5-méthoxycarbonylfurann-2-yle, 5-chlorofurann-2-yle, 5-bromofurann-2-yle, thiophan-2-yle, thiophan-3-yle, sulfolan-2-yle, sulfolan-3-yle, tétrahydrothiopyran-4-yle, tétrahydropyran-4-yle, tétrahydrofurann-2-yle, tétrahydrofurann-3-yle, 1-(4-méthylphényl)éthyle, 1-(3-méthylphényl)éthyle, 1-(2-méthylphényl)éthyle, 1-(4-chlorophényl)éthyle, 1-(3-chlorophényl)éthyle, 1-(2-chlorophényl)éthyle, benzyle, (4-fluorophényl)méthyle, (3-fluorophényl)méthyle, (2-fluorophényl)méthyle, (2,4-difluorophényl)méthyle, (3,5-difluorophényl)méthyle, (2,5-difluorophényl)méthyle, (2,6-difluorophényl)méthyle, (2,4,5-trifluorophényl)méthyle, (2,4,6-trifluorophényl)méthyle, (4-chlorophényl)méthyle, (3-chlorophényl)méthyle, (2-chlorophényl)méthyle, (2,4-dichlorophényl)méthyle, (3,5-dichlorophényl)méthyle, (2,5-dichlorophényl)méthyle, (2,6-dichlorophényl)méthyle, (2,4,5-trichlorophényl)méthyle, (2,4,6-trichlorophényl)méthyle, (4-bromophényl)méthyle, (3-bromophényl)méthyle, (2-bromophényl)méthyle, (4-iodophényl)méthyle, (3-iodophényl)méthyle, (2-iodophényl)méthyle, (3-chloro-5-trifluorométhylpyridin-2-yl)méthyle, (2-bromo-4-fluorophénl)méthyle, (2-bromo-4-chlorophényl)méthyle, (3-bromo-4-fluorophényl)méthyle, (3-bromo-4-chlorophényl)méthyle, (3-bromo-5-fluorophényl)méthyle, (3-bromo-5-chlorophényl)méthyle, (2-fluoro-4-bromophényl)méthyle, (2-chloro-4-bromophényl)méthyle, (3-fluoro-4-bromophényl)méthyle, (3-chloro-4-bromophényl)méthyle, (2-chloro-4-fluorophényl)méthyle, (3-chloro-4-fluorophényl)méthyle, (2-fluoro-3-chlorophényl)méthyle, (2-fluoro-4-chlorophényl)méthyle, (2-fluoro-5-chlorophényl)méthyle, (3-fluoro-4-chlorophényl)méthyle, (3-fluoro-5-chlorophényl)méthyle, (2-fluoro-6-chlorophényl)méthyle, 2-phényléth-1-yle, 3-trifluorométhyl-4-chlorophényle, 3-chloro-4-trifluorométhylphényle, 2-chloro-4-trifluorométhylphényle, 3,5-difluoropyridin-2-yle, (3,6-dichloropyridin-2-yl)méthyle, (4-trifluorométhylphényl)méthyle, (3-trifluorométhylphényl)méthyle, (2-trifluorométhylphényl)méthyle, (4-trifluorométhoxyphényl)méthyle, (3-trifluorométhoxyphényl)méthyle, (2-trifluorométhoxyphényl)méthyle, (4-méthoxyphényl)méthyle, (3-méthoxyphényl)méthyle, (2-méthoxyphényl)méthyle, (4-méthylphényl)méthyle, (3-méthylphényl)méthyle, (2-méthylphényl)méthyle, (4-cyanophényl)méthyle, (3-cyanophényl)méthyle, (2-cyanophényl)méthyle, (2,4-diéthylphényl)méthyle, (3,5-diéthylphényl)méthyle, (3,4-diméthylphényl)méthyle, (3,5-diméthoxyphényl)méthyle, 1-phényléth-1-yle, 1-(o-chlorophényl)éth-1-yle, 1,3-thiazol-2-yle, 4-méthyl-1,3-thiazol-2-yle, 1,3-thiazol-2-yle, méthylthiométhyle, éthylthiométhyle, éthylthioéthyle, méthylthioéthyle, n-propylthiométhyle, iso-propylthiométhyle, trifluorométhylthiométhyle, trifluorométhylthioéthyle, méthylcarbonylméthyle, éthylcarbonylméthyle, n-propylcarbonylméthyle, iso-propylcarbonylméthyle, méthylcarbonyléthyle, hydroxycarbonyle, méthoxycarbonyle, éthoxycarbonyle, n-propyloxycarbonyle, iso-propyloxycarbonyle, n-butyloxycarbonyle, tert-butyloxycarbonyle, allyloxycarbonyle, benzyloxycarbonyle, aminocarbonyle, méthylaminocarbonyle, éthylaminocarbonyle, n-propylaminocarbonyle, iso-propylaminocarbonyle, diméthylaminocarbonyle, diéthylaminocarbonyle, méthyl(éthyl)aminocarbonyle, cyclopropylaminocarbonyle, cyclobutylaminocarbonyle, cyclopentylaminocarbonyle, cyclohexylaminocarbonyle, allylaminocarbonyle, benzylaminocarbonyle, tert-butyloxycarbonylaminocarbonyle, hydroxycarbonylméthyle, méthoxycarbonylméthyle, éthoxycarbonylméthyle, n-propyloxycarbonylméthyle, iso-propyloxycarbonylméthyle, n-butyloxycarbonylméthyle, tert-butyloxycarbonylméthyle, allyloxycarbonylméthyle, benzyloxycarbonylméthyle, aminocarbonylméthyle, méthylaminocarbonylméthyle, éthylaminocarbonylméthyle, n-propylaminocarbonylméthyle, iso-propylaminocarbonylméthyle, diméthylaminocarbonylméthyle, diéthylaminocarbonylméthyle, méthyl(éthyl)aminocarbonylméthyle, cyclopropylaminocarbonylméthyle, cyclobutylaminocarbonylméthyle, cyclopentylaminocarbonylméthyle, cyclohexylaminocarbonylméthyle, allylaminocarbonylméthyle, benzylaminocarbonylméthyle, 1-(hydroxycarbonyl)éth-1-yle, 1-(méthoxycarbonyl)éth-1-yle, 1-(éthoxycarbonyl)éth-1-yle, 1-(tert-butyloxycarbonyl)éth-1-yle, 1-(benzyloxycarbonyl)éth-1-yle, 2-(hydroxycarbonyl)éth-1-yle, 2-(méthoxycarbonyl)éth-1-yle, 2-(éthoxycarbonyl)éth-1-yle, 2-(tert-butyloxycarbonyl)éth-1-yle, 2-(benzyloxycarbonyl)éth-1-yle, 1-(hydroxycarbonyl)prop-1-yle, 1-(méthoxycarbonyl)prop-1-yle, 1-(éthoxycarbonyl)prop-1-yle, 1-(tert-butyloxycarbonyl)prop-1-yle, 1-(benzyloxycarbonyl)prop-1-yle, 2-(hydroxycarbonyl)prop-1-yle, 2-(méthoxycarbonyl)prop-1-yle, 2-(éthoxycarbonyl)prop-1-yle, 2-(tert-butyloxycarbonyl)prop-1-yle, 2-(benzyloxycarbonyl)prop-1-yle, 1-(hydroxycarbonyl)prop-2-yle, 1-(méthoxycarbonyl)prop-2-yle, 1-(éthoxycarbonyl)prop-2-yle, 1-(tert-butyloxycarbonyl)prop-2-yle, 1-(benzyloxycarbonyl)prop-2-yle, 3-(hydroxycarbonyl)prop-1-yle, 3-(méthoxycarbonyl)prop-1-yle, 3-(éthoxycarbonyl)prop-1-yle, 3-(tert-butyloxycarbonyl)prop-1-yle, 3-(benzyloxycarbonyl)prop-1-yle, aminométhyle, 2-aminoéth-1-yle, 1-aminoéthl-yle, 1-aminoprop-1-yle, 3-aminoprop-1-yle, méthylaminométhyle, diméthylaminométhyle, diéthylaminométhyle, éthylaminométhyle, iso-propylaminométhyle, cyclopropylaminométhyle, cyclobutylaminométhyle, cyclopentylaminométhyle, cyclohexylaminométhyle, méthoxycarbonylaminométhyle, éthoxycarbonylaminométhyle, tert-butyloxycarbonylaminométhyle, méthylcarbonylaminométhyle, éthylcarbonylaminométhyle, n-propylcarbonylaminométhyle, iso-propylcarbonylaminométhyle, 2-(méthylamino)éth-1-yle, 2-(diéth-1-ylamino)éth-1-yle, 2-(diéthylamino)éth-1-yle, 2-(éthylamino)éth-1-yle, 2-(iso-propylamino)éth-1-yle, 2-(cyclopropylamino)éth-1-yle, 2-(cyclobutylamino)éth-1-yle, 2-(cyclopentylamino)éth-1-yle, 2-(cyclohexylamino)éth-1-yle, 2-(méthoxycarbonylamino)éth-1-yle, 2-(éthoxycarbonylamino)éth-1-yle, 2-(tert-butyloxycarbonylamino)éth-1-yle, 2-(méthylcarbonylamino)éth-1-yle, 2-(éthylcarbonylamino)éth-1-yle, 2-(n-propylcarbonylamino)éth-1-yle, 2-(iso-propylcarbonylamino)éth-1-yle, 1-(méthylamino)éth-1-yle, 1-(diéth-1-ylamino)éth-1-yle, 1-(diéthylamino)éth-1-yle, 1-(éthylamino)éth-1-yle, 1-(iso-propylamino)éth-1-yle, 1-(cyclopropylamino)éth-1-yle, 1-(cyclobutylamino)éth-1-yle, 1-(cyclopentylamino)éth-1-yle, 1-(cyclohexylamino)éth-1-yle, 1-(méthoxycarbonylamino)éth-1-yle, 1-(éthoxycarbonylamino)éth-1-yle, 1-(tert-butyloxycarbonylamino)éth-1-yle, 1-(méthylcarbonylamino)éth-1-yle, 1-(éthylcarbonylamino)éth-1-yle, 1-(n-propylcarbonylamino)éth-1-yle, 1-(iso-propylcarbonylamino)éth-1-yle ou
R⁵ et R⁶ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁵ et R⁶ forment, avec l'atome de carbone auquel ils sont liés, une double liaison le cas échéant substituée davantage par R¹⁹ et R²⁰, selon la formule suivante (I') 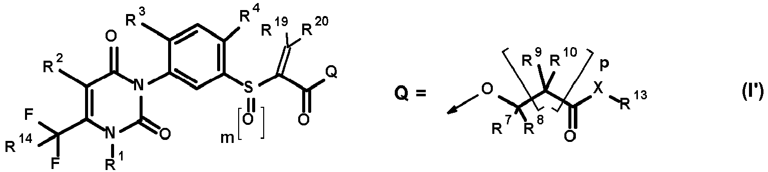
R¹⁴ représente hydrogène, fluor, chlore, brome, trifluorométhyle, méthoxy, éthoxy, n-propyloxy, n-butyloxy,
m représente 0, 1, 2,
R¹⁹ et R²⁰ représentent, indépendamment l'un de l'autre, hydrogène, fluor, chlore, brome, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, cyclopropyle, cyclobutyle, cyclopentyle, cyclohexyle, trifluorométhyle, difluorométhyle, pentafluoroéthyle, éthényle, 1-propényle, 1-méthyléthényle, 1-butényle, phényle ou
R¹⁹ et R²⁰ forment, avec l'atome de carbone auquel ils sont liés, un cycle monocyclique ou bicyclique de 3 à 10 chaînons, complètement saturé ou le cas échéant interrompu par des hétéroatomes et le cas échéant substitué davantage,
et Q représente un des groupements Q-1 à Q-300 spécifiquement mentionnés ci-après :
| | | | | |
|---|---|---|---|---|
| 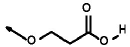 | 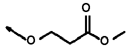 | 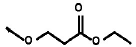 | 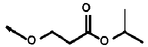 | 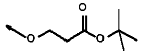 |
| Q-1 | Q-2 | Q-3 | Q-4 | Q-5 |
| | | | | |
| 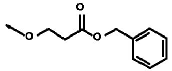 | 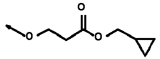 | 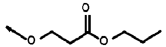 | 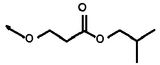 | 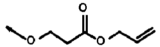 |
| Q-6 | Q-7 | Q-8 | Q-9 | Q-10 |
| | | | | |
| 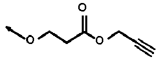 | 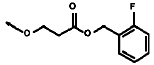 | 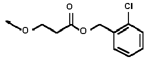 | 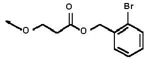 | 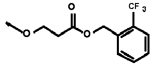 |
| Q-11 | Q-12 | Q-13 | Q-14 | Q-15 |
| | | | | |
| 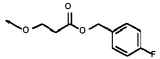 | 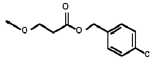 | 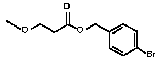 | 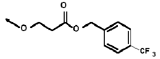 | 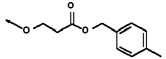 |
| Q-16 | Q-17 | Q-18 | Q-19 | Q-20 |
| | | | | |
| 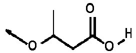 | 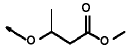 | 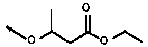 | 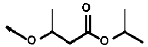 | 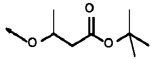 |
| Q-21 | Q-22 | Q-23 | Q-24 | Q-25 |
| | | | | |
| 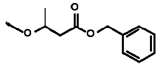 | 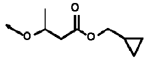 | 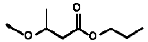 | 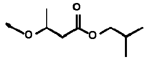 | 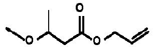 |
| Q-26 | Q-27 | Q-28 | Q-29 | Q-30 |
| 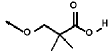 | 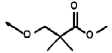 | 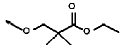 | 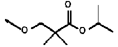 | 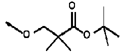 |
| Q-31 | Q-32 | Q-33 | Q-34 | Q-35 |
| | | | | |
| 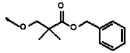 | 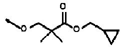 | 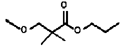 | 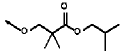 | 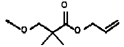 |
| Q-36 | Q-37 | Q-38 | Q-39 | Q-40 |
| | | | | |
| 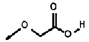 | 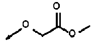 | 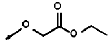 | 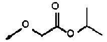 | 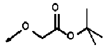 |
| Q-41 | Q-42 | Q-43 | Q-44 | Q-45 |
| | | | | |
| 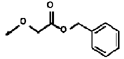 | 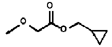 | 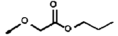 | 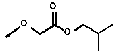 | 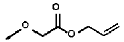 |
| Q-46 | Q-47 | Q-48 | Q-49 | Q-50 |
| | | | | |
| 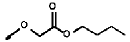 | 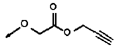 | 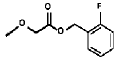 | 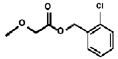 | 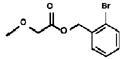 |
| Q-51 | Q-52 | Q-53 | Q-54 | Q-55 |
| | | | | |
| 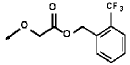 | 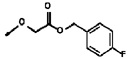 | 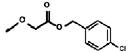 | 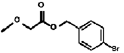 | 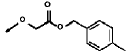 |
| Q-56 | Q-57 | Q-58 | Q-59 | Q-50 |
| | | | | |
| 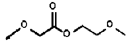 | 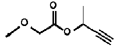 | 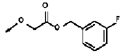 | 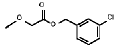 | 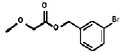 |
| Q-61 | Q-62 | Q-63 | Q-64 | Q-65 |
| | | | | |
| 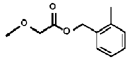 | 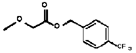 | 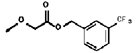 | 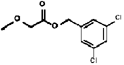 | 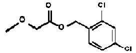 |
| Q-66 | Q-67 | Q-68 | Q-69 | Q-70 |
| 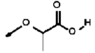 | 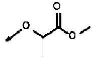 | 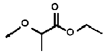 | 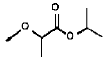 | 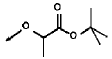 |
| Q-71 | Q-72 | Q-73 | Q-74 | Q-75 |
| | | | | |
| 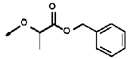 | 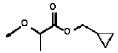 | 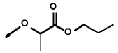 | 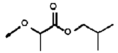 | 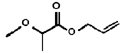 |
| Q-76 | Q-77 | Q-78 | Q-79 | Q-80 |
| | | | | |
| 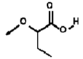 | 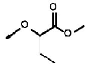 | 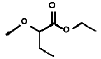 | 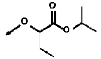 | 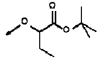 |
| Q-81 | Q-82 | Q-83 | Q-84 | Q-85 |
| | | | | |
| 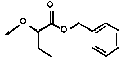 | 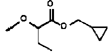 | 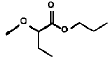 | 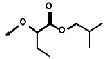 | 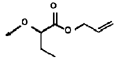 |
| Q-86 | Q-87 | Q-88 | Q-89 | Q-90 |
| | | | | |
| 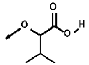 | 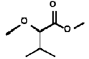 | 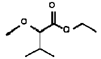 | 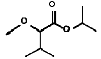 | 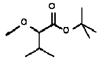 |
| Q-91 | Q-92 | Q-93 | Q-94 | Q-95 |
| | | | | |
| 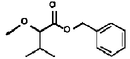 | 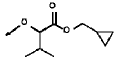 | 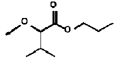 | 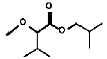 | 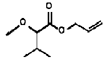 |
| Q-96 | Q-97 | Q-98 | Q-99 | Q-100 |
| | | | | |
| 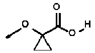 | 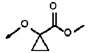 | 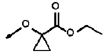 | 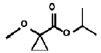 | 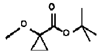 |
| Q-101 | Q-102 | Q-103 | Q-104 | Q-105 |
| | | | | |
| 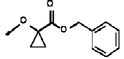 | 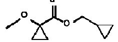 | 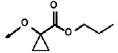 | 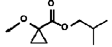 | 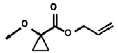 |
| Q-106 | Q-107 | Q-108 | Q-109 | Q-110 |
| 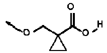 | 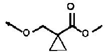 | 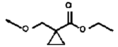 | 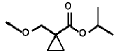 | 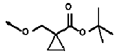 |
| Q-111 | Q-112 | Q-113 | Q-114 | Q-115 |
| | | | | |
| 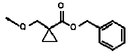 | 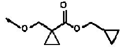 | 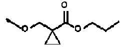 | 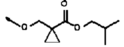 | 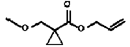 |
| Q-116 | Q-117 | Q-118 | Q-119 | Q-120 |
| | | | | |
| 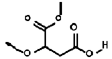 | 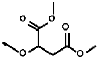 | 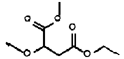 | 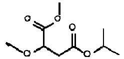 | 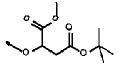 |
| Q-121 | Q-122 | Q-123 | Q-124 | Q-125 |
| | | | | |
| 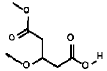 | 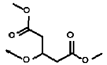 | 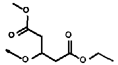 | 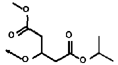 | 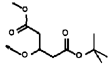 |
| Q-126 | Q-127 | Q-128 | Q-129 | Q-130 |
| | | | | |
| 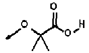 | 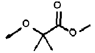 | 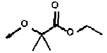 | 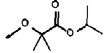 | 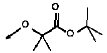 |
| Q-131 | Q-132 | Q-133 | Q-134 | Q-135 |
| | | | | |
| 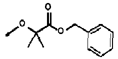 | 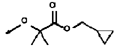 | 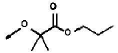 | 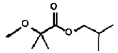 | 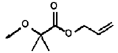 |
| Q-136 | Q-137 | Q-138 | Q-139 | Q-140 |
| | | | | |
| 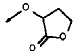 | 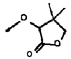 | 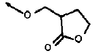 | 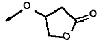 | 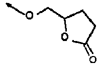 |
| Q-141 | Q-142 | Q-143 | Q-144 | Q-145 |
| | | | | |
| 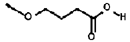 | 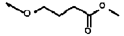 | 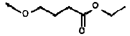 | 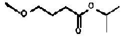 | 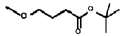 |
| Q-146 | Q-147 | Q-148 | Q-149 | Q-150 |
| 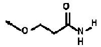 | 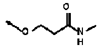 | 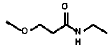 | 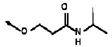 | 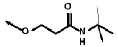 |
| Q-151 | Q-152 | Q-153 | Q-154 | Q-155 |
| | | | | |
| 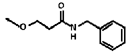 | 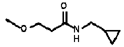 | 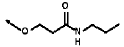 | 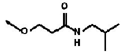 | 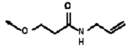 |
| Q-156 | Q-157 | Q-158 | Q-159 | Q-160 |
| | | | | |
| 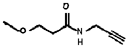 | 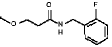 | 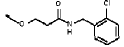 | 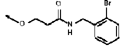 | 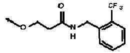 |
| Q-161 | Q-162 | Q-163 | Q-164 | Q-165 |
| | | | | |
| 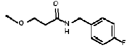 | 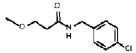 | 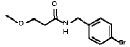 | 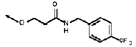 | 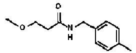 |
| Q-166 | Q-167 | Q-168 | Q-169 | Q-170 |
| | | | | |
| 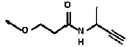 | 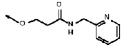 | 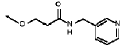 | 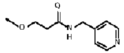 | 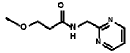 |
| Q-171 | Q-172 | Q-173 | Q-174 | Q-175 |
| | | | | |
| 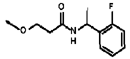 | 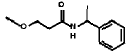 | 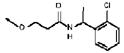 | 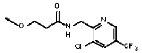 | 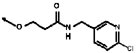 |
| Q-176 | Q-177 | Q-178 | Q-179 | Q-180 |
| | | | | |
| 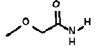 | 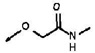 | 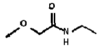 | 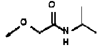 | 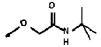 |
| Q-181 | Q-182 | Q-183 | Q-184 | Q-185 |
| | | | | |
| 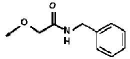 | 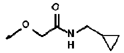 | 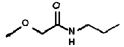 | 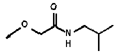 | 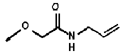 |
| Q-186 | Q-187 | Q-188 | Q-189 | Q-190 |
| 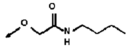 | 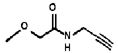 | 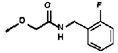 | 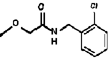 | 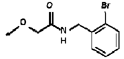 |
| Q-191 | Q-192 | Q-193 | Q-194 | Q-195 |
| | | | | |
| 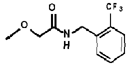 | 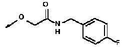 | 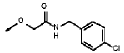 | 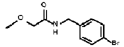 | 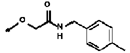 |
| Q-196 | Q-197 | Q-198 | Q-199 | Q-200 |
| | | | | |
| 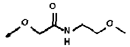 | 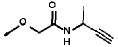 | 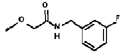 | 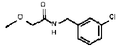 | 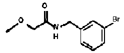 |
| Q-201 | Q-202 | Q-203 | Q-204 | Q-205 |
| | | | | |
| 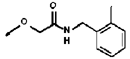 | 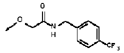 | 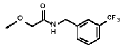 | 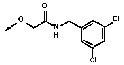 | 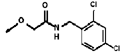 |
| Q-206 | Q-207 | Q-208 | Q-209 | Q-210 |
| | | | | |
| 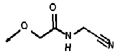 | 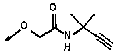 | 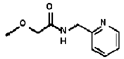 | 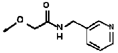 | 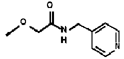 |
| Q-211 | Q-212 | Q-213 | Q-214 | Q-215 |
| | | | | |
| 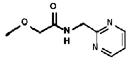 | 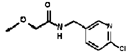 | 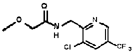 | 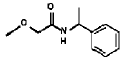 | 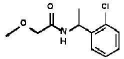 |
| Q-216 | Q-217 | Q-218 | Q-219 | Q-220 |
| | | | | |
| 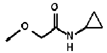 | 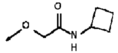 | 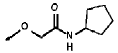 | 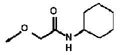 | 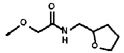 |
| Q-221 | Q-222 | Q-223 | Q-224 | Q-225 |
| | | | | |
| 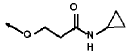 | 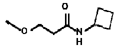 | 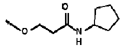 | 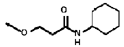 | 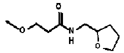 |
| Q-226 | Q-227 | Q-228 | Q-229 | Q-230 |
| 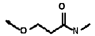 | 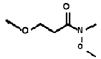 | 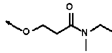 | 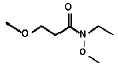 | 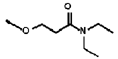 |
| Q-231 | Q-232 | Q-233 | Q-234 | Q-235 |
| | | | | |
| 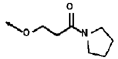 | 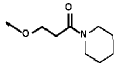 | 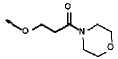 | 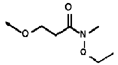 | 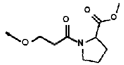 |
| Q-236 | Q-237 | Q-238 | Q-239 | Q-240 |
| | | | | |
| 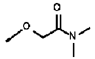 | 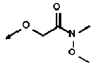 | 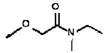 | 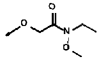 | 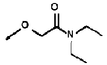 |
| Q-241 | Q-242 | Q-243 | Q-244 | Q-245 |
| | | | | |
| 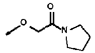 | 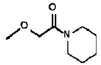 | 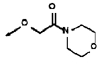 | 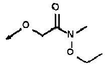 | 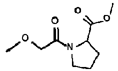 |
| Q-246 | Q-247 | Q-248 | Q-249 | Q-250 |
| | | | | |
| 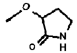 | 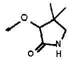 | 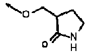 | 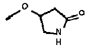 | 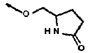 |
| Q-251 | Q-252 | Q-253 | Q-254 | Q-255 |
| | | | | |
| 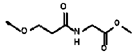 | 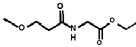 | 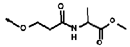 | 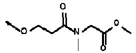 | 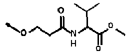 |
| Q-256 | Q-257 | Q-258 | Q-259 | Q-260 |
| | | | | |
| 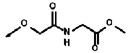 | 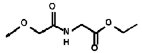 | 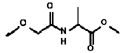 | 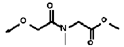 | 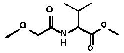 |
| Q-261 | Q-262 | Q-263 | Q-264 | Q-265 |
| | | | | |
| 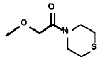 | 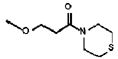 | 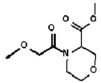 | 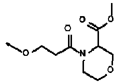 | 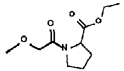 |
| Q-266 | Q-267 | Q-268 | Q-269 | Q-270 |
| 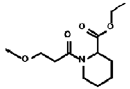 | 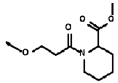 | 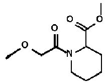 | 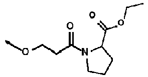 | 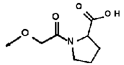 |
| Q-271 | Q-272 | Q-273 | Q-274 | Q-275 |
| | | | | |
| 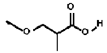 | 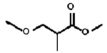 | 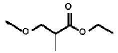 | 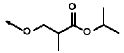 | 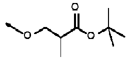 |
| Q-276 | Q-277 | Q-278 | Q-279 | Q-280 |
| | | | | |
| 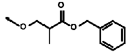 | 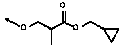 | 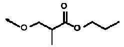 | 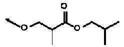 | 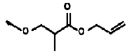 |
| Q-281 | Q-282 | Q-283 | Q-284 | Q-285 |
| | | | | |
| 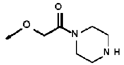 | 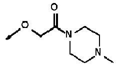 | 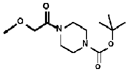 | 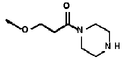 | 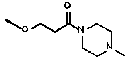 |
| Q-286 | Q-287 | Q-288 | Q-289 | Q-290 |
| | | | | |
| 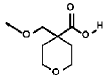 | 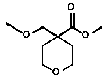 | 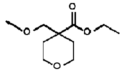 | 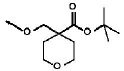 | 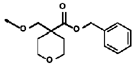 |
| Q-291 | Q-292 | Q-293 | Q-294 | Q-295 |
| | | | | |
| 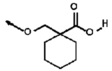 | 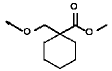 | 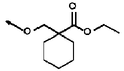 | 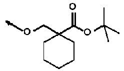 | 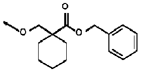 |
| Q-296 | Q-297 | Q-298 | Q-299 | Q-300 |

5. Composés de formule générale (I) selon la revendication 1 et/ou leur sel, **caractérisé en ce que**
R¹ représente méthyle, éthyle, n-propyle, 1-méthyléthyle, n-butyle, 1-méthylpropyle, 2-méthylpropyle, 1,1-diméthyléthyle, n-pentyle, amino, diméthylamino, diéthylamino,
R² représente hydrogène, méthyle, éthyle, n-propyle, iso-propyle,
R³ représente hydrogène, fluor, chlore, brome, méthoxy, éthoxy,
R⁴ représente halogène, cyano, C(O)NH₂, C(S)NH₂, difluorométhyle, trifluorométhyle, éthynyle, propyn-1-yle,
R⁵ et R⁶ représentent, indépendamment l'un de l'autre, hydrogène, fluor, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, cyclopropyle, cyclobutyle, cyclopentyle, cyclohexyle, cyclopropylméthyle, cyclobutylméthyle, cyclopentylméthyle, cyclohexylméthyle, éthényle, 1-propényle, 2-propényle, 1-méthyléthényle, 1-butényle, 2-butényle, 3-butényle, 1-méthyl-1-propényle, 2-méthyl-1-propényle, 1-méthyl-2-propényle, 2-méthyl-2-propényle, 1-pentényle, 2-pentényle, 3-pentényle, 4-pentényle, 1-méthyl-1-butényle, 2-méthyl-1-butényle, 3-méthyl-1-butényle, 1-méthyl-2-butényle, 2-méthyl-2-butényle, 3-méthyl-2-butényle, 1-méthyl-3-butényle, 2-méthyl-3-butényle, 3-méthyl-3-butényle, 1,1-diméthyl-2-propényle, 1,2-diméthyl-1-propényle, 1,2-diméthyl-2-propényle, 1-éthyl-1-propényle, 1-éthyl-2-propényle, éthynyle, 1-propynyle, 2-propynyle, 1-butynyle, 2-butynyle, 3-butynyle, 1-méthyl-2-propynyle, 1-pentynyle, 2-pentynyle, 3-pentynyle, 4-pentynyle, 1-méthyl-2-butynyle, 1-méthyl-3-butynyle, 2-méthyl-3-butynyle, 3-méthyl-1-butynyle, 1,1-diméthyl-2-propynyle, 1-éthyl-2-propynyle, trifluorométhyle, pentafluoroéthyle, 1,1,2,2-tétrafluoroéthyle, heptafluoropropyle, nonafluorobutyle, chlorodifluorométhyle, bromodifluorométhyle, dichlorofluorométhyle, iododifluorométhyle, bromofluorométhyle, 1-fluoroéthyle, 2-fluoroéthyle, fluorométhyle, difluorométhyle, 2,2-difluoroéthyle, 2,2,2-trifluoroéthyle, difluoro-tert-butyle, méthoxy, éthoxy, n-propyloxy, iso-propyloxy, n-butyloxy, tert-butyloxy, méthoxyméthyle, éthoxyméthyle, n-propyloxyméthyle, iso-propyloxyméthyle, méthoxyéthyle, éthoxyéthyle, n-propyloxyéthyle, iso-propyloxyéthyle, méthoxy-n-propyle, méthoxydifluorométhyle, éthoxydifluorométhyle, n-propyloxydifluorométhyle, n-butyloxydifluorométhyle, trifluorométhoxyméthyle, trifluorométhoxyéthyle, trifluorométhoxy-n-propyle, phényle, 2-fluoro-phényle, 3-fluoro-phényle, 4-fluoro-phényle, 2,4-difluoro-phényle, 2,5-difluoro-phényle, 2,6-difluoro-phényle, 2,3-difluoro-phényle, 3,4-difluoro-phényle, 3,5-difluoro-phényle, 2,4,5-trifluoro-phényle, 3,4,5-trifluoro-phényle, 2-chloro-phényle, 3-chloro-phényle, 4-chloro-phényle, 2,4-dichloro-phényle, 2,5-dichloro-phényle, 2,6-dichloro-phényle, 2,3-dichloro-phényle, 3,4-dichloro-phényle, 3,5-dichloro-phényle, 2,4,5-trichloro-phényle, 3,4,5-trichloro-phényle, 2,4,6-trichloro-phényle, pyridin-2-yle, pyridin-3-yle, pyridin-4-yle, thiophén-2-yle, thiophén-3-yle, furann-2-yle, tétrahydrofurann-2-yle, tétrahydrofurann-3-yle, 1-(4-méthylphényl)éthyle, 1-(3-méthylphényl)éthyle, 1-(2-méthylphényl)éthyle, 1-(4-chlorophényl)éthyle, 1-(3-chlorophényl)éthyle, 1-(2-chlorophényl)éthyle, benzyle, (4-fluorophényl)méthyle, (3-fluorophényl)méthyle, (2-fluorophényl)méthyle, (2,4-difluorophényl)méthyle, (3,5-difluorophényl)méthyle, (2,5-difluorophényl)méthyle, (2,6-difluorophényl)méthyle, (2,4,5-trifluorophényl)méthyle, (2,4,6-trifluorophényl)méthyle, (4-chlorophényl)méthyle, (3-chlorophényl)méthyle, (2-chlorophényl)méthyle, (2,4-dichlorophényl)méthyle, (3,5-dichlorophényl)méthyle, (2,5-dichlorophényl)méthyle, (2,6-dichlorophényl)méthyle, (2,4,5-trichlorophényl)méthyle, (2,4,6-trichlorophényl)méthyle, méthylthiométhyle, éthylthiométhyle, éthylthioéthyle, méthylthioéthyle, n-propylthiométhyle, iso-propylthiométhyle, trifluorométhylthiométhyle, trifluorométhylthioéthyle, méthylcarbonylméthyle, éthylcarbonylméthyle, n-propylcarbonylméthyle, iso-propylcarbonylméthyle, méthylcarbonyléthyle, hydroxycarbonyle, méthoxycarbonyle, éthoxycarbonyle, n-propyloxycarbonyle, iso-propyloxycarbonyle, n-butyloxycarbonyle, tert-butyloxycarbonyle, allyloxycarbonyle, benzyloxycarbonyle, aminocarbonyle, méthylaminocarbonyle, éthylaminocarbonyle, n-propylaminocarbonyle, iso-propylaminocarbonyle, diméthylaminocarbonyle, diéthylaminocarbonyle, méthyl(éthyl)aminocarbonyle, cyclopropylaminocarbonyle, cyclobutylaminocarbonyle, cyclopentylaminocarbonyle, cyclohexylaminocarbonyle, allylaminocarbonyle, benzylaminocarbonyle, tert-butyloxycarbonylaminocarbonyle, hydroxycarbonylméthyle, méthoxycarbonylméthyle, éthoxycarbonylméthyle, n-propyloxycarbonylméthyle, iso-propyloxycarbonylméthyle, n-butyloxycarbonylméthyle, tert-butyloxycarbonylméthyle, allyloxycarbonylméthyle, benzyloxycarbonylméthyle, aminocarbonylméthyle, méthylaminocarbonylméthyle, éthylaminocarbonylméthyle, n-propylaminocarbonylméthyle, iso-propylaminocarbonylméthyle, diméthylaminocarbonylméthyle, diéthylaminocarbonylméthyle, méthyl(éthyl)aminocarbonylméthyle, cyclopropylaminocarbonylméthyle, cyclobutylaminocarbonylméthyle, cyclopentylaminocarbonylméthyle, cyclohexylaminocarbonylméthyle, allylaminocarbonylméthyle, benzylaminocarbonylméthyle, 1-(hydroxycarbonyl)éth-1-yle, 1-(méthoxycarbonyl)éth-1-yle, 1-(éthoxycarbonyl)éth-1-yle, 1-(tert-butyloxycarbonyl)éth-1-yle, 1-(benzyloxycarbonyl)éth-1-yle, 2-(hydroxycarbonyl)éth-1-yle, 2-(méthoxycarbonyl)éth-1-yle, 2-(éthoxycarbonyl)éth-1-yle, 2-(tert-butyloxycarbonyl)éth-1-yle, 2-(benzyloxycarbonyl)éth-1-yle, 1-(hydroxycarbonyl)prop-1-yle, 1-(méthoxycarbonyl)prop-1-yle, 1-(éthoxycarbonyl)prop-1-yle, 1-(tert-butyloxycarbonyl)prop-1-yle, 1-(benzyloxycarbonyl)prop-1-yle, 2-(hydroxycarbonyl)prop-1-yle, 2-(méthoxycarbonyl)prop-1-yle, 2-(éthoxycarbonyl)prop-1-yle, 2-(tert-butyloxycarbonyl)prop-1-yle, 2-(benzyloxycarbonyl)prop-1-yle, 1-(hydroxycarbonyl)prop-2-yle, 1-(méthoxycarbonyl)prop-2-yle, 1-(éthoxycarbonyl)prop-2-yle, 1-(tert-butyloxycarbonyl)prop-2-yle, 1-(benzyloxycarbonyl)prop-2-yle, 3-(hydroxycarbonyl)prop-1-yle, 3-(méthoxycarbonyl)prop-1-yle, 3-(éthoxycarbonyl)prop-1-yle, 3-(tert-butyloxycarbonyl)prop-1-yle, 3-(benzyloxycarbonyl)prop-1-yle, aminométhyle, 2-aminoéth-1-yle, 1-aminoéth-1-yle, 1-aminoprop-1-yle, 3-aminoprop-1-yle, méthylaminométhyle, diméthylaminométhyle, diéthylaminométhyle, éthylaminométhyle, iso-propylaminométhyle, cyclopropylaminométhyle, cyclobutylaminométhyle, cyclopentylaminométhyle, cyclohexylaminométhyle, méthoxycarbonylaminométhyle, éthoxycarbonylaminométhyl tert-butyloxycarbonylaminométhyle, méthylcarbonylaminométhyle, éthylcarbonylaminométhyle, n-propylcarbonylaminométhyle, iso-propylcarbonylaminométhyle, 2-(méthylamino)éth-1-yle, 2-(diéth-1-ylamino)éth-1-yle, 2-(diéthylamino)éth-1-yle, 2-(éthylamino)éth-1-yle, 2-(iso-propylamino)éth-1-yle, 2-(cyclopropylamino)éth-1-yle, 2-(cyclobutylamino)éth-1-yle, 2-(cyclopentylamino)éth-1-yle, 2-(cyclohexylamino)éth-1-yle, 2-(méthoxycarbonylamino)éth-1-yle, 2-(éthoxycarbonylamino)éth-1-yle, 2-(tert-butyloxycarbonylamino)éth-1-yle, 2-(méthylcarbonylamino)éth-1-yle, 2-(éthylcarbonylamino)éth-1-yle, 2-(n-propylcarbonylamino)éth-1-yle, 2-(iso-propylcarbonylamino)éth-1-yle, 1-(méthylamino)éth-1-yle, 1-(diéth-1-ylamino)éth-1-yle, 1-(diéthylamino)éth-1-yle, 1-(éthylamino)éth-1-yle, 1-(iso-propylamino)éth-1-yle, 1-(cyclopropylamino)éth-1-yle, 1-(cyclobutylamino)éth-1-yle, 1-(cyclopentylamino)éth-1-yle, 1-(cyclohexylamino)éth-1-yle, 1-(méthoxycarbonylamino)éth-1-yle, 1-(éthoxycarbonylamino)éth-1-yle, 1-(tert-butyloxycarbonylamino)éth-1-yle, 1-(méthylcarbonylamino)éth-1-yle, 1-(éthylcarbonylamino)éth-1-yle, 1-(n-propylcarbonylamino)éth-1-yle, 1-(iso-propylcarbonylamino)éth-1-yle ou
R⁵ et R⁶ forment, avec l'atome de carbone auquel ils sont liés, un cycle monocyclique ou bicyclique de 3 à 10 chaînons, complètement saturé ou partiellement saturé, le cas échéant interrompu par des hétéroatomes et le cas échéant substitué davantage ou
R⁵ et R⁶ forment, avec l'atome de carbone auquel ils sont liés, une double liaison le cas échéant substituée davantage par R¹⁹ et R²⁰, selon la formule suivante (I') 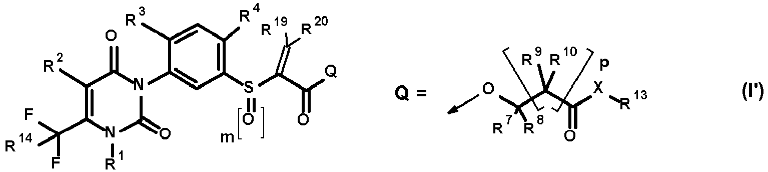
R¹⁴ représente hydrogène, fluor, chlore, brome, trifluorométhyle, méthoxy, éthoxy, n-propyloxy, n-butyloxy,
m représente 0, 1, 2,
R¹⁹ et R²⁰ représentent, indépendamment l'un de l'autre, hydrogène, fluor, chlore, brome, méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, cyclopropyle, cyclobutyle, cyclopentyle, cyclohexyle, trifluorométhyle, difluorométhyle, pentafluoroéthyle, éthényle, 1-propényle, 1-méthyléthényle, 1-butényle, phényle ou
R¹⁹ et R²⁰ forment, avec l'atome de carbone auquel ils sont liés, un cycle monocyclique ou bicyclique de 3 à 10 chaînons, complètement saturé ou le cas échéant interrompu par des hétéroatomes et le cas échéant substitué davantage
et
Q représente un des groupements Q-1 à Q-300 mentionnés dans la dans revendication 4.

6. Utilisation d'un ou de plusieurs composés de formule générale (I) et/ou de leurs sels tels que définis dans l'une quelconque des revendications 1 à 5 comme herbicide et/ou régulateur de la croissance de plantes, de préférence dans la culture de plantes utiles et/ou décoratives.

7. Agent herbicide et/ou régulateur de la croissance de plantes, caractérisé en ce l'agent contient un ou plusieurs composés de formule (I) et/ou leurs sels, tels que définis dans l'une quelconque des revendications 1 à 5 et une ou plusieurs autres substances choisies dans les groupes (i) et/ou (ii) :
(i) une ou plusieurs substances actives d'un point de vue agrochimique, de préférence choisies dans le groupe constitué par les insecticides, les acaricides, les nématicides, d'autres herbicides, les fongicides, les phytoprotecteurs, les engrais et/ou d'autres régulateurs de croissance,
(ii) un ou plusieurs adjuvants de formulation usuels dans la protection des plantes.

8. Procédé pour lutter contre des plantes nuisibles ou pour la régulation de la croissance de plantes, **caractérisé en ce qu'**une quantité active
- d'un ou de plusieurs composés de formule (I) et/ou de leurs sels tels que définis dans l'une quelconque des revendications 1 à 5 ou
- d'un agent selon la revendication 7,
est appliquée sur les plantes, les graines de plantes, le sol dans ou sur lequel les plantes croissent ou sur la surface de culture.
